# EUROPEAN PATENT SPECIFICATION

(11) **EP 3 362 560 B1**
(45) Date of publication and mention of the grant of the patent: **10.08.2022**
(21) Application number: 16791150.2
(22) Date of filing: 11.10.2016
(51) Int. Cl.: C12N 15/11

(54) **PROTECTED DNA TEMPLATES FOR GENE MODIFICATION AND INCREASED HOMOLOGOUS RECOMBINATION IN CELLS AND METHODS OF USE**
GESCHÜTZTE DNA-SCHABLONEN ZUR GENMODIFIZIERUNG UND ERHÖHTEN HOMOLOGEN REKOMBINATION IN ZELLEN UND VERFAHREN ZUR VERWENDUNG
MATRICES ADN PROTÉGÉES POUR MODIFICATION GÉNIQUE ET RECOMBINAISON HOMOLOGUE ACCRUE DANS LES CELLULES ET LEURS PROCÉDÉS D'UTILISATION

(30) Priority: 12.10.2015 US 201562240140 P
(43) Date of publication of application: 22.08.2018
(73) Proprietor: DuPont US Holding, LLC, Wilmington, DE 19805 (US)
(72) Inventor: FRISCH, Ryan L., Newark, Delaware 19702 (US)
(74) Representative: Dehns
(86) International application number: PCT/US2016/056404
(87) International publication number: WO 2017/066175

(56) References cited:
- WO-A1-2016/094867
- AYAL HENDEL ET AL: "Chemically modified guide RNAs enhance CRISPR-Cas genome editing in human primary cells", NATURE BIOTECHNOLOGY, vol. 33, no. 9, 29 June 2015 (2015-06-29), pages 985-989, XP055233915, US ISSN: 1087-0156, DOI: 10.1038/nbt.3290
- Ellen Prediger: "Modifications that block nuclease degradation", , 14 January 2014 (2014-01-14), XP002765968, Retrieved from the Internet: URL:https://www.idtdna.com/pages/decoded/d ecoded-articles/core-concepts/decoded/2014 /01/14/modification-highlight-modification s-that-block-nuclease-degradation [retrieved on 2017-01-16]
- S. W. CHO ET AL: "Analysis of off-target effects of CRISPR/Cas-derived RNA-guided endonucleases and nickases", GENOME RESEARCH, vol. 24, no. 1, 19 November 2013 (2013-11-19), pages 132-141, XP055227885, US ISSN: 1088-9051, DOI: 10.1101/gr.162339.113
- PELLAGATTI ANDREA ET AL: "Application of CRISPR/Cas9 genome editing to the study and treatment of disease", ARCHIVES OF TOXICOLOGY, vol. 89, no. 7, July 2015 (2015-07), pages 1023-1034, XP002765969,

## Description

### FIELD

The disclosure relates to the field of molecular biology, in particular, to methods for altering the genome of a cell. Specifically, this invention pertains to the use of protected DNA templates in combination with guide polynucleotide/ Cas complexes for genetic modification in microbial cells.

### REFERENCE TO SEQUENCE LISTING SUBMITTED ELECTRONICALLY

The official copy of the sequence listing is submitted electronically via EFS-Web as an ASCII formatted sequence listing with a file named CL6641WOPCT2_SEQLISTING.txt created on October 07, 2016 and having a size 188 kilobytes and is filed concurrently with the specification. The sequence listing contained in this ASCII formatted document is part of the specification as filed.

### CROSS-REFERENCE TO RELATED APPLICATION

This application claims the benefit of U.S. Provisional Application No. 62/240,140, filed October 12, 2015.

### BACKGROUND

Recombinant DNA technology has made it possible to modify (edit), insert and/ or delete DNA sequences at targeted locations in DNA sequences and genomic sequences. Site-specific integration techniques, which employ site-specific recombination systems, as well as other types of recombination technologies, have been used to inhibit gene expression as well as generate targeted modifications of polynucleotides of interest in a variety of organisms. Inhibition of gene expression can be accomplished, for example, by interrupting or deleting the DNA sequence of the gene, resulting in "knock-out" of the gene (Austin et al., Nat. Genetics 36:921-924). Gene knock-outs mostly have been carried out through homologous recombination (HR), a technique applicable across a wide array of organisms from bacteria to mammals. Inserting DNA sequences into a genome resulting in genetic "knock-in", can also be performed by HR. Genome-editing techniques such as designer zinc finger nucleases (ZFNs), transcription activator-like effector nucleases (TALENs), homing meganucleases, or guided Cas9 systems are available for producing targeted genome perturbations.

Although gene modification by HR is a powerful tool, it can be a complex, labor-intensive procedure and generally difficult to scale-up in a cost-effective manner. This difficulty is exacerbated in organisms in which HR is not efficient. Such low efficiency typically forces practitioners to rely on selectable phenotypes or exogenous markers to help identify cells in which a desired HR event occurred.

There still remains a need for new genome engineering technologies that increase the robustness of homologous recombination and that are affordable, easy to set up, scalable, and amenable to targeting multiple positions within the genome of an organism

### BRIEF SUMMARY

Methods are provided for modifying a nucleotide sequence in the genome of a microbial cell. The methods employ a guide polynucleotide, a protected polynucleotide modification template and a Cas endonuclease to modify a nucleotide sequence and/or to increase the frequency of homologous directed repair. The methods can further be used to decrease the frequency of off-site integration of any modification template. The present disclosure also describes methods for selecting a microbial cell comprising a modified target site in its genome and methods for selecting a microbial cell comprising a polynucleotide of interest inserted into a target site in its genome.

Embodiments of the present invention are defined in the claims. In particular, in one embodiment of the disclosure, the method comprises a method for selecting a microbial cell comprising a modified nucleotide sequence in its genome, the method comprising: a.) providing a guide polynucleotide, at least one protected polynucleotide modification template and a Cas endonuclease to a microbial cell, wherein said Cas endonuclease and guide polynucleotide can form a complex capable of introducing a single or double-strand break at a target site in genome of said cell, wherein said protected polynucleotide modification template comprises at least one nucleotide modification of said nucleotide sequence; and, b.) selecting a cell from step (a) comprising said modified nucleotide sequence wherein the protected polynucleotide modification template is a double stranded linear molecule comprising at least one phosphorothiate bond at the 5' end of at least one strand, or a single stranded linear molecule comprising at least one phosphorothioate bond at its 5' end. The protected polynucleotide modification template can be a linear polynucleotide comprising at least one protection molecule at its 5' end, or both 5' and 3' ends The at least one nucleotide modification of the protected polynucleotide template can be selected from the group consisting of (i) a replacement of at least one nucleotide, (ii) a deletion of at least one nucleotide, (iii) an insertion of at least one nucleotide, and (iv) any combination of (i) - (iii).

In one embodiment of the disclosure, the method further comprises determining the frequency of Homologous Directed Repair (HDR) and Non-Homologous End Joining (NHEJ) in said cell.

In one embodiment of the disclosure, the method further comprises determining the frequency of off-site integration of the protected polynucleotide modification template in said cell. The frequency of off-site integration of the protected polynucleotide modification template in said cell can be decreased when compared to the frequency of off-site integration derived from a control method having all the same components and steps as said method except for using an unprotected (control) polynucleotide modification template.

In one embodiment of the disclosure, the method comprises a method for selecting a microbial cell comprising a polynucleotide of interest inserted into a target site in its genome, the method comprising: a) providing a guide polynucleotide, a protected polynucleotide donor DNA and a Cas endonuclease to a cell, wherein said Cas endonuclease and guide polynucleotide can form a complex capable of introducing a single or double-strand break at a target site in the genome of said cell, wherein said protected polynucleotide donor DNA comprises a polynucleotide of interest to be inserted into the genome of said cell; and, b) selecting a microbial cell from step (a) comprising a polynucleotide of interest inserted into a target site in its genome, wherein the protected polynucleotide donor DNA is a double stranded linear molecule comprising at least one phosphorothioate bond at the 5' end of at least one strand, or a single stranded linear molecule comprising at least one phosphorothioate bond at its 5' end.

Additional embodiments of the methods of the present disclosure are shown herein.

### BRIEF DESCRIPTION OF THE DRAWINGS AND THE SEQUENCE LISTING

The disclosure can be more fully understood from the following detailed description and the accompanying drawings and Sequence Listing, which form a part of this application. The sequence descriptions and sequence listing attached hereto comply with the rules governing nucleotide and amino acid sequence disclosures in patent applications as set forth in 37 C.F.R. §§1.821-1.825. The sequence descriptions contain the three letter codes for amino acids as defined in 37 C.F.R. §§ 1.821-1.825.

### Figures

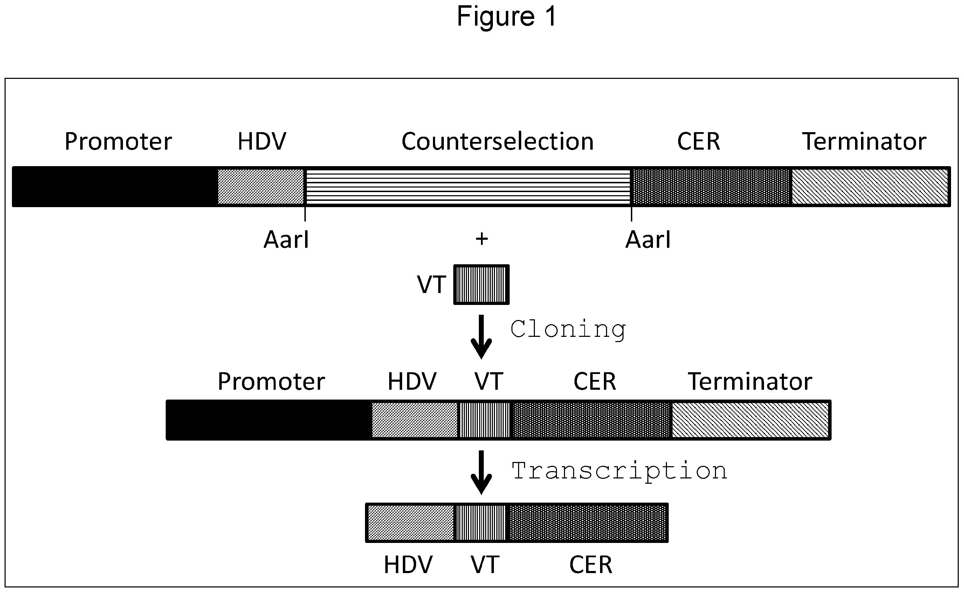

Figure 1 depicts the structure of a high throughput gRNA cloning cassette (for example, but not limiting to, SEQ ID NO: 12 on pRF291. The cassette is composed of a promoter (shown in solid black), DNA encoding a 5' ribozyme (shown in solid gray), a counter selection cassette flanked by two restriction sites (shown in horizontal line fill), a DNA encoding the CER domain (shown as CER) and a transcriptional terminator (dot fill). When a DNA duplex containing a variable targeting domain with the correct overhanging ends (VT, shown as vertical stripe fill) is mixed with a plasmid containing a cassette in the presence of the restriction enzyme and DNA ligase, the counterselection cassette (horizontal stripe fill) can be replaced by the VT domain (Vertical stripe). These events can be selected *in vitro* by selecting for the absence of the counter selection cassette. The product is a functional gRNA expression cassette.

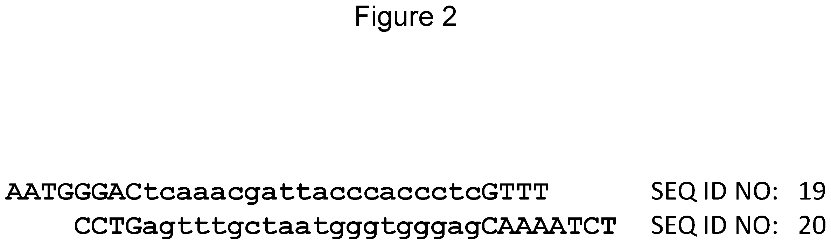

Figure 2 depicts a variable targeting domain duplex (SEQ ID NO:19 and SEQ ID NO:20)for use with the high-throughput plasmid pRF291 comprising SEQ ID NO:19 and SEQ ID NO:20)

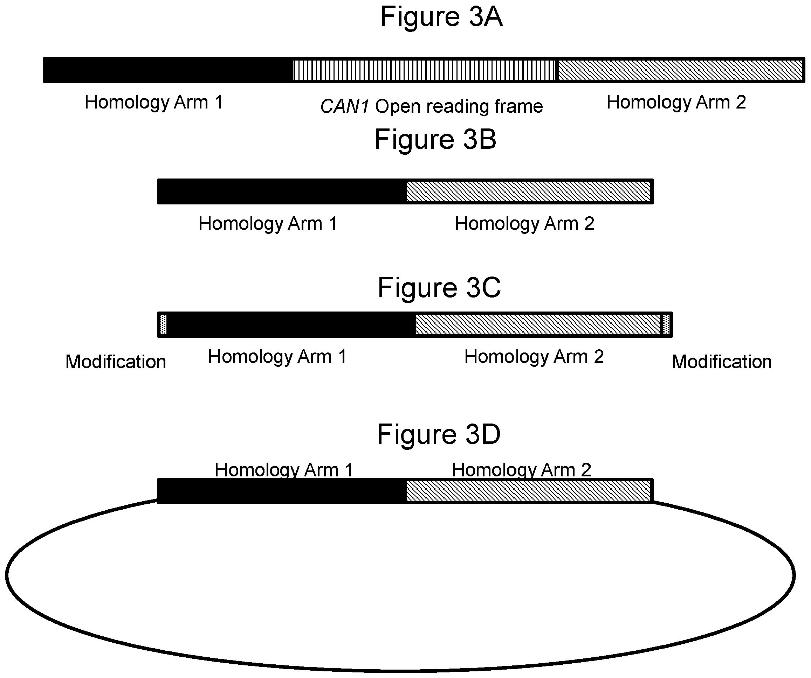

Figure 3A-3D depicts different polynucleotide modification templates. Figure 3A depicts the wild type (WT) *CAN1* locus with the *CAN1* open reading frame (Vertical stripe fill) flanked by Homology arm 1 (Solid black fill) and Homology arm 2 (Diagonal stripe fill). Figure 3B depicts an unprotected (unmodified) polynucleotide modification template composed of two homology arms (Arm 1, solid black fill and Arm 2, diagonal stripe fill). Figure 3C depicts an protected polynucleotide modification template composed of two homology arms (Arm 1, solid black fill and Arm 2, diagonal stripe fill) with the 5' and 3' ends containing a desired modification (protection) to the DNA (dot fill). Figure 3D depicts a protected polynucleotide modification template composed of two homology arms (Arm 1, solid black fill and Arm 2, diagonal stripe fill) that has been made into a circular molecule.

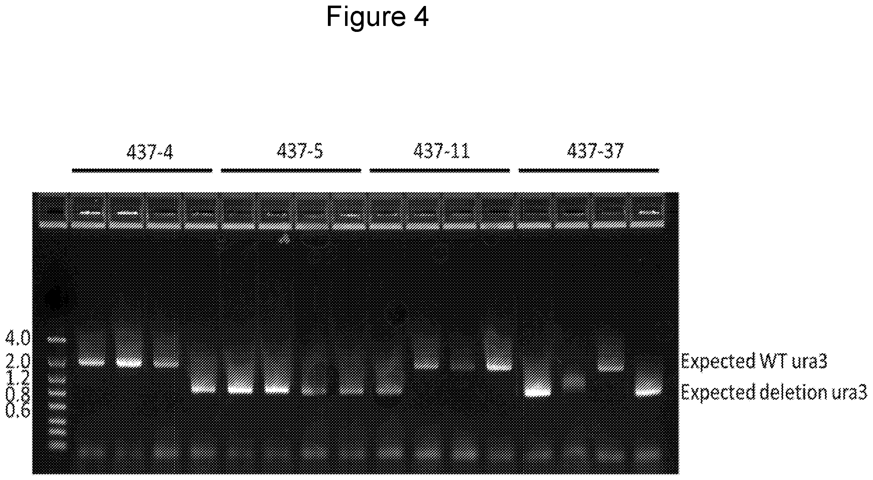

Figure 4: Example PCR of the *URA3* locus from cells treated with pRF437 showing colonies containing indel mutations with a band at the expected size for the WT *URA3* locus and colonies containing deletion of the *URA3* ORF by HDR containing the expected smaller band.

### Sequences

**Table 1. Summary of Nucleic Acid and Protein SEQ ID Numbers**

| Description | Nucleic acid SEQ ID NO. | Protein SEQ ID NO. |
|---|---|---|
| Cas9 endonuclease, *Streptococcus pyogenes* | | 1 |
| Yarrowia codon optimized Cas9 | 2 | |
| SV40 Nuclear localization signal | | 3 |
| FBA1 promoter | 4 | |
| Yarrowia optimized expression cassette | 5 | |
| pZufCas9 | 6 | |
| Aarl-removal 1 primer | 7 | |
| Aarl-removal 2 primer | 8 | |
| pRF109 | 9 | |
| Aar1- Cas9 ORF (Aar1-Cas9CG gene) | 10 | |
| pRF141 | 11 | |
| high-throughput cloning cassette | 12 | |
| yl52 promoter | 13 | |
| DNA encoding the HDV ribozyme | 14 | |
| rpsL counterselectable marker | 15 | |
| DNA encoding Cas9 CER domain | 16 | |
| SUP4 terminator | 17 | |
| pRF291 | 18 | |
| Can1-1F | 19 | |
| Can1-1R | 20 | |
| DNA encoding Can1-1 VT domain | 21 | |
| Can1-1 target site | 22 | |
| CAN1 gene, *Yarrowia lipolytica* | 23 | |
| pRF303 | 24 | |
| can1 upstream homology arm | 25 | |
| Can1 upstream forward | 26 | |
| Can1 upstream reverse | 27 | |
| Can1 downstream homology arm | 28 | |
| Can1 downstream homology arm forward primer | 29 | |
| Can1 downstream homology arm reverse primer | 30 | |
| Can1 polynucleotide modification template (editing template) cloning fragment | 31 | |
| pUC18 | 32 | |
| pRF80 | 33 | |
| Can1 polynucleotide modification template | 34 | |
| C3S forward with /5SpC3/ upstream of first 5'base (A) listed in SEQ ID NO:35 (/5SpC3/AGCTTGCT AC GTT AGGAGAA) | 35 | |
| C3S reverse with /5SpC3/ upstream of first 5'base (T) listed in SEQ ID NO:36 | 36 | |
| (/5SpC3/TATGAGCTTATCCTGTATCG) | | |
| PT forward primer wherein first five 5' nucleotides are modified (A*G*C*T*T*GCTACGTTAGGAGAA) | 37 | |
| PT reverse wherein first five 5' nucleotides are modified (T*A*T*G*A*GCTTATCCTGTATCG) | 38 | |
| CAN1 locus (colony PCR) | 39 | |
| unmodified forward primer | 40 | |
| unmodified reverse primer | 41 | |
| Can1 locus Forward | 42 | |
| Can1 locus reverse | 43 | |
| Can1 locus WT | 44 | |
| Can1 locus deletion | 45 | |
| Copy number analysis fragment | 46 | |
| Can1 copy number F | 47 | |
| Can1 copy number R | 48 | |
| Can1 copy number probe (6FAM-CTTTTCGCCCCCACTGCAGCC-TAMRA) | 49 | |
| TEF1 locus | 50 | |
| TEF1 forward | 51 | |
| TEF1 reverse | 52 | |
| TEF1 probe (6FAM-TGCTGGTGGTGTTGGTGAGTT-TAMRA) | 53 | |
| pRF434 | 54 | |
| Hygromycin resistance cassette | 55 | |
| URA3 locus, *Yarrowia lipolytica* | 56 | |
| ura3-1 target site, *Yarrowia lipolytica* | 57 | |
| ura3-1F | 58 | |
| ura3-1R | 59 | |
| DNA encoding Ura3-1 VT domain | 60 | |
| pRF421 | 61 | |
| URA3 upstream sequence | 62 | |
| URA3 downstream sequence | 63 | |
| URA3 deletion polynucleotide editing template | 64 | |
| pRF263 | 65 | |
| HY007 | 66 | |
| oligo 297 | 67 | |
| EcoRI flanked URA3 deletion template | 68 | |
| pRF437 | 69 | |
| oligo 308 | 70 | |
| oligo 309 | 71 | |
| URA3 locus deletion PCR product | 72 | |

### DETAILED DESCRIPTION

Methods are provided for modifying a nucleotide sequence in the genome of a cell. The methods employ a guide polynucleotide, a protected polynucleotide modification template and a Cas endonuclease to modify a nucleotide sequence and/or to increase the frequency of homologous directed repair. The methods can further be used to decrease the frequency if off-site integration of any modification template.

Many cell types, such as but not limiting to non-conventional yeast, plants, animals, in which Non-Homologous End Joining (NHEJ) predominates over Homology Directed Repair (HDR), and consequently gene editing based on repair of targeted DNA breaks will have a high background of NHEJ mutations in addition to precise gene edits based on a polynucleotide modification template.

Described herein are methods that use a protected polynucleotide modification template to increase the frequency of HDR leading to precise edits (nucleotide modifications) and/or to decreased off-site integration of the protected modification template.

The term "cell" herein refers to any type of cell such as a prokaryotic or eukaryotic cell. A eukaryotic cell has a nucleus and other membrane-enclosed structures (organelles), whereas a prokaryotic cell lacks a nucleus. A cell can be a mammalian cell or non-mammalian cell. Non-mammalian cells can be eukaryotic or prokaryotic. For example, a non-mammalian cell herein can refer to a microbial cell or cell of a non-mammalian multicellular organism such as a plant, insect, nematode, avian species, amphibian, reptile, or fish. A microbial cell herein can refer to a fungal cell (e.g., yeast cell), prokaryotic cell, protist cell (e.g., algal cell), euglenoid cell, stramenopile cell, or oomycete cell, for example. A prokaryotic cell herein can refer to a bacterial cell or archaeal cell, for example.

The term "yeast" herein refers to fungal species that predominantly exist in unicellular form. Yeast can alternatively be referred to as "yeast cells". A yeast herein can be characterized as either a conventional yeast or non-conventional yeast, for example.

The term "conventional yeast" ("model yeast") herein generally refers to Saccharomyces or Schizosaccharomyces yeast species. Conventional yeast in certain embodiments are yeast that favor homologous recombination (HR) DNA repair processes over repair processes mediated by non-homologous end-joining (NHEJ).

The term "non-conventional yeast" herein refers to any yeast that is not a "conventional" ("model") yeast such as a *Saccharomyces* (e.g., *S*. *cerevisiae*, which is also known as budding yeast, baker's yeast, and/or brewer's yeast) or *Schizosaccharomyces* (e.g., *S*. *pombe*, which is also known as fission yeast) species. A non-conventional yeast in certain aspects herein can be one that reproduces asexually (anamorphic) or sexually (teleomorphic). While non-conventional yeast herein typically exist in unicellular form, certain types of these yeast may optionally be able to form pseudohyphae (strings of connected budding cells). In still further aspects, a non-conventional yeast may be haploid or diploid, and/or may have the ability to exist in either of these ploidy forms. Non-conventional yeast are described in Non-Conventional Yeasts in Genetics, Biochemistry and Biotechnology: Practical Protocols (K. Wolf, K.D. Breunig, G. Barth, Eds., Springer-Verlag, Berlin, Germany, 2003) and Spencer et al. (Appl. Microbiol. Biotechnol. 58:147-156). Non-conventional yeast in certain embodiments may additionally (or alternatively) be yeast that favor NHEJ DNA repair processes over repair processes mediated by HR. Definition of a non-conventional yeast along these lines - preference of NHEJ over HR - is further disclosed by Chen et al. (PLoS ONE 8:e57952). Preferred non-conventional yeast herein are those of the genus Yarrowia (e.g., Yarrowia lipolytica).

CRISPR loci (Clustered Regularly Interspaced Short Palindromic Repeats) (also known as SPIDRs--SPacer Interspersed Direct Repeats) constitute a family of DNA loci. CRISPR loci consist of short and highly conserved DNA repeats (typically 24 to 40 bp, repeated from 1 to 140 times - also referred to as CRISPR-repeats) which are partially palindromic. The repeated sequences (usually specific to a species) are interspaced by variable sequences of constant length (typically 20 to 58 bp depending on the CRISPR locus (WO2007/025097, published March 1, 2007). Bacteria and archaea have evolved adaptive immune defenses termed clustered regularly interspaced short palindromic repeats (CRISPR)/CRISPR-associated (Cas) systems that use short RNA to direct degradation of foreign nucleic acids ((Horvath and Barrangou, Science 327:167-170; Karginov and Hannon, Mol. Cell 37:7-19). WO2007/025097, published March 1, 2007). The type II CRISPR/Cas system from bacteria employs a crRNA (CRISPR RNA) and tracrRNA (trans-activating CRISPR RNA) to guide the Cas endonuclease to its DNA target. The crRNA contains a region complementary to one strand of the double strand DNA target and a region that base pairs with the tracrRNA (trans-activating CRISPR RNA) forming a RNA duplex that directs the Cas endonuclease to cleave the DNA target.

Cas gene includes a gene that is generally coupled, associated or close to, or in the vicinity of flanking CRISPR loci. The terms "Cas gene", "CRISPR-associated (Cas) gene" are used interchangeably herein. A comprehensive review of the Cas protein family is presented in Haft et al. (2005) Computational Biology, PLoS Comput Biol 1(6): e60. doi:10.1371/journal.pcbi.0010060. As described therein, 41 CRISPR-associated (Cas) gene families are described, in addition to the four previously known gene families. It shows that CRISPR systems belong to different classes, with different repeat patterns, sets of genes, and species ranges. The number of Cas genes at a given CRISPR locus can vary between species.

The term Cas endonuclease herein refers to a protein encoded by a Cas (CRISPR-associated) gene. A Cas endonuclease, when in complex with a suitable polynucleotide component, is capable of recognizing, binding to, and optionally nicking or cleaving all or part of a specific DNA target sequence.

As used herein, the terms "guide polynucleotide/Cas endonuclease complex", "guide polynucleotide/Cas endonuclease system", " guide polynucleotide/Cas complex", "guide polynucleotide/Cas system", "guided Cas system" are used interchangeably herein and refer to at least one guide polynucleotide and at least one Cas endonuclease that are capable of forming a complex, wherein said guide polynucleotide/Cas endonuclease complex can direct the Cas endonuclease to a DNA target site, enabling the Cas endonuclease to recognize, bind to, and optionally nick or cleave (introduce a single or double strand break) the DNA target site. A guide polynucleotide/Cas endonuclease complex herein can comprise Cas protein(s) and suitable polynucleotide component(s) of any of the four known CRISPR systems (Horvath and Barrangou, Science 327:167-170) such as a type I, II, or III CRISPR system. A Cas endonuclease unwinds the DNA duplex at the target sequence and optionally cleaves at least one DNA strand, as mediated by recognition of the target sequence by a polynucleotide (such as, but not limited to, a crRNA or guide RNA) that is in complex with the Cas protein. Such recognition and cutting of a target sequence by a Cas endonuclease typically occurs if the correct protospacer-adjacent motif (PAM) is located at or adjacent to the 3' end of the DNA target sequence. Alternatively, a Cas protein herein may lack DNA cleavage or nicking activity, but can still specifically bind to a DNA target sequence when complexed with a suitable RNA component. (See also U.S. Patent Application US 2015-0082478 A1, published on March 19, 2015 and US 2015-0059010 A1, published on February 26, 2015).

A guide polynucleotide/Cas endonuclease complex can cleave one or both strands of a DNA target sequence. A guide polynucleotide/Cas endonuclease complex that can cleave both strands of a DNA target sequence typically comprises a Cas protein that has all of its endonuclease domains in a functional state (e.g., wild type endonuclease domains or variants thereof retaining some or all activity in each endonuclease domain). Thus, a wild type Cas protein (e.g., a Cas9 protein disclosed herein), or a variant thereof retaining some or all activity in each endonuclease domain of the Cas protein, is a suitable example of a Cas endonuclease that can cleave both strands of a DNA target sequence. A Cas9 protein comprising functional RuvC and HNH nuclease domains is an example of a Cas protein that can cleave both strands of a DNA target sequence. A guide polynucleotide/Cas endonuclease complex that can cleave one strand of a DNA target sequence can be characterized herein as having nickase activity (e.g., partial cleaving capability). A Cas nickase typically comprises one functional endonuclease domain that allows the Cas to cleave only one strand (i.e., make a nick) of a DNA target sequence. For example, a Cas9 nickase may comprise (i) a mutant, dysfunctional RuvC domain and (ii) a functional HNH domain (e.g., wild type HNH domain). As another example, a Cas9 nickase may comprise (i) a functional RuvC domain (e.g., wild type RuvC domain) and (ii) a mutant, dysfunctional HNH domain. Non-limiting examples of Cas9 nickases suitable for use herein are disclosed by Gasiunas et al. (Proc. Natl. Acad. Sci. U.S.A. 109:E2579-E2586), Jinek et al. (Science 337:816-821), Sapranauskas et al. (Nucleic Acids Res. 39:9275-9282) and in U.S. Patent Appl. Publ. No. 2014/0189896.

A pair of Cas9 nickases can be used to increase the specificity of DNA targeting. In general, this can be done by providing two Cas9 nickases that, by virtue of being associated with RNA components with different guide sequences, target and nick nearby DNA sequences on opposite strands in the region for desired targeting. Such nearby cleavage of each DNA strand creates a double strand break (i.e., a DSB with single-stranded overhangs), which is then recognized as a substrate for non-homologous-end-joining, NHEJ (leading to indel formation) or homologous recombination, HR. Each nick in these embodiments can be at least about 5, 10, 15, 20, 30, 40, 50, 60, 70, 80, 90, or 100 (or any integer between 5 and 100) bases apart from each other, for example. One or two Cas9 nickase proteins herein can be used in a Cas9 nickase pair. For example, a Cas9 nickase with a mutant RuvC domain, but functioning HNH domain (i.e., Cas9 HNH+/RuvC-), could be used (e.g., *Streptococcus pyogenes* Cas9 HNH+/RuvC-). Each Cas9 nickase (e.g., Cas9 HNH+/RuvC-) would be directed to specific DNA sites nearby each other (up to 100 base pairs apart) by using suitable RNA components herein with guide RNA sequences targeting each nickase to each specific DNA site.

A Cas protein can be part of a fusion protein comprising one or more heterologous protein domains (e.g., 1, 2, 3, or more domains in addition to the Cas protein). Such a fusion protein may comprise any additional protein sequence, and optionally a linker sequence between any two domains, such as between Cas and a first heterologous domain. Examples of protein domains that may be fused to a Cas protein herein include, without limitation, epitope tags (e.g., histidine [His], V5, FLAG, influenza hemagglutinin [HA], myc, VSV-G, thioredoxin [Trx]), reporters (e.g., glutathione-5-transferase [GST], horseradish peroxidase [HRP], chloramphenicol acetyltransferase [CAT], beta-galactosidase, beta-glucuronidase [GUS], luciferase, green fluorescent protein [GFP], HcRed, DsRed, cyan fluorescent protein [CFP], yellow fluorescent protein [YFP], blue fluorescent protein [BFP]), and domains having one or more of the following activities: methylase activity, demethylase activity, transcription activation activity (e.g., VP16 or VP64), transcription repression activity, transcription release factor activity, histone modification activity, RNA cleavage activity and nucleic acid binding activity. A Cas protein can also be in fusion with a protein that binds DNA molecules or other molecules, such as maltose binding protein (MBP), S-tag, Lex A DNA binding domain (DBD), GAL4A DNA binding domain, and herpes simplex virus (HSV) VP16.

A Cas protein herein can be from any of the following genera: *Aeropyrum, Pyrobaculum, Sulfolobus, Archaeoglobus, Haloarcula, Methanobacteriumn, Methanococcus, Methanosarcina, Methanopyrus, Pyrococcus, Picrophilus, Thernioplasnia, Corynebacterium, Mycobacterium, Streptomyces, Aquifrx, Porphvromonas, Chlorobium, Thermus, Bacillus, Listeria, Staphylococcus, Clostridium, Thermoanaerobacter, Mycoplasma, Fusobacterium, Azarcus, Chromobacterium, Neisseria, Nitrosomonas, Desulfovibrio, Geobacter, Myrococcus, Campylobacter, Wolinella, Acinetobacter, Erwinia, Escherichia, Legionella, Methylococcus, Pasteurella, Photobacterium, Salmonella, Xanthomonas, Yersinia, Streptococcus, Treponema, Francisella,* or *Thermotoga.* Alternatively, a Cas protein herein can be encoded, for example, by any of SEQ ID NOs:462-465, 467-472, 474-477, 479-487, 489-492, 494-497, 499-503, 505-508, 510-516, or 517-521 as disclosed in U.S. Appl. Publ. No. 2010/0093617.

A guide polynucleotide/Cas endonuclease complex in certain embodiments can bind to a DNA target site sequence, but does not cleave any strand at the target site sequence. Such a complex may comprise a Cas protein in which all of its nuclease domains are mutant, dysfunctional. For example, a Cas9 protein herein that can bind to a DNA target site sequence, but does not cleave any strand at the target site sequence, may comprise both a mutant, dysfunctional RuvC domain and a mutant, dysfunctional HNH domain. A Cas protein herein that binds, but does not cleave, a target DNA sequence can be used to modulate gene expression, for example, in which case the Cas protein could be fused with a transcription factor (or portion thereof) (e.g., a repressor or activator, such as any of those disclosed herein).

The Cas endonuclease gene can be a gene encoding a Type II Cas9 endonuclease , such as but not limited to, Cas9 genes listed in SEQ ID NOs: 462, 474, 489, 494, 499, 505, and 518 of WO2007/025097published March 1, 2007. The Cas endonuclease gene can be operably linked to a SV40 nuclear targeting signal upstream of the Cas codon region and a bipartite VirD2 nuclear localization signal (Tinland et al. (1992) Proc. Natl. Acad. Sci. USA 89:7442-6) downstream of the Cas codon region. "Cas9" (formerly referred to as Cas5, Csn1, or Csx12) herein refers to a Cas endonuclease of a type II CRISPR system that forms a complex with a crNucleotide and a tracrNucleotide, or with a single guide polynucleotide, for specifically recognizing and cleaving all or part of a DNA target sequence. Cas9 protein comprises a RuvC nuclease domain and an HNH (H-N-H) nuclease domain, each of which can cleave a single DNA strand at a target sequence (the concerted action of both domains leads to DNA double-strand cleavage, whereas activity of one domain leads to a nick). In general, the RuvC domain comprises subdomains I, II and III, where domain I is located near the N-terminus of Cas9 and subdomains II and III are located in the middle of the protein, flanking the HNH domain (Hsu et al, Cell 157:1262-1278). ). A type II CRISPR system includes a DNA cleavage system utilizing a Cas9 endonuclease in complex with at least one polynucleotide component. For example, a Cas9 can be in complex with a CRISPR RNA (crRNA) and a trans-activating CRISPR RNA (tracrRNA). In another example, a Cas9 can be in complex with a single guide RNA.

The amino acid sequence of a Cas9 protein described herein, as well as certain other Cas proteins herein, may be derived from a *Streptococcus* (e.g., *S*. *pyogenes, S. pneumoniae, S. thermophilus, S. agalactiae, S. parasanguinis, S. oralis, S. salivarius, S*. *macacae, S. dysgalactiae, S. anginosus, S. constellatus, S. pseudoporcinus, S. mutans*), *Listeria* (e.g., *L. innocua*), *Spiroplasma* (e.g., *S*. *apis, S*. *syrphidicola*), *Peptostreptococcaceae, Atopobium, Porphyromonas* (e.g., *P. catoniae*), *Prevotella* (e.g., *P. intermedia*), *Veillonella, Treponema* (e.g., *T. socranskii, T. denticola*), *Capnocytophaga, Finegoldia* (e.g., *F. magna*), *Coriobacteriaceae* (e.g., *C. bacterium*), *Olsenella* (e.g., *O*. *profusa*), *Haemophilus* (e.g., *H. sputorum, H. pittmaniae*), *Pasteurella* (e.g., *P. bettyae*), *Olivibacter* (e.g., *O*. *sitiensis*), *Epilithonimonas* (e.g., *E*. *tenax*), *Mesonia* (e.g., *M. mobilis*), *Lactobacillus* (e.g., *L. plantarum*), *Bacillus* (e.g., *B. cereus*), *Aquimarina* (e.g., *A. muelleri*), *Chryseobacterium* (e.g., *C. palustre*), *Bacteroides* (e.g., *B. graminisolvens*), *Neisseria* (e.g., *N. meningitidis*), *Francisella* (e.g., *F. novicida*), or *Flavobacterium* (e.g., *F. frigidarium, F. soli*) species, for example. An *S*. *pyogenes* Cas9 is preferred in certain aspects herein. As another example, a Cas9 protein can be any of the Cas9 proteins disclosed in Chylinski et al. (RNA Biology 10:726-737).

Accordingly, the sequence of a Cas9 protein herein can comprise, for example, any of the Cas9 amino acid sequences disclosed in GenBank Accession Nos. G3ECR1 (*S*. *thermophilus),* WP_026709422, WP_027202655, WP_027318179, WP_027347504, WP_027376815, WP_027414302, WP_027821588, WP_027886314, WP_027963583, WP_028123848, WP_028298935, Q03JI6 (*S*. *thermophilus),* EGP66723, EGS38969, EGV05092, EHI65578 (*S*. *pseudoporcinus*), EIC75614 (*S*. *oralis),* EID22027 (*S*. *constellatus*), EIJ69711, EJP22331 (*S*. *oralis*), EJP26004 (*S*. *anginosus*), EJP30321, EPZ44001 (*S*. *pyogenes),* EPZ46028 (*S*. *pyogenes),* EQL78043 (*S*. *pyogenes),* EQL78548 (*S*. *pyogenes),* ERL10511, ERL12345, ERL19088 (*S*. *pyogenes),* ESA57807 (*S*. *pyogenes),* ESA59254 (*S*. *pyogenes),* ESU85303 (*S*. *pyogenes),* ETS96804, UC75522, EGR87316 (*S*. *dysgalactiae*), EGS33732, EGV01468 (*S*. *oralis),* EHJ52063 (*S*. *macacae*), EID26207 (*S*. *oralis),* EID33364, EIG27013 (S. *parasanguinis*), EJF37476, EJO19166 *(Streptococcus* sp. BS35b), EJU16049, EJU32481, YP_006298249, ERF61304, ERK04546, ETJ95568 (*S*. *agalactiae*), TS89875, ETS90967 *(Streptococcus* sp. SR4), ETS92439, EUB27844 *(Streptococcus* sp. BS21), AFJ08616, EUC82735 *(Streptococcus* sp. CM6), EWC92088, EWC94390, EJP25691, YP_008027038, YP_008868573, AGM26527, AHK22391, AHB36273, Q927P4, G3ECR1, or Q99ZW2 (*S*. *pyogenes).* A variant of any of these Cas9 protein sequences may be used, but should have specific binding activity, and optionally endonucleolytic activity, toward DNA when associated with an RNA component herein. Such a variant may comprise an amino acid sequence that is at least about 80%, 81%, 82%, 83%, 84%, 85%, 86%, 87%, 88%, 89%, 90%, 91%, 92%, 93%, 94%, 95%, 96%, 97%, 98%, or 99% identical to the amino acid sequence of the reference Cas9.

Alternatively, a Cas9 protein herein can be encoded by any of SEQ ID NOs:462 (*S*. *thermophilus),* 474 (*S*. *thermophilus),* 489 (*S*. *agalactiae*), 494 (*S*. *agalactiae*), 499 (*S*. *mutans*), 505 (*S*. *pyogenes),* or 518 (*S*. *pyogenes)* as disclosed in U.S. Appl. Publ. No. 2010/0093617, for example. Alternatively still, a Cas9 protein may comprise an amino acid sequence that is at least about 80%, 81%, 82%, 83%, 84%, 85%, 86%, 87%, 88%, 89%, 90%, 91%, 92%, 93%, 94%, 95%, 96%, 97%, 98%, or 99% identical to any of the foregoing amino acid sequences, for example. Such a variant Cas9 protein should have specific binding activity, and optionally cleavage or nicking activity, toward DNA when associated with an RNA component herein.

The origin of a Cas protein used herein (e.g., Cas9) may be from the same species from which the RNA component(s) is derived, or it can be from a different species. For example, an RGEN comprising a Cas9 protein derived from a *Streptococcus* species (e.g., *S*. *pyogenes* or *S*. *thermophilus*) may be complexed with at least one RNA component having a sequence (e.g., crRNA repeat sequence, tracrRNA sequence) derived from the same *Streptococcus* species. Alternatively, the origin of a Cas protein used herein (e.g., Cas9) may be from a different species from which the RNA component(s) is derived (the Cas protein and RNA component(s) may be heterologous to each other); such heterologous Cas/RNA component RGENs should have DNA targeting activity.

Determining binding activity and/or endonucleolytic activity of a Cas protein herein toward a specific target DNA sequence may be assessed by any suitable assay known in the art, such as disclosed in U.S. Patent No. 8697359, which is disclosed herein by reference. A determination can be made, for example, by expressing a Cas protein and suitable RNA component in a non-conventional yeast, and then examining the predicted DNA target site for the presence of an indel (a Cas protein in this particular assay would have complete endonucleolytic activity [double-strand cleaving activity]). Examining for the presence of an indel at the predicted target site could be done via a DNA sequencing method or by inferring indel formation by assaying for loss of function of the target sequence, for example. In another example, Cas protein activity can be determined by expressing a Cas protein and suitable RNA component in a non-conventional yeast that has been provided a donor DNA comprising a sequence homologous to a sequence in, at or near the target site. The presence of donor DNA sequence at the target site (such as would be predicted by successful HR between the donor and target sequences) would indicate that targeting occurred.

A Cas protein herein such as a Cas9 typically further comprises a heterologous nuclear localization sequence (NLS). A heterologous NLS amino acid sequence herein may be of sufficient strength to drive accumulation of a Cas protein in a detectable amount in the nucleus of a yeast cell herein, for example. An NLS may comprise one (monopartite) or more (e.g., bipartite) short sequences (e.g., 2 to 20 residues) of basic, positively charged residues (e.g., lysine and/or arginine), and can be located anywhere in a Cas amino acid sequence but such that it is exposed on the protein surface. An NLS may be operably linked to the N-terminus or C-terminus of a Cas protein herein, for example. Two or more NLS sequences can be linked to a Cas protein, for example, such as on both the N- and C-termini of a Cas protein. Non-limiting examples of suitable NLS sequences herein include those disclosed in U.S. Patent Nos. 6660830 and 7309576 (e.g., Table 1 therein).

The Cas endonuclease can comprise a modified form of the Cas9 polypeptide. The modified form of the Cas9 polypeptide can include an amino acid change (e.g., deletion, insertion, or substitution) that reduces the naturally-occurring nuclease activity of the Cas9 protein. For example, in some instances, the modified form of the Cas9 protein has less than 50%, less than 40%, less than 30%, less than 20%, less than 10%, less than 5%, or less than 1% of the nuclease activity of the corresponding wild-type Cas9 polypeptide (US patent application US20140068797 A1, published on March 6, 2014). In some cases, the modified form of the Cas9 polypeptide has no substantial nuclease activity and is referred to as catalytically "inactivated Cas9" or "deactivated cas9 (dCas9)." Catalytically inactivated Cas9 variants include Cas9 variants that contain mutations in the HNH and RuvC nuclease domains. These catalytically inactivated Cas9 variants are capable of interacting with sgRNA and binding to the target site in vivo but cannot cleave either strand of the target DNA.

A catalytically inactive Cas9 can be fused to a heterologous sequence (US patent application US20140068797 A1, published on March 6, 2014). Suitable fusion partners include, but are not limited to, a polypeptide that provides an activity that indirectly increases transcription by acting directly on the target DNA or on a polypeptide (e.g., a histone or other DNA-binding protein) associated with the target DNA. Additional suitable fusion partners include, but are not limited to, a polypeptide that provides for methyltransferase activity, demethylase activity, acetyltransferase activity, deacetylase activity, kinase activity, phosphatase activity, ubiquitin ligase activity, deubiquitinating activity, adenylation activity, deadenylation activity, SUMOylating activity, deSUMOylating activity, ribosylation activity, deribosylation activity, myristoylation activity, or demyristoylation activity. Further suitable fusion partners include, but are not limited to, a polypeptide that directly provides for increased transcription of the target nucleic acid (e.g., a transcription activator or a fragment thereof, a protein or fragment thereof that recruits a transcription activator, a small molecule/drug-responsive transcription regulator, etc.). A catalytically inactive Cas9 can also be fused to a Fokl nuclease to generate double strand breaks (Guilinger et al. Nature biotechnology, volume 32, number 6, June 2014).

Any guided endonuclease can be used in the methods disclosed herein. Such endonucleases include, but are not limited to Cas9 and Cpf1 endonucleases. Many endonucleases have been described to date that can recognize specific PAM sequences (see for example -US patent application14/772711 filed March 12, 2014 and Zetsche B et al. 2015. Cell 163, 1013) and cleave the target DNA at a specific positions. It is understood that based on the methods and embodiments described herein utilizing a guided Cas system one can now tailor these methods such that they can utilize any guided endonuclease system.

The terms "functional fragment", "fragment that is functionally equivalent" and "functionally equivalent fragment" of a Cas endonuclease are used interchangeably herein, and refer to a portion or subsequence of the Cas endonuclease sequence of the present disclosure in which the ability to recognize, bind to, and optionally nick or cleave (introduce a single or double strand break in) the target site is retained.

The terms "functional variant ", "Variant that is functionally equivalent" and "functionally equivalent variant" of a Cas endonuclease are used interchangeably herein, and refer to a variant of the Cas endonuclease of the present disclosure in which the ability to recognize, bind to, and optionally nick or cleave (introduce a single or double strand break in) the target site is retained. Fragments and variants can be obtained via methods such as site-directed mutagenesis and synthetic construction.

The Cas endonuclease gene includes a Yarrowia codon optimized *Streptococcus pyogenes* Cas9 gene that can recognize any genomic sequence of the form N(12-30)NGG can in principle be targeted or a Cas9 endonuclease originated from an organism selected from the group consisting of *Brevibacillus laterosporus, Lactobacillus reuteri* Mlc3, *Lactobacillus rossiae* DSM 15814, *Pediococcus pentosaceus* SL4, *Lactobacillus nodensis* JCM 14932, *Sulfurospirillum* sp. SCADC, *Bifidobacterium thermophilum* DSM 20210, *Loktanella vestfoldensis, Sphingomonas sanxanigenens* NX02, *Epilithonimonas tenax* DSM 16811, *Sporocytophaga myxococcoides* and *Psychroflexus torquis* ATCC 700755, wherein said Cas9 endonuclease can form a guide RNA/Cas endonuclease complex capable of recognizing, binding to, and optionally nicking or cleaving all or part of a DNA target sequence. (US provisional patent application BB2475

The Cas endonuclease can be provided to a cell by any method known in the art, for example, but not limited to transient introduction methods, transfection and/or topical application or indirectly via recombination constructs.

Endonucleases are enzymes that cleave the phosphodiester bond within a polynucleotide chain, and include restriction endonucleases that cleave DNA at specific sites without damaging the bases. Restriction endonucleases include Type I, Type II, Type III, and Type IV endonucleases, which further include subtypes. In the Type I and Type III systems, both the methylase and restriction activities are contained in a single complex. Endonucleases also include meganucleases, also known as homing endonucleases (HEases), which like restriction endonucleases, bind and cut at a specific recognition site, however the recognition sites for meganucleases are typically longer, about 18 bp or more (patent application WO-PCT PCT/US12/30061 filed on March 22, 2012). Meganucleases have been classified into four families based on conserved sequence motifs, the families are the LAGLIDADG, GIY-YIG, H-N-H, and His-Cys box families. These motifs participate in the coordination of metal ions and hydrolysis of phosphodiester bonds. HEases are notable for their long recognition sites, and for tolerating some sequence polymorphisms in their DNA substrates. The naming convention for meganuclease is similar to the convention for other restriction endonuclease. Meganucleases are also characterized by prefix F-, I-, or PI- for enzymes encoded by free-standing ORFs, introns, and inteins, respectively. One step in the recombination process involves polynucleotide cleavage at or near the recognition site. This cleaving activity can be used to produce a double-strand break. For reviews of site-specific recombinases and their recognition sites, see, Sauer (1994) Curr Op Biotechnol 5:521-7; and Sadowski (1993) FASEB 7:760-7. In some examples the recombinase is from the Integrase or Resolvase families.

TAL effector nucleases are a new class of sequence-specific nucleases that can be used to make double-strand breaks at specific target sequences in the genome of a non-conventional yeast or other organism. (Miller et al. (2011) Nature Biotechnology 29:143-148). Zinc finger nucleases (ZFNs) are engineered double-strand break inducing agents comprised of a zinc finger DNA binding domain and a double-strand-break-inducing agent domain. Recognition site specificity is conferred by the zinc finger domain, which typically comprising two, three, or four zinc fingers, for example having a C2H2 structure, however other zinc finger structures are known and have been engineered. Zinc finger domains are amenable for designing polypeptides which specifically bind a selected polynucleotide recognition sequence. ZFNs include an engineered DNA-binding zinc finger domain linked to a non-specific endonuclease domain, for example nuclease domain from a Type IIs endonuclease such as Fokl. Additional functionalities can be fused to the zinc-finger binding domain, including transcriptional activator domains, transcription repressor domains, and methylases. In some examples, dimerization of nuclease domain is required for cleavage activity. Each zinc finger recognizes three consecutive base pairs in the target DNA. For example, a 3 finger domain recognized a sequence of 9 contiguous nucleotides, with a dimerization requirement of the nuclease, two sets of zinc finger triplets are used to bind an 18 nucleotide recognition sequence.

As used herein, the term "guide polynucleotide", relates to a polynucleotide sequence that can form a complex with a Cas endonuclease and enables the Cas endonuclease to recognize, bind to, and optionally cleave a DNA target site. The guide polynucleotide can be a single molecule or a double molecule. The guide polynucleotide sequence can be a RNA sequence, a DNA sequence, or a combination thereof (a RNA-DNA combination sequence). Optionally, the guide polynucleotide can comprise at least one nucleotide, phosphodiester bond or linkage modification such as, but not limited, to Locked Nucleic Acid (LNA), 5-methyl dC, 2,6-Diaminopurine, 2'-Fluoro A, 2'-Fluoro U, 2'-O-Methyl RNA, phosphorothioate bond, linkage to a cholesterol molecule, linkage to a polyethylene glycol molecule, linkage to a spacer 18 (hexaethylene glycol chain) molecule, or 5' to 3' covalent linkage resulting in circularization. A guide polynucleotide that solely comprises ribonucleic acids is also referred to as a "guide RNA" or "gRNA" ( See also U.S. Patent Application US 2015-0082478 A1, published on March 19, 2015 and US 2015-0059010 A1, published on February 26, 2015.

The guide polynucleotide can be a double molecule (also referred to as duplex guide polynucleotide) comprising a crNucleotide sequence and a tracrNucleotide sequence. The crNucleotide includes a first nucleotide sequence domain (referred to as Variable Targeting domain or VT domain) that can hybridize to a nucleotide sequence in a target DNA and a second nucleotide sequence (also referred to as a tracr mate sequence) that is part of a Cas endonuclease recognition (CER) domain. The tracr mate sequence can hybridized to a tracrNucleotide along a region of complementarity and together form the Cas endonuclease recognition domain or CER domain. The CER domain is capable of interacting with a Cas endonuclease polypeptide. The crNucleotide and the tracrNucleotide of the duplex guide polynucleotide can be RNA, DNA, and/or RNA-DNA- combination sequences. In some embodiments, the crNucleotide molecule of the duplex guide polynucleotide is referred to as "crDNA" (when composed of a contiguous stretch of DNA nucleotides) or "crRNA" (when composed of a contiguous stretch of RNA nucleotides), or "crDNA-RNA" (when composed of a combination of DNA and RNA nucleotides). The crNucleotide can comprise a fragment of the cRNA naturally occurring in Bacteria and Archaea. The size of the fragment of the cRNA naturally occurring in Bacteria and Archaea that can be present in a crNucleotide disclosed herein can range from, but is not limited to, 2, 3, 4, 5, 6, 7, 8, 9,10, 11, 12, 13, 14, 15, 16, 17, 18, 19, 20 or more nucleotides. In some embodiments the tracrNucleotide is referred to as "tracrRNA" (when composed of a contiguous stretch of RNA nucleotides) or "tracrDNA" (when composed of a contiguous stretch of DNA nucleotides) or "tracrDNA-RNA" (when composed of a combination of DNA and RNA nucleotides. In one embodiment, the RNA that guides the RNA/ Cas9 endonuclease complex is a duplexed RNA comprising a duplex crRNA-tracrRNA.

The tracrRNA (trans-activating CRISPR RNA) contains, in the 5'-to-3' direction, (i) a sequence that anneals with the repeat region of CRISPR type II crRNA and (ii) a stem loop-containing portion (Deltcheva et al., Nature 471:602-607). The duplex guide polynucleotide can form a complex with a Cas endonuclease, wherein said guide polynucleotide/Cas endonuclease complex (also referred to as a guide polynucleotide/Cas endonuclease system) can direct the Cas endonuclease to a genomic target site, enabling the Cas endonuclease to recognize, bind to, and optionally nick or cleave (introduce a single or double strand break) into the target site. ( See also U.S. Patent Application US 2015-0082478 A1, published on March 19, 2015 and US 2015-0059010 A1, published on February 26, 2015.)

The guide polynucleotide can also be a single molecule (also referred to as single guide polynucleotide) comprising a crNucleotide sequence linked to a tracrNucleotide sequence. The single guide polynucleotide comprises a first nucleotide sequence domain (referred to as Variable Targeting domain or VT domain) that can hybridize to a nucleotide sequence in a target DNA and a Cas endonuclease recognition domain (CER domain), that interacts with a Cas endonuclease polypeptide. By "domain" it is meant a contiguous stretch of nucleotides that can be RNA, DNA, and/or RNA-DNA-combination sequence. The VT domain and /or the CER domain of a single guide polynucleotide can comprise a RNA sequence, a DNA sequence, or a RNA-DNA-combination sequence. The single guide polynucleotide being comprised of sequences from the crNucleotide and the tracrNucleotide may be referred to as "single guide RNA" (when composed of a contiguous stretch of RNA nucleotides) or "single guide DNA" (when composed of a contiguous stretch of DNA nucleotides) or "single guide RNA-DNA" (when composed of a combination of RNA and DNA nucleotides). The single guide polynucleotide can form a complex with a Cas endonuclease, wherein said guide polynucleotide/Cas endonuclease complex (also referred to as a guide polynucleotide/Cas endonuclease system) can direct the Cas endonuclease to a genomic target site, enabling the Cas endonuclease to recognize, bind to, and optionally nick or cleave (introduce a single or double strand break) the target site. (See also U.S. Patent Application US 2015-0082478 A1, published on March 19, 2015 and US 2015-0059010 A1, published on February 26, 2015.)

The term "variable targeting domain" or "VT domain" is used interchangeably herein and includes a nucleotide sequence that can hybridize (is complementary) to one strand (nucleotide sequence) of a double strand DNA target site. The % complementation between the first nucleotide sequence domain (VT domain ) and the target sequence can be at least 50%, 51%, 52%, 53%, 54%, 55%, 56%, 57%, 58%, 59%, 60%, 61%, 62%, 63%, 63%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100%. The variable targeting domain can be at least 12, 13, 14, 15, 16, 17, 18, 19, 20, 21, 22, 23, 24, 25, 26, 27, 28, 29 or 30 nucleotides in length. In some embodiments, the variable targeting domain comprises a contiguous stretch of 12 to 30 nucleotides. The variable targeting domain can be composed of a DNA sequence, a RNA sequence, a modified DNA sequence, a modified RNA sequence, or any combination thereof.

The term "Cas endonuclease recognition domain" or "CER domain" (of a guide polynucleotide) is used interchangeably herein and includes a nucleotide sequence that interacts with a Cas endonuclease polypeptide. A CER domain comprises a tracrNucleotide mate sequence followed by a tracrNucleotide sequence. The CER domain can be composed of a DNA sequence, a RNA sequence, a modified DNA sequence, a modified RNA sequence (see for example US 2015-0059010 A1, published on February 26, 2015), or any combination thereof.

The nucleotide sequence linking the crNucleotide and the tracrNucleotide of a single guide polynucleotide can comprise a RNA sequence, a DNA sequence, or a RNA-DNA combination sequence. In one embodiment, the nucleotide sequence linking the crNucleotide and the tracrNucleotide of a single guide polynucleotide can be at least 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 78, 79, 80, 81, 82, 83, 84, 85, 86, 87, 88, 89, 90, 91, 92, 93, 94, 95, 96, 97, 98, 99 or 100 nucleotides in length. In another embodiment, the nucleotide sequence linking the crNucleotide and the tracrNucleotide of a single guide polynucleotide can comprise a tetraloop sequence, such as, but not limiting to a GAAA tetraloop sequence.

Nucleotide sequence modification of the guide polynucleotide, VT domain and/or CER domain can be selected from, but not limited to , the group consisting of a 5' cap, a 3' polyadenylated tail, a riboswitch sequence, a stability control sequence, a sequence that forms a dsRNA duplex, a modification or sequence that targets the guide poly nucleotide to a subcellular location, a modification or sequence that provides for tracking , a modification or sequence that provides a binding site for proteins , a Locked Nucleic Acid (LNA), a 5-methyl dC nucleotide, a 2,6-Diaminopurine nucleotide, a 2'-Fluoro A nucleotide, a 2'-Fluoro U nucleotide; a 2'-O-Methyl RNA nucleotide, a phosphorothioate bond, linkage to a cholesterol molecule, linkage to a polyethylene glycol molecule, linkage to a spacer 18 molecule, a 5' to 3' covalent linkage, or any combination thereof. These modifications can result in at least one additional beneficial feature, wherein the additional beneficial feature is selected from the group of a modified or regulated stability, a subcellular targeting, tracking, a fluorescent label, a binding site for a protein or protein complex, modified binding affinity to complementary target sequence, modified resistance to cellular degradation, and increased cellular permeability.

The terms "functional fragment", "fragment that is functionally equivalent" and "functionally equivalent fragment" of a guide RNA, crRNA or tracrRNA are used interchangeably herein, and refer to a portion or subsequence of the guide RNA, crRNA or tracrRNA , respectively, of the present disclosure in which the ability to function as a guide RNA, crRNA or tracrRNA, respectively, is retained.

The terms "functional variant ", "Variant that is functionally equivalent" and "functionally equivalent variant" of a guide RNA, crRNA or tracrRNA (respectively) are used interchangeably herein, and refer to a variant of the guide RNA, crRNA or tracrRNA, respectively, of the present disclosure in which the ability to function as a guide RNA, crRNA or tracrRNA, respectively, is retained.

The terms "single guide RNA" and "sgRNA" are used interchangeably herein and relate to a synthetic fusion of two RNA molecules, a crRNA (CRISPR RNA) comprising a variable targeting domain (linked to a tracr mate sequence that hybridizes to a tracrRNA), fused to a tracrRNA (trans-activating CRISPR RNA). The single guide RNA can comprise a crRNA or crRNA fragment and a tracrRNA or tracrRNA fragment of the type II CRISPR/Cas system that can form a complex with a type II Cas endonuclease, wherein said guide RNA/Cas endonuclease complex can direct the Cas endonuclease to a DNA target site, enabling the Cas endonuclease to recognize, bind to, and optionally nick or cleave (introduce a single or double strand break) the DNA target site.

The terms "guide RNA/Cas endonuclease complex", "guide RNA/Cas endonuclease system", " guide RNA/Cas complex", "guide RNA/Cas system", "gRNA/Cas complex", "gRNA/Cas system", "RNA-guided endonuclease" , "RGEN" are used interchangeably herein and refer to at least one RNA component and at least one Cas endonuclease that are capable of forming a complex, wherein said guide RNA/Cas endonuclease complex can direct the Cas endonuclease to a DNA target site, enabling the Cas endonuclease to recognize, bind to, and optionally nick or cleave (introduce a single or double strand break) the DNA target site. An RNA component of an RGEN contains a ribonucleotide sequence that is complementary to a strand of a DNA target sequence. This complementary RNA sequence is also referred to herein as a "variable targeting domain" sequence. A guide RNA/Cas endonuclease complex herein can comprise Cas protein(s) and suitable RNA component(s) of any of the four known CRISPR systems (Horvath and Barrangou, Science 327:167-170) such as a type I, II, or III CRISPR system. A guide RNA/Cas endonuclease complex can comprise a Type II Cas9 endonuclease and at least one RNA component (e.g., a crRNA and tracrRNA, or a gRNA). (See also U.S. Patent Application US 2015-0082478 A1, published on March 19, 2015 and US 2015-0059010 A1, published on February 26, 2015).

The guide polynucleotide can be introduced into a cell transiently, as single stranded polynucleotide or a double stranded polynucleotide, using any method known in the art such as, but not limited to, particle bombardment, *Agrobacterium transformation* or topical applications. The guide polynucleotide can also be introduced indirectly into a cell by introducing a recombinant DNA molecule (via methods such as, but not limited to, particle bombardment or *Agrobacterium transformation*) comprising a heterologous nucleic acid fragment encoding a guide polynucleotide, operably linked to a specific promoter that is capable of transcribing the guide RNA in said cell. The specific promoter can be, but is not limited to, a RNA polymerase III promoter, which allow for transcription of RNA with precisely defined, unmodified, 5'- and 3'-ends (DiCarlo et al., Nucleic Acids Res. 41: 4336-4343; Ma et al., Mol. Ther. Nucleic Acids 3:e161).

The terms "target site", "target sequence", "target site sequence, "target DNA", "target locus", "genomic target site", "genomic target sequence", "genomic target locus" and "protospacer", are used interchangeably herein and refer to a polynucleotide sequence such as, but not limited to, a nucleotide sequence on a chromosome, episome, or any other DNA molecule in the genome (including chromosomal, choloroplastic, mitochondrial DNA, plasmid DNA) of a cell, at which a guide polynucleotide/Cas endonuclease complex can recognize, bind to, and optionally nick or cleave . The target site can be an endogenous site in the genome of a cell, or alternatively, the target site can be heterologous to the cell and thereby not be naturally occurring in the genome of the cell, or the target site can be found in a heterologous genomic location compared to where it occurs in nature. As used herein, terms "endogenous target sequence" and "native target sequence" are used interchangeable herein to refer to a target sequence that is endogenous or native to the genome of a cell and is at the endogenous or native position of that target sequence in the genome of the cell. Cells include, but are not limited to, human, non-human, animal, bacterial, archaeal, fungal, insect, yeast, non-conventional yeast, plant cells, plants, seeds as well as microorganisms produced by the methods described herein. An "artificial target site" or "artificial target sequence" are used interchangeably herein and refer to a target sequence that has been introduced into the genome of a cell. Such an artificial target sequence can be identical in sequence to an endogenous or native target sequence in the genome of a cell but be located in a different position (i.e., a non-endogenous or non-native position) in the genome of a cell.

An "altered target site", "altered target sequence", "modified target site", "modified target sequence" are used interchangeably herein and refer to a target sequence as disclosed herein that comprises at least one alteration when compared to non-altered target sequence. Such "alterations" include, for example: (i) replacement of at least one nucleotide, (ii) a deletion of at least one nucleotide, (iii) an insertion of at least one nucleotide, or (iv) any combination of (i) - (iii).

The length of the target DNA sequence (target site) can vary, and includes, for example, target sites that are at least 12, 13, 14, 15, 16, 17, 18, 19, 20, 21, 22, 23, 24, 25, 26, 27, 28, 29, 30 or more nucleotides in length. It is further possible that the target site can be palindromic, that is, the sequence on one strand reads the same in the opposite direction on the complementary strand. The nick/cleavage site can be within the target sequence or the nick/cleavage site could be outside of the target sequence. In another variation, the cleavage could occur at nucleotide positions immediately opposite each other to produce a blunt end cut or, in other Cases, the incisions could be staggered to produce single-stranded overhangs, also called "sticky ends", which can be either 5' overhangs, or 3' overhangs. Active variants of genomic target sites can also be used. Such active variants can comprise at least 65%, 70%, 75%, 80%, 85%, 90%, 91%, 92%, 93%, 94%, 95%, 96%, 97%, 98%, 99% or more sequence identity to the given target site, wherein the active variants retain biological activity and hence are capable of being recognized and cleaved by an Cas endonuclease. Assays to measure the single or double-strand break of a target site by an endonuclease are known in the art and generally measure the overall activity and specificity of the agent on DNA substrates containing recognition sites.

An "episome" herein refers to a DNA molecule that can exist in a yeast cell autonomously (can replicate and pass on to daughter cells) apart from the chromosomes of the yeast cell. Episomal DNA can be either native or heterologous to a yeast cell. Examples of native episomes herein include mitochondrial DNA (mtDNA). Examples of heterologous episomes herein include plasmids and yeast artificial chromosomes (YACs).

A "protospacer adjacent motif" (PAM) herein refers to a short nucleotide sequence adjacent to a target sequence (protospacer) that is recognized (targeted) by a guide polynucleotide/Cas endonuclease system described herein. The Cas endonuclease may not successfully recognize a target DNA sequence if the target DNA sequence is not followed by a PAM sequence. The sequence and length of a PAM herein can differ depending on the Cas protein or Cas protein complex used. The PAM sequence can be of any length but is typically 1, 2, 3, 4, 5, 6, 7, 8, 9, 10, 11, 12, 13, 14, 15, 16, 17, 18, 19 or 20 nucleotides long.

The terms "5'-cap" and "7-methylguanylate (m7G) cap" are used interchangeably herein. A 7-methylguanylate residue is located on the 5' terminus of messenger RNA (mRNA) in eukaryotes. RNA polymerase II (Pol II) transcribes mRNA in eukaryotes. Messenger RNA capping occurs generally as follows: The most terminal 5' phosphate group of the mRNA transcript is removed by RNA terminal phosphatase, leaving two terminal phosphates. A guanosine monophosphate (GMP) is added to the terminal phosphate of the transcript by a guanylyl transferase, leaving a 5'-5' triphosphate-linked guanine at the transcript terminus. Finally, the 7-nitrogen of this terminal guanine is methylated by a methyl transferase.

The terminology "not having a 5'-cap" herein is used to refer to RNA having, for example, a 5'-hydroxyl group instead of a 5'-cap. Such RNA can be referred to as "uncapped RNA", for example. Uncapped RNA can better accumulate in the nucleus following transcription, since 5'-capped RNA is subject to nuclear export. One or more RNA components herein are uncapped.

The terms "ribozyme" and "ribonucleic acid enzyme" are used interchangeably herein. A ribozyme refers to one or more RNA sequences that form secondary, tertiary, and/or quaternary structure(s) that can cleave RNA at a specific site. A ribozyme herein can be a hammerhead (HH) ribozyme, hepatitis delta virus (HDV) ribozyme, group I intron ribozyme, RnaseP ribozyme, or hairpin ribozyme, for example. A ribozyme includes a "self-cleaving ribozyme" that is capable of cleaving RNA at a cis-site relative to the ribozyme sequence (i.e., auto-catalytic, or self-cleaving). The general nature of ribozyme nucleolytic activity has been described (e.g., Lilley, Biochem. Soc. Trans. 39:641-646). A "hammerhead ribozyme" (HHR) herein may comprise a small catalytic RNA motif made up of three base-paired stems and a core of highly conserved, non-complementary nucleotides that are involved in catalysis. Pley et al. (Nature 372:68-74) and Hammann et al. (RNA 18:871-885), disclose hammerhead ribozyme structure and activity. Other non-limiting examples of ribozymes herein include Varkud satellite (VS) ribozymes, glucosamine-6-phosphate activated ribozymes (glmS), and CPEB3 ribozymes. Lilley (Biochem. Soc. Trans. 39:641-646) discloses information pertaining to ribozyme structure and activity. Examples of ribozymes that should be suitable for use herein include ribozymes disclosed in EP0707638 and U.S. Patent Nos. 6063566, 5580967, 5616459, and 5688670.

A hammerhead ribozyme herein may comprise a "minimal hammerhead" sequence as disclosed by Scott et al. (Cell 81:991-1002), for example. A hammerhead ribozyme may be a type I, type II, or type III hammerhead ribozyme, for example, as disclosed in Hammann et al. (RNA 18:871-885). Multiple means for identifying DNA encoding a hammerhead ribozyme are disclosed in Hammann et al., which can be utilized accordingly herein. A hammerhead ribozyme herein may be derived from a virus, viroid, plant virus satellite RNA, prokaryote (e.g., Archaea, cyanobacteria, acidobacteria), or eukaryote such as a plant (e.g., Arabidopsis thaliana, carnation), protist (e.g., amoeba, euglenoid), fungus (e.g., Aspergillus, Y. lipolytica), amphibian (e.g., newt, frog), schistosome, insect (e.g., cricket), mollusc, mammal (e.g., mouse, human), or nematode, for example.

A hammerhead ribozyme herein typically comprises three base-paired helices, each respectively referred to as helix I, II and III, separated by short linkers of conserved sequences. The three types of hammerhead ribozymes (I-III) are generally based on which helix the 5' and 3' ends of the ribozyme are comprised in. For example, if the 5' and 3' ends of a hammerhead ribozyme sequence contribute to stem I, then it can be referred to as a type I hammerhead ribozyme. Of the three possible topological types, type I can be found in the genomes of prokaryotes, eukaryotes and RNA plant pathogens, whereas type II hammerhead ribozymes have only been described in prokaryotes, and type III hammerhead ribozymes are mostly found in plants, plant pathogens and prokaryotes. A hammerhead ribozyme in certain embodiments is a type I hammerhead ribozyme.

The sequence encoding a hammerhead ribozyme can comprise at least about 40, 50, 60, 70, 80, 90, 100, 110, 120, 130, 140, or 150 (or any integer between 40 and 150) nucleotides, 40-100 nucleotides, or 40-60 nucleotides.

In one embodiment of the disclosure, the method comprises a method of targeting an RNA-guided endonuclease (RGEN) to a target site sequence on a chromosome or episome in a non-conventional yeast, said method comprising providing to said yeast a first recombinant DNA construct comprising a DNA sequence encoding a Cas endonuclease, a protected polynucleotide modification template, and at least a second recombinant DNA construct comprising a DNA sequence encoding a ribozyme upstream of an RNA component, wherein the RNA transcribed from the second recombinant DNA construct autocatalytically removes the ribozyme to yield said RNA component , wherein the RNA component and the Cas9 endonuclease can form an RGEN that can bind to all or part of the target site sequence.

In certain embodiments, a DNA polynucleotide comprising a ribozyme-guide RNA cassette could comprise a suitable transcription termination sequence downstream of the guide RNA component sequence. Examples of transcription termination sequences useful herein are disclosed in U.S. Pat. Appl. Publ. No. 2014/0186906. For example, an *S*. *cerevisiae* Sup4 gene transcription terminator sequence can be used. Such embodiments typically do not comprise a ribozyme sequence located downstream from a ribozyme-RNA component cassette. Also, such embodiments typically comprise 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, or more residues following the end of the RNA component sequence, depending on the choice of terminator sequence. These additional residues can be all U residues, or at least 90%, 91%, 92%, 93%, 94%, 95%, 96%, 97%, 98%, or 99% U residues, for example, depending on the choice of terminator sequence. Alternatively, a ribozyme sequence (e.g., hammerhead or HDV ribozyme) can be 3' of (e.g., 1, 2, 3, 4, 5, 6, 7, 8, 9, 10 or more nucleotides) the RNA component sequence; the RNA component sequence in such embodiments is flanked by upstream and downstream ribozymes. A 3' ribozyme sequence can be positioned accordingly such that it cleaves itself from the RNA component sequence; such cleavage would render a transcript ending exactly at the end of the RNA component sequence, or with 1, 2, 3, 4, 5, 6, 7, 8, 9, 10, 11, 12, 13, 14, 15, or more residues following the end of the RNA component sequence, for example.

The terms "targeting", "gene targeting" and "DNA targeting" are used interchangeably herein. DNA targeting herein may be the specific introduction of a knock-out, edit, or knock-in at a particular DNA sequence, such as in a chromosome or plasmid of a cell. In general, DNA targeting can be performed herein by cleaving one or both strands at a specific DNA sequence in a cell with a Cas protein associated with a suitable polynucleotide component. Such DNA cleavage, if a double-strand break (DSB), can prompt NHEJ or HDR processes which can lead to modifications at the target site.

The terms "knock-out", "gene knock-out" and "genetic knock-out" are used interchangeably herein. A knock-out represents a DNA sequence of a cell that has been rendered partially or completely inoperative by targeting with a Cas protein; such a DNA sequence prior to knock-out could have encoded an amino acid sequence, or could have had a regulatory function (e.g., promoter), for example. A knock-out may be produced by an indel (insertion or deletion of nucleotide bases in a target DNA sequence through NHEJ), or by specific removal of sequence that reduces or completely destroys the function of sequence at or near the targeting site. An indel may be of 1, 2, 3, 4, 5, 6, 7, 8, 9, 10, or more bases, for example. An indel in certain embodiments can be even larger, at least about 20, 30, 40, 50, 60, 70, 80, 90, or 100 bases If an indel is introduced within an open reading frame (ORF) of a gene, oftentimes the indel disrupts wild type expression of protein encoded by the ORF by creating a frameshift mutation.

The guide polynucleotide/Cas endonuclease system can be used in combination with a co-delivered polynucleotide modification template to allow for editing (modification) of a genomic nucleotide sequence of interest. (See also U.S. Patent Application US 2015-0082478 A1, published on March 19, 2015 and WO2015/026886 A1, published on February 26, 2015.)

A "modified nucleotide" or "edited nucleotide" refers to a nucleotide sequence of interest that comprises at least one alteration when compared to its non-modified nucleotide sequence. Such "alterations" include, for example: (i) replacement of at least one nucleotide, (ii) a deletion of at least one nucleotide, (iii) an insertion of at least one nucleotide, or (iv) any combination of (i) - (iii).

The term "polynucleotide modification template" includes a polynucleotide that comprises at least one nucleotide modification when compared to the nucleotide sequence to be edited. A nucleotide modification can be at least one nucleotide substitution (replacement of at least one nucleotide), one nucleotide addition (insertion of at least one nucleotide), a deletion of at least one nucleotide, or any combination thereof. Optionally, the polynucleotide modification template can further comprise homologous nucleotide sequences flanking the at least one nucleotide modification, wherein the flanking homologous nucleotide sequences provide sufficient homology to the desired nucleotide sequence to be edited. A polynucleotide modification template that does not comprise a protection at its 5' or 3' end is referred to as an "unprotected polynucleotide modification template".

The terms "protected polynucleotide modification template" or " protected polynucleotide editing template" are used interchangeably herein and refer to a polynucleotide modification template molecule that has at least one modification (referred to as a protection or protection molecule) at at least its 5' end, or both its 5' and 3' end, and is a double stranded linear molecule comprising at least one phosphorothioate bond at the 5' end of at least one strand, or a single stranded linear molecule comprising at least one phosphorothioate bond at its 5' end. A protection at the 5' or 3' end includes any modification to the polynucleotide modification template that renders the template more stable (protected) as is evidence by increased HDR, decreased NHEJ, or decreased off-site integration, or any one combination thereof. The protection molecule (modification) can alter template stability by protecting the template from exonucleases within the cell and/or alter the ability of the template to act as a substrate for non-homologous end-joining (NHEJ). As an alternative, the protected polynucleotide modification templates may interact better with the proteins of homology directed repair or more poorly with the proteins of non-homologous end-joining when compared to the unprotected polynucleotide donor.

In some cells, the polynucleotide modification template can be incorporated (for example via NHEJ) into other spontaneous locations of DNA damage. The incorporation of a fragment of DNA via NHEJ has a final DNA ligation step where the 5' phosphate and 3' hydroxyl group of the DNA ends are being joined. In the case of protected polynucleotide modification templates, a suitable 5' phosphate group may not be available or may be blocked by modifications, thereby preventing off-site integration of the template.

The term "increased" as used herein may refer to a quantity or activity that is at least about 1%, 2%, 3%, 4%, 5%, 6%, 7%, 8%, 9%, 10%, 11%, 12%, 13%, 14%, 15%, 16%, 17%, 18%, 19%, 20%, 21% ,22% , 23%, 24%, 25%, 26%, 27%, 28%, 29%, 30%, 31%, 32%, 33%, 34%, 35%, 36%, 37%, 38%, 39%, 40%, 45%, 50%, 55%, 60%, 65%, 70%, 75%, 80%, 85%, 90%, 95%, 100%, 200%, or 250% more than the quantity or activity for which the increased quantity or activity is being compared. The terms "increased", "elevated", "enhanced", "greater than", and "improved" are used interchangeably herein. The term "increased" can be used to characterize the expression of a polynucleotide encoding a protein, for example, where "increased expression" can also mean "over-expression".

Non-limiting examples of protected polynucleotide modification templates are linear polynucleotide modification templates comprising at least one protection molecule consisting of at least 1, 2, 3, 4, or five 5' most phophodiester bonds replaced with phosphorothioate bonds on each strand.

In one embodiment, the disclosure describes a method for selecting a microbial cell comprising a modified nucleotide sequence in its genome, the method comprising:
a.) providing a guide polynucleotide, a protected polynucleotide modification template and a Cas endonuclease to a microbial cell, wherein said Cas endonuclease and guide polynucleotide can form a complex capable of introducing a single or double-strand break at a target site in genome of said cell, wherein said protected polynucleotide modification template comprises at least one nucleotide modification of said nucleotide sequence; and, b.) selecting a cell from step (a) comprising said modified nucleotide sequence, wherein the protected polynucleotide modification template is a double stranded linear molecule comprising at least one phosphorothioate bond at the 5' end of at least one strand, or a single stranded linear molecule comprising at least one phosphorothioate bond at its 5' end. The method can further comprise determining the frequency of Homologous Directed Repair (HDR) and Non-Homologous End Joining (NHEJ) in said cell.

Using the methods described herein, the frequency of HDR can be increased by at least about 1%, 2%, 3%, 4%, 5%, 6%, 7%, 8%, 9%, 10%, 11%, 12%, 13%, 14%, 15%, 16%, 17%, 18%, 19%, 20%, 21% ,22% , 23%, 24%, 25%, 26%, 27%, 28%, 29%, 30%, 31%, 32%, 33%, 34%, 35%, 36%, 37%, 38%, 39%, 40%, 45%, 50%, 55%, 60%, 65%, 70%, 75%, 80%, 85%, 90%, 95%, 100%, 200%, or 250% when compared to the frequency of HDR derived from a control method having all the same components and steps as the method described herein except for using an unprotected (control) polynucleotide modification template.

Using the methods described herein, the frequency of NHEJ can be decreased by at least about 1%, 2%, 3%, 4%, 5%, 6%, 7%, 8%, 9%, 10%, 11%, 12%, 13%, 14%, 15%, 16%, 17%, 18%, 19%, 20%, 21% ,22% , 23%, 24%, 25%, 26%, 27%, 28%, 29%, 30%, 31%, 32%, 33%, 34%, 35%, 36%, 37%, 38%, 39%, 40%, 45%, 50%, 55%, 60%, 65%, 70%, 75%, 80%, 85%, 90%, 95% when compared to the frequency of NHEJ derived from a control method having all the same components and steps as the method of described herein except for using an unprotected (control) polynucleotide modification template.

In one embodiment, the disclosure describes a method for editing a nucleotide sequence in the genome of a microbial cell, the method comprising providing a guide polynucleotide, a protected polynucleotide modification template, and at least one Cas endonuclease to a microbial cell, wherein the Cas endonuclease is capable of introducing a single or double-strand break at a target sequence in the genome of said cell, wherein said polynucleotide modification template includes at least one nucleotide modification of said nucleotide sequence and wherein the protected polynucleotide modification template is a double stranded linear molecule comprising at least one phosphorothioate bond at the 5' end of at least one strand, or a single stranded linear molecule comprising at least one phosphorothioate bond at its 5' end. The nucleotide to be edited can be located within or outside a target site recognized and cleaved by a Cas endonuclease. In one embodiment, the at least one nucleotide modification is not a modification at a target site recognized and cleaved by a Cas endonuclease. In another embodiment, there are at least 1, 2, 3, 4, 5, 6, 7, 8, 9, 10, 11, 12, 13, 14, 15, 16, 17, 18, 19, 20, 21, 22, 23, 24, 25, 26, 27, 30, 40, 50, 100, 200, 300, 400, 500, 600, 700, 900 or 1000 nucleotides between the at least one nucleotide to be edited and the genomic target site.

Cells for use in the methods of the present invention include, but are not limited to, bacterial, archaeal, fungal, microbial, and yeast, as well as microorganisms produced by the methods described herein. Examples of yeast herein include conventional yeast and non-conventional yeast. Conventional yeast in certain embodiments are yeast that favor homologous recombination (HR) DNA repair processes over repair processes mediated by non-homologous end-joining (NHEJ). Examples of conventional yeast herein include species of the genera Saccharomyces (e.g., *S*. *cerevisiae, which is also known as budding yeast, baker's yeast, and*/*or brewer's yeast; S. bayanus; S. boulardii; S. bulderi; S. cariocanus; S. cariocus; S. chevalieri; S*. *dairenensis; S. ellipsoideus; S. eubayanus; S. exiguus; S. florentinus; S. kluyveri; S. martiniae; S*. *monacensis; S. norbensis; S*. *paradoxus; S*. *pastorianus; S. spencerorum; S. turicensis; S. unisporus; S. uvarum; S*. *zonatus) and Schizosaccharomyces (e.g., S. pombe, which is also known as fission yeast; S*. *cryophilus; S. japonicus; S*. *octosporus*).

A non-conventional yeast herein is not a conventional yeast such as a *Saccharomyces* (e.g., *S*. *cerevisiae*) or *Schizosaccharomyces* (e.g., *S*. *pombe*) species. Non-conventional yeast in certain embodiments can be yeast that favor NHEJ DNA repair processes over repair processes mediated by HR. Conventional yeasts such as *S*. *cerevisiae* and *S*. *pombe* typically exhibit specific integration of donor DNA with short flanking homology arms (30-50 bp) with efficiencies routinely over 70%, whereas non-conventional yeasts such as *Pichia pastoris*, *Pichia stipitis, Hansenula polymorpha*, *Yarrowia lipolytica* and *Kluyveromyces lactis* usually show specific integration with similarly structured donor DNA at efficiencies of less than 1% (Chen et al., *PLoS ONE* 8:e57952). Thus, a preference for HR processes can be gauged, for example, by transforming yeast with a suitable donor DNA and determining the degree to which it is specifically recombined with a genomic site predicted to be targeted by the donor DNA. A preference for NHEJ (or low preference for HR), for example, would be manifest if such an assay yielded a high degree of random integration of the donor DNA in the yeast genome. Assays for determining the rate of specific (HR-mediated) and/or random (NHEJ-mediated) integration of DNA in yeast are known in the art (e.g., Ferreira and Cooper, Genes Dev. 18:2249-2254; Corrigan et al., PLoS ONE 8:e69628; Weaver et al., Proc. Natl. Acad. Sci. U.S.A. 78:6354-6358; Keeney and Boeke, Genetics 136:849-856).

Given their low level of HR activity, non-conventional yeast herein can (i) exhibit a rate of specific targeting by a suitable donor DNA having 30-50 bp flanking homology arms of less than about 1%, 2%, 3%, 4%, 5%,, 6%, 7%, or 8%, for example, and/or (ii) exhibit a rate of random integration of the foregoing donor DNA of more than about 65%, 66%, 67%, 68%, 69%, 70%, 71%, 72%, 73%, 74%, or 75%, for example. These rates of (i) specific targeting and/or (ii) random integration of a suitable donor DNA can characterize a non-conventional yeast as it exists before being provided an RGEN as disclosed herein. An aim for providing an RGEN to a non-conventional yeast in certain embodiments is to create site-specific DNA single-strand breaks (SSB) or double-strand breaks (DSB) for biasing the yeast toward HR at the specific site. Thus, providing a suitable RGEN in a non-conventional yeast typically should allow the yeast to exhibit an increased rate of HR with a particular donor DNA. Such an increased rate can be at least about 2-, 3-, 4-, 5-, 6-, 7-, 8-, 9-, or 10-fold higher than the rate of HR in a suitable control (e.g., same non-conventional yeast transformed with the same donor DNA, but lacking a suitable RGEN).

The methods described herein employ a guide polynucleotide, a protected polynucleotide modification template and a Cas endonuclease to modify a nucleotide sequence and/or to increase the frequency of homologous directed repair. The protected polynucleotide template can comprise two homology arms separated by at least one heterologous gene expression cassette. The methods can further be used to decrease the frequency if off-site integration of any modification template.

In one embodiment, the disclosure describes a method for selecting a microbial cell comprising a modified nucleotide sequence in its genome, the method comprising a) providing a guide polynucleotide, at least one protected polynucleotide modification template and a Cas endonuclease to a microbial cell, wherein said Cas endonuclease and guide polynucleotide can form a complex capable of introducing a single or double-strand break at a target site in genome of said cell, wherein said protected polynucleotide modification template comprises at least one nucleotide modification of said nucleotide sequence wherein the protected polynucleotide modification template is a double stranded linear molecule comprising at least one phosphorothioate bond at the 5' end of at least one strand, or a single stranded linear molecule comprising at least one phosphorothioate bond at its 5' end; b) selecting a cell from step (a) comprising said modified nucleotide sequence, and c) further determining the frequency of off-site integration of the protected polynucleotide modification template in said cell.

The frequency of off-site integration of the protected polynucleotide modification template in said cell can be decreased by 1%, 2%, 3%, 4%, 5%, 6%, 7%, 8%, 9%, 10%, 11%, 12%, 13%, 14%, 15%, 16%, 17%, 18%, 19%, 20%, 21% ,22% , 23%, 24%, 25%, 26%, 27%, 28%, 29%, 30%, 31%, 32%, 33%, 34%, 35%, 36%, 37%, 38%, 39%, 40%, 45%, 50%, 55%, 60%, 65%, 70%, 75%, 80%, 85%, 90%, 95%or 100% when compared to the frequency of off-site integration derived from a control method having all the same components as the methods described herein except for using an unprotected (control) polynucleotide modification template.

The terms "knock-in", "gene knock-in, "gene insertion" and "genetic knock-in" are used interchangeably herein. A knock-in represents the replacement or insertion of a DNA sequence at a specific DNA sequence in cell by targeting with a Cas protein (by HR, wherein a suitable donor DNA polynucleotide is also used). Examples of knock-ins are a specific insertion of a heterologous amino acid coding sequence in a coding region of a gene, or a specific insertion of a transcriptional regulatory element in a genetic locus.

Various methods and compositions can be employed to obtain a cell or organism having a polynucleotide of interest inserted in a target site for a Cas endonuclease. Such methods can employ homologous recombination to provide integration of the polynucleotide of Interest at the target site. In one method provided, a polynucleotide of interest is provided to the organism cell in a donor DNA construct. As used herein, "donor DNA" or "donor polynucleotide" is a DNA construct that comprises a polynucleotide of interest to be inserted into the target site of a Cas endonuclease. The donor DNA construct can further comprise a first and a second region of homology that flank the polynucleotide of Interest. The first and second regions of homology of the donor DNA share homology to a first and a second genomic region, respectively, present in or flanking the target site of the cell or organism genome. By "homology" is meant DNA sequences that are similar. For example, a "region of homology to a genomic region" that is found on the donor DNA is a region of DNA that has a similar sequence to a given "genomic region" in the cell or organism genome. A region of homology can be of any length that is sufficient to promote homologous recombination at the cleaved target site. For example, the region of homology can comprise at least 5-10, 5-15, 5-20, 5-25, 5-30, 5-35, 5-40, 5-45, 5- 50, 5-55, 5-60, 5-65, 5- 70, 5-75, 5-80, 5-85, 5-90, 5-95, 5-100, 5-200, 5-300, 5-400, 5-500, 5-600, 5-700, 5-800, 5-900, 5-1000, 5-1100, 5-1200, 5-1300, 5-1400, 5-1500, 5-1600, 5-1700, 5-1800, 5-1900, 5-2000, 5-2100, 5-2200, 5-2300, 5-2400, 5-2500, 5-2600, 5-2700, 5-2800, 5-2900, 5-3000, 5-3100 or more bases in length such that the region of homology has sufficient homology to undergo homologous recombination with the corresponding genomic region. "Sufficient homology" indicates that two polynucleotide sequences have sufficient structural similarity to act as substrates for a homologous recombination reaction. The structural similarity includes overall length of each polynucleotide fragment, as well as the sequence similarity of the polynucleotides. Sequence similarity can be described by the percent sequence identity over the whole length of the sequences, and/or by conserved regions comprising localized similarities such as contiguous nucleotides having 100% sequence identity, and percent sequence identity over a portion of the length of the sequences.

The amount of homology or sequence identity shared by a target and a donor polynucleotide can vary and includes total lengths and/or regions having unit integral values in the ranges of about 1-20 bp, 20-50 bp, 50-100 bp, 75-150 bp, 100-250 bp, 150-300 bp, 200-400 bp, 250-500 bp, 300-600 bp, 350-750 bp, 400-800 bp, 450-900 bp, 500-1000 bp, 600-1250 bp, 700-1500 bp, 800-1750 bp, 900-2000 bp, 1-2.5 kb, 1.5-3 kb, 2-4 kb, 2.5-5 kb, 3-6 kb, 3.5-7 kb, 4-8 kb, 5-10 kb, or up to and including the total length of the target site. These ranges include every integer within the range, for example, the range of 1-20 bp includes 1, 2, 3, 4, 5, 6, 7, 8, 9, 10, 11, 12, 13, 14, 15, 16, 17, 18, 19 and 20 bps. The amount of homology can also described by percent sequence identity over the full aligned length of the two polynucleotides which includes percent sequence identity of about at least 50%, 55%, 60%, 65%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100%. Sufficient homology includes any combination of polynucleotide length, global percent sequence identity, and optionally conserved regions of contiguous nucleotides or local percent sequence identity, for example sufficient homology can be described as a region of 75-150 bp having at least 80% sequence identity to a region of the target locus. Sufficient homology can also be described by the predicted ability of two polynucleotides to specifically hybridize under high stringency conditions, see, for example, Sambrook et al., (1989) Molecular Cloning: A Laboratory Manual, (Cold Spring Harbor Laboratory Press, NY); Current Protocols in Molecular Biology, Ausubel et al., Eds (1994) Current Protocols, (Greene Publishing Associates, Inc. and John Wiley & Sons, Inc.); and, Tijssen (1993) Laboratory Techniques in Biochemistry and Molecular Biology--Hybridization with Nucleic Acid Probes, (Elsevier, New York).

A donor DNA polynucleotide can have two homologous sequences separated by a sequence that is heterologous to sequence at a target site. These two homologous sequences of such a donor polynucleotide can be referred to as "homology arms", which flank the heterologous sequence. HR between a target site and a donor polynucleotide with two homology arms typically results in the replacement of a sequence at the target site with the heterologous sequence of the donor polynucleotide (target site sequence located between DNA sequences homologous to the homology arms of the donor polynucleotide is replaced by the heterologous sequence of the donor polynucleotide). In a donor polynucleotide with two homology arms, the arms can be separated by 1 or more nucleotides (i.e., the heterologous sequence in the donor polynucleotide can be at least 1 nucleotide in length). Various HR procedures that can be performed in a non-conventional yeast herein are disclosed, for example, in DNA Recombination: Methods and Protocols: 1st Edition (H. Tsubouchi, Ed., Springer-Verlag, New York, 2011).

As used herein, a "genomic region" is a segment of a chromosome in the genome of a cell that is present on either side of the target site or, alternatively, also comprises a portion of the target site. The genomic region can comprise at least 5-10, 5-15, 5-20, 5-25, 5-30, 5-35, 5-40, 5-45, 5- 50, 5-55, 5-60, 5-65, 5- 70, 5-75, 5-80, 5-85, 5-90, 5-95, 5-100, 5-200, 5-300, 5-400, 5-500, 5-600, 5-700, 5-800, 5-900, 5-1000, 5-1100, 5-1200, 5-1300, 5-1400, 5-1500, 5-1600, 5-1700, 5-1800, 5-1900, 5-2000, 5-2100, 5-2200, 5-2300, 5-2400, 5-2500, 5-2600, 5-2700, 5-2800. 5-2900, 5-3000, 5-3100 or more bases such that the genomic region has sufficient homology to undergo homologous recombination with the corresponding region of homology.

Polynucleotides of interest and/or traits can be stacked together in a complex trait locus as described in US-2013-0263324-A1, published 03 Oct 2013 and in PCT/US13/22891, published January 24, 2013. The guide polynucleotide/Cas9 endonuclease system described herein provides for an efficient system to generate double strand breaks and allows for traits to be stacked in a complex trait locus.

The guide polynucleotide/Cas endonuclease system can be used for introducing one or more polynucleotides of interest or one or more traits of interest into one or more target sites by providing one or more guide polynucleotides, one or more Cas endonucleases, and optionally one or more donor DNAs to a cell. ((as described in US patent application No. 14/463,687, filed August 20, 2014.

The structural similarity between a given genomic region and the corresponding region of homology found on the donor DNA can be any degree of sequence identity that allows for homologous recombination to occur. For example, the amount of homology or sequence identity shared by the "region of homology" of the donor DNA and the "genomic region" of the organism genome can be at least 50%, 55%, 60%, 65%, 70%, 75%, 80%, 81%, 82%, 83%, 84%, 85%, 86%, 87%, 88%, 89%, 90%, 91%, 92%, 93%, 94%, 95%, 96%, 97%, 98%, 99% or 100% sequence identity, such that the sequences undergo homologous recombination

The region of homology on the donor DNA can have homology to any sequence flanking the target site. While in some embodiments the regions of homology share significant sequence homology to the genomic sequence immediately flanking the target site, it is recognized that the regions of homology can be designed to have sufficient homology to regions that may be further 5' or 3' to the target site. In still other embodiments, the regions of homology can also have homology with a fragment of the target site along with downstream genomic regions. In one embodiment, the first region of homology further comprises a first fragment of the target site and the second region of homology comprises a second fragment of the target site, wherein the first and second fragments are dissimilar.

As used herein, "homologous recombination" includes the exchange of DNA fragments between two DNA molecules at the sites of homology. The frequency of homologous recombination is influenced by a number of factors. Different organisms vary with respect to the amount of homologous recombination and the relative proportion of homologous to non-homologous recombination. Generally, the length of the region of homology affects the frequency of homologous recombination events: the longer the region of homology, the greater the frequency. The length of the homology region needed to observe homologous recombination is also species-variable. In many cases, at least 5 kb of homology has been utilized, but homologous recombination has been observed with as little as 25-50 bp of homology. See, for example, Singer et al., (1982) Cell 31:25-33; Shen and Huang, (1986) Genetics 112:441-57; Watt et al., (1985) Proc. Natl. Acad. Sci. USA 82:4768-72, Sugawara and Haber, (1992) Mol Cell Biol 12:563-75, Rubnitz and Subramani, (1984) Mol Cell Biol 4:2253-8; Ayares et al., (1986) Proc. Natl. Acad. Sci. USA 83:5199-203; Liskay et al., (1987) Genetics 115:161-7.

Homology-directed repair (HDR) is a mechanism in cells to repair double-stranded and single stranded DNA breaks. Homology-directed repair includes homologous recombination (HR) and single-strand annealing (SSA) (Lieber. 2010 Annu. Rev. Biochem . 79:181-211). The most common form of HDR is called homologous recombination (HR), which has the longest sequence homology requirements between the donor and acceptor DNA. Other forms of HDR include single-stranded annealing (SSA) and breakage-induced replication, and these require shorter sequence homology relative to HR. Homology-directed repair at nicks (single-stranded breaks) can occur via a mechanism distinct from HDR at double-strand breaks (Davis and Maizels. PNAS (0027-8424), 111 (10), p. E924-E932.

Alteration of the genome of a cell, for example, through homologous recombination (HR), is a powerful tool for genetic engineering. The parameters for homologous recombination in plants have primarily been investigated by rescuing introduced truncated selectable marker genes. In these experiments, the homologous DNA fragments were typically between 0.3 kb to 2 kb. Observed frequencies for homologous recombination were on the order of 10⁻⁴ to 10⁻⁵. See, for example, Halfter et al., (1992) Mol Gen Genet 231:186-93; Offringa et al., (1990) EMBO J 9:3077-84; Offringa et al., (1993) Proc. Natl. Acad. Sci. USA 90:7346-50; Paszkowski et al., (1988) EMBO J 7:4021-6; Hourda and Paszkowski, (1994) Mol Gen Genet 243:106-11; and Risseeuw et al., (1995) Plant J 7:109-19.

Homologous recombination has been demonstrated in insects. In Drosophila, Dray and Gloor found that as little as 3 kb of total template:target homology sufficed to copy a large non-homologous segment of DNA into the target with reasonable efficiency (Dray and Gloor, (1997) Genetics 147:689-99). Using FLP-mediated DNA integration at a target FRT in Drosophila, Golic et al., showed integration was approximately 10-fold more efficient when the donor and target shared 4.1 kb of homology as compared to 1.1 kb of homology (Golic et al., (1997) Nucleic Acids Res 25:3665). Data from Drosophila indicates that 2-4 kb of homology is sufficient for efficient targeting, but there is some evidence that much less homology may suffice, on the order of about 30 bp to about 100 bp (Nassif and Engels, (1993) Proc. Natl. Acad. Sci. USA 90:1262-6; Keeler and Gloor, (1997) Mol Cell Biol 17:627-34).

Homologous recombination has also been accomplished in other organisms. For example, at least 150-200 bp of homology was required for homologous recombination in the parasitic protozoan Leishmania (Papadopoulou and Dumas, (1997) Nucleic Acids Res 25:4278-86). In the filamentous fungus Aspergillus nidulans, gene replacement has been accomplished with as little as 50 bp flanking homology (Chaveroche et al., (2000) Nucleic Acids Res 28:e97). Targeted gene replacement has also been demonstrated in the ciliate Tetrahymena thermophila (Gaertig et al., (1994) Nucleic Acids Res 22:5391-8). In mammals, homologous recombination has been most successful in the mouse using pluripotent embryonic stem cell lines (ES) that can be grown in culture, transformed, selected and introduced into a mouse embryo. Embryos bearing inserted transgenic ES cells develop as genetically offspring. By interbreeding siblings, homozygous mice carrying the selected genes can be obtained. An overview of the process is provided in Watson et al., (1992) Recombinant DNA, 2nd Ed., (Scientific American Books distributed by WH Freeman & Co.); Capecchi, (1989) Trends Genet 5:70-6; and Bronson, (1994) J Biol Chem 269:27155-8. Homologous recombination in mammals other than mouse has been limited by the lack of stem cells capable of being transplanted to oocytes or developing embryos. However, McCreath et al., Nature 405:1066-9 (2000) reported successful homologous recombination in sheep by transformation and selection in primary embryo fibroblast cells.

Error-prone DNA repair mechanisms can produce mutations at double-strand break sites. The Non-Homologous-End-Joining (NHEJ) pathways are the most common repair mechanism to bring the broken ends together (Bleuyard et al., (2006) DNA Repair 5:1-12). The structural integrity of chromosomes is typically preserved by the repair, but deletions, insertions, or other rearrangements are possible. The two ends of one double-strand break are the most prevalent substrates of NHEJ (Kirik et al., (2000) EMBO J 19:5562-6), however if two different double-strand breaks occur, the free ends from different breaks can be ligated and result in chromosomal deletions (Siebert and Puchta, (2002) Plant Cell 14:1121-31), or chromosomal translocations between different chromosomes (Pacher et al., (2007) Genetics 175:21-9).

Episomal DNA molecules can also be ligated into the double-strand break, for example, integration of T-DNAs into chromosomal double-strand breaks (Chilton and Que, (2003) Plant Physiol 133:956-65; Salomon and Puchta, (1998) EMBO J 17:6086-95). Once the sequence around the double-strand breaks is altered, for example, by exonuclease activities involved in the maturation of double-strand breaks, gene conversion pathways can restore the original structure if a homologous sequence is available, such as a homologous chromosome in non-dividing somatic cells, or a sister chromatid after DNA replication (Molinier et al., (2004) Plant Cell 16:342-52). Ectopic and/or epigenic DNA sequences may also serve as a DNA repair template for homologous recombination (Puchta, (1999) Genetics 152:1173-81).

Once a double-strand break is induced in the DNA, the cell's DNA repair mechanism is activated to repair the break. Error-prone DNA repair mechanisms can produce mutations at double-strand break sites. The most common repair mechanism to bring the broken ends together is the nonhomologous end-joining (NHEJ) pathway (Bleuyard et al., (2006) DNA Repair 5:1-12). The structural integrity of chromosomes is typically preserved by the repair, but deletions, insertions, or other rearrangements are possible (Siebert and Puchta, (2002) Plant Cell 14:1121-31; Pacher et al., (2007) Genetics 175:21-9).

Alternatively, the double-strand break can be repaired by homologous recombination between homologous DNA sequences. Once the sequence around the double-strand break is altered, for example, by exonuclease activities involved in the maturation of double-strand breaks, gene conversion pathways can restore the original structure if a homologous sequence is available, such as a homologous chromosome in non-dividing somatic cells, or a sister chromatid after DNA replication (Molinier et al., (2004) Plant Cell 16:342-52). Ectopic and/or epigenic DNA sequences may also serve as a DNA repair template for homologous recombination (Puchta, (1999) Genetics 152:1173-81).

DNA double-strand breaks appear to be an effective factor to stimulate homologous recombination pathways (Puchta et al., (1995) Plant Mol Biol 28:281-92; Tzfira and White, (2005) Trends Biotechnol 23:567-9; Puchta, (2005) J Exp Bot 56:1 - 14). Using DNA-breaking agents, a two- to nine-fold increase of homologous recombination was observed between artificially constructed homologous DNA repeats in plants (Puchta et al., (1995) Plant Mol Biol 28:281-92). In maize protoplasts, experiments with linear DNA molecules demonstrated enhanced homologous recombination between plasmids (Lyznik et al., (1991) Mol Gen Genet 230:209-18).

The donor DNA may be introduced by any means known in the art. The donor DNA may be provided by any transformation method known in the art including, for example, Agrobacterium-mediated transformation or biolistic particle bombardment. The donor DNA may be present transiently in the cell or it could be introduced via a viral replicon.

Further uses for guide RNA/Cas endonuclease systems have been described (See U.S. Patent Application US 2015-0082478 A1, published on March 19, 2015, WO2015/026886 A1, published on February 26, 2015, US 2015-0059010 A1, published on February 26, 2015, US application 62/023246, filed on July 07, 2014, and US application 62/036,652, filed on August 13, 2014) and include but are not limited to modifying or replacing nucleotide sequences of interest (such as a regulatory elements), insertion of polynucleotides of interest, gene knock-out, gene-knock in, modification of splicing sites and/or introducing alternate splicing sites, modifications of nucleotide sequences encoding a protein of interest, amino acid and/or protein fusions, and gene silencing by expressing an inverted repeat into a gene of interest.

In one embodiment of the disclosure, the method comprises a method for selecting a microbial cell comprising a modified nucleotide sequence in its genome, the method comprising: a.) providing a guide polynucleotide, a protected polynucleotide modification template and a Cas endonuclease to a microbial cell, wherein said Cas endonuclease and guide polynucleotide can form a complex capable of introducing a single or double-strand break at a target site in genome of said cell, wherein said protected polynucleotide modification template comprises at least one nucleotide modification of said nucleotide sequence; and, b.) selecting a cell from step (a) comprising said modified nucleotide sequence wherein the protected polynucleotide modification template is a double stranded linear molecule comprising at least one phosphorothioate bond at the 5' end of at least one strand, or a single stranded linear molecule comprising at least one phosphorothioate bond at its 5' end. The protected polynucleotide modification template can be a linear polynucleotide comprising at least one protection molecule at its 5' end, or both 5' and 3' endsThe at least one nucleotide modification of the protected polynucleotide template can be selected from the group consisting of (i) a replacement of at least one nucleotide, (ii) a deletion of at least one nucleotide, (iii) an insertion of at least one nucleotide, and (iv) any combination of (i) - (iii).

The methods described herein employ a guide polynucleotide, a protected polynucleotide modification template and a Cas endonuclease and can be used for genome engineering (such as introducing polynucleotides of interest, editing genes or modifying gened that are part of a metabolic pathway.

Methods described herein can be used for metabolic pathway engineering (metabolic engineering) and/or to produce recombinant microbial cells that have been genetically engineered. The recombinant microbial cell in certain embodiments may be one that has been genetically engineered by deleting genes for metabolic engineering using the methods described herein. The recombinant microbial cell in certain embodiments may be one that has been genetically engineered to produce an increased amount of total lipids and/or fatty acids such as PUFAs. For example, a fatty acid or PUFA biosynthetic pathway, or portion thereof, may be introduced to the organism by inserting coding sequences for certain pathway enzymes, such as fatty acid desaturases and elongases. One or a combination of the following enzymes may be genetically introduced to the oleaginous yeast cell to provide a PUFA biosynthetic pathway therein: delta-4 desaturase, delta-5 desaturase, delta-6 desaturase, delta-12 desaturase, delta-15 desaturase, delta-17 desaturase, delta-9 desaturase, delta-8 desaturase, delta-9 elongase, C14/16 elongase, C16/18 elongase, C18/20 elongase, C20/22 elongase. One or more of these enzymes may be from a heterologous source. Example PUFA biosynthetic pathways may contain both a delta-9 elongase and delta-8 desaturase (e.g., refer to U.S. Pat. Appl. Publ. No. 2011-0055973), or both a delta-6 desaturase and delta-6 elongase. Alternatively, the recombinant microbial cell may be modified to have increased total lipids and/or PUFA levels by introducing or deleting genes, other than those encoding desaturases or elongases, that regulate fatty acid biosynthesis.

The recombinant microbial cell used in certain embodiments may be a Yarrowia cell that produces an oil comprising at least 28 percent EPA measured as a weight percent of the dry cell weight and that comprise a down-regulation of an endogenous polynucleotide sequence encoding Sou2 sorbitol utilization protein, and at least one polynucleotide sequence encoding an active LPCAT enzyme comprising at least one amino acid mutation in a membrane-bound O-acyltransferase motif. (PCT/US2013/07895, filed December 18, 2013).

The recombinant microbial cell used can be a cell of a yeast, mold, fungus, oomycete, bacteria, algae, stramenopile, or protist (e.g., euglenoid). In certain embodiments, the recombinant microbial cell is an oleaginous microbial cell, such as an oleaginous yeast cell. Examples of oleaginous yeast include species of the genera Yarrowia, Candida, Rhodotorula, Rhodosporidium, Cryptococcus, Trichosporon and Lipomyces. More specific examples of oleaginous yeast include Rhodosporidium toruloides, Lipomyces starkeyii, L. lipoferus, Candida revkaufi, C. pulcherrima, C. tropicalis, C. utilis, Trichosporon pullans, T. cutaneum, Rhodotorula glutinus and R. graminis, for example. Examples of fungal cells in certain embodiments include species of the genera Fusarium (e.g., Fusarium lateritium), Mortierella (e.g., Mortierella alpina) and Mucor (e.g., Mucor rouxii and Mucor circinelloides). The microbial cell can be of the genera Entomophthora, Pythium and Porphyridium in other embodiments of the disclosed invention.

Polynucleotides of interest are further described herein and include polynucleotides reflective of the commercial markets and interests of those involved in the development of the crop. Crops and markets of interest change, and as developing nations open up world markets, new crops and technologies will emerge also. In addition, as our understanding of agronomic traits and characteristics such as yield and heterosis increase, the choice of genes for genetic engineering will change accordingly.

Methods for identifying at least one cell, comprising in its genome, a polynucleotide of interest integrated at the target site can also be used. A variety of methods are available for identifying those cells with insertion into the genome at or near to the target site without using a screenable marker phenotype. Such methods can be viewed as directly analyzing a target sequence to detect any change in the target sequence, including but not limited to PCR methods, sequencing methods, nuclease digestion, Southern blots, and any combination thereof. See, for example, US Patent Application 12/147,834to the extent necessary for the methods described herein.

Polynucleotides/polypeptides of interest include, but are not limited to, microbial metabolic pathway genes, herbicide-resistance coding sequences, insecticidal coding sequences, nematicidal coding sequences, antimicrobial coding sequences, antifungal coding sequences, antiviral coding sequences, abiotic and biotic stress tolerance coding sequences, or sequences modifying plant traits such as yield, grain quality, nutrient content, starch quality and quantity, nitrogen fixation and/or utilization, fatty acids, and oil content and/or composition. General categories of genes of interest include, for example, those genes involved in information, such as zinc fingers, those involved in communication, such as kinases, and those involved in housekeeping, such as heat shock proteins. More specific categories of transgenes, for example, include genes encoding important traits for agronomics, insect resistance, disease resistance, herbicide resistance, fertility or sterility, grain characteristics, and commercial products. Genes of interest include, generally, those involved in oil, starch, carbohydrate, or nutrient metabolism as well as those affecting kernel size, sucrose loading, and the like that can be stacked or used in combination with other traits, such as but not limited to herbicide resistance, described herein.

Agronomically important traits such as oil, starch, and protein content can be genetically altered in addition to using traditional breeding methods. Modifications include increasing content of oleic acid, saturated and unsaturated oils, increasing levels of lysine and sulfur, providing essential amino acids, and also modification of starch. Hordothionin protein modifications are described in U.S. Patent Nos. 5,703,049, 5,885,801, 5,885,802, and 5,990,389.

Furthermore, it is recognized that the polynucleotide of interest may also comprise antisense sequences complementary to at least a portion of the messenger RNA (mRNA) for a targeted gene sequence of interest. Antisense nucleotides are constructed to hybridize with the corresponding mRNA. Modifications of the antisense sequences may be made as long as the sequences hybridize to and interfere with expression of the corresponding mRNA. In this manner, antisense constructions having 70%, 80%, or 85% sequence identity to the corresponding antisense sequences may be used. Furthermore, portions of the antisense nucleotides may be used to disrupt the expression of the target gene. Generally, sequences of at least 50 nucleotides, 100 nucleotides, 200 nucleotides, or greater may be used.

In addition, the polynucleotide of interest may also be used in the sense orientation to suppress the expression of endogenous genes in an organism of interest. Methods for suppressing gene expression in microorganisms and plants using polynucleotides in the sense orientation are known in the art. See, U.S. Patent Nos. 5,283,184 and 5,034,323.

The polynucleotide of interest can also be a phenotypic marker. A phenotypic marker is screenable or a selectable marker that includes visual markers and selectable markers whether it is a positive or negative selectable marker. Any phenotypic marker can be used. Specifically, a selectable or screenable marker comprises a DNA segment that allows one to identify, or select for or against a molecule or a cell that contains it, often under particular conditions. These markers can encode an activity, such as, but not limited to, production of RNA, peptide, or protein, or can provide a binding site for RNA, peptides, proteins, inorganic and organic compounds or compositions and the like.

Examples of selectable markers include, but are not limited to, DNA segments that comprise restriction enzyme sites; DNA segments that encode products which provide resistance against otherwise toxic compounds including antibiotics, such as, spectinomycin, ampicillin, kanamycin, tetracycline, Basta, neomycin phosphotransferase II (NEO) and hygromycin phosphotransferase (HPT)); DNA segments that encode products which are otherwise lacking in the recipient cell (e.g., tRNA genes, auxotrophic markers); DNA segments that encode products which can be readily identified (e.g., phenotypic markers such as β-galactosidase, GUS; fluorescent proteins such as green fluorescent protein (GFP), cyan (CFP), yellow (YFP), red (RFP), and cell surface proteins); the generation of new primer sites for PCR (e.g., the juxtaposition of two DNA sequence not previously juxtaposed), the inclusion of DNA sequences not acted upon or acted upon by a restriction endonuclease or other DNA modifying enzyme, chemical, etc.; and, the inclusion of a DNA sequences required for a specific modification (e.g., methylation) that allows its identification.

Additional selectable markers include genes that confer resistance to herbicidal compounds, such as glufosinate ammonium, bromoxynil, imidazolinones, and 2,4-dichlorophenoxyacetate (2,4-D). See for example, Yarranton, (1992) Curr Opin Biotech 3:506-11; Christopherson et al., (1992) Proc. Natl. Acad. Sci. USA 89:6314-8; Yao et al., (1992) Cell 71:63-72; Reznikoff, (1992) Mol Microbiol 6:2419-22; Hu et al., (1987) Cell 48:555-66; Brown et al., (1987) Cell 49:603-12; Figge et al., (1988) Cell 52:713-22; Deuschle et al., (1989) Proc. Natl. Acad. Sci. USA 86:5400-4; Fuerst et al., (1989) Proc. Natl. Acad. Sci. USA 86:2549-53; Deuschle et al., (1990) Science 248:480-3; Gossen, (1993) Ph.D. Thesis, University of Heidelberg; Reines et al., (1993) Proc. Natl. Acad. Sci. USA 90:1917-21; Labow et al., (1990) Mol Cell Biol 10:3343-56; Zambretti et al., (1992) Proc. Natl. Acad. Sci. USA 89:3952-6; Baim et al., (1991) Proc. Natl. Acad. Sci. USA 88:5072-6; Wyborski et al., (1991) Nucleic Acids Res 19:4647-53; Hillen and Wissman, (1989) Topics Mol Struc Biol 10:143-62; Degenkolb et al., (1991) Antimicrob Agents Chemother 35:1591-5; Kleinschnidt et al., (1988) Biochemistry 27:1094-104; Bonin, (1993) Ph.D. Thesis, University of Heidelberg; Gossen et al., (1992) Proc. Natl. Acad. Sci. USA 89:5547-51; Oliva et al., (1992) Antimicrob Agents Chemother 36:913-9; Hlavka et al., (1985) Handbook of Experimental Pharmacology, Vol. 78 (Springer-Verlag, Berlin); Gill et al., (1988) Nature 334:721-4. Commercial traits can also be encoded on a gene or genes that could increase for example, starch for ethanol production, or provide expression of proteins. Another important commercial use of transformed micororganisms or plants is the production of polymers and bioplastics such as described in U.S. Patent No. 5,602,321. Genes such as β-Ketothiolase, PHBase (polyhydroxyburyrate synthase), and acetoacetyl-CoA reductase (see Schubert et al. (1988) J. Bacteriol. 170:5837-5847) facilitate expression of polyhyroxyalkanoates (PHAs).

Selection methods for use herein are resistance to kanamycin, hygromycin and the amino glycoside G418, as well as ability to grow on media lacking uracil, leucine, lysine, tryptophan or histidine. In alternate embodiments, 5-fluoroorotic acid (5-fluorouracil-6-carboxylic acid monohydrate [5-FOA]) is used for selection of yeast Ura- mutants (U.S. Pat. Appl. Publ. No. 2009-0093543), or a native acetohydroxyacid synthase (or acetolactate synthase; E.C. 4.1.3.18) that confers sulfonyl urea herbicide resistance (Intl. Appl. Publ. No. WO 2006/052870) is utilized for selection of transformants. A unique method of "recycling" a pair of preferred selection markers for their use in multiple sequential transformations, by use of site-specific recombinase systems, is also taught in U.S. Pat. Appl. Publ. No. 2009-0093543.

It may be desirable to manipulate a number of different genetic elements in the disclosed embodiments that control aspects of transcription, RNA stability, translation, protein stability and protein location, oxygen limitation and secretion from the host cell. More specifically, gene expression may be controlled by altering the following: the nature of the relevant promoter and terminator sequences; the number of copies of the cloned gene; whether the gene is plasmid-borne or integrated into the genome of the host cell; the final cellular location of the synthesized foreign protein; the efficiency of translation in the host organism; the intrinsic stability of the cloned gene protein within the host cell; and the codon usage within the cloned gene, such that its frequency approaches the frequency of preferred codon usage of the host cell.

Promoters useful to drive expression of heterologous genes in microbial host cells are numerous and known to those skilled in the art. Expression can be accomplished in an induced or constitutive fashion. Induced expression can be accomplished by inducing the activity of a regulatable promoter operably linked to the gene of interest, while constitutive expression can be achieved by the use of a constitutive promoter operably linked to the gene of interest. Virtually any promoter (i.e., native, synthetic, or chimeric) capable of directing expression of a gene is suitable, although transcriptional and translational regulatory regions from the host species may be particularly useful.

In general, the terminator can be derived from the 3' region of the gene from which the promoter was obtained or from a different gene. A large number of terminators are known and function satisfactorily in a variety of hosts, when utilized both in the same and different genera and species from which they were derived. The terminator usually is selected more as a matter of convenience rather than because of any particular property. Preferably, the terminator is derived from a yeast gene. The terminator can also be synthetic, as one of skill in the art can utilize available information to design and synthesize a terminator. A terminator may be unnecessary, but it is preferred.

Although not intended to be limiting, preferred promoters and terminators for use in a recombinant microbial host cell of the genus *Yarrowia* are those taught in U.S. Pat. Appl. Publ. Nos. 2009-0093543, 2010-0068789, 2011-0059496, 2012-0252079, 2012-0252093, 2013-0089910 and 2013-0089911.

The transgenes, recombinant DNA molecules, DNA sequences of interest, and polynucleotides of interest can be comprise one or more DNA sequences for gene silencing. Methods for gene silencing involving the expression of DNA sequences in cells and organisms are known in the art include, but are not limited to, cosuppression, antisense suppression, double-stranded RNA (dsRNA) interference, hairpin RNA (hpRNA) interference, intron-containing hairpin RNA (ihpRNA) interference, transcriptional gene silencing, and micro RNA (miRNA) interference

As used herein, "nucleic acid" means a polynucleotide and includes a single or a double-stranded polymer of deoxyribonucleotide or ribonucleotide bases. Nucleic acids may also include fragments and modified nucleotides.

The terms "polynucleotide", "nucleic acid sequence", "nucleotide sequence" and "nucleic acid fragment" are used interchangeably to denote a polymer of RNA and/or DNA that is single- or double-stranded, optionally containing synthetic, non-natural, or altered nucleotide bases. Nucleotides (usually found in their 5'-monophosphate form) are referred to by their single letter designation as follows: "A" for adenosine or deoxyadenosine (for RNA or DNA, respectively), "C" for cytosine or deoxycytosine, "G" for guanosine or deoxyguanosine, "U" for uridine, "T" for deoxythymidine, "R" for purines (A or G), "Y" for pyrimidines (C or T), "K" for G or T, "H" for A or C or T, "I" for inosine, and "N" for any nucleotide (e.g., N can be A, C, T, or G, if referring to a DNA sequence; N can be A, C, U, or G, if referring to an RNA sequence). Any RNA sequence (e.g., crRNA, tracrRNA, gRNA) disclosed herein may be encoded by a suitable DNA sequence.

### "Open reading frame" is abbreviated ORF.

The terms "subfragment that is functionally equivalent" and "functionally equivalent subfragment" are used interchangeably herein. These terms refer to a portion or subsequence of an isolated nucleic acid fragment in which the ability to alter gene expression or produce a certain phenotype is retained whether or not the fragment or subfragment encodes an active enzyme. For example, the fragment or subfragment can be used in the design of genes to produce the desired phenotype in a microbe or plant. Genes can be designed for use in suppression by linking a nucleic acid fragment or subfragment thereof, whether or not it encodes an active enzyme, in the sense or antisense orientation relative to a promoter sequence.

The term "conserved domain" or "motif" means a set of amino acids conserved at specific positions along an aligned sequence of evolutionarily related proteins. While amino acids at other positions can vary between homologous proteins, amino acids that are highly conserved at specific positions indicate amino acids that are essential to the structure, the stability, or the activity of a protein. Because they are identified by their high degree of conservation in aligned sequences of a family of protein homologues, they can be used as identifiers, or "signatures", to determine if a protein with a newly determined sequence belongs to a previously identified protein family.

Polynucleotide and polypeptide sequences, variants thereof, and the structural relationships of these sequences can be described by the terms "homology", "homologous", "substantially identical", "substantially similar" and "corresponding substantially" which are used interchangeably herein. These refer to polypeptide or nucleic acid fragments wherein changes in one or more amino acids or nucleotide bases do not affect the function of the molecule, such as the ability to mediate gene expression or to produce a certain phenotype. These terms also refer to modification(s) of nucleic acid fragments that do not substantially alter the functional properties of the resulting nucleic acid fragment relative to the initial, unmodified fragment. These modifications include deletion, substitution, and/or insertion of one or more nucleotides in the nucleic acid fragment.

Substantially similar nucleic acid sequences encompassed may be defined by their ability to hybridize (under moderately stringent conditions, e.g., 0.5X SSC, 0.1% SDS, 60°C) with the sequences exemplified herein, or to any portion of the nucleotide sequences disclosed herein and which are functionally equivalent to any of the nucleic acid sequences disclosed herein. Stringency conditions can be adjusted to screen for moderately similar fragments, such as homologous sequences from distantly related organisms, to highly similar fragments, such as genes that duplicate functional enzymes from closely related organisms. Post-hybridization washes determine stringency conditions.

The term "selectively hybridizes" includes reference to hybridization, under stringent hybridization conditions, of a nucleic acid sequence to a specified nucleic acid target sequence to a detectably greater degree (e.g., at least 2-fold over background) than its hybridization to non-target nucleic acid sequences and to the substantial exclusion of non-target nucleic acids. Selectively hybridizing sequences typically have about at least 80% sequence identity, or 90% sequence identity, up to and including 100% sequence identity (i.e., fully complementary) with each other.

The term "stringent conditions" or "stringent hybridization conditions" includes reference to conditions under which a probe will selectively hybridize to its target sequence in an *in vitro* hybridization assay. Stringent conditions are sequence-dependent and will be different in different circumstances. By controlling the stringency of the hybridization and/or washing conditions, target sequences can be identified which are 100% complementary to the probe (homologous probing). Alternatively, stringency conditions can be adjusted to allow some mismatching in sequences so that lower degrees of similarity are detected (heterologous probing). Generally, a probe is less than about 1000 nucleotides in length, optionally less than 500 nucleotides in length.

Typically, stringent conditions will be those in which the salt concentration is less than about 1.5 M Na ion, typically about 0.01 to 1.0 M Na ion concentration (or other salt(s)) at pH 7.0 to 8.3, and at least about 30°C for short probes (e.g., 10 to 50 nucleotides) and at least about 60°C for long probes (e.g., greater than 50 nucleotides). Stringent conditions may also be achieved with the addition of destabilizing agents such as formamide. Exemplary low stringency conditions include hybridization with a buffer solution of 30 to 35% formamide, 1 M NaCl, 1% SDS (sodium dodecyl sulphate) at 37°C, and a wash in 1X to 2X SSC (20X SSC = 3.0 M NaCl/0.3 M trisodium citrate) at 50 to 55°C. Exemplary moderate stringency conditions include hybridization in 40 to 45% formamide, 1 M NaCl, 1% SDS at 37°C, and a wash in 0.5X to 1X SSC at 55 to 60°C. Exemplary high stringency conditions include hybridization in 50% formamide, 1 M NaCl, 1% SDS at 37°C, and a wash in 0.1X SSC at 60 to 65°C.

"Sequence identity" or "identity" in the context of nucleic acid or polypeptide sequences refers to the nucleic acid bases or amino acid residues in two sequences that are the same when aligned for maximum correspondence over a specified comparison window.

The term "percentage of sequence identity" refers to the value determined by comparing two optimally aligned sequences over a comparison window, wherein the portion of the polynucleotide or polypeptide sequence in the comparison window may comprise additions or deletions (i.e., gaps) as compared to the reference sequence (which does not comprise additions or deletions) for optimal alignment of the two sequences. The percentage is calculated by determining the number of positions at which the identical nucleic acid base or amino acid residue occurs in both sequences to yield the number of matched positions, dividing the number of matched positions by the total number of positions in the window of comparison and multiplying the results by 100 to yield the percentage of sequence identity. Useful examples of percent sequence identities include, but are not limited to, 50%, 55%, 60%, 65%, 70%, 75%, 80%, 85%, 90% or 95%, or any integer percentage from 50% to 100%. These identities can be determined using any of the programs described herein.

Sequence alignments and percent identity or similarity calculations may be determined using a variety of comparison methods designed to detect homologous sequences including, but not limited to, the MegAlign^{™} program of the LASERGENE bioinformatics computing suite (DNASTAR Inc., Madison, WI). Within the context of this application it will be understood that where sequence analysis software is used for analysis, that the results of the analysis will be based on the "default values" of the program referenced, unless otherwise specified. As used herein "default values" will mean any set of values or parameters that originally load with the software when first initialized.

The "Clustal V method of alignment" corresponds to the alignment method labeled Clustal V (described by Higgins and Sharp, (1989) CABIOS 5:151-153; Higgins et al., (1992) Comput Appl Biosci 8:189-191) and found in the MegAlign^{™} program of the LASERGENE bioinformatics computing suite (DNASTAR Inc., Madison, WI). For multiple alignments, the default values correspond to GAP PENALTY=10 and GAP LENGTH PENALTY=10. Default parameters for pairwise alignments and calculation of percent identity of protein sequences using the Clustal method are KTUPLE=1, GAP PENALTY=3, WINDOW=5 and DIAGONALS SAVED=5. For nucleic acids these parameters are KTUPLE=2, GAP PENALTY=5, WINDOW=4 and DIAGONALS SAVED=4. After alignment of the sequences using the Clustal V program, it is possible to obtain a "percent identity" by viewing the "sequence distances" table in the same program.

The "Clustal W method of alignment" corresponds to the alignment method labeled Clustal W (described by Higgins and Sharp, (1989) CABIOS 5:151-153; Higgins et al., (1992) Comput Appl Biosci 8:189-191) and found in the MegAlign^{™} v6.1 program of the LASERGENE bioinformatics computing suite (DNASTAR Inc., Madison, WI). Default parameters for multiple alignment (GAP PENALTY=10, GAP LENGTH PENALTY=0.2, Delay Divergen Seqs (%)=30, DNA Transition Weight=0.5, Protein Weight Matrix=Gonnet Series, DNA Weight Matrix=IUB ). After alignment of the sequences using the Clustal W program, it is possible to obtain a "percent identity" by viewing the "sequence distances" table in the same program.

Unless otherwise stated, sequence identity/similarity values provided herein refer to the value obtained using GAP Version 10 (GCG, Accelrys, San Diego, CA) using the following parameters: % identity and % similarity for a nucleotide sequence using a gap creation penalty weight of 50 and a gap length extension penalty weight of 3, and the nwsgapdna.cmp scoring matrix; % identity and % similarity for an amino acid sequence using a GAP creation penalty weight of 8 and a gap length extension penalty of 2, and the BLOSUM62 scoring matrix (Henikoff and Henikoff, (1989) Proc. Natl. Acad. Sci. USA 89:10915). GAP uses the algorithm of Needleman and Wunsch, (1970) J Mol Biol 48:443-53, to find an alignment of two complete sequences that maximizes the number of matches and minimizes the number of gaps. GAP considers all possible alignments and gap positions and creates the alignment with the largest number of matched bases and the fewest gaps, using a gap creation penalty and a gap extension penalty in units of matched bases.

"BLAST" is a searching algorithm provided by the National Center for Biotechnology Information (NCBI) used to find regions of similarity between biological sequences. The program compares nucleotide or protein sequences to sequence databases and calculates the statistical significance of matches to identify sequences having sufficient similarity to a query sequence such that the similarity would not be predicted to have occurred randomly. BLAST reports the identified sequences and their local alignment to the query sequence.

It is well understood by one skilled in the art that many levels of sequence identity are useful in identifying polypeptides from other species or modified naturally or synthetically wherein such polypeptides have the same or similar function or activity. Useful examples of percent identities include, but are not limited to, 50%, 55%, 60%, 65%, 70%, 75%, 80%, 85%, 90% or 95%, or any integer percentage from 50% to 100%. Indeed, any integer amino acid identity from 50% to 100% may be useful in describing the present disclosure, such as 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99%.

"Gene" includes a nucleic acid fragment that expresses a functional molecule such as, but not limited to, a specific protein, including regulatory sequences preceding (5' non-coding sequences) and following (3' non-coding sequences) the coding sequence. "Native gene" refers to a gene as found in nature with its own regulatory sequences.

A "mutated gene" is a gene that has been altered through human intervention. Such a "mutated gene" has a sequence that differs from the sequence of the corresponding non-mutated gene by at least one nucleotide addition, deletion, or substitution. In certain embodiments of the disclosure, the mutated gene comprises an alteration that results from a guide polynucleotide/Cas endonuclease system as disclosed herein. A mutated cell is a cell comprising a mutated gene.

As used herein, a "targeted mutation" is a mutation in a native gene that was made by altering a target sequence within the native gene using a method involving a double-strand-break-inducing agent that is capable of inducing a double-strand break in the DNA of the target sequence as disclosed herein or known in the art.

The guide RNA/Cas endonuclease induced targeted mutation can occur in a nucleotide sequence that is located within or outside a genomic target site that is recognized and cleaved by a Cas endonuclease.

The term "genome" as it applies to a cell encompasses not only chromosomal DNA found within the nucleus, but organelle DNA found within subcellular components (e.g., mitochondria, or plastid) of the cell.

A "codon-modified gene" or "codon-preferred gene" or "codon-optimized gene" is a gene having its frequency of codon usage designed to mimic the frequency of preferred codon usage of the host cell.

An "allele" is one of several alternative forms of a gene occupying a given locus on a chromosome. When all the alleles present at a given locus on a chromosome are the same, that organism is homozygous at that locus. If the alleles present at a given locus on a chromosome differ, that organism is heterozygous at that locus.

"Coding sequence" refers to a polynucleotide sequence which codes for a specific amino acid sequence. "Regulatory sequences" refer to nucleotide sequences located upstream (5' non-coding sequences), within, or downstream (3' non-coding sequences) of a coding sequence, and which influence the transcription, RNA processing or stability, or translation of the associated coding sequence. Regulatory sequences may include, but are not limited to: promoters, translation leader sequences, 5' untranslated sequences, 3' untranslated sequences, introns, polyadenylation target sequences, RNA processing sites, effector binding sites, and stem-loop structures.

A promoter is a region of DNA involved in recognition and binding of RNA polymerase and other proteins to initiate transcription. The promoter sequence consists of proximal and more distal upstream elements, the latter elements often referred to as enhancers. An "enhancer" is a DNA sequence that can stimulate promoter activity, and may be an innate element of the promoter or a heterologous element inserted to enhance the level or tissue-specificity of a promoter. Promoters may be derived in their entirety from a native gene, or be composed of different elements derived from different promoters found in nature, and/or comprise synthetic DNA segments. It is understood by those skilled in the art that different promoters may direct the expression of a gene in different tissues or cell types, or at different stages of development, or in response to different environmental conditions. It is further recognized that since in most cases the exact boundaries of regulatory sequences have not been completely defined, DNA fragments of some variation may have identical promoter activity. Promoters that cause a gene to be expressed in most cell types at most times are commonly referred to as "constitutive promoters".

A "strong promoter" as used herein refers to a promoter that can direct a relatively large number of productive initiations per unit time, and/or is a promoter driving a higher level of gene transcription than the average transcription level of the genes in a cell.

A plant promoter is a promoter capable of initiating transcription in a plant cell, for a review of plant promoters, see, Potenza et al., (2004) In Vitro Cell Dev Biol 40:1-22. Constitutive promoters include, for example, the core promoter of the Rsyn7 promoter and other constitutive promoters disclosed in WO99/43838 and U.S. Patent No. 6,072,050; the core CaMV 35S promoter (Odell et al., (1985) Nature 313:810-2); rice actin (McElroy et al., (1990) Plant Cell 2:163-71); ubiquitin (Christensen et al., (1989) Plant Mol Biol 12:619-32; Christensen et al., (1992) Plant Mol Biol 18:675-89); pEMU (Last et al., (1991) Theor Appl Genet 81:581-8); MAS (Velten et al., (1984) EMBO J 3:2723-30); ALS promoter (U.S. Patent No. 5,659,026), and the like. Other constitutive promoters are described in, for example, U.S. Patent Nos. 5,608,149; 5,608,144; 5,604,121; 5,569,597; 5,466,785; 5,399,680; 5,268,463; 5,608,142 and 6,177,611. In some examples an inducible promoter may be used. Pathogen-inducible promoters induced following infection by a pathogen include, but are not limited to those regulating expression of PR proteins, SAR proteins, beta-1,3-glucanase, chitinase, *etc.*

Chemical-regulated promoters can be used to modulate the expression of a gene in a plant through the application of an exogenous chemical regulator. The promoter may be a chemical-inducible promoter, where application of the chemical induces gene expression, or a chemical-repressible promoter, where application of the chemical represses gene expression. Chemical-inducible promoters include, but are not limited to, the maize In2-2 promoter, activated by benzene sulfonamide herbicide safeners (De Veylder et al., (1997) Plant Cell Physiol 38:568-77), the maize GST promoter (GST-II-27, WO93/01294), activated by hydrophobic electrophilic compounds used as pre-emergent herbicides, and the tobacco PR-1a promoter (Ono et al., (2004) Biosci Biotechnol Biochem 68:803-7) activated by salicylic acid. Other chemical-regulated promoters include steroid-responsive promoters (see, for example, the glucocorticoid-inducible promoter (Schena et al., (1991) Proc. Natl. Acad. Sci. USA 88:10421-5; McNellis et al., (1998) Plant J 14:247-257); tetracycline-inducible and tetracycline-repressible promoters (Gatz et al., (1991) Mol Gen Genet 227:229-37; U.S. Patent Nos. 5,814,618 and 5,789,156).

Tissue-preferred promoters can be utilized to target enhanced expression within a particular plant tissue. Tissue-preferred promoters include, for example, Kawamata et al., (1997) Plant Cell Physiol 38:792-803; and Guevara-Garcia et al., (1993) Plant J 4:495-505. Seed-preferred promoters include both seed-specific promoters active during seed development, as well as seed-germinating promoters active during seed germination. See, Thompson et al., (1989) BioEssays 10:108.

The term "inducible promoter" refers to promoters that selectively express a coding sequence or functional RNA in response to the presence of an endogenous or exogenous stimulus, for example by chemical compounds (chemical inducers) or in response to environmental, hormonal, chemical, and/or developmental signals. Inducible or regulated promoters include, for example, promoters induced or regulated by light, heat, stress, flooding or drought, salt stress, osmotic stress, phytohormones, wounding, or chemicals such as ethanol, abscisic acid (ABA), jasmonate, salicylic acid, or safeners. An example of a stress-inducible is RD29A promoter (Kasuga et al. (1999) Nature Biotechnol. 17:287-91New promoters of various types useful in plant cells are constantly being discovered; numerous examples may be found in the compilation by Okamuro and Goldberg, (1989) In The Biochemistry of Plants, Vol. 115, Stumpf and Conn, eds (New York, NY: Academic Press), pp. 1-82.

"Translation leader sequence" refers to a polynucleotide sequence located between the promoter sequence of a gene and the coding sequence. The translation leader sequence is present in the mRNA upstream of the translation start sequence. The translation leader sequence may affect processing of the primary transcript to mRNA, mRNA stability or translation efficiency. Examples of translation leader sequences have been described (e.g., Turner and Foster, (1995) Mol Biotechnol 3:225-236).

"3' non-coding sequences", "transcription terminator" or "termination sequences" refer to DNA sequences located downstream of a coding sequence and include polyadenylation recognition sequences and other sequences encoding regulatory signals capable of affecting mRNA processing or gene expression. The polyadenylation signal is usually characterized by affecting the addition of polyadenylic acid tracts to the 3' end of the mRNA precursor. The use of different 3' non-coding sequences is exemplified by Ingelbrecht et a/., (1989) Plant Cell 1:671-680.

"RNA transcript" refers to the product resulting from RNA polymerase-catalyzed transcription of a DNA sequence. When the RNA transcript is a perfect complimentary copy of the DNA sequence, it is referred to as the primary transcript or pre-mRNA. A RNA transcript is referred to as the mature RNA or mRNA when it is a RNA sequence derived from post-transcriptional processing of the primary transcript pre mRNAt. "Messenger RNA" or "mRNA" refers to the RNA that is without introns and that can be translated into protein by the cell. "cDNA" refers to a DNA that is complementary to, and synthesized from, a mRNA template using the enzyme reverse transcriptase. The cDNA can be single-stranded or converted into double-stranded form using the Klenow fragment of DNA polymerase I. "Sense" RNA refers to RNA transcript that includes the mRNA and can be translated into protein within a cell or *in vitro.* "Antisense RNA" refers to an RNA transcript that is complementary to all or part of a target primary transcript or mRNA, and that blocks the expression of a target gene (see, e.g., U.S. Patent No. 5,107,065). The complementarity of an antisense RNA may be with any part of the specific gene transcript, i.e., at the 5' non-coding sequence, 3' non-coding sequence, introns, or the coding sequence. "Functional RNA" refers to antisense RNA, ribozyme RNA, or other RNA that may not be translated but yet has an effect on cellular processes. The terms "complement" and "reverse complement" are used interchangeably herein with respect to mRNA transcripts, and are meant to define the antisense RNA of the message.

The terms "control cell" and "suitable control cell" are used interchangeably herein and may be referenced with respect to a cell in which a particular modification (e.g., over-expression of a polynucleotide, down-regulation of a polynucleotide) has been made (i.e., an "experimental cell"). A control cell may be any cell that does not have or does not express the particular modification of the experimental cell. Thus, a control cell may be an untransformed wild type cell or may be genetically transformed but does not express the genetic transformation. For example, a control cell may be a direct parent of the experimental cell, which direct parent cell does not have the particular modification that is in the experimental cell. Alternatively, a control cell may be a parent of the experimental cell that is removed by one or more generations. Alternatively still, a control cell may be a sibling of the experimental cell, which sibling does not comprise the particular modification that is present in the experimental cell.

The term "operably linked" refers to the association of nucleic acid sequences on a single nucleic acid fragment so that the function of one is regulated by the other. For example, a promoter is operably linked with a coding sequence when it is capable of regulating the expression of that coding sequence (i.e., the coding sequence is under the transcriptional control of the promoter). Coding sequences can be operably linked to regulatory sequences in a sense or antisense orientation. In another example, the complementary RNA regions can be operably linked, either directly or indirectly, 5' to the target mRNA, or 3' to the target mRNA, or within the target mRNA, or a first complementary region is 5' and its complement is 3' to the target mRNA.

Standard recombinant DNA and molecular cloning techniques used herein are well known in the art and are described more fully in Sambrook et al., Molecular Cloning: A Laboratory Manual; Cold Spring Harbor Laboratory: Cold Spring Harbor, NY (1989). Transformation methods are well known to those skilled in the art and are described *infra.*

"PCR" or "polymerase chain reaction" is a technique for the synthesis of specific DNA segments and consists of a series of repetitive denaturation, annealing, and extension cycles. Typically, a double-stranded DNA is heat denatured, and two primers complementary to the 3' boundaries of the target segment are annealed to the DNA at low temperature, and then extended at an intermediate temperature. One set of these three consecutive steps is referred to as a "cycle".

The term "recombinant" refers to an artificial combination of two otherwise separated segments of sequence, e.g., by chemical synthesis, or manipulation of isolated segments of nucleic acids by genetic engineering techniques.

The terms "plasmid", "vector" and "cassette" refer to an extra chromosomal element often carrying genes that are not part of the central metabolism of the cell, and usually in the form of double-stranded DNA. Such elements may be autonomously replicating sequences, genome integrating sequences, phage, or nucleotide sequences, in linear or circular form, of a single- or double-stranded DNA or RNA, derived from any source, in which a number of nucleotide sequences have been joined or recombined into a unique construction which is capable of introducing a polynucleotide of interest into a cell. "Transformation cassette" refers to a specific vector containing a gene and having elements in addition to the gene that facilitates transformation of a particular host cell. "Expression cassette" refers to a specific vector containing a gene and having elements in addition to the gene that allow for expression of that gene in a host.

The term "transformation" as used herein refers to the transfer of a nucleic acid molecule into a host organism. The nucleic acid molecule may be a plasmid that replicates autonomously, or it may integrate into the genome of the host organism. The terms "recombinant DNA molecule", "recombinant construct", "expression construct", " construct", "construct", and "recombinant DNA construct" are used interchangeably herein. A recombinant construct comprises an artificial combination of nucleic acid fragments, e.g., regulatory and coding sequences that are not all found together in nature. For example, a construct may comprise regulatory sequences and coding sequences that are derived from different sources, or regulatory sequences and coding sequences derived from the same source, but arranged in a manner different than that found in nature. Such a construct may be used by itself or may be used in conjunction with a vector. If a vector is used, then the choice of vector is dependent upon the method that will be used to transform host cells as is well known to those skilled in the art. For example, a plasmid vector can be used. The skilled artisan is well aware of the genetic elements that must be present on the vector in order to successfully transform, select and propagate host cells. The skilled artisan will also recognize that different independent transformation events may result in different levels and patterns of expression (Jones et a/., (1985) EMBO J 4:2411-2418; De Almeida et a/., (1989) Mol Gen Genetics 218:78-86), and thus that multiple events are typically screened in order to obtain lines displaying the desired expression level and pattern. Such screening may be accomplished standard molecular biological, biochemical, and other assays including Southern analysis of DNA, Northern analysis of mRNA expression, PCR, real time quantitative PCR (qPCR), reverse transcription PCR (RT-PCR), immunoblotting analysis of protein expression, enzyme or activity assays, and/or phenotypic analysis.

The term "expression", as used herein, refers to the production of a functional end-product (e.g., an mRNA, guide RNA, or a protein) in either precursor or mature form.

The term "providing" includes providing a nucleic acid (e.g., expression construct) or peptide, polypeptide or protein to a cell. Providing includes reference to the incorporation of a nucleic acid or polypeptide into a eukaryotic or prokaryotic cell where the nucleic acid may be incorporated into the genome of the cell, and includes reference to the transient provision of a nucleic acid or protein to the cell. Providing includes reference to stable or transient transformation methods, transfection, transduction, microinjection, electroporation, viral methods, *Agrobacterium*-mediated transformation, ballistic particle acceleration as well as sexually crossing. Thus, "providing" in the context of inserting a nucleic acid fragment (e.g., a recombinant DNA construct/expression construct, guide RNA, guide DNA, template DNA, donor DNA) into a cell, includes "transfection" or "transformation" or "transduction" and includes reference to the incorporation of a nucleic acid fragment into a eukaryotic or prokaryotic cell where the nucleic acid fragment may be incorporated into the genome of the cell (e.g., chromosome, plasmid, plastid, or mitochondrial DNA), converted into an autonomous replicon, or transiently expressed (e.g., transfected mRNA).

A variety of methods are known for contacting, providing, and/or introducing a composition (such as a nucleotide sequence, a peptide or a polypeptide) into an organisms including stable transformation methods, transient transformation methods, virus-mediated methods, sexual crossing and sexual breeding. Stable transformation indicates that the introduced polynucleotide integrates into the genome of the organism and is capable of being inherited by progeny thereof. Transient transformation indicates that the introduced composition is only temporarily expressed or present in the organism.

Protocols for contacting, providing, introducing polynucleotides and polypeptides to cells or organisms are known. and include microinjection (Crossway et al., (1986) Biotechniques 4:320-34 and U.S. Patent No. 6,300,543), meristem transformation (U.S. Patent No. 5,736,369), electroporation (Riggs et al., (1986) Proc. Natl. Acad. Sci. USA 83:5602-6, Agrobacterium-mediated transformation (U.S. Patent Nos. 5,563,055 and 5,981,840), direct gene transfer (Paszkowski et al., (1984) EMBO J 3:2717-22), and ballistic particle acceleration (U.S. Patent Nos. 4,945,050; 5,879,918; 5,886,244; 5,932,782; Tomes et al., (1995) "Direct DNA Transfer into Intact Plant Cells via Microprojectile Bombardment" in Plant Cell, Tissue, and Organ Culture: Fundamental Methods, ed. Gamborg & Phillips (Springer-Verlag, Berlin); McCabe et al., (1988) Biotechnology 6:923-6; Weissinger et al., (1988) Ann Rev Genet 22:421-77; Sanford et al., (1987) Particulate Science and Technology 5:27-37 (onion); Christou et al., (1988) Plant Physiol 87:671-4 (soybean); Finer and McMullen, (1991) In Vitro Cell Dev Biol 27P:175-82 (soybean); Singh et al., (1998) Theor Appl Genet 96:319-24 (soybean); Datta et al., (1990) Biotechnology 8:736-40 (rice); Klein et al., (1988) Proc. Natl. Acad. Sci. USA 85:4305-9 (maize); Klein et al., (1988) Biotechnology 6:559-63 (maize); U.S. Patent Nos. 5,240,855; 5,322,783 and 5,324,646; Klein et al., (1988) Plant Physiol 91:440-4 (maize); Fromm et al., (1990) Biotechnology 8:833-9 (maize); Hooykaas-Van Slogteren et al., (1984) Nature 311:763-4; U.S. Patent No. 5,736,369 (cereals); Bytebier et al., (1987) Proc. Natl. Acad. Sci. USA 84:5345-9 (*Liliaceae*); De Wet et al., (1985) in The Experimental Manipulation of Ovule Tissues, ed. Chapman et al., (Longman, New York), pp. 197-209 (pollen); Kaeppler et al., (1990) Plant Cell Rep 9:415-8) and Kaeppler et al., (1992) Theor Appl Genet 84:560-6 (whisker-mediated transformation); D'Halluin et al., (1992) Plant Cell 4:1495-505 (electroporation); Li et al., (1993) Plant Cell Rep 12:250-5; Christou and Ford (1995) Annals Botany 75:407-13 (rice) and Osjoda et al., (1996) Nat Biotechnol 14:745-50 (maize via *Agrobacterium tumefaciens*), chemical transformation ( lithium acetate transformation [Methods in Enzymology, 194:186-187 (1991)). As an example, U.S. Patent Nos. 4,880,741 and 5,071,764, and Chen et al. (1997, Appl. Microbiol. Biotechnol. 48:232-235), describe integration techniques for *Y. lipolytica,* based on linearized fragments of DNA.

Alternatively, polynucleotides may be introduced into cells or organisms by contacting cells or organisms with a virus or viral nucleic acids. Generally, such methods involve incorporating a polynucleotide within a viral DNA or RNA molecule. In some examples a polypeptide of interest may be initially synthesized as part of a viral polyprotein, which is later processed by proteolysis *in vivo* or *in vitro* to produce the desired recombinant protein. Methods for introducing polynucleotides into plants and expressing a protein encoded therein, involving viral DNA or RNA molecules, are known, see, for example, U.S. Patent Nos. 5,889,191, 5,889,190, 5,866,785, 5,589,367 and 5,316,931. Transient transformation methods include, but are not limited to, the introduction of polypeptides, such as a double-strand break inducing agent, directly into the organism, the introduction of polynucleotides such as DNA and/or RNA polynucleotides, and the introduction of the RNA transcript, such as an mRNA encoding a double-strand break inducing agent, into the organism. Such methods include, for example, microinjection or particle bombardment. See, for example Crossway et al., (1986) Mol Gen Genet 202:179-85; Nomura et al., (1986) Plant Sci 44:53-8; Hepler et al., (1994) Proc. Natl. Acad. Sci. USA 91:2176-80; and, Hush et al., (1994) J Cell Sci 107:775-84.

Nucleid acids and proteins can be provided to a cell by any method known in the art such as, but not limited to, methods using molecules to facilitate the uptake of anyone or all components of a guided Cas system (protein and/or nucleic acids), such as cell-penetrating peptides, nanocariers. See also US20110035836 Nanocarier based plant transfection and transduction, and EP 2821486 A1, Method of introducing nucleic acid into plant cells.

Providing a guide RNA/Cas endonuclease complex to a cell includes providing the individual components of said complex to the cell either directly or via recombination constructs, and includes providing the whole complex to the cell as well.

"Mature" protein refers to a post-translationally processed polypeptide (i.e., one from which any pre- or pro-peptides present in the primary translation product have been removed). "Precursor" protein refers to the primary product of translation of mRNA (i.e., with pre- and propeptides still present). Pre- and propeptides may be but are not limited to intracellular localization signals.

"Stable transformation" refers to the transfer of a nucleic acid fragment into a genome of a host organism, including both nuclear and organellar genomes, resulting in genetically stable inheritance. In contrast, "transient transformation" refers to the transfer of a nucleic acid fragment into the nucleus, or other DNA-containing organelle, of a host organism resulting in gene expression without integration or stable inheritance. Host organisms containing the transformed nucleic acid fragments are referred to as "transgenic" organisms.

The term "plant" refers to whole plants, plant organs, plant tissues, seeds, plant cells, seeds and progeny of the same. Plant cells include, without limitation, cells from seeds, suspension cultures, embryos, meristematic regions, callus tissue, leaves, roots, shoots, gametophytes, sporophytes, pollen and microspores. Plant parts include differentiated and undifferentiated tissues including, but not limited to roots, stems, shoots, leaves, pollens, seeds, tumor tissue and various forms of cells and culture (e.g., single cells, protoplasts, embryos, and callus tissue). The plant tissue may be in plant or in a plant organ, tissue or cell culture. The term "plant organ" refers to plant tissue or a group of tissues that constitute a morphologically and functionally distinct part of a plant. The term "genome" refers to the entire complement of genetic material (genes and non-coding sequences) that is present in each cell of an organism, or virus or organelle; and/or a complete set of chromosomes inherited as a (haploid) unit from one parent. "Progeny" comprises any subsequent generation of a plant.

A transgenic plant includes, for example, a plant which comprises within its genome a heterologous polynucleotide introduced by a transformation step. The heterologous polynucleotide can be stably integrated within the genome such that the polynucleotide is passed on to successive generations. The heterologous polynucleotide may be integrated into the genome alone or as part of a recombinant DNA construct. A transgenic plant can also comprise more than one heterologous polynucleotide within its genome. Each heterologous polynucleotide may confer a different trait to the transgenic plant. A heterologous polynucleotide can include a sequence that originates from a foreign species, or, if from the same species, can be substantially modified from its native form. Transgenic can include any cell, cell line, callus, tissue, plant part or plant, the genotype of which has been altered by the presence of heterologous nucleic acid including those transgenics initially so altered as well as those created by sexual crosses or asexual propagation from the initial transgenic. The alterations of the genome (chromosomal or extra-chromosomal) by conventional plant breeding methods, by the genome editing procedure described herein that does not result in an insertion of a foreign polynucleotide, or by naturally occurring events such as random cross-fertilization, non-recombinant viral infection, non-recombinant bacterial transformation, non-recombinant transposition, or spontaneous mutation are not intended to be regarded as transgenic.

A fertile plant is a plant that produces viable male and female gametes and is self-fertile. Such a self-fertile plant can produce a progeny plant without the contribution from any other plant of a gamete and the genetic material contained therein. Male-sterile plants include plants that do not produce male gametes that are viable or otherwise capable of fertilization. Female-sterile plants include plants that do not produce female gametes that are viable or otherwise capable of fertilization. It is recognized that male-sterile and female-sterile plants can be female-fertile and male- fertile, respectively. It is further recognized that a male fertile (but female sterile) plant can produce viable progeny when crossed with a female fertile plant and that a female fertile (but male sterile) plant can produce viable progeny when crossed with a male fertile plant.

Conventional yeasts such as *Saccharomyces cerevisiae* and *Schizosaccharomyces pombe* typically exhibit specific integration of donor DNA with short flanking homology arms (30-50 bp) with efficiencies routinely over 70%, whereas non-conventional yeasts such as *Pichia pastoris, Hansenula polymorpha, Yarrowia lipolytica, Pichia stipitis* and *Kluyveromyces lactis* usually show specific integration with similarly structured donor DNA at efficiencies of less than 1% (Chen et al., PLoS ONE 8:e57952). Thus, a preference for HR processes can be gauged, for example, by transforming yeast with a suitable donor DNA and determining the degree to which it is specifically recombined with a genomic site predicted to be targeted by the donor DNA. A preference for NHEJ (or low preference for HR), for example, would be manifest if such an assay yielded a high degree of random integration of the donor DNA in the yeast genome. Assays for determining the rate of specific (HR-mediated) and/or random (NHEJ-mediated) integration of DNA in yeast are known in the art (e.g., Ferreira and Cooper, Genes Dev. 18:2249-2254; Corrigan et al., PLoS ONE 8:e69628; Weaver et al., Proc. Natl. Acad. Sci. U.S.A. 78:6354-6358; Keeney and Boeke, Genetics 136:849-856).

Given their low level of HR activity, non-conventional yeast herein can (i) exhibit a rate of specific targeting by a suitable template or donor DNA having 30-50 bp flanking homology arms of less than about 1%, 2%, 3%, 4%, 5%,, 6%, 7%, or 8%, for example, and/or (ii) exhibit a rate of random integration of the foregoing donor DNA of more than about 65%, 66%, 67%, 68%, 69%, 70%, 71%, 72%, 73%, 74%, or 75%, for example. These rates of (i) specific targeting and/or (ii) random integration of a suitable template or donor DNA can characterize a non-conventional yeast as it exists before being provided a guided Cas system as disclosed herein.

Non-limiting examples of non-conventional yeast herein include yeasts of the following genera: *Yarrowia, Pichia, Schwanniomyces, Kluyveromyces, Arxula, Trichosporon, Candida, Ustilago, Torulopsis, Zygosaccharomyces, Trigonopsis,* Cryptococcus, *Rhodotorula, Phaffia, Sporobolomyces,* and *Pachysolen.* A suitable example of a *Yarrowia* species is *Y. lipolytica.* Suitable examples of *Pichia* species include *P. pastoris, P. methanolica, P. stipitis, P. anomala* and *P. angusta.* Suitable examples of *Schwanniomyces* species include *S*. *castellii, S. alluvius, S. hominis, S. occidentalis,* S. capriottii, *S*. *etchellsii, S. polymorphus, S. pseudopolymorphus, S. vanrijiae* and *S*. *yamadae.* Suitable examples of *Kluyveromyces* species include K. lactis, K. marxianus, *K. fragilis, K. drosophilarum, K. thermotolerans, K. phaseolosporus, K. vanudenii, K. waltii, K. africanus* and *K. polysporus.* Suitable examples of *Arxula* species include *A. adeninivorans* and *A. terrestre.* Suitable examples of *Trichosporon* species include *T. cutaneum, T. capitatum, T. inkin* and *T. beemeri.* Suitable examples of *Candida* species include *C*. *albicans, C. ascalaphidarum, C. amphixiae, C. antarctica, C. argentea, C. atlantica, C. atmosphaerica, C. blattae, C. bromeliacearum, C. carpophila, C. carvajalis, C. cerambycidarum, C. chauliodes, C. corydali, C. dosseyi, C. dubliniensis, C. ergatensis, C. fructus, C. glabrata, C. fermentati, C. guilliermondii, C. haemulonii, C. insectamens, C. insectorum, C. intermedia, C. jeffresii, C. kefyr, C*. *keroseneae, C*. *krusei, C. lusitaniae, C. lyxosophila, C. maltosa, C. marina, C. membranifaciens, C. milleri, C. mogii, C. oleophila, C. oregonensis, C. parapsilosis, C. quercitrusa, C. rugosa, C*. *sake, C*. *shehatea, C. temnochilae, C. tenuis, C. theae, C. tolerans, C. tropicalis, C. tsuchiyae, C. sinolaborantium, C*. *sojae, C. subhashii, C. viswanathii, C. utilis, C. ubatubensis* and *C. zemplinina.* Suitable examples of *Ustilago* species include *U. avenae, U. esculenta, U. hordei, U. maydis, U. nuda* and *U. tritici.* Suitable examples of *Torulopsis* species include *T. geochares, T. azyma, T. glabrata* and *T. candida.* Suitable examples of *Zygosaccharomyces* species include *Z*. *bailii, Z. bisporus, Z. cidri, Z. fermentati, Z. florentinus, Z. kombuchaensis, Z. lentus, Z. mellis, Z. microellipsoides, Z. mrakii, Z. pseudorouxii* and *Z. rouxii.* Suitable examples of *Trigonopsis* species include *T. variabilis.* Suitable examples of *Cryptococcus* species include *C. laurentii, C. albidus, C. neoformans, C. gattii, C. uniguttulatus, C. adeliensis, C. aerius, C. albidosimilis, C. antarcticus, C. aquaticus, C. ater, C. bhutanensis, C. consortionis, C. curvatus, C. phenolicus, C. skinneri, C. terreus* and *C*. *vishniacci.* Suitable examples of *Rhodotorula* species include *R*. *acheniorum, R. tula, R. acuta, R. americana, R. araucariae, R. arctica, R. armeniaca, R. aurantiaca, R. auriculariae, R. bacarum, R. benthica, R. biourgei, R. bogoriensis, R. bronchialis, R. buffonii, R. calyptogenae, R. chungnamensis, R. cladiensis, R. corallina, R. cresolica, R. crocea, R. cycloclastica, R. dairenensis, R. diffluens, R. evergladiensis, R. ferulica, R. foliorum, R. fragaria, R. fujisanensis, R. futronensis, R. gelatinosa, R. glacialis, R. glutinis, R. gracilis, R. graminis, R. grinbergsii, R. himalayensis, R. hinnulea, R. histolytica, R. hylophila, R. incarnata, R. ingeniosa, R. javanica, R. koishikawensis, R. lactosa, R. lamellibrachiae, R. laryngis, R. lignophila, R. lini, R. longissima, R. ludwigii, R. lysinophila, R. marina, R. martyniae-fragantis, R. matritensis, R. meli, R. minuta, R. mucilaginosa, R. nitens, R. nothofagi, R. oryzae, R. pacifica, R. pallida, R. peneaus, R. philyla, R. phylloplana, R. pilatii, R. pilimanae, R. pinicola, R. plicata, R. polymorpha, R. psychrophenolica, R. psychrophila, R. pustula, R. retinophila, R. rosacea, R. rosulata, R. rubefaciens, R. rubella, R. rubescens, R. rubra, R. rubrorugosa, R. rufula, R. rutila, R. sanguinea, R. sanniei, R. sartoryi, R. silvestris, R. simplex, R. sinensis, R. slooffiae, R. sonckii, R. straminea, R. subericola, R. suganii, R. taiwanensis, R. taiwaniana, R. terpenoidalis, R. terrea, R. texensis, R. tokyoensis, R. ulzamae, R. vanillica, R. vuilleminii, R. yarrowii, R. yunnanensis* and *R. zsoltii.* Suitable examples of *Phaffia* species include *P. rhodozyma.* Suitable examples of *Sporobolomyces* species include *S*. *alborubescens, S*. *bannaensis, S. beijingensis, S*. *bischofiae, S. clavatus, S. coprosmae, S. coprosmicola, S. corallinus, S. dimmenae, S. dracophylli, S. elongatus, S. gracilis, S. inositophilus, S. johnsonii, S*. *koalae, S. magnisporus, S. novozealandicus, S*. *odorus, S. patagonicus, S. productus, S*. *roseus, S*. *sasicola, S*. *shibatanus, S. singularis, S. subbrunneus, S. symmetricus, S. syzygii, S*. *taupoensis, S*. *tsugae, S. xanthus* and *S*. *yunnanensis.* Suitable examples of *Pachysolen* species include *P. tannophilus.*

Examples of *Yarrowia lipolytica* (*Y*. *lipolytica* ) include the following isolates available from the American Type Culture Collection (ATCC, Manassas, VA): strain designations ATCC #20362, #8862, #8661, #8662, #9773, #15586, #16617, #16618, #18942, #18943, #18944, #18945, #20114, #20177, #20182, #20225, #20226, #20228, #20327, #20255, #20287, #20297, #20315, #20320, #20324, #20336, #20341, #20346, #20348, #20363, #20364, #20372, #20373, #20383, #20390, #20400, #20460, #20461, #20462, #20496, #20510, #20628, #20688, #20774, #20775, #20776, #20777, #20778, #20779, #20780, #20781, #20794, #20795, #20875, #20241, #20422, #20423, #32338, #32339, #32340, #32341, #34342, #32343, #32935, #34017, #34018, #34088, #34922, #34922, #38295, #42281, #44601, #46025, #46026, #46027, #46028, #46067, #46068, #46069, #46070, #46330, #46482, #46483, #46484, #46436, #60594, #62385, #64042, #74234, #76598, #76861, #76862, #76982, #90716, #90811, #90812, #90813, #90814, #90903, #90904, #90905, #96028, #201241, #201242, #201243, #201244, #201245, #201246, #201247, #201249, and/or #201847.

A *Y. lipolytica,* as well as any other non-conventional yeast herein, may be oleaginous (e.g., produce at least 25% of its dry cell weight as oil) and/or produce one or more polyunsaturated fatty acids (e.g., omega-6 or omega-3). Such oleaginy may be a result of the yeast being genetically engineered to produce an elevated amount of lipids compared to its wild type form. Examples of oleaginous *Y. lipolytica* strains are disclosed in U.S. Pat. Appl. Publ. Nos. 2009/0093543, 2010/0317072, 2012/0052537 and 2014/0186906.

Embodiments disclosed herein for non-conventional yeast can also be applied to other microorganisms such as fungi. Fungi in certain embodiments can be fungi that favor NHEJ DNA repair processes over repair processes mediated by HR. A fungus herein can be a Basidiomycetes, Zygomycetes, Chytridiomycetes, or Ascomycetes fungus. Examples of filamentous fungi herein include those of the genera *Trichoderma, Chrysosporium, Thielavia, Neurospora* (e.g., *N. crassa, N. sitophila*), *Cryphonectria* (e.g., *C. parasitica*)*, Aureobasidium* (e.g., *A. pullulans*), *Filibasidium, Piromyces, Cryplococcus, Acremonium, Tolypocladium, Scytalidium, Schizophyllum, Sporotrichum, Penicillium* (e.g., *P. bilaiae, P. camemberti, P. candidum, P. chrysogenum, P. expansum, P. funiculosum, P. glaucum, P. marneffei, P. roqueforti, P. verrucosum, P. viridicatum* ), *Gibberella* (e.g., *G. acuminata, G. avenacea, G. baccata, G. circinata, G. cyanogena, G. fujikuroi, G. intricans, G. pulicaris, G. stilboides, G. tricincta, G*. *zeae*), *Myceliophthora, Mucor* (e.g., *M. rouxii, M. circinelloides*), *Aspergillus* (e.g., *A. niger, A. oryzae, A. nidulans, A. flavus, A. lentulus, A. terreus, A. clavatus, A. fumigatus*), *Fusarium* (e.g., *F. graminearum, F. oxysporum, F. bubigenum, F. solani, F. oxysporum, F. verticillioides, F. proliferatum, F. venenatum*), and *Humicola*, and anamorphs and teleomorphs thereof. The genus and species of fungi herein can be defined, if desired, by morphology as disclosed in Barnett and Hunter (Illustrated Genera of Imperfect Fungi, 3rd Edition, Burgess Publishing Company, 1972). A fungus can optionally be characterized as a pest/pathogen, such as a pest/pathogen of an animal (e.g., human).

*Trichoderma* species in certain aspects herein include *T. aggressivum*, *T. amazonicum, T. asperellum, T. atroviride, T. aureoviride, T. austrokoningii, T. brevicompactum, T. candidum, T. caribbaeum, T. catoptron, T. cremeum, T. ceramicum, T. cerinum, T. chlorosporum, T. chromospermum, T. cinnamomeum, T. citrinoviride, T. crassum, T. cremeum, T. dingleyeae, T. dorotheae*, *T. effusum, T. erinaceum, T. estonicum, T. fertile, T. gelatinosus, T. ghanense, T. hamatum, T. harzianum, T. helicum, T. intricatum*, *T. konilangbra, T. koningii, T. koningiopsis, T. longibrachiatum, T. longipile, T. minutisporum, T. oblongisporum, T. ovalisporum, T. petersenii, T. phyllostahydis, T. piluliferum, T. pleuroticola, T. pleurotum, T. polysporum, T. pseudokoningii, T. pubescens, T. reesei, T. rogersonii, T. rossicum, T. saturnisporum*, *T. sinensis, T. sinuosum, T. spirale, T. stramineum, T. strigosum*, *T. stromaticum, T. surrotundum, T. taiwanense, T. thailandicum, T. thelephoricolum, T. theobromicola, T. tomentosum, T. velutinum, T. virens, T. viride* and *T. viridescens.* A *Trichoderma* species herein can be cultivated and/or manipulated as described in Trichoderma: Biology and Applications (P.K. Mukherjee et al., Eds., CABI, Oxfordshire, UK, 2013), for example.

A microbial cell in certain embodiments is an algal cell. For example, an algal cell can be from any of the following: Chlorophyta (green algae), Rhodophyta (red algae), Phaeophyceae (brown algae), Bacillariophycaeae (diatoms), and Dinoflagellata (dinoflagellates). An algal cell can be of a microalgae (e.g., phytoplankton, microphytes, or planktonic algae) or macroalgae (kelp, seaweed) in other aspects. As further examples, an algal cell herein can be a *Porphyra* (purple laver), *Palmaria* species such as *P. palmata* (dulse), *Arthrospira* species such as A. *platensis* (*spirulina*), *Chlorella* (e.g., *C. protothecoides*), *a Chondrus* species such as *C. crispus* (Irish moss), *Aphanizomenon, Sargassum, Cochayuyo, Botryococcus* (e.g., *B. braunii*), *Dunaliella* (e.g., *D. tertiolecta*), *Gracilaria, Pleurochrysis* (e.g., *P*. *carterae), Ankistrodesmus, Cyclotella, Hantzschia, Nannochloris, Nannochloropsis, Nitzschia, Phaeodactylum* (e.g., *P. tricornutum*), *Scenedesmus, Stichococcus, Tetraselmis* (e.g., *T. suecica*), *Thalassiosira* (e.g., *T. pseudonana*), *Crypthecodinium* (e.g., *C. cohnii*), *Neochloris* (e.g., *N. oleoabundans*), or *Schiochytrium.* An algal species herein can be cultivated and/or manipulated as described in Thompson (Algal Cell Culture. Encyclopedia of Life Support System (EOLSS), Biotechnology Vol 1, available at eolss.net/sample-chapters internet site), for example.

A protist cell herein can be selected from the class Ciliata (e.g., the genera *Tetrahymena, Paramecium, Colpidium, Colpoda, Glaucoma, Platyophrya, Vorticella, Potomacus*, Pseudocohnilembus, *Euplotes, Engelmaniella,* and *Stylonichia*), the subphylum Mastigophora (flagellates), the class Phytomastigophorea (e.g., the genera *Euglena, Astasia, Haematococcus,* and *Crypthecodinium*), the class Zoomastigophorea, the superclass Rhizopoda, the class Lobosea (e.g., the genus *Amoeba*), and the class Eumycetozoea (e.g., the genera *Dictyostelium* and *Physarum*), for example. Certain protist species herein can be cultivated and/or manipulated as described in ATCC® Protistology Culture Guide: tips and techniques for propagating protozoa and algae (2013, available at American Type Culture Collection internet site), for example. A protist can optionally be characterized as a pest/pathogen of a plant or animal (e.g., human) in certain embodiments.

A bacterial cell in certain embodiments can be those in the form of cocci, bacilli, spirochetes, spheroplasts, protoplasts, etc. Other non-limiting examples of bacteria include those that are Gram-negative and Gram-positive. Still other non-limiting examples of bacteria include those of the genera *Salmonella* (e.g., *S*. *typhi, S. enteritidis*), *Shigella* (e.g., *S*. *dysenteriae*), *Escherichia* (e.g., E. *coli*), *Enterobacter, Serratia, Proteus, Yersinia, Citrobacter, Edwardsiella, Providencia, Klebsiella, Hafnia, Ewingella,* Kluyvera, *Morganella, Planococcus, Stomatococcus, Micrococcus, Staphylococcus* (e.g., *S*. *aureus, S. epidermidis*), *Vibrio* (e.g., V. *cholerae*), *Aeromonas, Plessiomonas, Haemophilus* (e.g., *H. influenzae*), *Actinobacillus, Pasteurella, Mycoplasma* (e.g., *M. pneumonia*), *Ureaplasma, Rickettsia, Coxiella, Rochalimaea, Ehrlichia, Streptococcus* (e.g., *S*. *pyogenes, S*. *mutans, S. pneumoniae*), *Enterococcus* (e.g., *E. faecalis*), *Aerococcus, Gemella, Lactococcus* (e.g., *L. lactis*), *Leuconostoc* (e.g., *L. mesenteroides*), *Pedicoccus, Bacillus* (e.g., *B*. *cereus, B. subtilis, B. thuringiensis*), *Corynebacterium* (e.g., *C*. *diphtheriae*), *Arcanobacterium, Actinomyces, Rhodococcus, Listeria* (e.g., L. *monocytogenes*), *Erysipelothrix, Gardnerella, Neisseria* (e.g., *N. meningitidis, N. gonorrhoeae*), *Campylobacter, Arcobacter, Wolinella, Helicobacter* (e.g., *H. pylori*), *Achromobacter, Acinetobacter, Agrobacterium* (e.g., *A. tumefaciens*), *Alcaligenes, Chryseomonas, Comamonas, Eikenella, Flavimonas, Flavobacterium, Moraxella, Oligella, Pseudomonas* (e.g., *P. aeruginosa*), *Shewanella, Weeksella, Xanthomonas, Bordetella, Franciesella, Brucella, Legionella, Afipia, Bartonella, Calymmatobacterium, Cardiobacterium, Streptobacillus, Spirillum, Peptostreptococcus, Peptococcus, Sarcinia, Coprococcus, Ruminococcus, Propionibacterium, Mobiluncus, Bifidobacterium, Eubacterium, Lactobacillus* (e.g., L. *lactis, L. acidophilus*), *Rothia, Clostridium* (e.g., *C. botulinum, C. perfringens*), *Bacteroides, Porphyromonas, Prevotella, Fusobacterium, Bilophila, Leptotrichia, Wolinella, Acidaminococcus, Megasphaera, Veilonella, Norcardia, Actinomadura, Norcardiopsis, Streptomyces, Micropolysporas, Thermoactinomycetes, Mycobacterium* (e.g., *M. tuberculosis, M. bovis, M. leprae*), *Treponema, Borrelia* (e.g., *B. burgdorferi*), *Leptospira, and Chlamydiae.* A bacteria can optionally be characterized as a pest/pathogen of a plant or animal (e.g., human) in certain embodiments. Bacteria can be comprised in a mixed microbial population (e.g., containing other bacteria, or containing yeast and/or other bacteria) in certain embodiments.

An archaeal cell in certain embodiments can be from any Archaeal phylum, such as Euryarchaeota, Crenarchaeota, Nanoarchaeota, Korarchaeota, Aigarchaeota, or Thaumarchaeota. Archaeal cells herein can be extremophilic (e.g., able to grow and/or thrive in physically or geochemically extreme conditions that are detrimental to most life), for example. Some examples of extremophilic archaea include those that are thermophilic (e.g., can grow at temperatures between 45-122 °C), hyperthermophilic (e.g., can grow at temperatures between 80-122 °C), acidophilic (e.g., can grow at pH levels of 3 or below), alkaliphilic (e.g., can grow at pH levels of 9 or above), and/or halophilic (e.g., can grow in high salt concentrations [e.g., 20-30% NaCl]). Examples of archaeal species include those of the genera *Halobacterium* (e.g., *H. volcanii*), *Sulfolobus* (e.g., *S*. *solfataricus, S. acidocaldarius*), *Thermococcus* (e.g., *T. alcaliphilus, T. celer, T. chitonophagus, T. gammatolerans, T. hydrothermalis, T. kodakarensis, T. litoralis, T. peptonophilus, T. profundus, T. stetteri*), *Methanocaldococcus* (e.g., *M. thermolithotrophicus, M. jannaschii*), *Methanococcus* (e.g., *M. maripaludis*), *Methanothermobacter* (e.g., *M. marburgensis, M. thermautotrophicus*), *Archaeoglobus* (e.g., *A. fulgidus*), *Nitrosopumilus* (e.g., *N*. *maritimus*), *Metallosphaera* (e.g., *M. sedula*), *Ferroplasma, Thermoplasma, Methanobrevibacter* (e.g., *M. smithii*), and *Methanosphaera* (e.g., *M. stadtmanae*).

Mammalian cells can be human, non-human primate (e.g., monkey, ape), rodent (e.g., mouse, rat, hamster, guinea pig), rabbit, dog, cat, cow, pig, horse, goat, or sheep cells. Other examples of mammalian cells herein include primary epithelial cells (e.g., keratinocytes, cervical epithelial cells, bronchial epithelial cells, tracheal epithelial cells, kidney epithelial cells, retinal epithelial cells); established cell lines (e.g., 293 embryonic kidney cells, HeLa cervical epithelial cells, PER-C6 retinal cells, MDBK, CRFK, MDCK, CHO, BeWo, Chang cells, Detroit 562, Hep-2, KB, LS 180, LS 174T, NCI-H-548, RPMI 2650, SW-13, T24, WI-28 VA13, 2RA, WISH, BS-C-I, LLC-MK2, Clone M-3, RAG, TCMK-1, LLC-PK1, PK-15, GH1, GH3, L2, LLC-RC 256, MH1C1, XC, MDOK, VSW, TH-I, B1 cells); any epithelial, mesenchymal (e.g., fibroblast), neural, or muscular cell from any tissue or organ (e.g., skin, heart; liver; kidney; colon; intestine; esophagus; stomach; neural tissue such as brain or spinal cord; lung; vascular tissue; lymphoid tissue such as lymph gland, adenoid, tonsil, bone marrow, or blood; spleen); and fibroblast or fibroblast-like cell lines (e.g., TRG-2, IMR-33, Don cells, GHK-21, citrullinemia cells, Dempsey cells, Detroit 551, Detroit 510, Detroit 525, Detroit 529, Detroit 532, Detroit 539, Detroit 548, Detroit 573, HEL 299, IMR-90, MRC-5, WI-38, WI-26, MiCl1, CV-1, COS-1, COS-3, COS-7, Vero, DBS-FrhL-2, BALB/3T3, F9, SV-T2, M-MSV-BALB/3T3, K-BALB, BLO-11, NOR-10, C3H/IOTI/2, HSDM1C3, KLN205, McCoy cells, Mouse L cells, SCC-PSA1, Swiss/3T3 cells, Indian muntjac cells, SIRC, Jensen cells). Methods of culturing and manipulating mammalian cells lines are known in the art.

In certain embodiments, a cell can be of any pathogen and/or pest of an animal or plant. Examples of such pathogens/pests include various types of bacteria, fungi, yeast, protists, nematodes, and insects. Those skilled in the art would recognize examples of such pathogens/pests disclosed above.

A "centimorgan" (cM) or "map unit" is the distance between two linked genes, markers, target sites, loci, or any pair thereof, wherein 1% of the products of meiosis are recombinant. Thus, a centimorgan is equivalent to a distance equal to a 1% average recombination frequency between the two linked genes, markers, target sites, loci, or any pair thereof.

The guide RNA/Cas system described herein is especially useful for genome engineering, especially microbial and plant genome engineering, in circumstances where nuclease off-target cutting can be toxic to the targeted cells. In one embodiment of the guide RNA/Cas system described herein, an expression-optimized Cas9 gene, is stably integrated into the target genome, e.g. Yarrowia's genome. Expression of the Cas9 gene is under control of a promoter, e.g. a Yarrowia promoter,. In the absence of the guide RNA or crRNA, the Cas9 protein is not able to cut DNA and therefore its presence in the cell should have little or no consequence. Hence a key advantage of the guide RNA/Cas system described herein is the ability to create and maintain a cell line or organism capable of efficient expression of the Cas9 protein with little or no consequence to cell viability.

A guide RNA/Cas system mediating gene targeting can be used in methods for directing transgene insertion and / or for producing complex transgenic trait loci comprising multiple transgenes in a fashion similar as disclosed in WO2013/0198888 (published August 1, 2013) where instead of using a double strand break inducing agent to introduce a gene of interest, a guide RNA/Cas system as disclosed herein is used. A complex transgenic trait locus includes a genomic locus that has multiple transgenes genetically linked to each other. By inserting independent transgenes within 0.1, 0.2, 0.3, 0.4, 0.5 , 1.0, 2, or even 5 centimorgans (cM) from each other, the transgenes can be bred as a single genetic locus (see, for example, U.S. patent application 13/427,138) or PCT application PCT/US2012/030061.

Chromosomal intervals that correlate with a phenotype or trait of interest can be identified. A variety of methods well known in the art are available for identifying chromosomal intervals. The boundaries of such chromosomal intervals are drawn to encompass markers that will be linked to the gene controlling the trait of interest. In other words, the chromosomal interval is drawn such that any marker that lies within that interval (including the terminal markers that define the boundaries of the interval) can be used as a marker for northern leaf blight resistance. In one embodiment, the chromosomal interval comprises at least one QTL, and furthermore, may indeed comprise more than one QTL. Close proximity of multiple QTLs in the same interval may obfuscate the correlation of a particular marker with a particular QTL, as one marker may demonstrate linkage to more than one QTL. Conversely, e.g., if two markers in close proximity show co-segregation with the desired phenotypic trait, it is sometimes unclear if each of those markers identifies the same QTL or two different QTL. The term "quantitative trait locus" or "QTL" refers to a region of DNA that is associated with the differential expression of a quantitative phenotypic trait in at least one genetic background, e.g., in at least one breeding population. The region of the QTL encompasses or is closely linked to the gene or genes that affect the trait in question. An "allele of a QTL" can comprise multiple genes or other genetic factors within a contiguous genomic region or linkage group, such as a haplotype. An allele of a QTL can denote a haplotype within a specified window wherein said window is a contiguous genomic region that can be defined, and tracked, with a set of one or more polymorphic markers. A haplotype can be defined by the unique fingerprint of alleles at each marker within the specified window.

A variety of methods are available to identify those cells having an altered genome at or near a target site without using a screenable marker phenotype. Such methods can be viewed as directly analyzing a target sequence to detect any change in the target sequence, including but not limited to PCR methods, sequencing methods, nuclease digestion, Southern blots, and any combination thereof.

Proteins may be altered in various ways including amino acid substitutions, deletions, truncations, and insertions. Methods for such manipulations are generally known. For example, amino acid sequence variants of the protein(s) can be prepared by mutations in the DNA. Methods for mutagenesis and nucleotide sequence alterations include, for example, Kunkel, (1985) Proc. Natl. Acad. Sci. USA 82:488-92; Kunkel et a/., (1987) Meth Enzymol 154:367-82; U.S. Patent No. 4,873,192; Walker and Gaastra, eds. (1983) Techniques in Molecular Biology (MacMillan Publishing Company, New York) and the references cited therein. Guidance regarding amino acid substitutions not likely to affect biological activity of the protein is found, for example, in the model of Dayhoff et a/., (1978) Atlas of Protein Sequence and Structure (Natl Biomed Res Found, Washington, D.C.). Conservative substitutions, such as exchanging one amino acid with another having similar properties, may be preferable. Conservative deletions, insertions, and amino acid substitutions are not expected to produce radical changes in the characteristics of the protein, and the effect of any substitution, deletion, insertion, or combination thereof can be evaluated by routine screening assays. Assays for double-strand-break-inducing activity are known and generally measure the overall activity and specificity of the agent on DNA substrates containing target sites.

A variety of methods are known for the introduction of nucleotide sequences and polypeptides into an organism, including, for example, transformation, sexual crossing, and the introduction of the polypeptide, DNA, or mRNA into the cell.

Methods for contacting, providing, and/or introducing a composition into various organisms are known and include but are not limited to, stable transformation methods, transient transformation methods, virus-mediated methods, and sexual breeding. Stable transformation indicates that the introduced polynucleotide integrates into the genome of the organism and is capable of being inherited by progeny thereof. Transient transformation indicates that the introduced composition is only temporarily expressed or present in the organism.

Protocols for introducing polynucleotides and polypeptides into plants may vary depending on the type of plant or plant cell targeted for transformation, such as monocot or dicot. Suitable methods of introducing polynucleotides and polypeptides into plant cells and subsequent insertion into the plant genome include microinjection (Crossway et al., (1986) Biotechniques 4:320-34 and U.S. Patent No. 6,300,543), meristem transformation (U.S. Patent No. 5,736,369), electroporation (Riggs et al., (1986) Proc. Natl. Acad. Sci. USA 83:5602-6, *Agrobacterium*-mediated transformation (U.S. Patent Nos. 5,563,055 and 5,981,840), direct gene transfer (Paszkowski et al., (1984) EMBO J 3:2717-22), and ballistic particle acceleration (U.S. Patent Nos. 4,945,050; 5,879,918; 5,886,244; 5,932,782; Tomes et al., (1995) "Direct DNA Transfer into Intact Plant Cells via Microprojectile Bombardment" in Plant Cell, Tissue, and Organ Culture: Fundamental Methods, ed. Gamborg & Phillips (Springer-Verlag, Berlin); McCabe et al., (1988) Biotechnology 6:923-6; Weissinger et al., (1988) Ann Rev Genet 22:421-77; Sanford et al., (1987) Particulate Science and Technology 5:27-37 (onion); Christou et al., (1988) Plant Physiol 87:671-4 (soybean); Finer and McMullen, (1991) In Vitro Cell Dev Biol 27P:175-82 (soybean); Singh et al., (1998) Theor Appl Genet 96:319-24 (soybean); Datta et al., (1990) Biotechnology 8:736-40 (rice); Klein et al., (1988) Proc. Natl. Acad. Sci. USA 85:4305-9 (maize); Klein et al., (1988) Biotechnology 6:559-63 (maize); U.S. Patent Nos. 5,240,855; 5,322,783 and 5,324,646; Klein et al., (1988) Plant Physiol 91:440-4 (maize); Fromm et al., (1990) Biotechnology 8:833-9 (maize); Hooykaas-Van Slogteren et al., (1984) Nature 311:763-4; U.S. Patent No. 5,736,369 (cereals); Bytebier et al., (1987) Proc. Natl. Acad. Sci. USA 84:5345-9 (*Liliaceae*); De Wet et al., (1985) in The Experimental Manipulation of Ovule Tissues, ed. Chapman et al., (Longman, New York), pp. 197-209 (pollen); Kaeppler et al., (1990) Plant Cell Rep 9:415-8) and Kaeppler et al., (1992) Theor Appl Genet 84:560-6 (whisker-mediated transformation); D'Halluin et al., (1992) Plant Cell 4:1495-505 (electroporation); Li et al., (1993) Plant Cell Rep 12:250-5; Christou and Ford (1995) Annals Botany 75:407-13 (rice) and Osjoda et al., (1996) Nat Biotechnol 14:745-50 (maize via *Agrobacterium tumefaciens*).

Alternatively, polynucleotides may be introduced into plants by contacting plants with a virus or viral nucleic acids. Generally, such methods involve incorporating a polynucleotide within a viral DNA or RNA molecule. In some examples a polypeptide of interest may be initially synthesized as part of a viral polyprotein, which is later processed by proteolysis *in vivo* or *in vitro* to produce the desired recombinant protein. Methods for introducing polynucleotides into plants and expressing a protein encoded therein, involving viral DNA or RNA molecules, are known, see, for example, U.S. Patent Nos. 5,889,191, 5,889,190, 5,866,785, 5,589,367 and 5,316,931. Transient transformation methods include, but are not limited to, the introduction of polypeptides, such as a double-strand break inducing agent, directly into the organism, the introduction of polynucleotides such as DNA and/or RNA polynucleotides, and the introduction of the RNA transcript, such as an mRNA encoding a double-strand break inducing agent, into the organism. Such methods include, for example, microinjection or particle bombardment. See, for example Crossway et al., (1986) Mol Gen Genet 202:179-85; Nomura et al., (1986) Plant Sci 44:53-8; Hepler et al., (1994) Proc. Natl. Acad. Sci. USA 91:2176-80; and, Hush et al., (1994) J Cell Sci 107:775-84.

The term "dicot" refers to the subclass of angiosperm plants also knows as "dicotyledoneae" and includes reference to whole plants, plant organs (e.g., leaves, stems, roots, etc.), seeds, plant cells, and progeny of the same. Plant cell, as used herein includes, without limitation, seeds, suspension cultures, embryos, meristematic regions, callus tissue, leaves, roots, shoots, gametophytes, sporophytes, pollen, and microspores.

The term "crossed" or "cross" or "crossing" in the context of this disclosure means the fusion of gametes via pollination to produce progeny (i.e., cells, seeds, or plants). The term encompasses both sexual crosses (the pollination of one plant by another) and selfing (self-pollination, i.e., when the pollen and ovule (or microspores and megaspores) are from the same plant or genetically identical plants).

The term "introgression" refers to the transmission of a desired allele of a genetic locus from one genetic background to another. For example, introgression of a desired allele at a specified locus can be transmitted to at least one progeny plant via a sexual cross between two parent plants, where at least one of the parent plants has the desired allele within its genome. Alternatively, for example, transmission of an allele can occur by recombination between two donor genomes, e.g., in a fused protoplast, where at least one of the donor protoplasts has the desired allele in its genome. The desired allele can be, e.g., a transgene, a modified (mutated or edited) native allele, or a selected allele of a marker or QTL.

Standard DNA isolation, purification, molecular cloning, vector construction, and verification/characterization methods are well established, see, for example Sambrook et al., (1989) Molecular Cloning: A Laboratory Manual, (Cold Spring Harbor Laboratory Press, NY). Vectors and constructs include circular plasmids, and linear polynucleotides, comprising a polynucleotide of interest and optionally other components including linkers, adapters, regulatory or analysis. In some examples a recognition site and/or target site can be contained within an intron, coding sequence, 5' UTRs, 3' UTRs, and/or regulatory regions.

Any plant can be used, including monocot and dicot plants. Examples of monocot plants that can be used include, but are not limited to, corn (Zea *mays*), rice (*Oryza sativa*), rye (*Secale cereale*), sorghum (*Sorghum bicolor, Sorghum vulgare*), millet (e.g., pearl millet (*Pennisetum glaucum*), proso millet (*Panicum miliaceum*), foxtail millet (*Setaria italica*), finger millet (*Eleusine coracana*)), wheat (*Triticum aestivum*), sugarcane (*Saccharum spp.*), oats (*Avena*), barley (*Hordeum*), switchgrass (*Panicum virgatum*), pineapple (*Ananas comosus*), banana (*Musa spp*.), palm, ornamentals, turfgrasses, and other grasses. Examples of dicot plants that can be used include, but are not limited to, soybean (*Glycine max*), canola (*Brassica napus* and *B. campestris*), alfalfa (*Medicago sativa*), tobacco (*Nicotiana tabacum*), *Arabidopsis* (*Arabidopsis thaliana*), sunflower (*Helianthus annuus*), cotton (*Gossypium arboreum*), and peanut (*Arachis hypogaea*), tomato (*Solanum lycopersicum*), potato (*Solanum tuberosum*) *etc.*

The meaning of abbreviations is as follows: "sec" means second(s), "min" means minute(s), "h" means hour(s), "d" means day(s), "µL" means microliter(s), "mL" means milliliter(s), "L" means liter(s), "µM" means micromolar, "mM" means millimolar, "M" means molar, "mmol" means millimole(s), "µmole" mean micromole(s), "g" means gram(s), "µg" means microgram(s), "ng" means nanogram(s), "U" means unit(s), "bp" means base pair(s) and "kb" means kilobase(s).

### EXAMPLES

In the following Examples, unless otherwise stated, parts and percentages are by weight and degrees are Celsius. It should be understood that these Examples, while indicating embodiments of the disclosure, are given by way of illustration only. From the above discussion and these Examples, one skilled in the art can make various changes and modifications of the disclosure to adapt it to various usages and conditions. Such modifications are also intended to fall within the scope of the appended claims.

### EXAMPLE 1

### Cas9 HDV-gRNA expression plasmid targeting Can1.

This example discusses the use of single guide RNAs (sgRNAs) that are flanked on the 5' end by a hepatitis delta virus (HDV) ribozyme. The HDV ribozyme cleaves 5' of its own sequence removing any preceding RNA sequence but leaving the HDV sequence fused to the 5' end of the gRNA.

In order to test a sgRNA/Cas endonuclease system in *Yarrowia,* the Cas9 gene from *Streptococcus pyogenes* M1 GAS (SF370 (SEQ ID NO: 1) was *Yarrowia* codon optimized per standard techniques known in the art (SEQ ID NO: 2). In order to localize the Cas9 protein to the nucleus of the cells, *Simian virus 40* (SV40) monopartite (PKKKRKV, SEQ ID NO: 3) nuclear localization signal was incorporated at the carboxy terminus of the Cas9 protein. The Yarrowia codon optimized Cas9 gene was fused to a Yarrowia constitutive promoter, FBA1 (SEQ ID NO: 4), by standard molecular biology techniques. An example of a Yarrowia codon optimized Cas9 expression cassette containing the FBA1 promoter and the Yarrowia optimized Cas9-NLS fusion is shown in SEQ ID NO: 5. The Cas9 expression cassette was cloned into the plasmid pZuf resulting in pZufCas9 (SEQ ID NO 6).

Plasmid pZuf-Cas9CS (SEQ ID NO: 6) was mutagenized using Agilent QuickChange and the following primers Aarl-removal-1 (AGAAGTATCCTACCATCTACcatctccGAAAGAAACTCGTCGATTCC, SEQ ID NO: 7) and Aarl-removal-2 (GGAATCGACGAGTTTCTTTCggagatgGTAGATGGTAGGATACTTCT, SEQ ID NO: 8) to remove the endogenous Aarl site present in the *Yarrowia* codon optimized Cas9 gene (SEQ ID NO: 2) present in pZuf-Cas9CS (SEQ ID NO: 6) generating pRF109 (SEQ ID NO: 9). The modified Aar1-Cas9CS gene (SEQ ID NO: 10) was cloned as a Ncol/Notl fragment from pRF109 (SEQ ID NO: 9) into the Ncol/Notl site of pZufCas9CS (SEQU ID NO: 6) replacing the existing Cas9 gene (SEQ ID NO: 2) with the Aar1- Cas9 gene (SEQ ID NO: 10) generating pRF141 (SEQ ID NO: 11).

The high throughput variable targeting domain (VT) cloning cassette (Figure 1, SEQ ID NO: 12) is composed of the yl52 promoter (SEQ ID NO: 13), the DNA sequence encoding the HDV ribozyme (SEQ ID NO: 14), the *Escherichia coli* counterselection cassette *rpsL* (SEQ ID NO: 15), the DNA encoding the Cas9 CER domain (SEQ ID NO: 16) and the *S*. *cerevisiae SUP4* terminator (SEQ ID NO: 17). Flanking the ends of the high-throughput cloning cassette (SEQ ID NO: 12) are Pacl and Clal restriction enzyme recognition sites. The high-throughput cloning cassette (SEQ ID NO: 12) was cloned into the Pacl/Clal sites of pRF141 (SEQ ID NO: 11) to generate pRF291 (SEQ ID NO 14). The *rpsL* counterselection cassette (SEQ ID NO: 15) contains a WT copy of the E. *coli rpsL* gene with its native promoter and terminator. *rpsL* encodes the S12 ribosomal protein subunit (Escherichia coli and Salmonella typhimurium: Cellular and Molecular Biology, 1987 American Society of Microbiology). Some mutations in the S12 subunit cause resistance to the antibiotic streptomycin (Ozaki, M., et al. (1969). "Identification and functional characterization of the protein controlled by the streptomycin-resistant locus in E. coli." Nature 222(5191): 333-339). in a recessive manner (Lederberg, J. (1951). "Streptomycin resistance; a genetically recessive mutation." J Bacteriol 61(5): 549-550.) such that if a wild-type copy of the *rpsL* gene is present the strain is phenotypically sensitive to streptomycin Lederberg, J. (1951). "Streptomycin resistance; a genetically recessive mutation." J Bacteriol 61(5): 549-550.). Common cloning strains such as Top10 (Life technologies) have a mutated copy of *rpsL* on their chromosome such that the cells are resistant to streptomycin.

Cloning a variable targeting domain into pRF291 requires two partially complimentary oligonucleotides that when annealed they contain the desired variable targeting domain as well as the correct overhangs for cloning into the two Aarl sites present in the high-throughput cloning cassette. Two oligonucleotides, Can1-1F ( AATGGGACtcaaacgattacccaccctcGTTT, SEQ ID NO: 19) and Can1-1R (TCTAAAACgagggtgggtaatcgtttgaGTCC, SEQ ID NO: 20) containing the DNA encoding the variable targeting domain Can1-1 (SEQ ID NO: 21) which targets the Can1-1 target site (SEQ ID NO: 22) in the *CAN1* gene of *Yarrowia lipolytica* (SEQ ID NO: 23), were resuspended in duplex buffer (30 mM HEPES pH 7.5, 100 mM Sodium Acetate) at 100 µM. Can1-1F (SEQ ID NO: 19) and Can1-1R (SEQ ID NO: 20) were mixed at a final concentration of 50µM each in a single tube, heated to 95°C for 5 minutes and cooled to 25°C at 0.1°C/min to anneal the two oligonucleotides to form a small duplex DNA molecule (Figure 2). A single tube digestion/ligation reaction was created containing 50 ng of pRF291, 2.5 µM of the small duplex DNA composed of Can1-1F (SEQ ID NO: 19) and Can1-1R (SEQ ID NO: 20), 1× T4 ligase buffer (50 mM Tris-HCI, 10 mM MgCl2, 1 mM ATP, 10 mM DTT pH 7.5), 0.5 µM Aarl oligonucleotide, 2 units Aarl, 40 units T4 DNA ligase in a 20 µl final volume. A second control reaction lacking the duplexed Can1-1F and Can1-1R duplex was also assembled. The reactions were incubated at 37°C for 30 minutes. 10 µl of each reaction was transformed into Top10 E. *coli* cells as previously described (Green, M. R. & Sambrook, J. Molecular Cloning: A Laboratory Manual. Fourth Edition edn, (Cold Spring Harbor Laboratory Press, 2012)). In order to select for the presence of pRF291 where the duplex of Can1-1F (SEQ ID NO: 19) and Can1-1R (SEQ ID NO: 20) had replaced the *rpsL* counterselection marker flanked by Aarl restriction sites (Figure 1) cells were plated on lysogeny broth solidified with 1.5% (w/v) Bacto agar containing 100µg/ml Ampicillin and 50µg/ml Streptomycin. The presence of pRF291 containing the high-throughput cloning cassette yielded colonies phenotypically resistant to the antibiotic ampicillin but sensitive to the antibiotic streptomycin due to the presence of the counterselection cassette on the plasmid. However, in cases where the counterselection cassette was removed via the Aarl enzyme and the Can1-1 variable targeting domain containing duplex DNA was ligated into the site (removing the recognition sequences for Aarl) the cells transformed with the plasmid had an ampicillin resistant, streptomycin resistant phenotype (Figure 1). pRF291 containing the Can1-1 variable targeting domain replacing the counterselection cassette created a recombinant Can1-1 gRNA expression cassette (SEQ ID NO: 19) containing the yl52 promoter (SEQ ID NO: 13) fused to the DNA encoding the HDV ribozyme (SEQ ID NO: 14) fused to the DNA encoding the Can1-1 variable targeting domain (SEQ ID NO: 21) fused to the DNA encoding the CER domain (SEQ ID NO: 16) fused to the *SUP4* terminator (SEQ ID NO: 17). The plasmid containing this construct, pRF303 (SEQ ID NO: 24) was used to target the *CAN1* gene (SEQ ID NO: 23) of *Yarrowia lipolytica* with Cas9.

### EXAMPLE 2

### Generation of protected polynucleotide modification templates.

During the repair of Cas9/gRNA generated DNA double-stranded breaks (DSBs) the preference for most eukaryotic cell types to use the non-homologous end-joining pathway for the repair of double-stranded DNA breaks (DSBs) typically yields a large background of NHEJ derived mutations (indels) with few colonies using the proteins of homology-directed recombination (HDR) to repair the break. This means in the typically gene editing experiment using and editing template to make a templated change at a DSB many events must be screened to find events where the Cas9/gRNA generated DSB was repaired using HDR. The use of protected polynucleotide editing templates provides a method of increasing the frequency of HDR repair of Cas9/gRNA generated DSBs decreasing the number of events that must be screened to find an event with the desired edit. This example describes the generation of three different types of polynucleotide modification template molecules that have modified ends that renders them less prone to degradation, referred to as "protected polynucleotide modification templates". These protected polynucleotide modification templates can potentially alter template stability by protecting the template from exonuclease activity within the cell and/or alter the ability of the template to act as a substrate for non-homologous end-joining (NHEJ). If the desired gene editing event is a deletion of the region between two different homology arms (Figure 3A) the editing template will contain the two homology arms linked with no intervening sequence (Figure 3B) The three types of protected polynucleotide modification templates are 1) a circular DNA template with no available double strand ends (Figure 3D) a linear double stranded DNA template modified with a three carbon alkane spacer on the 5' end of each strand (Figure 3C), and 3) a linear double stranded DNA template with the five 5' most phophodiester bonds replaced with phosphorothioate bonds on each strand (Figure 3C).

A unprotected (unmodified) polynucleotide modification template was generated by making two PCR products, one, the 629 bp ending 2 bp 5' of the CAN1 open reading frame (SEQ ID NO: 25) which was amplified from *Yarrowia lipolytica* ATCC20362 genomic DNA using standard techniques (primers used, GGGAAGCTTGCTACGTTAGGAGAAGACGC (forward, SEQ ID NO: 26) and GGAGAGAGCGTCGGGAGTGGTCGGATGGATGGAGACG (reverse, SEQ ID NO:27)). The reverse primer adds 17 nucleotides complementary to the sequence 37 bp 3' of the *CAN1* open reading frame and the forward primer adds a 5' HinDIII recognition site. The second PCR product, consisting of 637 bp starting 37 basepairs 3' of the CAN1 open-reading frame (SEQ ID NO: 28). This PCR product was amplified from *Yarrowia lipolytica* ATCC20362 genomic DNA using standard techniques (primers used, CGTCTCCATCCATCCGACCACTCCCGACGCTCTCTCC (forward, SEQ ID NO: 29) and CCATACATCCTTCCACCACTGC (reverse, SEQ ID NO: 30)). The forward primer adds the 20 nucleotides complementary to the region ending 2 bp 5' of the *CAN1* open reading frame. Both the upstream (SEQ ID NO: 25) and the downstream PCR product (SEQ ID NO:28) were purified using Zymo clean and concentrate columns. 10ng of each PCR product were mixed in a new PCR reaction. The 3' 37 nucleotides of the upstream product is identical to the 5' 37 nucleotides of the downstream product. The upstream and downstream fragments were used to prime each other creating a single product representing the unprotected polynucleotide modification template (SEQ ID NO: 31) by synthesis from overlapping ends containing both the upstream and downstream sequences (Horton et al (2013) Biotechniques 54(3):129-133) (Figure 3B). The complete non-protected (control) polynucleotide modification template was digested with HinDIII and cloned into the HinDIII site of pUC18 (SEQ ID NO: 32) using standard techniques generating plasmid pRF80 (SEQ ID NO: 33). The plasmid pRF80 represents a double stranded circular protected polynucleotide modification template (Figure 3D) comprising a 1210 bp DNA fragment (SEQ ID NO: 34) that when used as a template for homology directed repair (HDR) will lead to the deletion of the entire *CAN1* open reading frame.

Linear protected polynucleotide modification templates were generated from the cloned template contained on pRF80 (SEQ ID NO: 33). The template contained on pRF80 was PCR amplified using standard techniques and chemically synthesized, modified oligonucleotides as primers (IDT). To generate a linear protected polynucleotide modification template with the 5' ends modified with an alkane spacer the template from pRF80 was amplified (primers used /5SpC3/AGCTTGCTACGTTAGGAGAA, forward (SEQ ID NO: 35) and /5SpC3/TATGAGCTTATCCTGTATCG, reverse (SEQ ID NO:36)) to generate a 1215bp PCR containing the *CAN1* ORF deletion template (SEQ ID NO: 34) with 5' alkane spacers on the 5' end of each strand. A second linear protected polynucleotide modification template was generated in essentially the same manner using pRF80 (SEQ ID NO: 33) as a PCR template. The linear template was amplified with chemically synthesized primers (IDT) in which the five 5' most phosphodiester bonds have been replaced with phosphorothioate bonds (*) (primers used; A*G*C*T*T*GCTACGTTAGGAGAA, forward (SEQ ID NO: 37) and T*A*T*G*A*GCTTATCCTGTATCG, reverse (SEQ ID NO: 38)). The resulting product is the 1215bp *CAN1* deletion protected polynucleotide modification template (SEQ ID NO: 31) containing five phosphorothioate bonds at the 5' most ends of each strand. An unmodified (unprotected, control) template was amplified from pRF80 using chemically synthesized oligonucleotide primers with no modifications and standard techniques (primers used, AGCTTGCTACGTTAGGAGAA, forward (SEQ ID NO: 40) and TATGAGCTTATCCTGTATCG, reverse (SEQ ID NO: 41) to yield an unprotected 1215bp linear *CAN1* deletion polynucleotide modification template (editing template) (SEQ ID NO: 31). PCR reactions of linear templates were purified using Zymo clean and concentrate 25 columns and eluted in 25µl of 10mM Tris 1mM EDTA pH8.0.

### EXAMPLE 3

### Precise gene editing using protected polynucleotide modification templates in combination with Cas9/gRNA targeting

In this example *Yarrowia* lipolytica cells were transformed with targeting plasmids in the presence and absence of protected and unprotected (unmodified) polynucleotide modification templates to determine the effect of using a protected template (instead of an unprotected (control) template) on HDR and NHEJ frequency. Increases in HDR frequencies and the simultaneous decrease in NHEJ frequency would greatly decrease the NHEJ-derived background present in typical Cas9/gRNA gene editing experiments. Cells were phenotypically scored for Canavanine resistance to determine overall targeting efficiency (representative of NHEJ frequency + HDR frequency). Colony PCR of the *CAN1* locus (SEQ ID NO: 39) was performed to determine frequency of repair of the Cas9/gRNA generated double-strand break by HDR and NHEJ.

A *uracil* auxotrophic strain of *Yarrowia lipolytica* ATCC20362 was grown for 24 hours on YPD medium plates (Teknova) at 30°C. 1 loop of cells were resuspended in transformation buffer (35% polyethylene glycol average molecular weight of 3550, 100 mM lithium acetate, 100 mM dithiothreitol, 10 mM Tris, 1mM EDTA pH 6.0). 100 µl of cell suspension was mixed with 100 ng of either pRF291 (SEQ ID NO:18) (Cas9 expression, no gRNA) or pRF303 (SEQ ID NO: 24) (Cas9 expression, Can1-1 gRNA expression) with either no polynucleotide modification template, 1 µg unprotected linear polynucleotide modification template (SEQ ID NO: 31), 1 µg C3S protected linear polynucleotide modification template (template comprising a 3 carbon alkaline spacer at the 5' end of SEQ ID NO: 31), 1µg PT protected linear polynucleotide modification template (template comprising a phosphorothiate bond at the first five 5' nucleotides of SEQ ID NO: 31) or 5 µg of a circular protected polynucleotide modification template , pRF80 (SEQ ID NO 33) . Transformation mixtures were incubated at 39°C for 1 hour at 800 RPM. Transformation mixtures were plated on complete minimal medium plates lacking uracil (Teknova) to select for cells transformed with plasmid DNA. Plates were incubated at 30°C for 48 hours. 24 colonies from each transformation were streak purified on complete minimal plates lacking uracil (Teknova) for single colonies. 4 single colonies from each streak purified colony (96 for each transformation) were patched to complete minimal plates lacking arginine containing 60µg/ml L-canavanine. L-canavanine is toxic to cells with a functional *CAN1* gene which is an importer of arginine and L-canavanine to the cells. Cells containing a loss of function allele in the *CAN1* gene will be phenotypically resistant to the presence of L-canavanine in the medium and will form colonies on plates containing L-canavanine. Cells containing a wild-type copy of the *CAN1* gene will be unable to grow on medium containing L-canavanine. The mode of action of L-canavanine is well known (Rosenthal G.A., The Biological effects and mode of action of L-Canavanine, a structural analog of L-arginine, The quarterly review of biology, volume 52, 1977, 155-178). The frequency of Canavanine resistance by transformation treatment is given in Table 2.

**Table 2: Canavanine resistance by transformation treatment.**

| Plasm id | Polynucleotide modification template | Canavanine resistance frequency ± Standard deviation¹ |
|---|---|---|
| pRF291 | None | 0±0 |
| pRF291 | unprotected linear (control) | 0±0 |
| pRF291 | C3S linear protected | 0.005±0.008 |
| pRF291 | PT linear protected | 0±0 |
| pRF291 | pRF80 circular protected | 0±0 |
| pRF303 | None | 0.80 ± 0.10 |
| pRF303 | unprotected linear (control) | 0.72 ± 0.11 |
| pRF303 | C3S linear protected | 0.78 ± 0.18 |
| pRF303 | PT linear protected | 0.68 ± 0.13 |
| pRF303 | pRF80 circular protected | 0.74 ± 0.13 |

| | | |
|---|---|---|
| ¹Results represent the average and standard deviation for at least two independent repeats in each case Each experimental repeat screened a total of 80 colonies. | | |

Cells transformed with pRF291 (SEQ ID NO: 18) which carries a Cas9 expression cassette but lacks a functional gRNA targeting the *CAN1* gene did not give rise to Canavanine resistance cells with the exception of a single instance of a canavanine resistant colony in a single experiment with the C3S polynucleotide modification template (Table 2). Cells transformed with pRF303 (SEQ ID NO: 24) in the presence or absence of unprotected or protected polynucleotide modification templates gave similar frequencies of canavanine resistant colonies (Table 2) suggesting that the presence of a polynucleotide modification template in the transformation mix did not alter the ability of Cas9/gRNA to induced targeted double strand breaks.

To determine the frequency of homologous directed repair (HDR) and NHEJ at the targeted double-strand breaks generated by Cas9/gRNA at the Can1-1 target site in the presence of unprotected (control) or protected polynucleotide modification protected modification templates Yarrowia colony PCR of the *CAN1* locus (SEQ ID NO: 44) (primers used, GGAAGGCACATATGGCAAGG, forward (SEQ ID NO: 42) and GTAAGAGTGGTTTGCTCCAGG, reverse (SEQ ID NO: 43)) was performed using standard techniques. If the *CAN1* locus was not modified or contained a small indel generated by NHEJ the colony PCR result would give a band similar in size to the WT *CAN1* locus at an apparent size of 2125 bp (SEQ ID NO: 44). If the Cas9/gRNA generated double-strand break had been repaired using the unprotected or protected modification templates, the PCR would generate a smaller *CAN1* locus product 392bp indicating the deletion of the entire open-reading frame (SEQ ID NO: 45). Colony PCR was performed on all canavanine resistant colonies from cells transformed with pRF303 in the presence or absence of the template and the fraction of cells where the Cas9/gRNA generated double-strand break was repaired via HDR or NHEJ with the polynucleotide modification template was determined (Table 3).

**Table 3: Frequency of HDR of Cas9/gRNA generated double strand break by polynucleotide modification template type.**

| Polynucleotide modification template | HDR frequency ± Standard deviation² | NHEJ Frequency ± Standard deviation² | Ratio of HDR in protected vs unprotected template |
|---|---|---|---|
| None | 0.00 ± 0.00 | 1.00±0.00 | - |
| unprotected linear | 0.09 ± 0.02 | 0.91±0.06 | - |
| C3S protected linear | 0.08 ± 0.03 | 0.92±0.06 | 1.0 |
| PT protected linear | 0.19 ± 0.05 | 0.81±0.12 | 2.1 |
| pRF80 protected circular | 0.13 ± 0.04 | 0.88±0.06 | 1.4 |

| | | | |
|---|---|---|---|
| ²Results represent the average and standard error of the mean for at least two independent repeats in each case. Each experimental repeat screened a total of 80 colonies. | | | |

Cells treated with unprotected polynucleotide modification template or the C3S protected linear template had similar frequencies of repair of the Cas9/gRNA generated DSB by HDR (Table 3). Cells treated with the PT linear protected modification polynucleotide template or the pRF80 circular protected modification polynucleotide modification template had HDR frequencies of the Cas9/gRNA generated DSBs 2.1 fold and 1.4 fold higher than the unprotected (control) linear polynucleotide modification template, respectively. In the repair of Cas9/gRNA generated double-stranded breaks linear protected templates with 5' phosphorothioate modifications or circular, non-replicating protected templates provide substantial increases in the fraction of breaks repaired via HDR with 200% and 140% of the frequency of unprotected linear polynucleotide modification templates. NHEJ is the predominate DNA DSB repair pathway in most eukaryotic cells including *Yarrowia lipolytica.* The use of protected DNA modification templates increases the frequency of HDR repair of Cas9/gRNA generated DSBs by as much as 2 fold, allowing for screening a smaller number of events to find the correct, template repair of the DSB.

An additional complication of repair of Cas9/gRNA generated double-stranded breaks using polynucleotide modification templates is the possibility that the template can be incorporated by the NHEJ pathway at other regions of DNA damage resulting in off-site integration. In order to determine the frequency at which this off-site integration occurs in the cells treated with polynucleotide modification templates, relative copy number analysis was performed looking for a 62bp fragment of the polynucleotide modification template (SEQ ID NO: 46). Relative copy number analysis was performed on colonies from cells treated with pRF303 (SEQ ID NO: 24) and a linear control polynucleotide modification polynucleotide (SEQ ID NO: 34), the linear protected polynucleotide modification template, PT (SEQ ID NO: 34), and the circular protected polynucleotide modification template, pRF80 (SEQ ID NO: 33). If the polynucleotide modification template is only incorporated during the HDR of the Cas9/gRNA double strand break at the *CAN1* locus (SEQ ID NO: 39) the cell will only carry a single copy of the copy number analysis fragment (SEQ ID NO: 46). However, if the cell incorporates additional copies of the polynucleotide modification template elsewhere in the genome due to the activity of the NHEJ pathway, additional copies of the fragment will be present and the cell will return a higher relative copy number. Briefly, genomic DNA was isolate from colonies that scored positive for HDR of the Can1-1 Cas9/gRNA targeted double strand break using standard techniques. 1 µl of the genomic DNA from each colony was added to three replicate qPCR reactions for both the *CAN1* locus (SEQ ID NO: 46), (primers used, AGCGCCAAACCCAAAGC, forward (SEQ ID NO: 47), CTTGCCATATGTGCCTTCCA, reverse (SEQ ID NO: 48), and 6FAM-CTTTTCGCCCCCACTGCAGCC-TAMRA, probe (SEQ ID NO: 49)) or as a control the *TEF1* locus (SEQ ID NO: 50) (primers used, CGACTGTGCCATCCTCATCA, forward (SEQ ID NO: 51), TGACCGTCCTTGGAGATACCA, reverse (SEQ ID NO: 52) and 6FAM-TGCTGGTGGTGTTGGTGAGTT-TAMRA, probe (SEQ ID NO: 53)). Reactions were run in TaqMAN universal PCR master mix (ABI life technologies) on a life technologies Quant Studio 7 instrument using the following cycling conditions 95°C for 10 minutes followed by 40 cycles of 95°C for 15 seconds, 60°C for 1 minute. 6FAM fluorescence from the probes was monitored through the 40 cycles of the PCR and Ct values were collected. Relative gene copy number was determined by the ΔΔCt method (User Bulletin #2 ABI PRISM 7700 Sequence Detection System (Updated 2001)). Briefly, TEF1 Ct values were used to normalize the data for differences in cell copy number between genomic DNA samples. Genomic DNA from a wildtype strain was used as a reference for the relative quantification for the *CAN1* copy number fragment (SEQ ID NO: 46). Software on the Quant studio 7 calculated relative gene copy number and corresponding error for each sample relative to the wildtype strain. The colonies were separated into two bins, those with less than 2 relative copies and those with 2 or more relative copies. The first bin indicates that the polynucleotide modification template was only used for HDR repair of the Cas9/gRNA generated double strand break at the Can1-1 target site (SEQ ID NO: 22) and was not integrated elsewhere in the genome by NHEJ. The second bin represents cells where the polynucleotide modification template was used to repair the Cas9/gRNA generated double-strand break at the Can1-1 target site (SEQ ID NO: 22) and was integrated at least once somewhere else in the genome via a NHEJ mechanism. The results of the copy number analysis are presented in Table 4.

**Table 4: Copy number analysis of the CAN1 locus among colonies the HDR repair of the Cas9/gRNA generated D**

| Editing template | Fraction of cells with single copies of CAN1 (%) | Fraction of cells with >2 copies of CAN1 (%) |
|---|---|---|
| unprotected linear | 63 | 37 |
| PT protected linear | 60 | 40 |
| pRF80 protected circular | 100 | 0 |

Copy number analysis was performed using a qPCR target that was present both in the CAN1 locus of Yarrowia lipolytica as well as present on all polynucleotide modification templates (protected and unprotected). If the polynucleotide modification template was used for HDR of the CAN1 locus in the cells, the copy number of the target will remain 1. If the polynucleotide editing template was also inserted elsewhere in the Yarrowia genome the copy number will be at least 2 indicating the copy present in the CAN1 locus and the copy of the polynucleotide editing template inserted by NHEJ elsewhere in the genome.

Both the unprotected linear polynucleotide modification template and the PT template yield approximately 60% of colonies with a single copy of the *CAN1* polynucleotide modification template indicating that the polynucleotide modification template was used for HDR of the Cas9/gRNA generated DSB but did not integrate in the genome Table 4). The circular protected polynucleotide modification template, pRF80, demonstrated 100 percent of the colonies with only a single copy of the *CAN1* locus indicating that the circular template was only used for HDR of the Cas9/gRNA generated break at Can1-1 and was not integrated elsewhere in the chromosome.

The protected polynucleotide modification templates gave superior results compared to unprotected polynucleotide modification templates in surprising ways. Linear protected templates in which the five 5' phosphodiester bonds on each strand have been replaced with phosphorothioate bonds give more than twice as many colonies with the Cas9/gRNA generated DSB repaired via HDR than an unprotected template without causing a change in the integration of the linear template elsewhere in the chromosome. The protected polynucleotide modification template which is circular instead of linear gave a 40 percent improvement in the frequency of HDR of the Cas9/gRNA generated double strand break at Can1-1 (Table 3) and gave a 60 percent improvement in the number of colonies with no off-site integration of the polynucleotide modification template (Table 4).

### EXAMPLE 4

### Precise genome editing using protected polynucleotide modification templates contained on the Cas9/sgRNA plasmid

In this example the URA3 gene of *Yarrowia lipolytica* is targeted for precise genome editing using a protected polynucleotide editing template that is part of a circular DNA molecule containing the Cas9 expression cassette and the sgRNA expression cassette.

Plasmid pRF434 (SEQ ID NO: 54) was constructed by replacing the *URA3* selectable marker present in pRF291 (SEQ ID NO: 18) between the Pacl and Pmel restriction sites with a hygromycin resistance expression cassette (SEQ ID NO: 55). This plasmid allows the high throughput cloning of variable targeting domains in the same fashion as pRF291 (Figure 1). Within the *Yarrowia lipolytica URA3* locus (SEQ ID NO: 56) exists the target site Ura3-1 (SEQ ID NO: 57). Two oligos Ura3-1F (SEQ ID NO: 58) and URA3-1R (SEQ ID NO: 59) containing the DNA encoding the variable targeting domain corresponding to the Ura3-1 target site (SEQ ID NO: 57) were resuspended in duplex buffer (30 mM HEPES pH 7.5, 100 mM Sodium Acetate) at 100 µM. Ura3-1F (SEQ ID NO: 58) and Ura3-1R (SEQ ID NO: 59) were mixed at a final concentration of 50µM each in a single tube, heated to 95°C for 5 minutes and cooled to 25°C at 0.1°C/min to anneal the two nucleotides to form a small duplex DNA molecule. A single tube digestion/ligation reaction was created containing 50 ng of pRF434, 2.5 µM of the small duplex DNA composed of Ura3-1F (SEQ ID NO: 58) and Ura3-1R (SEQ ID NO: 59), 1× T4 ligase buffer (50mM Tris-HCl, 10 mM MgCl₂, 1 mM ATP, 10 mM DTT pH 7.5), 0.5 µM Aarl oligonucleotide, 2 units Aarl, 40 units T4 DNA ligase in a final volume of 20µl. A control reaction lacked the small DNA duplex of Ura3-1F (SEQ ID NO: 58) and URA3-1R (SEQ ID NO: 59). The reactions were incubated at 37°C for 1 hour and then transformed into Top10 E. *coli* cells as previously described (Green, M. R. & Sambrook, J. Molecular Cloning: A Laboratory Manual. Fourth Edition edn, (Cold Spring Harbor Laboratory Press, 2012)). In order to select for the presence of pRF434 where the duplex of Ura3-1F (SEQ ID NO: 58) and Ura3-1R (SEQ ID NO: 59) had replaced the *rpsL* counterselection marker flanked by Aarl restriction sites (Figure 1) cells were plated on lysogeny broth solidified with 1.5% (w/v) Bacto agar containing 100 µg/ml Ampicillin and 50 µg/ml streptomycin. The presence of pRF434 (SEQ ID NO: 54) containing the high-throughput cloning cassette yielded colonies phenotypically resistant to ampicillin but sensitive to streptomycin due to the counterselection cassette and do not form colonies in the presence of streptomycin. However, in cases where the counterselection cassette was removed via the Aarl enzyme and the Ura3-1 duplex DNA was ligated into the site (removing the Aarl recognition sites) the transformed cells have an ampicillin-resistant, streptomycin-resistant phenotype and form colonies in the presence of ampicillin and streptomycin. pRF434 (SEQ ID NO: 54) containing the DNA encoding the Ura3-1 variable targeting domain in the Aarl sites creates a recombinant HDV-sgRNA expression cassette containing the yl52 promoter (SEQ ID NO: 13) fused to the DNA encoding the HDV ribozyme (SEQ ID NO: 14) fused to the DNA encoding the Ura3-1 VT domain (SEQ ID NO: 60) fused to the DNA encoding the CER domain (SEQ ID NO: 16), fused to the *SUP4* terminator (SEQ ID NO 17). The plasmid containing this construct, pRF421 (SEQ ID NO: 61) was used to target the URA3 locus (SEQ ID NO: 56) of *Yarrowia lipolytica.*

In order to construct a protected polynucleotide editing template targeting the *URA3* locus (SEQ ID NO: 56) the 378bp upstream of the URA3 open reading frame (SEQ ID NO: 62) were fused to the 255bp downstream of the *URA3* stop codon and the DNA encoding the stop codon (SEQ ID NO: 63). This DNA represents a polynucleotide modification template that can delete the URA3 open reading frame leaving only the stop codon. The polynucleotide editing template was synthesized chemically (IDT) with 5' EcoRI and 3' HinDIII restriction sites (SEQ ID NO: 64). The construct was cloned into the EcoRI/HinDIII sites of pUC18 (SEQ ID NO: 32) generating plasmid pRF263 (SEQ ID NO: 65). The polynucleotide editing template was amplified from pRF263 using primers HY007 (SEQ ID NO: 66) and oligo 297 (SEQ ID NO: 67) to generate the URA3 deletion polynucleotide modification template flanked by 5' and 3' EcoRI sites (SEQ ID NO: 68). The EcoRI flanked URA3 deletion polynucleotide editing template was cloned into the EcoRI site of pRF421 (SEQ ID NO: 61) to generate pRF437 (SEQ ID NO: 69).

Prototrophic *Yarrowia lipolytica* ATCC20362 cells were grown for 24 hours on YPD medium plates (Teknova) at 30°C. 1 loop of cells were resuspended in transformation buffer (35% polyethylene glycol average molecular weight of 3550, 100 mM lithium acetate, 100 mM dithiothreitol, 10 mM Tris, 1mM EDTA pH 6.0). 100 µl of cell suspension was mixed with 100ng of either pRF421 (SEQ ID NO: 61), pRF434 (SEQ ID NO: 54), pRF437 (SEQ ID NO: 69), or no DNA. Cells were heat shocked at 39°C 800RPM for 1 hour. 1ml of YPD medium (Teknova) was added to each transformation. Cells were grown at 30°C 220RPM for 4 hours to allow expression of the hygromycin resistance cassette. Cells were plated on YPD medium containing 250 mg/L of hygromycin sulfate (calbiochem). Colonies were allowed to form at 30°C. 48 colonies from each transformation (with the exception of no DNA which had 0 colonies) were patched to YPD medium plates (Teknova) and CM plates containing 450 mg/L 5-fluoroorotic acid (5FOA). 5FOA selects against cells with a functional URA3 gene. From the patches it was possible to score the efficiency of URA3 inactivation by pRF434 (SEQ ID NO: 54), pRF421 (SEQ ID NO: 61), and pRF437 (SEQ ID NO 69) (table 5).

**Table 5: Frequency of 5FOA resistant colonies among pRF434, pRF421, and pRF437 transformants.**

| Plasmid | VT domain | Protected modification template | 5FOA resistance ± Range¹ |
|---|---|---|---|
| pRF434 | None | None | 0.00 ± 0.00 |
| pRF421 | Ura3-1 | None | 0.86 ± 0.03 |
| pRF437 | Ura3-1 | circular within plasm id | 0.84 ± 0.11 |

| | | | |
|---|---|---|---|
| ¹Results represent the average and range for two independent repeats in each case. Each replicate screened at least 48 colonies. | | | |

The presence of the protected polynucleotide modification template within the context of the Cas9/sgRNA plasmid did not affect the frequency of targeting at the *URA3* locus using a sgRNA containing the Ura3-1 variable targeting domain (Table 5). The frequency of 5FOA resistance represents total targeting frequency and includes mutants generated by repair of the Cas9/gRNA DSB by the NHEJ pathway and the HDR pathway. To determine the frequency of repair of the Cas9/sgRNA generated DSB by the HDR and NHEJ pathway PCR amplification of the *URA3* locus in 5FOA resistant colonies was performed using oligonucleotide primers 308 (SEQ ID NO: 70) and 309 (SEQ ID NO: 71). Typically Cas9/sgRNA breaks repaired by the NHEJ pathway result in the deletion or insertion of a few nucleotides resulting in small indels and when the entire locus is amplified the product appears WT (SEQ ID NO: 56) in size (1714bp). In cases where the Cas9/sgRNA generated DSB has been repaired via HDR with the protected polynucleotide editing template the amplified URA3 locus is reduced in size (859bp) (SEQ ID NO: 72) due to deletion of the URA3 open reading frame. An example of the PCR of 5FOA resistant colonies from cells transformed with pRF437 (SEQ ID NO:69) is shown in Figure 4.

The overall frequency of HDR among the 5FOA resistant colonies is shown in Table 6.

**Table 6: HDR using protected polynucleotide editing templates.**

| Plasmid | VT domain | Protected modification template | HDR Frequency ± Range¹ |
|---|---|---|---|
| pRF434 | None | None | ND |
| pRF421 | Ura3-1 | None | ND |
| pRF437 | Ura3-1 | circular within plasm id | 0.84 ± 0.19 |

| | | | |
|---|---|---|---|
| ¹Values reported represent average and range from two independent replicates. Each replicate screened at least 48 colonies. | | | |

By placing the polynucleotide editing template within a replicating circular DNA the ends are protected and the modification template persists in the cells yielding over 80% of the colonies having repair of the Cas9/sgRNA generated DSB via the HDR pathway and therefore 15% of the colonies repaired the Cas9/sgRNA generated break by NHEJ.

## Claims

1. A method for selecting a microbial cell comprising a modified nucleotide sequence in its genome, the method comprising:
(a) providing a guide polynucleotide, at least one protected polynucleotide modification template and a Cas endonuclease to a microbial cell, wherein said Cas endonuclease and guide polynucleotide can form a complex capable of introducing a single or double-strand break at a target site in genome of said cell, wherein said protected polynucleotide modification template comprises at least one nucleotide modification of said nucleotide sequence; and,
(b) selecting a cell from step (a) comprising said modified nucleotide sequence,
wherein the protected polynucleotide modification template is a double stranded linear molecule comprising at least one phosphorothioate bond at the 5' end of at least one strand, or a single stranded linear molecule comprising at least one phosphorothioate bond at its 5' end.

2. The method of claim 1, wherein the at least one nucleotide modification of the protected polynucleotide template is selected from the group consisting of (i) a replacement of at least one nucleotide, (ii) a deletion of at least one nucleotide, (iii) an insertion of at least one nucleotide, and (iv) any combination of (i) - (iii).

3. The method of claim 1 or 2, further determining the frequency of Homologous Directed Repair (HDR) and/or Non-Homologous End Joining (NHEJ) in said cell.

4. The method of claim 3, wherein the frequency of HDR is increased when compared to the frequency of HDR derived from a control method having all the same components and steps as the method of any one of claims 1 to 3 except for using an unprotected (control) polynucleotide modification template.

5. The method of claim 3, wherein the frequency of NHEJ is decreased when compared to the frequency of NHEJ derived from a control method having all the same components and steps as the method of any one of claims 1 to 3 except for using an unprotected (control) polynucleotide modification template.

6. The method of any one of claims 1 to 5, further determining the frequency of off-site integration of the protected polynucleotide modification template in said cell.

7. The method of claim 6, wherein the frequency of off-site integration of the protected polynucleotide modification template in said cell is decreased when compared to the frequency of off-site integration derived from a control method having all the same components and steps as the method of claim 1 except for using an unprotected (control) polynucleotide modification template.

8. A method for selecting a microbial cell comprising a polynucleotide of interest inserted into a target site in its genome, the method comprising:
(a) providing a guide polynucleotide, at least one protected polynucleotide donor DNA and a Cas endonuclease to a cell, wherein said Cas endonuclease and guide polynucleotide can form a complex capable of introducing a single or double-strand break at a target site in the genome of said cell, wherein said protected polynucleotide donor DNA comprises a polynucleotide of interest to be inserted into the genome of said cell; and,
(b) selecting a microbial cell from step (a) comprising a polynucleotide of interest inserted into a target site in its genome,
wherein the protected polynucleotide donor DNA is a double stranded linear molecule comprising at least one phosphorothioate bond at the 5' end of at least one strand, or a single stranded linear molecule comprising at least one phosphorothioate bond at its 5' end.

9. The method of any one of claims 1 to 8, wherein the microbial cell is a non-conventional yeast, wherein the non-conventional yeast is a yeast that is not a *Saccharomyces* or *Schizosaccharomyces* species.

10. The method of claim 9, wherein said yeast is a member of a genus selected from the group consisting of *Yarrowia, Pichia, Schwanniomyces, Kluyveromyces, Arxula, Trichosporon, Candida, Ustilago, Torulopsis, Zygosaccharomyces, Trigonopsis, Cryptococcus, Rhodotorula, Phaffia, Sporobolomyces, and Pachysolen.*

## Patentansprüche

1. Verfahren zur Selektion einer Mikrobenzelle, umfassend eine modifizierte Nukleotidsequenz in ihrem Genom, wobei das Verfahren umfasst:
(a) Bereitstellung eines Leitpolynukleotids, mindestens einer geschützten Polynukleotidmodifikationsschablone und einer Cas-Endonuklease an eine Mikrobenzelle, wobei die Cas-Endonuklease und das Leitpolynukleotid einen Komplex bilden können, der in der Lage ist, einen Einzel- oder Doppelstrangbruch an einer Zielstelle im Genom der Zelle einzuführen, wobei die geschützte Polynukleotidmodifikationsschablone mindestens eine Nukleotidmodifikation der Nukleotidsequenz umfasst; und,
(b) Selektion einer Zelle aus Schritt (a), die die modifizierte Nukleotidsequenz umfasst,
wobei die geschützte Polynukleotidmodifikationsschablone ein lineares Doppelstrangmolekül, das mindestens eine Phosphorothioatbindung am 5'-Ende von mindestens einem Strang umfasst, oder ein lineares Einzelstrangmolekül, das mindestens eine Phosphorothioatbindung an seinem 5'-Ende umfasst, ist.

2. Verfahren nach Anspruch 1, wobei die mindestens eine Nukleotidmodifikation der geschützten Polynukleotidschablone ausgewählt ist aus der Gruppe bestehend aus (i) einem Ersatz von mindestens einem Nukleotid, (ii) einer Deletion von mindestens einem Nukleotid, (iii) einer Insertion von mindestens einem Nukleotid und (iv) einer beliebigen Kombination von (i) - (iii).

3. Verfahren nach Anspruch 1 oder 2, ferner Bestimmen der Häufigkeit von Homologous Directed Repair (HDR) und/oder Non-Homologous End Joining (NHEJ) in der Zelle.

4. Verfahren nach Anspruch 3, wobei die Häufigkeit von HDR erhöht ist, wenn sie mit der Häufigkeit von HDR verglichen wird, die von einem Kontrollverfahren mit all den gleichen Komponenten und Schritten wie beim Verfahren nach einem der Ansprüche 1 bis 3 abgeleitet ist, mit Ausnahme der Verwendung einer ungeschützten (Kontroll-) Polynukleotidmodifikationsschablone.

5. Verfahren nach Anspruch 3, wobei die Häufigkeit von NHEJ verringert ist, wenn sie mit der Häufigkeit von NHEJ verglichen wird, die von einem Kontrollverfahren mit all den gleichen Komponenten und Schritten wie beim Verfahren nach einem der Ansprüche 1 bis 3 abgeleitet ist, mit Ausnahme der Verwendung einer ungeschützten (Kontroll-) Polynukleotidmodifikationsschablone.

6. Verfahren nach einem der Ansprüche 1 bis 5, ferner Bestimmen der Häufigkeit der Off-Site-Integration der geschützten Polynukleotidmodifikationsschablone in der Zelle.

7. Verfahren nach Anspruch 6, wobei die Häufigkeit der Off-Site-Integration der geschützten Polynukleotidmodifikationsschablone in der Zelle verringert ist, wenn sie mit der Häufigkeit der Off-Site-Integration verglichen wird, die von einem Kontrollverfahren mit all den gleichen Komponenten und Schritten wie beim Verfahren nach Anspruch 1 abgeleitet ist, mit Ausnahme der Verwendung einer ungeschützten (Kontroll-) Polynukleotidmodifikationsschablone.

8. Verfahren zur Selektion einer Mikrobenzelle umfassend ein Polynukleotid von Interesse, das in eine Zielstelle in ihrem Genom inseriert ist, wobei das Verfahren umfasst:
(a) Bereitstellung eines Leitpolynukleotids, mindestens einer geschützten Polynukleotid-Donor-DNA und einer Cas-Endonuklease an eine Zelle, wobei die Cas-Endonuklease und das Leitpolynukleotid einen Komplex bilden können, der in der Lage ist, einen Einzel- oder Doppelstrangbruch an einer Zielstelle im Genom der Zelle einzuführen, wobei die geschützte Polynukleotid-Donor-DNA ein in das Genom der Zelle einzuführendes Polynukleotid von Interesse umfasst; und,
(b) Selektion einer Mikrobenzelle aus Schritt (a), die ein Polynukleotid von Interesse umfasst, das in eine Zielstelle in ihrem Genom inseriert ist,
wobei die geschützte Polynukleotid-Donor-DNA ein lineares Doppelstrangmolekül, das mindestens eine Phosphorothioatbindung am 5'-Ende von mindestens einem Strang umfasst, oder ein lineares Einzelstrangmolekül, das mindestens eine Phosphorothioatbindung an seinem 5'-Ende umfasst, ist.

9. Verfahren nach einem der Ansprüche 1 bis 8, wobei die Mikrobenzelle eine nicht-konventionelle Hefe ist, wobei die nicht-konventionelle Hefe eine Hefe ist, die nicht eine *Saccharomyces-* oder *Schizosaccharomyces-*Spezies ist.

10. Verfahren nach Anspruch 9, wobei die Hefe ein Mitglied einer Gattung ist, ausgewählt aus der Gruppe bestehend aus *Yarrowia, Pichia, Schwanniomyces, Kluyveromyces, Arxula, Trichosporon, Candida, Ustilago, Torulopsis, Zygosaccharomyces, Trigonopsis, Cryptococcus, Rhodotorula, Phaffia, Sporobolomyces* und *Pachysolen.*

## Revendications

1. Procédé de sélection d'une cellule comprenant une séquence nucléotidique modifiée dans son génome, le procédé comprenant :
a. la fourniture d'un polynucléotide guide, d'au moins une matrice de modification de polynucléotide protégé et d'une endonucléase Cas à une cellule, dans lequel lesdits endonucléase Cas et polynucléotide guide peuvent former un complexe capable d'introduire une cassure simple ou double brin à un site cible dans le génome de ladite cellule, dans lequel ladite matrice de modification de polynucléotide protégé comprend au moins une modification de nucléotide de ladite séquence nucléotidique ; et,
b. la sélection d'une cellule provenant de l'étape (a) comprenant ladite séquence nucléotidique modifiée,
dans lequel ladite matrice de modification de polynucléotide protégé est une molécule linéaire double brin comprenant au moins une liaison phosphorothioate à l'extrémité 5' d'au moins un brin, ou une molécule linéaire simple brin comprenant au moins une liaison phosphorothioate à son extrémité 5'.

2. Procédé selon la revendication 1, dans lequel l'au moins une modification de nucléotide de la matrice de polynucléotide protégé est choisie dans le groupe constitué de (i) un remplacement d'au moins un nucléotide, (ii) une délétion d'au moins un nucléotide, (iii) une insertion d'au moins un nucléotide, et (iv) une combinaison quelconque de (i) à (iii).

3. Procédé selon la revendication 1 ou 2, déterminant en outre la fréquence de réparation dirigée par homologie (HDR) et/ou de jonction d'extrémités non homologues (NHEJ) dans ladite cellule.

4. Procédé selon la revendication 3, dans lequel la fréquence de HDR est augmentée par rapport à la fréquence de HDR dérivée d'un procédé témoin ayant l'ensemble des mêmes composants et étapes que le procédé selon l'une quelconque des revendications 1 à 3 à l'exception de l'utilisation d'une matrice de modification de polynucléotide non protégé (témoin).

5. Procédé selon la revendication 3, dans lequel la fréquence de NHEJ est diminuée par rapport à la fréquence de NHEJ dérivée d'un procédé témoin ayant l'ensemble des mêmes composants et étapes que le procédé selon l'une quelconque des revendications 1 à 3 à l'exception de l'utilisation d'une matrice de modification de polynucléotide non protégé (témoin).

6. Procédé selon l'une quelconque des revendications 1 à 5 déterminant en outre la fréquence d'intégration hors site de la matrice de modification de polynucléotide protégé dans ladite cellule.

7. Procédé selon la revendication 6, dans lequel la fréquence d'intégration hors site de la matrice de modification de polynucléotide protégé dans ladite cellule est diminuée par rapport à la fréquence d'intégration hors site dérivée d'un procédé témoin ayant l'ensemble des mêmes composants et étapes que le procédé selon la revendication 1 à l'exception de l'utilisation d'une matrice de modification de polynucléotide non protégé (témoin).

8. Procédé de sélection d'une cellule microbienne comprenant un polynucléotide d'intérêt inséré dans un site cible dans son génome, le procédé comprenant :
a. la fourniture d'un polynucléotide guide, d'au moins un ADN donneur de polynucléotide protégé et d'une endonucléase Cas à une cellule, dans lequel lesdits endonucléase Cas et polynucléotide guide peuvent former un complexe capable d'introduire une cassure simple ou double brin à un site cible dans le génome de ladite cellule, dans lequel ledit ADN donneur de polynucléotide protégé comprend un polynucléotide d'intérêt devant être inséré dans le génome de ladite cellule ; et,
b. la sélection d'une cellule microbienne provenant de l'étape (a) comprenant un polynucléotide d'intérêt inséré dans un site cible dans son génome,
dans lequel l'ADN donneur de polynucléotide protégé est une molécule linéaire double brin comprenant au moins une liaison phosphorothioate à l'extrémité 5' d'au moins un brin, ou une molécule linéaire simple brin comprenant au moins une liaison phosphorothioate à son extrémité 5'.

9. Procédé selon l'une quelconque des revendications 1 à 8, dans lequel la cellule microbienne est une levure non conventionnelle, dans lequel la levure non conventionnelle est une levure qui n'est pas d'une espèce *Saccharomyces* ou *Schizosaccharomyces.*

10. Procédé selon la revendication 9, ladite levure étant un membre d'un genre choisi dans le groupe constitué de *Yarrowia, Pichia, Schwanniomyces, Kluyveromyces, Arxula, Trichosporon, Candida, Ustilago, Torulopsis, Zygosaccharomyces, Trigonopsis, Cryptococcus, Rhodotorula, Phaffia, Sporobolomyces* et *Pachysolen.*


### SEQUENCE LISTING

<110> E. I. du Pont de Nemours and Company Frisch, Ryan L.
<120> PROTECTED DNA TEMPLATES FOR GENE MODIFICATION AND INCREASED HOMOLOGOUS RECOMBINATION IN CELLS AND METHODS OF USE
<130> CL6441-WO-PCT
<150> US 62/240,140
   <151> 2015-10-12
<160> 72
<170> PatentIn version 3.5
<210> 1
   <211> 1372
   <212> PRT
   <213> Streptococcus pyogenes
<400> 1
   Met Asp Lys Lys Tyr Ser Ile Gly Leu Asp Ile Gly Thr Asn Ser Val
   1               5                   10                  15
   Gly Trp Ala Val Ile Thr Asp Glu Tyr Lys Val Pro Ser Lys Lys Phe
               20                  25                  30
   Lys Val Leu Gly Asn Thr Asp Arg His Ser Ile Lys Lys Asn Leu Ile
           35                  40                  45
   Gly Ala Leu Leu Phe Asp Ser Gly Glu Thr Ala Glu Ala Thr Arg Leu
       50                  55                  60
   Lys Arg Thr Ala Arg Arg Arg Tyr Thr Arg Arg Lys Asn Arg Ile Cys
   65                  70                  75                  80
   Tyr Leu Gln Glu Ile Phe Ser Asn Glu Met Ala Lys Val Asp Asp Ser
                   85                  90                  95
   Phe Phe His Arg Leu Glu Glu Ser Phe Leu Val Glu Glu Asp Lys Lys
               100                 105                 110
   His Glu Arg His Pro Ile Phe Gly Asn Ile Val Asp Glu Val Ala Tyr
           115                 120                 125
   His Glu Lys Tyr Pro Thr Ile Tyr His Leu Arg Lys Lys Leu Val Asp
       130                 135                 140
   Ser Thr Asp Lys Ala Asp Leu Arg Leu Ile Tyr Leu Ala Leu Ala His
   145                 150                 155                 160
   Met Ile Lys Phe Arg Gly His Phe Leu Ile Glu Gly Asp Leu Asn Pro
                   165                 170                 175
   Asp Asn Ser Asp Val Asp Lys Leu Phe Ile Gln Leu Val Gln Thr Tyr
               180                 185                 190
   Asn Gln Leu Phe Glu Glu Asn Pro Ile Asn Ala Ser Gly Val Asp Ala
           195                 200                 205
   Lys Ala Ile Leu Ser Ala Arg Leu Ser Lys Ser Arg Arg Leu Glu Asn
       210                 215                 220
   Leu Ile Ala Gln Leu Pro Gly Glu Lys Lys Asn Gly Leu Phe Gly Asn
   225                 230                 235                 240
   Leu Ile Ala Leu Ser Leu Gly Leu Thr Pro Asn Phe Lys Ser Asn Phe
                   245                 250                 255
   Asp Leu Ala Glu Asp Ala Lys Leu Gln Leu Ser Lys Asp Thr Tyr Asp
               260                 265                 270
   Asp Asp Leu Asp Asn Leu Leu Ala Gln Ile Gly Asp Gln Tyr Ala Asp
           275                 280                 285
   Leu Phe Leu Ala Ala Lys Asn Leu Ser Asp Ala Ile Leu Leu Ser Asp
       290                 295                 300
   Ile Leu Arg Val Asn Thr Glu Ile Thr Lys Ala Pro Leu Ser Ala Ser
   305                 310                 315                 320
   Met Ile Lys Arg Tyr Asp Glu His His Gln Asp Leu Thr Leu Leu Lys
                   325                 330                 335
   Ala Leu Val Arg Gln Gln Leu Pro Glu Lys Tyr Lys Glu Ile Phe Phe
               340                 345                 350
   Asp Gln Ser Lys Asn Gly Tyr Ala Gly Tyr Ile Asp Gly Gly Ala Ser
           355                 360                 365
   Gln Glu Glu Phe Tyr Lys Phe Ile Lys Pro Ile Leu Glu Lys Met Asp
       370                 375                 380
   Gly Thr Glu Glu Leu Leu Val Lys Leu Asn Arg Glu Asp Leu Leu Arg
   385                 390                 395                 400
   Lys Gln Arg Thr Phe Asp Asn Gly Ser Ile Pro His Gln Ile His Leu
                   405                 410                 415
   Gly Glu Leu His Ala Ile Leu Arg Arg Gln Glu Asp Phe Tyr Pro Phe
               420                 425                 430
   Leu Lys Asp Asn Arg Glu Lys Ile Glu Lys Ile Leu Thr Phe Arg Ile
           435                 440                 445
   Pro Tyr Tyr Val Gly Pro Leu Ala Arg Gly Asn Ser Arg Phe Ala Trp
       450                 455                 460
   Met Thr Arg Lys Ser Glu Glu Thr Ile Thr Pro Trp Asn Phe Glu Glu
   465                 470                 475                 480
   Val Val Asp Lys Gly Ala Ser Ala Gln Ser Phe Ile Glu Arg Met Thr
                   485                 490                 495
   Asn Phe Asp Lys Asn Leu Pro Asn Glu Lys Val Leu Pro Lys His Ser
               500                 505                 510
   Leu Leu Tyr Glu Tyr Phe Thr Val Tyr Asn Glu Leu Thr Lys Val Lys
           515                 520                 525
   Tyr Val Thr Glu Gly Met Arg Lys Pro Ala Phe Leu Ser Gly Glu Gln
       530                 535                 540
   Lys Lys Ala Ile Val Asp Leu Leu Phe Lys Thr Asn Arg Lys Val Thr
   545                 550                 555                 560
   Val Lys Gln Leu Lys Glu Asp Tyr Phe Lys Lys Ile Glu Cys Phe Asp
                   565                 570                 575
   Ser Val Glu Ile Ser Gly Val Glu Asp Arg Phe Asn Ala Ser Leu Gly
               580                 585                 590
   Thr Tyr His Asp Leu Leu Lys Ile Ile Lys Asp Lys Asp Phe Leu Asp
           595                 600                 605
   Asn Glu Glu Asn Glu Asp Ile Leu Glu Asp Ile Val Leu Thr Leu Thr
       610                 615                 620
   Leu Phe Glu Asp Arg Glu Met Ile Glu Glu Arg Leu Lys Thr Tyr Ala
   625                 630                 635                 640
   His Leu Phe Asp Asp Lys Val Met Lys Gln Leu Lys Arg Arg Arg Tyr
                   645                 650                 655
   Thr Gly Trp Gly Arg Leu Ser Arg Lys Leu Ile Asn Gly Ile Arg Asp
               660                 665                 670
   Lys Gln Ser Gly Lys Thr Ile Leu Asp Phe Leu Lys Ser Asp Gly Phe
           675                 680                 685
   Ala Asn Arg Asn Phe Met Gln Leu Ile His Asp Asp Ser Leu Thr Phe
       690                 695                 700
   Lys Glu Asp Ile Gln Lys Ala Gln Val Ser Gly Gln Gly Asp Ser Leu
   705                 710                 715                 720
   His Glu His Ile Ala Asn Leu Ala Gly Ser Pro Ala Ile Lys Lys Gly
                   725                 730                 735
   Ile Leu Gln Thr Val Lys Val Val Asp Glu Leu Val Lys Val Met Gly
               740                 745                 750
   Arg His Lys Pro Glu Asn Ile Val Ile Glu Met Ala Arg Glu Asn Gln
           755                 760                 765
   Thr Thr Gln Lys Gly Gln Lys Asn Ser Arg Glu Arg Met Lys Arg Ile
       770                 775                 780
   Glu Glu Gly Ile Lys Glu Leu Gly Ser Gln Ile Leu Lys Glu His Pro
   785                 790                 795                 800
   Val Glu Asn Thr Gln Leu Gln Asn Glu Lys Leu Tyr Leu Tyr Tyr Leu
                   805                 810                 815
   Gln Asn Gly Arg Asp Met Tyr Val Asp Gln Glu Leu Asp Ile Asn Arg
               820                 825                 830
   Leu Ser Asp Tyr Asp Val Asp His Ile Val Pro Gln Ser Phe Leu Lys
           835                 840                 845
   Asp Asp Ser Ile Asp Asn Lys Val Leu Thr Arg Ser Asp Lys Asn Arg
       850                 855                 860
   Gly Lys Ser Asp Asn Val Pro Ser Glu Glu Val Val Lys Lys Met Lys
   865                 870                 875                 880
   Asn Tyr Trp Arg Gln Leu Leu Asn Ala Lys Leu Ile Thr Gln Arg Lys
                   885                 890                 895
   Phe Asp Asn Leu Thr Lys Ala Glu Arg Gly Gly Leu Ser Glu Leu Asp
               900                 905                 910
   Lys Ala Gly Phe Ile Lys Arg Gln Leu Val Glu Thr Arg Gln Ile Thr
           915                 920                 925
   Lys His Val Ala Gln Ile Leu Asp Ser Arg Met Asn Thr Lys Tyr Asp
       930                 935                 940
   Glu Asn Asp Lys Leu Ile Arg Glu Val Lys Val Ile Thr Leu Lys Ser
   945                 950                 955                 960
   Lys Leu Val Ser Asp Phe Arg Lys Asp Phe Gln Phe Tyr Lys Val Arg
                   965                 970                 975
   Glu Ile Asn Asn Tyr His His Ala His Asp Ala Tyr Leu Asn Ala Val
               980                 985                 990
   Val Gly Thr Ala Leu Ile Lys Lys  Tyr Pro Lys Leu Glu  Ser Glu Phe
           995                 1000                1005
   Val Tyr  Gly Asp Tyr Lys Val  Tyr Asp Val Arg Lys  Met Ile Ala
       1010                1015                1020
   Lys Ser  Glu Gln Glu Ile Gly  Lys Ala Thr Ala Lys  Tyr Phe Phe
       1025                1030                1035
   Tyr Ser  Asn Ile Met Asn Phe  Phe Lys Thr Glu Ile  Thr Leu Ala
       1040                1045                1050
   Asn Gly  Glu Ile Arg Lys Arg  Pro Leu Ile Glu Thr  Asn Gly Glu
       1055                1060                1065
   Thr Gly  Glu Ile Val Trp Asp  Lys Gly Arg Asp Phe  Ala Thr Val
       1070                1075                1080
   Arg Lys  Val Leu Ser Met Pro  Gln Val Asn Ile Val  Lys Lys Thr
       1085                1090                1095
   Glu Val  Gln Thr Gly Gly Phe  Ser Lys Glu Ser Ile  Leu Pro Lys
       1100                1105                1110
   Arg Asn  Ser Asp Lys Leu Ile  Ala Arg Lys Lys Asp  Trp Asp Pro
       1115                1120                1125
   Lys Lys  Tyr Gly Gly Phe Asp  Ser Pro Thr Val Ala  Tyr Ser Val
       1130                1135                1140
   Leu Val  Val Ala Lys Val Glu  Lys Gly Lys Ser Lys  Lys Leu Lys
       1145                1150                1155
   Ser Val  Lys Glu Leu Leu Gly  Ile Thr Ile Met Glu  Arg Ser Ser
       1160                1165                1170
   Phe Glu  Lys Asn Pro Ile Asp  Phe Leu Glu Ala Lys  Gly Tyr Lys
       1175                1180                1185
   Glu Val  Lys Lys Asp Leu Ile  Ile Lys Leu Pro Lys  Tyr Ser Leu
       1190                1195                1200
   Phe Glu  Leu Glu Asn Gly Arg  Lys Arg Met Leu Ala  Ser Ala Gly
       1205                1210                1215
   Glu Leu  Gln Lys Gly Asn Glu  Leu Ala Leu Pro Ser  Lys Tyr Val
       1220                1225                1230
   Asn Phe  Leu Tyr Leu Ala Ser  His Tyr Glu Lys Leu  Lys Gly Ser
       1235                1240                1245
   Pro Glu  Asp Asn Glu Gln Lys  Gln Leu Phe Val Glu  Gln His Lys
       1250                1255                1260
   His Tyr  Leu Asp Glu Ile Ile  Glu Gln Ile Ser Glu  Phe Ser Lys
       1265                1270                1275
   Arg Val  Ile Leu Ala Asp Ala  Asn Leu Asp Lys Val  Leu Ser Ala
       1280                1285                1290
   Tyr Asn  Lys His Arg Asp Lys  Pro Ile Arg Glu Gln  Ala Glu Asn
       1295                    1300                    1305
   Ile Ile  His Leu Phe Thr Leu  Thr Asn Leu Gly Ala  Pro Ala Ala
       1310                    1315                    1320
   Phe Lys  Tyr Phe Asp Thr Thr  Ile Asp Arg Lys Arg  Tyr Thr Ser
       1325                    1330                    1335
   Thr Lys  Glu Val Leu Asp Ala  Thr Leu Ile His Gln  Ser Ile Thr
       1340                    1345                    1350
   Gly Leu  Tyr Glu Thr Arg Ile  Asp Leu Ser Gln Leu  Gly Gly Asp
       1355                    1360                    1365
   Ser Arg  Ala Asp
       1370
<210> 2
   <211> 4140
   <212> DNA
   <213> Artificial sequence
<220>
   <223> Yarrowia codon optimized Cas9
<400> 2
   atggacaaga aatactccat cggcctggac attggaacca actctgtcgg ctgggctgtc 60
   atcaccgacg agtacaaggt gccctccaag aaattcaagg tcctcggaaa caccgatcga 120
   cactccatca agaaaaacct cattggtgcc ctgttgttcg attctggcga gactgccgaa 180
   gctaccagac tcaagcgaac tgctcggcga cgttacaccc gacggaagaa ccgaatctgc 240
   tacctgcagg agatcttttc caacgagatg gccaaggtgg acgattcgtt ctttcatcga 300
   ctggaggaat ccttcctcgt cgaggaagac aagaaacacg agcgtcatcc catctttggc 360
   aacattgtgg acgaggttgc ttaccacgag aagtatccta ccatctacca tctccgaaag 420
   aaactcgtcg attccaccga caaggcggat ctcagactta tctacctcgc tctggcacac 480
   atgatcaagt ttcgaggtca tttcctcatc gagggcgatc tcaatcccga caacagcgat 540
   gtggacaagc tgttcattca gctcgttcag acctacaacc agctgttcga ggaaaacccc 600
   atcaatgcct ccggagtcga tgcaaaggcc atcttgtctg ctcgactctc gaagagcaga 660
   cgactggaga acctcattgc ccaacttcct ggcgagaaaa agaacggact gtttggcaac 720
   ctcattgccc tttctcttgg tctcacaccc aacttcaagt ccaacttcga tctggcggag 780
   gacgccaagc tccagctgtc caaggacacc tacgacgatg acctcgacaa cctgcttgca 840
   cagattggcg atcagtacgc cgacctgttt ctcgctgcca agaacctttc ggatgctatt 900
   ctcttgtctg acattctgcg agtcaacacc gagatcacaa aggctcccct ttctgcctcc 960
   atgatcaagc gatacgacga gcaccatcag gatctcacac tgctcaaggc tcttgtccga 1020
   cagcaactgc ccgagaagta caaggagatc tttttcgatc agtcgaagaa cggctacgct 1080
   ggatacatcg acggcggagc ctctcaggaa gagttctaca agttcatcaa gccaattctc 1140
   gagaagatgg acggaaccga ggaactgctt gtcaagctca atcgagagga tctgcttcgg 1200
   aagcaacgaa ccttcgacaa cggcagcatt cctcatcaga tccacctcgg tgagctgcac 1260
   gccattcttc gacgtcagga agacttctac ccctttctca aggacaaccg agagaagatc 1320
   gagaagattc ttacctttcg aatcccctac tatgttggtc ctcttgccag aggaaactct 1380
   cgatttgctt ggatgactcg aaagtccgag gaaaccatca ctccctggaa cttcgaggaa 1440
   gtcgtggaca agggtgcctc tgcacagtcc ttcatcgagc gaatgaccaa cttcgacaag 1500
   aatctgccca acgagaaggt tcttcccaag cattcgctgc tctacgagta ctttacagtc 1560
   tacaacgaac tcaccaaagt caagtacgtt accgagggaa tgcgaaagcc tgccttcttg 1620
   tctggcgaac agaagaaagc cattgtcgat ctcctgttca agaccaaccg aaaggtcact 1680
   gttaagcagc tcaaggagga ctacttcaag aaaatcgagt gtttcgacag cgtcgagatt 1740
   tccggagttg aggaccgatt caacgcctct ttgggcacct atcacgatct gctcaagatt 1800
   atcaaggaca aggattttct cgacaacgag gaaaacgagg acattctgga ggacatcgtg 1860
   ctcactctta ccctgttcga agatcgggag atgatcgagg aacgactcaa gacatacgct 1920
   cacctgttcg acgacaaggt catgaaacaa ctcaagcgac gtagatacac cggctgggga 1980
   agactttcgc gaaagctcat caacggcatc agagacaagc agtccggaaa gaccattctg 2040
   gactttctca agtccgatgg ctttgccaac cgaaacttca tgcagctcat tcacgacgat 2100
   tctcttacct tcaaggagga catccagaag gcacaagtgt ccggtcaggg cgacagcttg 2160
   cacgaacata ttgccaacct ggctggttcg ccagccatca agaaaggcat tctccagact 2220
   gtcaaggttg tcgacgagct ggtgaaggtc atgggacgtc acaagcccga gaacattgtg 2280
   atcgagatgg ccagagagaa ccagacaact caaaagggtc agaaaaactc gcgagagcgg 2340
   atgaagcgaa tcgaggaagg catcaaggag ctgggatccc agattctcaa ggagcatccc 2400
   gtcgagaaca ctcaactgca gaacgagaag ctgtatctct actatctgca gaatggtcga 2460
   gacatgtacg tggatcagga actggacatc aatcgtctca gcgactacga tgtggaccac 2520
   attgtccctc aatcctttct caaggacgat tctatcgaca acaaggtcct tacacgatcc    2580
   gacaagaaca gaggcaagtc ggacaacgtt cccagcgaag aggtggtcaa aaagatgaag    2640
   aactactggc gacagctgct caacgccaag ctcattaccc agcgaaagtt cgacaatctt    2700
   accaaggccg agcgaggcgg tctgtccgag ctcgacaagg ctggcttcat caagcgtcaa    2760
   ctcgtcgaga ccagacagat cacaaagcac gtcgcacaga ttctcgattc tcggatgaac    2820
   accaagtacg acgagaacga caagctcatc cgagaggtca aggtgattac tctcaagtcc    2880
   aaactggtct ccgatttccg aaaggacttt cagttctaca aggtgcgaga gatcaacaat    2940
   taccaccatg cccacgatgc ttacctcaac gccgtcgttg gcactgcgct catcaagaaa    3000
   taccccaagc tcgaaagcga gttcgtttac ggcgattaca aggtctacga cgttcgaaag    3060
   atgattgcca agtccgaaca ggagattggc aaggctactg ccaagtactt cttttactcc    3120
   aacatcatga actttttcaa gaccgagatc accttggcca acggagagat tcgaaagaga    3180
   ccacttatcg agaccaacgg cgaaactgga gagatcgtgt gggacaaggg tcgagacttt    3240
   gcaaccgtgc gaaaggttct gtcgatgcct caggtcaaca tcgtcaagaa aaccgaggtt    3300
   cagactggcg gattctccaa ggagtcgatt ctgcccaagc gaaactccga caagctcatc    3360
   gctcgaaaga aagactggga tcccaagaaa tacggtggct tcgattctcc taccgtcgcc    3420
   tattccgtgc ttgtcgttgc gaaggtcgag aagggcaagt ccaaaaagct caagtccgtc    3480
   aaggagctgc tcggaattac catcatggag cgatcgagct tcgagaagaa tcccatcgac    3540
   ttcttggaag ccaagggtta caaggaggtc aagaaagacc tcattatcaa gctgcccaag    3600
   tactctctgt tcgaactgga gaacggtcga aagcgtatgc tcgcctccgc tggcgagctg    3660
   cagaagggaa acgagcttgc cttgccttcg aagtacgtca actttctcta tctggcttct    3720
   cactacgaga agctcaaggg ttctcccgag gacaacgaac agaagcaact cttcgttgag    3780
   cagcacaaac attacctcga cgagattatc gagcagattt ccgagttttc gaagcgagtc    3840
   atcctggctg atgccaactt ggacaaggtg ctctctgcct acaacaagca tcgggacaaa    3900
   cccattcgag aacaggcgga gaacatcatt cacctgttta ctcttaccaa cctgggtgct    3960
   cctgcagctt tcaagtactt cgataccact atcgaccgaa agcggtacac atccaccaag    4020
   gaggttctcg atgccaccct gattcaccag tccatcactg gcctgtacga gacccgaatc    4080
   gacctgtctc agcttggtgg cgactccaga gccgatccca agaaaaagcg aaaggtctaa    4140
<210> 3
   <211> 7
   <212> PRT
   <213> SV40
<400> 3
   Pro Lys Lys Lys Arg Lys Val
   1               5
<210> 4
   <211> 543
   <212> DNA
   <213> YArrowia lipolytica
<400> 4
   tcgacgttta aaccatcatc taagggcctc aaaactacct cggaactgct gcgctgatct      60
   ggacaccaca gaggttccga gcactttagg ttgcaccaaa tgtcccacca ggtgcaggca     120
   gaaaacgctg gaacagcgtg tacagtttgt cttaacaaaa agtgagggcg ctgaggtcga     180
   gcagggtggt gtgacttgtt atagccttta gagctgcgaa agcgcgtatg gatttggctc     240
   atcaggccag attgagggtc tgtggacaca tgtcatgtta gtgtacttca atcgccccct     300
   ggatatagcc ccgacaatag gccgtggcct catttttttg ccttccgcac atttccattg      360
   ctcggtaccc acaccttgct tctcctgcac ttgccaacct taatactggt ttacattgac     420
   caacatctta caagcggggg gcttgtctag ggtatatata aacagtggct ctcccaatcg     480
   gttgccagtc tcttttttcc tttctttccc cacagattcg aaatctaaac tacacatcac      540
   acc                                                                   543
<210> 5
   <211> 4683
   <212> DNA
   <213> Artificial sequence
<220>
   <223> Yarrowia optimized expression cassette
<400> 5
   tcgacgttta aaccatcatc taagggcctc aaaactacct cggaactgct gcgctgatct      60
   ggacaccaca gaggttccga gcactttagg ttgcaccaaa tgtcccacca ggtgcaggca     120
   gaaaacgctg gaacagcgtg tacagtttgt cttaacaaaa agtgagggcg ctgaggtcga     180
   gcagggtggt gtgacttgtt atagccttta gagctgcgaa agcgcgtatg gatttggctc     240
   atcaggccag attgagggtc tgtggacaca tgtcatgtta gtgtacttca atcgccccct     300
   ggatatagcc ccgacaatag gccgtggcct catttttttg ccttccgcac atttccattg      360
   ctcggtaccc acaccttgct tctcctgcac ttgccaacct taatactggt ttacattgac     420
   caacatctta caagcggggg gcttgtctag ggtatatata aacagtggct ctcccaatcg     480
   gttgccagtc tcttttttcc tttctttccc cacagattcg aaatctaaac tacacatcac     540
   accatggaca agaaatactc catcggcctg gacattggaa ccaactctgt cggctgggct     600
   gtcatcaccg acgagtacaa ggtgccctcc aagaaattca aggtcctcgg aaacaccgat     660
   cgacactcca tcaagaaaaa cctcattggt gccctgttgt tcgattctgg cgagactgcc     720
   gaagctacca gactcaagcg aactgctcgg cgacgttaca cccgacggaa gaaccgaatc     780
   tgctacctgc aggagatctt ttccaacgag atggccaagg tggacgattc gttctttcat     840
   cgactggagg aatccttcct cgtcgaggaa gacaagaaac acgagcgtca tcccatcttt     900
   ggcaacattg tggacgaggt tgcttaccac gagaagtatc ctaccatcta ccacctgcga     960
   aagaaactcg tcgattccac cgacaaggcg gatctcagac ttatctacct cgctctggca    1020
   cacatgatca agtttcgagg tcatttcctc atcgagggcg atctcaatcc cgacaacagc    1080
   gatgtggaca agctgttcat tcagctcgtt cagacctaca accagctgtt cgaggaaaac    1140
   cccatcaatg cctccggagt cgatgcaaag gccatcttgt ctgctcgact ctcgaagagc    1200
   agacgactgg agaacctcat tgcccaactt cctggcgaga aaaagaacgg actgtttggc    1260
   aacctcattg ccctttctct tggtctcaca cccaacttca agtccaactt cgatctggcg    1320
   gaggacgcca agctccagct gtccaaggac acctacgacg atgacctcga caacctgctt    1380
   gcacagattg gcgatcagta cgccgacctg tttctcgctg ccaagaacct ttcggatgct    1440
   attctcttgt ctgacattct gcgagtcaac accgagatca caaaggctcc cctttctgcc    1500
   tccatgatca agcgatacga cgagcaccat caggatctca cactgctcaa ggctcttgtc    1560
   cgacagcaac tgcccgagaa gtacaaggag atctttttcg atcagtcgaa gaacggctac   1620
   gctggataca tcgacggcgg agcctctcag gaagagttct acaagttcat caagccaatt    1680
   ctcgagaaga tggacggaac cgaggaactg cttgtcaagc tcaatcgaga ggatctgctt    1740
   cggaagcaac gaaccttcga caacggcagc attcctcatc agatccacct cggtgagctg    1800
   cacgccattc ttcgacgtca ggaagacttc tacccctttc tcaaggacaa ccgagagaag   1860
   atcgagaaga ttcttacctt tcgaatcccc tactatgttg gtcctcttgc cagaggaaac    1920
   tctcgatttg cttggatgac tcgaaagtcc gaggaaacca tcactccctg gaacttcgag    1980
   gaagtcgtgg acaagggtgc ctctgcacag tccttcatcg agcgaatgac caacttcgac    2040
   aagaatctgc ccaacgagaa ggttcttccc aagcattcgc tgctctacga gtactttaca    2100
   gtctacaacg aactcaccaa agtcaagtac gttaccgagg gaatgcgaaa gcctgccttc    2160
   ttgtctggcg aacagaagaa agccattgtc gatctcctgt tcaagaccaa ccgaaaggtc    2220
   actgttaagc agctcaagga ggactacttc aagaaaatcg agtgtttcga cagcgtcgag    2280
   atttccggag ttgaggaccg attcaacgcc tctttgggca cctatcacga tctgctcaag    2340
   attatcaagg acaaggattt tctcgacaac gaggaaaacg aggacattct ggaggacatc    2400
   gtgctcactc ttaccctgtt cgaagatcgg gagatgatcg aggaacgact caagacatac    2460
   gctcacctgt tcgacgacaa ggtcatgaaa caactcaagc gacgtagata caccggctgg    2520
   ggaagacttt cgcgaaagct catcaacggc atcagagaca agcagtccgg aaagaccatt    2580
   ctggactttc tcaagtccga tggctttgcc aaccgaaact tcatgcagct cattcacgac    2640
   gattctctta ccttcaagga ggacatccag aaggcacaag tgtccggtca gggcgacagc    2700
   ttgcacgaac atattgccaa cctggctggt tcgccagcca tcaagaaagg cattctccag    2760
   actgtcaagg ttgtcgacga gctggtgaag gtcatgggac gtcacaagcc cgagaacatt    2820
   gtgatcgaga tggccagaga gaaccagaca actcaaaagg gtcagaaaaa ctcgcgagag    2880
   cggatgaagc gaatcgagga aggcatcaag gagctgggat cccagattct caaggagcat    2940
   cccgtcgaga acactcaact gcagaacgag aagctgtatc tctactatct gcagaatggt    3000
   cgagacatgt acgtggatca ggaactggac atcaatcgtc tcagcgacta cgatgtggac    3060
   cacattgtcc ctcaatcctt tctcaaggac gattctatcg acaacaaggt ccttacacga    3120
   tccgacaaga acagaggcaa gtcggacaac gttcccagcg aagaggtggt caaaaagatg    3180
   aagaactact ggcgacagct gctcaacgcc aagctcatta cccagcgaaa gttcgacaat    3240
   cttaccaagg ccgagcgagg cggtctgtcc gagctcgaca aggctggctt catcaagcgt    3300
   caactcgtcg agaccagaca gatcacaaag cacgtcgcac agattctcga ttctcggatg    3360
   aacaccaagt acgacgagaa cgacaagctc atccgagagg tcaaggtgat tactctcaag    3420
   tccaaactgg tctccgattt ccgaaaggac tttcagttct acaaggtgcg agagatcaac    3480
   aattaccacc atgcccacga tgcttacctc aacgccgtcg ttggcactgc gctcatcaag    3540
   aaatacccca agctcgaaag cgagttcgtt tacggcgatt acaaggtcta cgacgttcga   3600
   aagatgattg ccaagtccga acaggagatt ggcaaggcta ctgccaagta cttcttttac    3660
   tccaacatca tgaacttttt caagaccgag atcaccttgg ccaacggaga gattcgaaag    3720
   agaccactta tcgagaccaa cggcgaaact ggagagatcg tgtgggacaa gggtcgagac    3780
   tttgcaaccg tgcgaaaggt tctgtcgatg cctcaggtca acatcgtcaa gaaaaccgag      3840
   gttcagactg gcggattctc caaggagtcg attctgccca agcgaaactc cgacaagctc      3900
   atcgctcgaa agaaagactg ggatcccaag aaatacggtg gcttcgattc tcctaccgtc      3960
   gcctattccg tgcttgtcgt tgcgaaggtc gagaagggca agtccaaaaa gctcaagtcc      4020
   gtcaaggagc tgctcggaat taccatcatg gagcgatcga gcttcgagaa gaatcccatc      4080
   gacttcttgg aagccaaggg ttacaaggag gtcaagaaag acctcattat caagctgccc      4140
   aagtactctc tgttcgaact ggagaacggt cgaaagcgta tgctcgcctc cgctggcgag      4200
   ctgcagaagg gaaacgagct tgccttgcct tcgaagtacg tcaactttct ctatctggct      4260
   tctcactacg agaagctcaa gggttctccc gaggacaacg aacagaagca actcttcgtt      4320
   gagcagcaca aacattacct cgacgagatt atcgagcaga tttccgagtt ttcgaagcga      4380
   gtcatcctgg ctgatgccaa cttggacaag gtgctctctg cctacaacaa gcatcgggac      4440
   aaacccattc gagaacaggc ggagaacatc attcacctgt ttactcttac caacctgggt      4500
   gctcctgcag ctttcaagta cttcgatacc actatcgacc gaaagcggta cacatccacc      4560
   aaggaggttc tcgatgccac cctgattcac cagtccatca ctggcctgta cgagacccga      4620
   atcgacctgt ctcagcttgg tggcgactcc agagccgatc ccaagaaaaa gcgaaaggtc      4680
   taa                                                                    4683
<210> 6
   <211> 10706
   <212> DNA
   <213> Artificial sequence
<220>
   <223> pZufCas9
<400> 6
   catggacaag aaatactcca tcggcctgga cattggaacc aactctgtcg gctgggctgt      60
   catcaccgac gagtacaagg tgccctccaa gaaattcaag gtcctcggaa acaccgatcg     120
   acactccatc aagaaaaacc tcattggtgc cctgttgttc gattctggcg agactgccga     180
   agctaccaga ctcaagcgaa ctgctcggcg acgttacacc cgacggaaga accgaatctg     240
   ctacctgcag gagatctttt ccaacgagat ggccaaggtg gacgattcgt tctttcatcg     300
   actggaggaa tccttcctcg tcgaggaaga caagaaacac gagcgtcatc ccatctttgg     360
   caacattgtg gacgaggttg cttaccacga gaagtatcct accatctacc acctgcgaaa     420
   gaaactcgtc gattccaccg acaaggcgga tctcagactt atctacctcg ctctggcaca     480
   catgatcaag tttcgaggtc atttcctcat cgagggcgat ctcaatcccg acaacagcga     540
   tgtggacaag ctgttcattc agctcgttca gacctacaac cagctgttcg aggaaaaccc     600
   catcaatgcc tccggagtcg atgcaaaggc catcttgtct gctcgactct cgaagagcag     660
   acgactggag aacctcattg cccaacttcc tggcgagaaa aagaacggac tgtttggcaa     720
   cctcattgcc ctttctcttg gtctcacacc caacttcaag tccaacttcg atctggcgga     780
   ggacgccaag ctccagctgt ccaaggacac ctacgacgat gacctcgaca acctgcttgc     840
   acagattggc gatcagtacg ccgacctgtt tctcgctgcc aagaaccttt cggatgctat     900
   tctcttgtct gacattctgc gagtcaacac cgagatcaca aaggctcccc tttctgcctc     960
   catgatcaag cgatacgacg agcaccatca ggatctcaca ctgctcaagg ctcttgtccg    1020
   acagcaactg cccgagaagt acaaggagat ctttttcgat cagtcgaaga acggctacgc    1080
   tggatacatc gacggcggag cctctcagga agagttctac aagttcatca agccaattct    1140
   cgagaagatg gacggaaccg aggaactgct tgtcaagctc aatcgagagg atctgcttcg    1200
   gaagcaacga accttcgaca acggcagcat tcctcatcag atccacctcg gtgagctgca    1260
   cgccattctt cgacgtcagg aagacttcta cccctttctc aaggacaacc gagagaagat    1320
   cgagaagatt cttacctttc gaatccccta ctatgttggt cctcttgcca gaggaaactc    1380
   tcgatttgct tggatgactc gaaagtccga ggaaaccatc actccctgga acttcgagga    1440
   agtcgtggac aagggtgcct ctgcacagtc cttcatcgag cgaatgacca acttcgacaa    1500
   gaatctgccc aacgagaagg ttcttcccaa gcattcgctg ctctacgagt actttacagt    1560
   ctacaacgaa ctcaccaaag tcaagtacgt taccgaggga atgcgaaagc ctgccttctt    1620
   gtctggcgaa cagaagaaag ccattgtcga tctcctgttc aagaccaacc gaaaggtcac    1680
   tgttaagcag ctcaaggagg actacttcaa gaaaatcgag tgtttcgaca gcgtcgagat    1740
   ttccggagtt gaggaccgat tcaacgcctc tttgggcacc tatcacgatc tgctcaagat    1800
   tatcaaggac aaggattttc tcgacaacga ggaaaacgag gacattctgg aggacatcgt    1860
   gctcactctt accctgttcg aagatcggga gatgatcgag gaacgactca agacatacgc    1920
   tcacctgttc gacgacaagg tcatgaaaca actcaagcga cgtagataca ccggctgggg    1980
   aagactttcg cgaaagctca tcaacggcat cagagacaag cagtccggaa agaccattct    2040
   ggactttctc aagtccgatg gctttgccaa ccgaaacttc atgcagctca ttcacgacga    2100
   ttctcttacc ttcaaggagg acatccagaa ggcacaagtg tccggtcagg gcgacagctt    2160
   gcacgaacat attgccaacc tggctggttc gccagccatc aagaaaggca ttctccagac    2220
   tgtcaaggtt gtcgacgagc tggtgaaggt catgggacgt cacaagcccg agaacattgt      2280
   gatcgagatg gccagagaga accagacaac tcaaaagggt cagaaaaact cgcgagagcg      2340
   gatgaagcga atcgaggaag gcatcaagga gctgggatcc cagattctca aggagcatcc      2400
   cgtcgagaac actcaactgc agaacgagaa gctgtatctc tactatctgc agaatggtcg      2460
   agacatgtac gtggatcagg aactggacat caatcgtctc agcgactacg atgtggacca      2520
   cattgtccct caatcctttc tcaaggacga ttctatcgac aacaaggtcc ttacacgatc      2580
   cgacaagaac agaggcaagt cggacaacgt tcccagcgaa gaggtggtca aaaagatgaa      2640
   gaactactgg cgacagctgc tcaacgccaa gctcattacc cagcgaaagt tcgacaatct      2700
   taccaaggcc gagcgaggcg gtctgtccga gctcgacaag gctggcttca tcaagcgtca      2760
   actcgtcgag accagacaga tcacaaagca cgtcgcacag attctcgatt ctcggatgaa      2820
   caccaagtac gacgagaacg acaagctcat ccgagaggtc aaggtgatta ctctcaagtc      2880
   caaactggtc tccgatttcc gaaaggactt tcagttctac aaggtgcgag agatcaacaa      2940
   ttaccaccat gcccacgatg cttacctcaa cgccgtcgtt ggcactgcgc tcatcaagaa      3000
   ataccccaag ctcgaaagcg agttcgttta cggcgattac aaggtctacg acgttcgaaa      3060
   gatgattgcc aagtccgaac aggagattgg caaggctact gccaagtact tcttttactc      3120
   caacatcatg aactttttca agaccgagat caccttggcc aacggagaga ttcgaaagag      3180
   accacttatc gagaccaacg gcgaaactgg agagatcgtg tgggacaagg gtcgagactt      3240
   tgcaaccgtg cgaaaggttc tgtcgatgcc tcaggtcaac atcgtcaaga aaaccgaggt      3300
   tcagactggc ggattctcca aggagtcgat tctgcccaag cgaaactccg acaagctcat      3360
   cgctcgaaag aaagactggg atcccaagaa atacggtggc ttcgattctc ctaccgtcgc      3420
   ctattccgtg cttgtcgttg cgaaggtcga gaagggcaag tccaaaaagc tcaagtccgt      3480
   caaggagctg ctcggaatta ccatcatgga gcgatcgagc ttcgagaaga atcccatcga      3540
   cttcttggaa gccaagggtt acaaggaggt caagaaagac ctcattatca agctgcccaa      3600
   gtactctctg ttcgaactgg agaacggtcg aaagcgtatg ctcgcctccg ctggcgagct      3660
   gcagaaggga aacgagcttg ccttgccttc gaagtacgtc aactttctct atctggcttc      3720
   tcactacgag aagctcaagg gttctcccga ggacaacgaa cagaagcaac tcttcgttga      3780
   gcagcacaaa cattacctcg acgagattat cgagcagatt tccgagtttt cgaagcgagt      3840
   catcctggct gatgccaact tggacaaggt gctctctgcc tacaacaagc atcgggacaa      3900
   acccattcga gaacaggcgg agaacatcat tcacctgttt actcttacca acctgggtgc      3960
   tcctgcagct ttcaagtact tcgataccac tatcgaccga aagcggtaca catccaccaa      4020
   ggaggttctc gatgccaccc tgattcacca gtccatcact ggcctgtacg agacccgaat      4080
   cgacctgtct cagcttggtg gcgactccag agccgatccc aagaaaaagc gaaaggtcta      4140
   agcggccgca agtgtggatg gggaagtgag tgcccggttc tgtgtgcaca attggcaatc      4200
   caagatggat ggattcaaca cagggatata gcgagctacg tggtggtgcg aggatatagc      4260
   aacggatatt tatgtttgac acttgagaat gtacgataca agcactgtcc aagtacaata      4320
   ctaaacatac tgtacatact catactcgta cccgggcaac ggtttcactt gagtgcagtg      4380
   gctagtgctc ttactcgtac agtgtgcaat actgcgtatc atagtctttg atgtatatcg      4440
   tattcattca tgttagttgc gtacgagccg gaagcataaa gtgtaaagcc tggggtgcct      4500
   aatgagtgag ctaactcaca ttaattgcgt tgcgctcact gcccgctttc cagtcgggaa      4560
   acctgtcgtg ccagctgcat taatgaatcg gccaacgcgc ggggagaggc ggtttgcgta      4620
   ttgggcgctc ttccgcttcc tcgctcactg actcgctgcg ctcggtcgtt cggctgcggc      4680
   gagcggtatc agctcactca aaggcggtaa tacggttatc cacagaatca ggggataacg      4740
   caggaaagaa catgtgagca aaaggccagc aaaaggccag gaaccgtaaa aaggccgcgt      4800
   tgctggcgtt tttccatagg ctccgccccc ctgacgagca tcacaaaaat cgacgctcaa      4860
   gtcagaggtg gcgaaacccg acaggactat aaagatacca ggcgtttccc cctggaagct      4920
   ccctcgtgcg ctctcctgtt ccgaccctgc cgcttaccgg atacctgtcc gcctttctcc      4980
   cttcgggaag cgtggcgctt tctcatagct cacgctgtag gtatctcagt tcggtgtagg      5040
   tcgttcgctc caagctgggc tgtgtgcacg aaccccccgt tcagcccgac cgctgcgcct      5100
   tatccggtaa ctatcgtctt gagtccaacc cggtaagaca cgacttatcg ccactggcag      5160
   cagccactgg taacaggatt agcagagcga ggtatgtagg cggtgctaca gagttcttga      5220
   agtggtggcc taactacggc tacactagaa ggacagtatt tggtatctgc gctctgctga      5280
   agccagttac cttcggaaaa agagttggta gctcttgatc cggcaaacaa accaccgctg      5340
   gtagcggtgg tttttttgtt tgcaagcagc agattacgcg cagaaaaaaa ggatctcaag      5400
   aagatccttt gatcttttct acggggtctg acgctcagtg gaacgaaaac tcacgttaag      5460
   ggattttggt catgagatta tcaaaaagga tcttcaccta gatcctttta aattaaaaat      5520
   gaagttttaa atcaatctaa agtatatatg agtaaacttg gtctgacagt taccaatgct      5580
   taatcagtga ggcacctatc tcagcgatct gtctatttcg ttcatccata gttgcctgac      5640
   tccccgtcgt gtagataact acgatacggg agggcttacc atctggcccc agtgctgcaa      5700
   tgataccgcg agacccacgc tcaccggctc cagatttatc agcaataaac cagccagccg      5760
   gaagggccga gcgcagaagt ggtcctgcaa ctttatccgc ctccatccag tctattaatt      5820
   gttgccggga agctagagta agtagttcgc cagttaatag tttgcgcaac gttgttgcca      5880
   ttgctacagg catcgtggtg tcacgctcgt cgtttggtat ggcttcattc agctccggtt      5940
   cccaacgatc aaggcgagtt acatgatccc ccatgttgtg caaaaaagcg gttagctcct      6000
   tcggtcctcc gatcgttgtc agaagtaagt tggccgcagt gttatcactc atggttatgg 6060
   cagcactgca taattctctt actgtcatgc catccgtaag atgcttttct gtgactggtg 6120
   agtactcaac caagtcattc tgagaatagt gtatgcggcg accgagttgc tcttgcccgg 6180
   cgtcaatacg ggataatacc gcgccacata gcagaacttt aaaagtgctc atcattggaa 6240
   aacgttcttc ggggcgaaaa ctctcaagga tcttaccgct gttgagatcc agttcgatgt 6300
   aacccactcg tgcacccaac tgatcttcag catcttttac tttcaccagc gtttctgggt 6360
   gagcaaaaac aggaaggcaa aatgccgcaa aaaagggaat aagggcgaca cggaaatgtt 6420
   gaatactcat actcttcctt tttcaatatt attgaagcat ttatcagggt tattgtctca 6480
   tgagcggata catatttgaa tgtatttaga aaaataaaca aataggggtt ccgcgcacat 6540
   ttccccgaaa agtgccacct gacgcgccct gtagcggcgc attaagcgcg gcgggtgtgg 6600
   tggttacgcg cagcgtgacc gctacacttg ccagcgccct agcgcccgct cctttcgctt 6660
   tcttcccttc ctttctcgcc acgttcgccg gctttccccg tcaagctcta aatcgggggc 6720
   tccctttagg gttccgattt agtgctttac ggcacctcga ccccaaaaaa cttgattagg 6780
   gtgatggttc acgtagtggg ccatcgccct gatagacggt ttttcgccct ttgacgttgg 6840
   agtccacgtt ctttaatagt ggactcttgt tccaaactgg aacaacactc aaccctatct 6900
   cggtctattc ttttgattta taagggattt tgccgatttc ggcctattgg ttaaaaaatg 6960
   agctgattta acaaaaattt aacgcgaatt ttaacaaaat attaacgctt acaatttcca 7020
   ttcgccattc aggctgcgca actgttggga agggcgatcg gtgcgggcct cttcgctatt 7080
   acgccagctg gcgaaagggg gatgtgctgc aaggcgatta agttgggtaa cgccagggtt 7140
   ttcccagtca cgacgttgta aaacgacggc cagtgaattg taatacgact cactataggg 7200
   cgaattgggt accgggcccc ccctcgaggt cgatggtgtc gataagcttg atatcgaatt 7260
   catgtcacac aaaccgatct tcgcctcaag gaaacctaat tctacatccg agagactgcc 7320
   gagatccagt ctacactgat taattttcgg gccaataatt taaaaaaatc gtgttatata 7380
   atattatatg tattatatat atacatcatg atgatactga cagtcatgtc ccattgctaa 7440
   atagacagac tccatctgcc gcctccaact gatgttctca atatttaagg ggtcatctcg 7500
   cattgtttaa taataaacag actccatcta ccgcctccaa atgatgttct caaaatatat 7560
   tgtatgaact tatttttatt acttagtatt attagacaac ttacttgctt tatgaaaaac 7620
   acttcctatt taggaaacaa tttataatgg cagttcgttc atttaacaat ttatgtagaa 7680
   taaatgttat aaatgcgtat gggaaatctt aaatatggat agcataaatg atatctgcat 7740
   tgcctaattc gaaatcaaca gcaacgaaaa aaatcccttg tacaacataa atagtcatcg 7800
   agaaatatca actatcaaag aacagctatt cacacgttac tattgagatt attattggac 7860
   gagaatcaca cactcaactg tctttctctc ttctagaaat acaggtacaa gtatgtacta 7920
   ttctcattgt tcatacttct agtcatttca tcccacatat tccttggatt tctctccaat 7980
   gaatgacatt ctatcttgca aattcaacaa ttataataag atataccaaa gtagcggtat 8040
   agtggcaatc aaaaagcttc tctggtgtgc ttctcgtatt tatttttatt ctaatgatcc 8100
   attaaaggta tatatttatt tcttgttata taatcctttt gtttattaca tgggctggat 8160
   acataaaggt attttgattt aattttttgc ttaaattcaa tcccccctcg ttcagtgtca 8220
   actgtaatgg taggaaatta ccatactttt gaagaagcaa aaaaaatgaa agaaaaaaaa 8280
   aatcgtattt ccaggttaga cgttccgcag aatctagaat gcggtatgcg gtacattgtt 8340
   cttcgaacgt aaaagttgcg ctccctgaga tattgtacat ttttgctttt acaagtacaa 8400
   gtacatcgta caactatgta ctactgttga tgcatccaca acagtttgtt ttgttttttt 8460
   ttgttttttt tttttctaat gattcattac cgctatgtat acctacttgt acttgtagta 8520
   agccgggtta ttggcgttca attaatcata gacttatgaa tctgcacggt gtgcgctgcg 8580
   agttactttt agcttatgca tgctacttgg gtgtaatatt gggatctgtt cggaaatcaa 8640
   cggatgctca atcgatttcg acagtaatta attaagtcat acacaagtca gctttcttcg 8700
   agcctcatat aagtataagt agttcaacgt attagcactg tacccagcat ctccgtatcg 8760
   agaaacacaa caacatgccc cattggacag atcatgcgga tacacaggtt gtgcagtatc 8820
   atacatactc gatcagacag gtcgtctgac catcatacaa gctgaacaag cgctccatac 8880
   ttgcacgctc tctatataca cagttaaatt acatatccat agtctaacct ctaacagtta 8940
   atcttctggt aagcctccca gccagccttc tggtatcgct tggcctcctc aataggatct 9000
   cggttctggc cgtacagacc tcggccgaca attatgatat ccgttccggt agacatgaca 9060
   tcctcaacag ttcggtactg ctgtccgaga gcgtctccct tgtcgtcaag acccaccccg 9120
   ggggtcagaa taagccagtc ctcagagtcg cccttaggtc ggttctgggc aatgaagcca 9180
   accacaaact cggggtcgga tcgggcaagc tcaatggtct gcttggagta ctcgccagtg 9240
   gccagagagc ccttgcaaga cagctcggcc agcatgagca gacctctggc cagcttctcg 9300
   ttgggagagg ggactaggaa ctccttgtac tgggagttct cgtagtcaga gacgtcctcc 9360
   ttcttctgtt cagagacagt ttcctcggca ccagctcgca ggccagcaat gattccggtt 9420
   ccgggtacac cgtgggcgtt ggtgatatcg gaccactcgg cgattcggtg acaccggtac 9480
   tggtgcttga cagtgttgcc aatatctgcg aactttctgt cctcgaacag gaagaaaccg 9540
   tgcttaagag caagttcctt gagggggagc acagtgccgg cgtaggtgaa gtcgtcaatg 9600
   atgtcgatat gggttttgat catgcacaca taaggtccga ccttatcggc aagctcaatg 9660
   agctccttgg tggtggtaac atccagagaa gcacacaggt tggttttctt ggctgccacg 9720
   agcttgagca ctcgagcggc aaaggcggac ttgtggacgt tagctcgagc ttcgtaggag 9780
   ggcattttgg tggtgaagag gagactgaaa taaatttagt ctgcagaact ttttatcgga      9840
   accttatctg gggcagtgaa gtatatgtta tggtaatagt tacgagttag ttgaacttat      9900
   agatagactg gactatacgg ctatcggtcc aaattagaaa gaacgtcaat ggctctctgg      9960
   gcgtcgcctt tgccgacaaa aatgtgatca tgatgaaagc cagcaatgac gttgcagctg     10020
   atattgttgt cggccaaccg cgccgaaaac gcagctgtca gacccacagc ctccaacgaa     10080
   gaatgtatcg tcaaagtgat ccaagcacac tcatagttgg agtcgtactc caaaggcggc     10140
   aatgacgagt cagacagata ctcgtcgacg tttaaaccat catctaaggg cctcaaaact     10200
   acctcggaac tgctgcgctg atctggacac cacagaggtt ccgagcactt taggttgcac     10260
   caaatgtccc accaggtgca ggcagaaaac gctggaacag cgtgtacagt ttgtcttaac     10320
   aaaaagtgag ggcgctgagg tcgagcaggg tggtgtgact tgttatagcc tttagagctg     10380
   cgaaagcgcg tatggatttg gctcatcagg ccagattgag ggtctgtgga cacatgtcat     10440
   gttagtgtac ttcaatcgcc ccctggatat agccccgaca ataggccgtg gcctcatttt     10500
   tttgccttcc gcacatttcc attgctcggt acccacacct tgcttctcct gcacttgcca     10560
   accttaatac tggtttacat tgaccaacat cttacaagcg gggggcttgt ctagggtata     10620
   tataaacagt ggctctccca atcggttgcc agtctctttt ttcctttctt tccccacaga     10680
   ttcgaaatct aaactacaca tcacac                                          10706
<210> 7
   <211> 47
   <212> DNA
   <213> Artificial sequence
<220>
   <223> AarI-removal 1
<400> 7
   agaagtatcc taccatctac catctccgaa agaaactcgt cgattcc 47
<210> 8
   <211> 47
   <212> DNA
   <213> Artificial sequence
<220>
   <223> AarI-removal 2
<400> 8
   ggaatcgacg agtttctttc ggagatggta gatggtagga tacttct 47
<210> 9
   <211> 10706
   <212> DNA
   <213> Artificial sequence
<220>
   <223> pRF109
<400> 9
   catggacaag aaatactcca tcggcctgga cattggaacc aactctgtcg gctgggctgt      60
   catcaccgac gagtacaagg tgccctccaa gaaattcaag gtcctcggaa acaccgatcg     120
   acactccatc aagaaaaacc tcattggtgc cctgttgttc gattctggcg agactgccga     180
   agctaccaga ctcaagcgaa ctgctcggcg acgttacacc cgacggaaga accgaatctg     240
   ctacctgcag gagatctttt ccaacgagat ggccaaggtg gacgattcgt tctttcatcg     300
   actggaggaa tccttcctcg tcgaggaaga caagaaacac gagcgtcatc ccatctttgg     360
   caacattgtg gacgaggttg cttaccacga gaagtatcct accatctacc atctccgaaa     420
   gaaactcgtc gattccaccg acaaggcgga tctcagactt atctacctcg ctctggcaca     480
   catgatcaag tttcgaggtc atttcctcat cgagggcgat ctcaatcccg acaacagcga     540
   tgtggacaag ctgttcattc agctcgttca gacctacaac cagctgttcg aggaaaaccc     600
   catcaatgcc tccggagtcg atgcaaaggc catcttgtct gctcgactct cgaagagcag     660
   acgactggag aacctcattg cccaacttcc tggcgagaaa aagaacggac tgtttggcaa     720
   cctcattgcc ctttctcttg gtctcacacc caacttcaag tccaacttcg atctggcgga     780
   ggacgccaag ctccagctgt ccaaggacac ctacgacgat gacctcgaca acctgcttgc     840
   acagattggc gatcagtacg ccgacctgtt tctcgctgcc aagaaccttt cggatgctat     900
   tctcttgtct gacattctgc gagtcaacac cgagatcaca aaggctcccc tttctgcctc     960
   catgatcaag cgatacgacg agcaccatca ggatctcaca ctgctcaagg ctcttgtccg    1020
   acagcaactg cccgagaagt acaaggagat ctttttcgat cagtcgaaga acggctacgc    1080
   tggatacatc gacggcggag cctctcagga agagttctac aagttcatca agccaattct    1140
   cgagaagatg gacggaaccg aggaactgct tgtcaagctc aatcgagagg atctgcttcg    1200
   gaagcaacga accttcgaca acggcagcat tcctcatcag atccacctcg gtgagctgca    1260
   cgccattctt cgacgtcagg aagacttcta cccctttctc aaggacaacc gagagaagat    1320
   cgagaagatt cttacctttc gaatccccta ctatgttggt cctcttgcca gaggaaactc    1380
   tcgatttgct tggatgactc gaaagtccga ggaaaccatc actccctgga acttcgagga    1440
   agtcgtggac aagggtgcct ctgcacagtc cttcatcgag cgaatgacca acttcgacaa    1500
   gaatctgccc aacgagaagg ttcttcccaa gcattcgctg ctctacgagt actttacagt    1560
   ctacaacgaa ctcaccaaag tcaagtacgt taccgaggga atgcgaaagc ctgccttctt    1620
   gtctggcgaa cagaagaaag ccattgtcga tctcctgttc aagaccaacc gaaaggtcac    1680
   tgttaagcag ctcaaggagg actacttcaa gaaaatcgag tgtttcgaca gcgtcgagat    1740
   ttccggagtt gaggaccgat tcaacgcctc tttgggcacc tatcacgatc tgctcaagat    1800
   tatcaaggac aaggattttc tcgacaacga ggaaaacgag gacattctgg aggacatcgt    1860
   gctcactctt accctgttcg aagatcggga gatgatcgag gaacgactca agacatacgc    1920
   tcacctgttc gacgacaagg tcatgaaaca actcaagcga cgtagataca ccggctgggg    1980
   aagactttcg cgaaagctca tcaacggcat cagagacaag cagtccggaa agaccattct    2040
   ggactttctc aagtccgatg gctttgccaa ccgaaacttc atgcagctca ttcacgacga    2100
   ttctcttacc ttcaaggagg acatccagaa ggcacaagtg tccggtcagg gcgacagctt    2160
   gcacgaacat attgccaacc tggctggttc gccagccatc aagaaaggca ttctccagac    2220
   tgtcaaggtt gtcgacgagc tggtgaaggt catgggacgt cacaagcccg agaacattgt    2280
   gatcgagatg gccagagaga accagacaac tcaaaagggt cagaaaaact cgcgagagcg    2340
   gatgaagcga atcgaggaag gcatcaagga gctgggatcc cagattctca aggagcatcc    2400
   cgtcgagaac actcaactgc agaacgagaa gctgtatctc tactatctgc agaatggtcg    2460
   agacatgtac gtggatcagg aactggacat caatcgtctc agcgactacg atgtggacca    2520
   cattgtccct caatcctttc tcaaggacga ttctatcgac aacaaggtcc ttacacgatc    2580
   cgacaagaac agaggcaagt cggacaacgt tcccagcgaa gaggtggtca aaaagatgaa    2640
   gaactactgg cgacagctgc tcaacgccaa gctcattacc cagcgaaagt tcgacaatct    2700
   taccaaggcc gagcgaggcg gtctgtccga gctcgacaag gctggcttca tcaagcgtca    2760
   actcgtcgag accagacaga tcacaaagca cgtcgcacag attctcgatt ctcggatgaa    2820
   caccaagtac gacgagaacg acaagctcat ccgagaggtc aaggtgatta ctctcaagtc    2880
   caaactggtc tccgatttcc gaaaggactt tcagttctac aaggtgcgag agatcaacaa    2940
   ttaccaccat gcccacgatg cttacctcaa cgccgtcgtt ggcactgcgc tcatcaagaa    3000
   ataccccaag ctcgaaagcg agttcgttta cggcgattac aaggtctacg acgttcgaaa    3060
   gatgattgcc aagtccgaac aggagattgg caaggctact gccaagtact tcttttactc    3120
   caacatcatg aactttttca agaccgagat caccttggcc aacggagaga ttcgaaagag    3180
   accacttatc gagaccaacg gcgaaactgg agagatcgtg tgggacaagg gtcgagactt    3240
   tgcaaccgtg cgaaaggttc tgtcgatgcc tcaggtcaac atcgtcaaga aaaccgaggt    3300
   tcagactggc ggattctcca aggagtcgat tctgcccaag cgaaactccg acaagctcat    3360
   cgctcgaaag aaagactggg atcccaagaa atacggtggc ttcgattctc ctaccgtcgc    3420
   ctattccgtg cttgtcgttg cgaaggtcga gaagggcaag tccaaaaagc tcaagtccgt    3480
   caaggagctg ctcggaatta ccatcatgga gcgatcgagc ttcgagaaga atcccatcga    3540
   cttcttggaa gccaagggtt acaaggaggt caagaaagac ctcattatca agctgcccaa    3600
   gtactctctg ttcgaactgg agaacggtcg aaagcgtatg ctcgcctccg ctggcgagct    3660
   gcagaaggga aacgagcttg ccttgccttc gaagtacgtc aactttctct atctggcttc    3720
   tcactacgag aagctcaagg gttctcccga ggacaacgaa cagaagcaac tcttcgttga    3780
   gcagcacaaa cattacctcg acgagattat cgagcagatt tccgagtttt cgaagcgagt    3840
   catcctggct gatgccaact tggacaaggt gctctctgcc tacaacaagc atcgggacaa    3900
   acccattcga gaacaggcgg agaacatcat tcacctgttt actcttacca acctgggtgc    3960
   tcctgcagct ttcaagtact tcgataccac tatcgaccga aagcggtaca catccaccaa    4020
   ggaggttctc gatgccaccc tgattcacca gtccatcact ggcctgtacg agacccgaat    4080
   cgacctgtct cagcttggtg gcgactccag agccgatccc aagaaaaagc gaaaggtcta    4140
   agcggccgca agtgtggatg gggaagtgag tgcccggttc tgtgtgcaca attggcaatc    4200
   caagatggat ggattcaaca cagggatata gcgagctacg tggtggtgcg aggatatagc    4260
   aacggatatt tatgtttgac acttgagaat gtacgataca agcactgtcc aagtacaata    4320
   ctaaacatac tgtacatact catactcgta cccgggcaac ggtttcactt gagtgcagtg    4380
   gctagtgctc ttactcgtac agtgtgcaat actgcgtatc atagtctttg atgtatatcg    4440
   tattcattca tgttagttgc gtacgagccg gaagcataaa gtgtaaagcc tggggtgcct    4500
   aatgagtgag ctaactcaca ttaattgcgt tgcgctcact gcccgctttc cagtcgggaa    4560
   acctgtcgtg ccagctgcat taatgaatcg gccaacgcgc ggggagaggc ggtttgcgta    4620
   ttgggcgctc ttccgcttcc tcgctcactg actcgctgcg ctcggtcgtt cggctgcggc    4680
   gagcggtatc agctcactca aaggcggtaa tacggttatc cacagaatca ggggataacg 4740
   caggaaagaa catgtgagca aaaggccagc aaaaggccag gaaccgtaaa aaggccgcgt 4800
   tgctggcgtt tttccatagg ctccgccccc ctgacgagca tcacaaaaat cgacgctcaa 4860
   gtcagaggtg gcgaaacccg acaggactat aaagatacca ggcgtttccc cctggaagct 4920
   ccctcgtgcg ctctcctgtt ccgaccctgc cgcttaccgg atacctgtcc gcctttctcc 4980
   cttcgggaag cgtggcgctt tctcatagct cacgctgtag gtatctcagt tcggtgtagg 5040
   tcgttcgctc caagctgggc tgtgtgcacg aaccccccgt tcagcccgac cgctgcgcct 5100
   tatccggtaa ctatcgtctt gagtccaacc cggtaagaca cgacttatcg ccactggcag 5160
   cagccactgg taacaggatt agcagagcga ggtatgtagg cggtgctaca gagttcttga 5220
   agtggtggcc taactacggc tacactagaa ggacagtatt tggtatctgc gctctgctga 5280
   agccagttac cttcggaaaa agagttggta gctcttgatc cggcaaacaa accaccgctg 5340
   gtagcggtgg tttttttgtt tgcaagcagc agattacgcg cagaaaaaaa ggatctcaag 5400
   aagatccttt gatcttttct acggggtctg acgctcagtg gaacgaaaac tcacgttaag 5460
   ggattttggt catgagatta tcaaaaagga tcttcaccta gatcctttta aattaaaaat 5520
   gaagttttaa atcaatctaa agtatatatg agtaaacttg gtctgacagt taccaatgct 5580
   taatcagtga ggcacctatc tcagcgatct gtctatttcg ttcatccata gttgcctgac 5640
   tccccgtcgt gtagataact acgatacggg agggcttacc atctggcccc agtgctgcaa 5700
   tgataccgcg agacccacgc tcaccggctc cagatttatc agcaataaac cagccagccg 5760
   gaagggccga gcgcagaagt ggtcctgcaa ctttatccgc ctccatccag tctattaatt 5820
   gttgccggga agctagagta agtagttcgc cagttaatag tttgcgcaac gttgttgcca 5880
   ttgctacagg catcgtggtg tcacgctcgt cgtttggtat ggcttcattc agctccggtt 5940
   cccaacgatc aaggcgagtt acatgatccc ccatgttgtg caaaaaagcg gttagctcct 6000
   tcggtcctcc gatcgttgtc agaagtaagt tggccgcagt gttatcactc atggttatgg 6060
   cagcactgca taattctctt actgtcatgc catccgtaag atgcttttct gtgactggtg 6120
   agtactcaac caagtcattc tgagaatagt gtatgcggcg accgagttgc tcttgcccgg 6180
   cgtcaatacg ggataatacc gcgccacata gcagaacttt aaaagtgctc atcattggaa 6240
   aacgttcttc ggggcgaaaa ctctcaagga tcttaccgct gttgagatcc agttcgatgt 6300
   aacccactcg tgcacccaac tgatcttcag catcttttac tttcaccagc gtttctgggt 6360
   gagcaaaaac aggaaggcaa aatgccgcaa aaaagggaat aagggcgaca cggaaatgtt 6420
   gaatactcat actcttcctt tttcaatatt attgaagcat ttatcagggt tattgtctca 6480
   tgagcggata catatttgaa tgtatttaga aaaataaaca aataggggtt ccgcgcacat 6540
   ttccccgaaa agtgccacct gacgcgccct gtagcggcgc attaagcgcg gcgggtgtgg 6600
   tggttacgcg cagcgtgacc gctacacttg ccagcgccct agcgcccgct cctttcgctt 6660
   tcttcccttc ctttctcgcc acgttcgccg gctttccccg tcaagctcta aatcgggggc 6720
   tccctttagg gttccgattt agtgctttac ggcacctcga ccccaaaaaa cttgattagg 6780
   gtgatggttc acgtagtggg ccatcgccct gatagacggt ttttcgccct ttgacgttgg 6840
   agtccacgtt ctttaatagt ggactcttgt tccaaactgg aacaacactc aaccctatct 6900
   cggtctattc ttttgattta taagggattt tgccgatttc ggcctattgg ttaaaaaatg 6960
   agctgattta acaaaaattt aacgcgaatt ttaacaaaat attaacgctt acaatttcca 7020
   ttcgccattc aggctgcgca actgttggga agggcgatcg gtgcgggcct cttcgctatt 7080
   acgccagctg gcgaaagggg gatgtgctgc aaggcgatta agttgggtaa cgccagggtt 7140
   ttcccagtca cgacgttgta aaacgacggc cagtgaattg taatacgact cactataggg 7200
   cgaattgggt accgggcccc ccctcgaggt cgatggtgtc gataagcttg atatcgaatt 7260
   catgtcacac aaaccgatct tcgcctcaag gaaacctaat tctacatccg agagactgcc 7320
   gagatccagt ctacactgat taattttcgg gccaataatt taaaaaaatc gtgttatata 7380
   atattatatg tattatatat atacatcatg atgatactga cagtcatgtc ccattgctaa 7440
   atagacagac tccatctgcc gcctccaact gatgttctca atatttaagg ggtcatctcg 7500
   cattgtttaa taataaacag actccatcta ccgcctccaa atgatgttct caaaatatat 7560
   tgtatgaact tatttttatt acttagtatt attagacaac ttacttgctt tatgaaaaac 7620
   acttcctatt taggaaacaa tttataatgg cagttcgttc atttaacaat ttatgtagaa 7680
   taaatgttat aaatgcgtat gggaaatctt aaatatggat agcataaatg atatctgcat 7740
   tgcctaattc gaaatcaaca gcaacgaaaa aaatcccttg tacaacataa atagtcatcg 7800
   agaaatatca actatcaaag aacagctatt cacacgttac tattgagatt attattggac 7860
   gagaatcaca cactcaactg tctttctctc ttctagaaat acaggtacaa gtatgtacta 7920
   ttctcattgt tcatacttct agtcatttca tcccacatat tccttggatt tctctccaat 7980
   gaatgacatt ctatcttgca aattcaacaa ttataataag atataccaaa gtagcggtat 8040
   agtggcaatc aaaaagcttc tctggtgtgc ttctcgtatt tatttttatt ctaatgatcc 8100
   attaaaggta tatatttatt tcttgttata taatcctttt gtttattaca tgggctggat 8160
   acataaaggt attttgattt aattttttgc ttaaattcaa tcccccctcg ttcagtgtca 8220
   actgtaatgg taggaaatta ccatactttt gaagaagcaa aaaaaatgaa agaaaaaaaa 8280
   aatcgtattt ccaggttaga cgttccgcag aatctagaat gcggtatgcg gtacattgtt 8340
   cttcgaacgt aaaagttgcg ctccctgaga tattgtacat ttttgctttt acaagtacaa 8400
   gtacatcgta caactatgta ctactgttga tgcatccaca acagtttgtt ttgttttttt 8460
   ttgttttttt tttttctaat gattcattac cgctatgtat acctacttgt acttgtagta 8520
   agccgggtta ttggcgttca attaatcata gacttatgaa tctgcacggt gtgcgctgcg 8580
   agttactttt agcttatgca tgctacttgg gtgtaatatt gggatctgtt cggaaatcaa 8640
   cggatgctca atcgatttcg acagtaatta attaagtcat acacaagtca gctttcttcg 8700
   agcctcatat aagtataagt agttcaacgt attagcactg tacccagcat ctccgtatcg 8760
   agaaacacaa caacatgccc cattggacag atcatgcgga tacacaggtt gtgcagtatc 8820
   atacatactc gatcagacag gtcgtctgac catcatacaa gctgaacaag cgctccatac 8880
   ttgcacgctc tctatataca cagttaaatt acatatccat agtctaacct ctaacagtta 8940
   atcttctggt aagcctccca gccagccttc tggtatcgct tggcctcctc aataggatct 9000
   cggttctggc cgtacagacc tcggccgaca attatgatat ccgttccggt agacatgaca 9060
   tcctcaacag ttcggtactg ctgtccgaga gcgtctccct tgtcgtcaag acccaccccg 9120
   ggggtcagaa taagccagtc ctcagagtcg cccttaggtc ggttctgggc aatgaagcca 9180
   accacaaact cggggtcgga tcgggcaagc tcaatggtct gcttggagta ctcgccagtg 9240
   gccagagagc ccttgcaaga cagctcggcc agcatgagca gacctctggc cagcttctcg 9300
   ttgggagagg ggactaggaa ctccttgtac tgggagttct cgtagtcaga gacgtcctcc 9360
   ttcttctgtt cagagacagt ttcctcggca ccagctcgca ggccagcaat gattccggtt 9420
   ccgggtacac cgtgggcgtt ggtgatatcg gaccactcgg cgattcggtg acaccggtac 9480
   tggtgcttga cagtgttgcc aatatctgcg aactttctgt cctcgaacag gaagaaaccg 9540
   tgcttaagag caagttcctt gagggggagc acagtgccgg cgtaggtgaa gtcgtcaatg 9600
   atgtcgatat gggttttgat catgcacaca taaggtccga ccttatcggc aagctcaatg 9660
   agctccttgg tggtggtaac atccagagaa gcacacaggt tggttttctt ggctgccacg 9720
   agcttgagca ctcgagcggc aaaggcggac ttgtggacgt tagctcgagc ttcgtaggag 9780
   ggcattttgg tggtgaagag gagactgaaa taaatttagt ctgcagaact ttttatcgga 9840
   accttatctg gggcagtgaa gtatatgtta tggtaatagt tacgagttag ttgaacttat 9900
   agatagactg gactatacgg ctatcggtcc aaattagaaa gaacgtcaat ggctctctgg 9960
   gcgtcgcctt tgccgacaaa aatgtgatca tgatgaaagc cagcaatgac gttgcagctg 10020
   atattgttgt cggccaaccg cgccgaaaac gcagctgtca gacccacagc ctccaacgaa 10080
   gaatgtatcg tcaaagtgat ccaagcacac tcatagttgg agtcgtactc caaaggcggc 10140
   aatgacgagt cagacagata ctcgtcgacg tttaaaccat catctaaggg cctcaaaact 10200
   acctcggaac tgctgcgctg atctggacac cacagaggtt ccgagcactt taggttgcac 10260
   caaatgtccc accaggtgca ggcagaaaac gctggaacag cgtgtacagt ttgtcttaac 10320
   aaaaagtgag ggcgctgagg tcgagcaggg tggtgtgact tgttatagcc tttagagctg 10380
   cgaaagcgcg tatggatttg gctcatcagg ccagattgag ggtctgtgga cacatgtcat 10440
   gttagtgtac ttcaatcgcc ccctggatat agccccgaca ataggccgtg gcctcatttt 10500
   tttgccttcc gcacatttcc attgctcggt acccacacct tgcttctcct gcacttgcca 10560
   accttaatac tggtttacat tgaccaacat cttacaagcg gggggcttgt ctagggtata 10620
   tataaacagt ggctctccca atcggttgcc agtctctttt ttcctttctt tccccacaga 10680
   ttcgaaatct aaactacaca tcacac 10706
<210> 10
   <211> 4140
   <212> DNA
   <213> Artificial sequence
<220>
   <223> Aarl- Cas9 ORF
<400> 10
   atggacaaga aatactccat cggcctggac attggaacca actctgtcgg ctgggctgtc      60
   atcaccgacg agtacaaggt gccctccaag aaattcaagg tcctcggaaa caccgatcga     120
   cactccatca agaaaaacct cattggtgcc ctgttgttcg attctggcga gactgccgaa     180
   gctaccagac tcaagcgaac tgctcggcga cgttacaccc gacggaagaa ccgaatctgc     240
   tacctgcagg agatcttttc caacgagatg gccaaggtgg acgattcgtt ctttcatcga     300
   ctggaggaat ccttcctcgt cgaggaagac aagaaacacg agcgtcatcc catctttggc     360
   aacattgtgg acgaggttgc ttaccacgag aagtatccta ccatctacca tctccgaaag     420
   aaactcgtcg attccaccga caaggcggat ctcagactta tctacctcgc tctggcacac     480
   atgatcaagt ttcgaggtca tttcctcatc gagggcgatc tcaatcccga caacagcgat     540
   gtggacaagc tgttcattca gctcgttcag acctacaacc agctgttcga ggaaaacccc     600
   atcaatgcct ccggagtcga tgcaaaggcc atcttgtctg ctcgactctc gaagagcaga     660
   cgactggaga acctcattgc ccaacttcct ggcgagaaaa agaacggact gtttggcaac     720
   ctcattgccc tttctcttgg tctcacaccc aacttcaagt ccaacttcga tctggcggag     780
   gacgccaagc tccagctgtc caaggacacc tacgacgatg acctcgacaa cctgcttgca     840
   cagattggcg atcagtacgc cgacctgttt ctcgctgcca agaacctttc ggatgctatt     900
   ctcttgtctg acattctgcg agtcaacacc gagatcacaa aggctcccct ttctgcctcc 960
   atgatcaagc gatacgacga gcaccatcag gatctcacac tgctcaaggc tcttgtccga 1020
   cagcaactgc ccgagaagta caaggagatc tttttcgatc agtcgaagaa cggctacgct 1080
   ggatacatcg acggcggagc ctctcaggaa gagttctaca agttcatcaa gccaattctc 1140
   gagaagatgg acggaaccga ggaactgctt gtcaagctca atcgagagga tctgcttcgg 1200
   aagcaacgaa ccttcgacaa cggcagcatt cctcatcaga tccacctcgg tgagctgcac 1260
   gccattcttc gacgtcagga agacttctac ccctttctca aggacaaccg agagaagatc 1320
   gagaagattc ttacctttcg aatcccctac tatgttggtc ctcttgccag aggaaactct 1380
   cgatttgctt ggatgactcg aaagtccgag gaaaccatca ctccctggaa cttcgaggaa 1440
   gtcgtggaca agggtgcctc tgcacagtcc ttcatcgagc gaatgaccaa cttcgacaag 1500
   aatctgccca acgagaaggt tcttcccaag cattcgctgc tctacgagta ctttacagtc 1560
   tacaacgaac tcaccaaagt caagtacgtt accgagggaa tgcgaaagcc tgccttcttg 1620
   tctggcgaac agaagaaagc cattgtcgat ctcctgttca agaccaaccg aaaggtcact 1680
   gttaagcagc tcaaggagga ctacttcaag aaaatcgagt gtttcgacag cgtcgagatt 1740
   tccggagttg aggaccgatt caacgcctct ttgggcacct atcacgatct gctcaagatt 1800
   atcaaggaca aggattttct cgacaacgag gaaaacgagg acattctgga ggacatcgtg 1860
   ctcactctta ccctgttcga agatcgggag atgatcgagg aacgactcaa gacatacgct 1920
   cacctgttcg acgacaaggt catgaaacaa ctcaagcgac gtagatacac cggctgggga 1980
   agactttcgc gaaagctcat caacggcatc agagacaagc agtccggaaa gaccattctg 2040
   gactttctca agtccgatgg ctttgccaac cgaaacttca tgcagctcat tcacgacgat 2100
   tctcttacct tcaaggagga catccagaag gcacaagtgt ccggtcaggg cgacagcttg 2160
   cacgaacata ttgccaacct ggctggttcg ccagccatca agaaaggcat tctccagact 2220
   gtcaaggttg tcgacgagct ggtgaaggtc atgggacgtc acaagcccga gaacattgtg 2280
   atcgagatgg ccagagagaa ccagacaact caaaagggtc agaaaaactc gcgagagcgg 2340
   atgaagcgaa tcgaggaagg catcaaggag ctgggatccc agattctcaa ggagcatccc 2400
   gtcgagaaca ctcaactgca gaacgagaag ctgtatctct actatctgca gaatggtcga 2460
   gacatgtacg tggatcagga actggacatc aatcgtctca gcgactacga tgtggaccac 2520
   attgtccctc aatcctttct caaggacgat tctatcgaca acaaggtcct tacacgatcc 2580
   gacaagaaca gaggcaagtc ggacaacgtt cccagcgaag aggtggtcaa aaagatgaag 2640
   aactactggc gacagctgct caacgccaag ctcattaccc agcgaaagtt cgacaatctt 2700
   accaaggccg agcgaggcgg tctgtccgag ctcgacaagg ctggcttcat caagcgtcaa 2760
   ctcgtcgaga ccagacagat cacaaagcac gtcgcacaga ttctcgattc tcggatgaac 2820
   accaagtacg acgagaacga caagctcatc cgagaggtca aggtgattac tctcaagtcc 2880
   aaactggtct ccgatttccg aaaggacttt cagttctaca aggtgcgaga gatcaacaat 2940
   taccaccatg cccacgatgc ttacctcaac gccgtcgttg gcactgcgct catcaagaaa 3000
   taccccaagc tcgaaagcga gttcgtttac ggcgattaca aggtctacga cgttcgaaag 3060
   atgattgcca agtccgaaca ggagattggc aaggctactg ccaagtactt cttttactcc 3120
   aacatcatga actttttcaa gaccgagatc accttggcca acggagagat tcgaaagaga 3180
   ccacttatcg agaccaacgg cgaaactgga gagatcgtgt gggacaaggg tcgagacttt 3240
   gcaaccgtgc gaaaggttct gtcgatgcct caggtcaaca tcgtcaagaa aaccgaggtt 3300
   cagactggcg gattctccaa ggagtcgatt ctgcccaagc gaaactccga caagctcatc 3360
   gctcgaaaga aagactggga tcccaagaaa tacggtggct tcgattctcc taccgtcgcc 3420
   tattccgtgc ttgtcgttgc gaaggtcgag aagggcaagt ccaaaaagct caagtccgtc 3480
   aaggagctgc tcggaattac catcatggag cgatcgagct tcgagaagaa tcccatcgac 3540
   ttcttggaag ccaagggtta caaggaggtc aagaaagacc tcattatcaa gctgcccaag 3600
   tactctctgt tcgaactgga gaacggtcga aagcgtatgc tcgcctccgc tggcgagctg 3660
   cagaagggaa acgagcttgc cttgccttcg aagtacgtca actttctcta tctggcttct 3720
   cactacgaga agctcaaggg ttctcccgag gacaacgaac agaagcaact cttcgttgag 3780
   cagcacaaac attacctcga cgagattatc gagcagattt ccgagttttc gaagcgagtc 3840
   atcctggctg atgccaactt ggacaaggtg ctctctgcct acaacaagca tcgggacaaa 3900
   cccattcgag aacaggcgga gaacatcatt cacctgttta ctcttaccaa cctgggtgct 3960
   cctgcagctt tcaagtactt cgataccact atcgaccgaa agcggtacac atccaccaag 4020
   gaggttctcg atgccaccct gattcaccag tccatcactg gcctgtacga gacccgaatc 4080
   gacctgtctc agcttggtgg cgactccaga gccgatccca agaaaaagcg aaaggtctaa 4140
<210> 11
   <211> 10706
   <212> DNA
   <213> Artificial sequence
<220>
   <223> pRF141
<400> 11
   catggacaag aaatactcca tcggcctgga cattggaacc aactctgtcg gctgggctgt      60
   catcaccgac gagtacaagg tgccctccaa gaaattcaag gtcctcggaa acaccgatcg     120
   acactccatc aagaaaaacc tcattggtgc cctgttgttc gattctggcg agactgccga     180
   agctaccaga ctcaagcgaa ctgctcggcg acgttacacc cgacggaaga accgaatctg     240
   ctacctgcag gagatctttt ccaacgagat ggccaaggtg gacgattcgt tctttcatcg     300
   actggaggaa tccttcctcg tcgaggaaga caagaaacac gagcgtcatc ccatctttgg     360
   caacattgtg gacgaggttg cttaccacga gaagtatcct accatctacc atctccgaaa     420
   gaaactcgtc gattccaccg acaaggcgga tctcagactt atctacctcg ctctggcaca     480
   catgatcaag tttcgaggtc atttcctcat cgagggcgat ctcaatcccg acaacagcga     540
   tgtggacaag ctgttcattc agctcgttca gacctacaac cagctgttcg aggaaaaccc     600
   catcaatgcc tccggagtcg atgcaaaggc catcttgtct gctcgactct cgaagagcag     660
   acgactggag aacctcattg cccaacttcc tggcgagaaa aagaacggac tgtttggcaa     720
   cctcattgcc ctttctcttg gtctcacacc caacttcaag tccaacttcg atctggcgga     780
   ggacgccaag ctccagctgt ccaaggacac ctacgacgat gacctcgaca acctgcttgc     840
   acagattggc gatcagtacg ccgacctgtt tctcgctgcc aagaaccttt cggatgctat     900
   tctcttgtct gacattctgc gagtcaacac cgagatcaca aaggctcccc tttctgcctc     960
   catgatcaag cgatacgacg agcaccatca ggatctcaca ctgctcaagg ctcttgtccg    1020
   acagcaactg cccgagaagt acaaggagat ctttttcgat cagtcgaaga acggctacgc    1080
   tggatacatc gacggcggag cctctcagga agagttctac aagttcatca agccaattct    1140
   cgagaagatg gacggaaccg aggaactgct tgtcaagctc aatcgagagg atctgcttcg    1200
   gaagcaacga accttcgaca acggcagcat tcctcatcag atccacctcg gtgagctgca    1260
   cgccattctt cgacgtcagg aagacttcta cccctttctc aaggacaacc gagagaagat    1320
   cgagaagatt cttacctttc gaatccccta ctatgttggt cctcttgcca gaggaaactc    1380
   tcgatttgct tggatgactc gaaagtccga ggaaaccatc actccctgga acttcgagga    1440
   agtcgtggac aagggtgcct ctgcacagtc cttcatcgag cgaatgacca acttcgacaa    1500
   gaatctgccc aacgagaagg ttcttcccaa gcattcgctg ctctacgagt actttacagt    1560
   ctacaacgaa ctcaccaaag tcaagtacgt taccgaggga atgcgaaagc ctgccttctt    1620
   gtctggcgaa cagaagaaag ccattgtcga tctcctgttc aagaccaacc gaaaggtcac    1680
   tgttaagcag ctcaaggagg actacttcaa gaaaatcgag tgtttcgaca gcgtcgagat    1740
   ttccggagtt gaggaccgat tcaacgcctc tttgggcacc tatcacgatc tgctcaagat    1800
   tatcaaggac aaggattttc tcgacaacga ggaaaacgag gacattctgg aggacatcgt    1860
   gctcactctt accctgttcg aagatcggga gatgatcgag gaacgactca agacatacgc    1920
   tcacctgttc gacgacaagg tcatgaaaca actcaagcga cgtagataca ccggctgggg    1980
   aagactttcg cgaaagctca tcaacggcat cagagacaag cagtccggaa agaccattct    2040
   ggactttctc aagtccgatg gctttgccaa ccgaaacttc atgcagctca ttcacgacga    2100
   ttctcttacc ttcaaggagg acatccagaa ggcacaagtg tccggtcagg gcgacagctt    2160
   gcacgaacat attgccaacc tggctggttc gccagccatc aagaaaggca ttctccagac    2220
   tgtcaaggtt gtcgacgagc tggtgaaggt catgggacgt cacaagcccg agaacattgt    2280
   gatcgagatg gccagagaga accagacaac tcaaaagggt cagaaaaact cgcgagagcg    2340
   gatgaagcga atcgaggaag gcatcaagga gctgggatcc cagattctca aggagcatcc    2400
   cgtcgagaac actcaactgc agaacgagaa gctgtatctc tactatctgc agaatggtcg    2460
   agacatgtac gtggatcagg aactggacat caatcgtctc agcgactacg atgtggacca    2520
   cattgtccct caatcctttc tcaaggacga ttctatcgac aacaaggtcc ttacacgatc    2580
   cgacaagaac agaggcaagt cggacaacgt tcccagcgaa gaggtggtca aaaagatgaa    2640
   gaactactgg cgacagctgc tcaacgccaa gctcattacc cagcgaaagt tcgacaatct    2700
   taccaaggcc gagcgaggcg gtctgtccga gctcgacaag gctggcttca tcaagcgtca    2760
   actcgtcgag accagacaga tcacaaagca cgtcgcacag attctcgatt ctcggatgaa    2820
   caccaagtac gacgagaacg acaagctcat ccgagaggtc aaggtgatta ctctcaagtc    2880
   caaactggtc tccgatttcc gaaaggactt tcagttctac aaggtgcgag agatcaacaa    2940
   ttaccaccat gcccacgatg cttacctcaa cgccgtcgtt ggcactgcgc tcatcaagaa    3000
   ataccccaag ctcgaaagcg agttcgttta cggcgattac aaggtctacg acgttcgaaa    3060
   gatgattgcc aagtccgaac aggagattgg caaggctact gccaagtact tcttttactc    3120
   caacatcatg aactttttca agaccgagat caccttggcc aacggagaga ttcgaaagag    3180
   accacttatc gagaccaacg gcgaaactgg agagatcgtg tgggacaagg gtcgagactt    3240
   tgcaaccgtg cgaaaggttc tgtcgatgcc tcaggtcaac atcgtcaaga aaaccgaggt    3300
   tcagactggc ggattctcca aggagtcgat tctgcccaag cgaaactccg acaagctcat    3360
   cgctcgaaag aaagactggg atcccaagaa atacggtggc ttcgattctc ctaccgtcgc    3420
   ctattccgtg cttgtcgttg cgaaggtcga gaagggcaag tccaaaaagc tcaagtccgt    3480
   caaggagctg ctcggaatta ccatcatgga gcgatcgagc ttcgagaaga atcccatcga    3540
   cttcttggaa gccaagggtt acaaggaggt caagaaagac ctcattatca agctgcccaa    3600
   gtactctctg ttcgaactgg agaacggtcg aaagcgtatg ctcgcctccg ctggcgagct    3660
   gcagaaggga aacgagcttg ccttgccttc gaagtacgtc aactttctct atctggcttc    3720
   tcactacgag aagctcaagg gttctcccga ggacaacgaa cagaagcaac tcttcgttga 3780
   gcagcacaaa cattacctcg acgagattat cgagcagatt tccgagtttt cgaagcgagt 3840
   catcctggct gatgccaact tggacaaggt gctctctgcc tacaacaagc atcgggacaa 3900
   acccattcga gaacaggcgg agaacatcat tcacctgttt actcttacca acctgggtgc 3960
   tcctgcagct ttcaagtact tcgataccac tatcgaccga aagcggtaca catccaccaa 4020
   ggaggttctc gatgccaccc tgattcacca gtccatcact ggcctgtacg agacccgaat 4080
   cgacctgtct cagcttggtg gcgactccag agccgatccc aagaaaaagc gaaaggtcta 4140
   agcggccgca agtgtggatg gggaagtgag tgcccggttc tgtgtgcaca attggcaatc 4200
   caagatggat ggattcaaca cagggatata gcgagctacg tggtggtgcg aggatatagc 4260
   aacggatatt tatgtttgac acttgagaat gtacgataca agcactgtcc aagtacaata 4320
   ctaaacatac tgtacatact catactcgta cccgggcaac ggtttcactt gagtgcagtg 4380
   gctagtgctc ttactcgtac agtgtgcaat actgcgtatc atagtctttg atgtatatcg 4440
   tattcattca tgttagttgc gtacgagccg gaagcataaa gtgtaaagcc tggggtgcct 4500
   aatgagtgag ctaactcaca ttaattgcgt tgcgctcact gcccgctttc cagtcgggaa 4560
   acctgtcgtg ccagctgcat taatgaatcg gccaacgcgc ggggagaggc ggtttgcgta 4620
   ttgggcgctc ttccgcttcc tcgctcactg actcgctgcg ctcggtcgtt cggctgcggc 4680
   gagcggtatc agctcactca aaggcggtaa tacggttatc cacagaatca ggggataacg 4740
   caggaaagaa catgtgagca aaaggccagc aaaaggccag gaaccgtaaa aaggccgcgt 4800
   tgctggcgtt tttccatagg ctccgccccc ctgacgagca tcacaaaaat cgacgctcaa 4860
   gtcagaggtg gcgaaacccg acaggactat aaagatacca ggcgtttccc cctggaagct 4920
   ccctcgtgcg ctctcctgtt ccgaccctgc cgcttaccgg atacctgtcc gcctttctcc 4980
   cttcgggaag cgtggcgctt tctcatagct cacgctgtag gtatctcagt tcggtgtagg 5040
   tcgttcgctc caagctgggc tgtgtgcacg aaccccccgt tcagcccgac cgctgcgcct 5100
   tatccggtaa ctatcgtctt gagtccaacc cggtaagaca cgacttatcg ccactggcag 5160
   cagccactgg taacaggatt agcagagcga ggtatgtagg cggtgctaca gagttcttga 5220
   agtggtggcc taactacggc tacactagaa ggacagtatt tggtatctgc gctctgctga 5280
   agccagttac cttcggaaaa agagttggta gctcttgatc cggcaaacaa accaccgctg 5340
   gtagcggtgg tttttttgtt tgcaagcagc agattacgcg cagaaaaaaa ggatctcaag 5400
   aagatccttt gatcttttct acggggtctg acgctcagtg gaacgaaaac tcacgttaag 5460
   ggattttggt catgagatta tcaaaaagga tcttcaccta gatcctttta aattaaaaat 5520
   gaagttttaa atcaatctaa agtatatatg agtaaacttg gtctgacagt taccaatgct 5580
   taatcagtga ggcacctatc tcagcgatct gtctatttcg ttcatccata gttgcctgac 5640
   tccccgtcgt gtagataact acgatacggg agggcttacc atctggcccc agtgctgcaa 5700
   tgataccgcg agacccacgc tcaccggctc cagatttatc agcaataaac cagccagccg 5760
   gaagggccga gcgcagaagt ggtcctgcaa ctttatccgc ctccatccag tctattaatt 5820
   gttgccggga agctagagta agtagttcgc cagttaatag tttgcgcaac gttgttgcca 5880
   ttgctacagg catcgtggtg tcacgctcgt cgtttggtat ggcttcattc agctccggtt 5940
   cccaacgatc aaggcgagtt acatgatccc ccatgttgtg caaaaaagcg gttagctcct 6000
   tcggtcctcc gatcgttgtc agaagtaagt tggccgcagt gttatcactc atggttatgg 6060
   cagcactgca taattctctt actgtcatgc catccgtaag atgcttttct gtgactggtg 6120
   agtactcaac caagtcattc tgagaatagt gtatgcggcg accgagttgc tcttgcccgg 6180
   cgtcaatacg ggataatacc gcgccacata gcagaacttt aaaagtgctc atcattggaa 6240
   aacgttcttc ggggcgaaaa ctctcaagga tcttaccgct gttgagatcc agttcgatgt 6300
   aacccactcg tgcacccaac tgatcttcag catcttttac tttcaccagc gtttctgggt 6360
   gagcaaaaac aggaaggcaa aatgccgcaa aaaagggaat aagggcgaca cggaaatgtt 6420
   gaatactcat actcttcctt tttcaatatt attgaagcat ttatcagggt tattgtctca 6480
   tgagcggata catatttgaa tgtatttaga aaaataaaca aataggggtt ccgcgcacat 6540
   ttccccgaaa agtgccacct gacgcgccct gtagcggcgc attaagcgcg gcgggtgtgg 6600
   tggttacgcg cagcgtgacc gctacacttg ccagcgccct agcgcccgct cctttcgctt 6660
   tcttcccttc ctttctcgcc acgttcgccg gctttccccg tcaagctcta aatcgggggc 6720
   tccctttagg gttccgattt agtgctttac ggcacctcga ccccaaaaaa cttgattagg 6780
   gtgatggttc acgtagtggg ccatcgccct gatagacggt ttttcgccct ttgacgttgg 6840
   agtccacgtt ctttaatagt ggactcttgt tccaaactgg aacaacactc aaccctatct 6900
   cggtctattc ttttgattta taagggattt tgccgatttc ggcctattgg ttaaaaaatg 6960
   agctgattta acaaaaattt aacgcgaatt ttaacaaaat attaacgctt acaatttcca 7020
   ttcgccattc aggctgcgca actgttggga agggcgatcg gtgcgggcct cttcgctatt 7080
   acgccagctg gcgaaagggg gatgtgctgc aaggcgatta agttgggtaa cgccagggtt 7140
   ttcccagtca cgacgttgta aaacgacggc cagtgaattg taatacgact cactataggg 7200
   cgaattgggt accgggcccc ccctcgaggt cgatggtgtc gataagcttg atatcgaatt 7260
   catgtcacac aaaccgatct tcgcctcaag gaaacctaat tctacatccg agagactgcc 7320
   gagatccagt ctacactgat taattttcgg gccaataatt taaaaaaatc gtgttatata 7380
   atattatatg tattatatat atacatcatg atgatactga cagtcatgtc ccattgctaa 7440
   atagacagac tccatctgcc gcctccaact gatgttctca atatttaagg ggtcatctcg 7500
   cattgtttaa taataaacag actccatcta ccgcctccaa atgatgttct caaaatatat      7560
   tgtatgaact tatttttatt acttagtatt attagacaac ttacttgctt tatgaaaaac      7620
   acttcctatt taggaaacaa tttataatgg cagttcgttc atttaacaat ttatgtagaa      7680
   taaatgttat aaatgcgtat gggaaatctt aaatatggat agcataaatg atatctgcat      7740
   tgcctaattc gaaatcaaca gcaacgaaaa aaatcccttg tacaacataa atagtcatcg      7800
   agaaatatca actatcaaag aacagctatt cacacgttac tattgagatt attattggac      7860
   gagaatcaca cactcaactg tctttctctc ttctagaaat acaggtacaa gtatgtacta      7920
   ttctcattgt tcatacttct agtcatttca tcccacatat tccttggatt tctctccaat      7980
   gaatgacatt ctatcttgca aattcaacaa ttataataag atataccaaa gtagcggtat      8040
   agtggcaatc aaaaagcttc tctggtgtgc ttctcgtatt tatttttatt ctaatgatcc      8100
   attaaaggta tatatttatt tcttgttata taatcctttt gtttattaca tgggctggat      8160
   acataaaggt attttgattt aattttttgc ttaaattcaa tcccccctcg ttcagtgtca      8220
   actgtaatgg taggaaatta ccatactttt gaagaagcaa aaaaaatgaa agaaaaaaaa      8280
   aatcgtattt ccaggttaga cgttccgcag aatctagaat gcggtatgcg gtacattgtt      8340
   cttcgaacgt aaaagttgcg ctccctgaga tattgtacat ttttgctttt acaagtacaa      8400
   gtacatcgta caactatgta ctactgttga tgcatccaca acagtttgtt ttgttttttt      8460
   ttgttttttt tttttctaat gattcattac cgctatgtat acctacttgt acttgtagta      8520
   agccgggtta ttggcgttca attaatcata gacttatgaa tctgcacggt gtgcgctgcg      8580
   agttactttt agcttatgca tgctacttgg gtgtaatatt gggatctgtt cggaaatcaa      8640
   cggatgctca atcgatttcg acagtaatta attaagtcat acacaagtca gctttcttcg      8700
   agcctcatat aagtataagt agttcaacgt attagcactg tacccagcat ctccgtatcg      8760
   agaaacacaa caacatgccc cattggacag atcatgcgga tacacaggtt gtgcagtatc      8820
   atacatactc gatcagacag gtcgtctgac catcatacaa gctgaacaag cgctccatac      8880
   ttgcacgctc tctatataca cagttaaatt acatatccat agtctaacct ctaacagtta      8940
   atcttctggt aagcctccca gccagccttc tggtatcgct tggcctcctc aataggatct      9000
   cggttctggc cgtacagacc tcggccgaca attatgatat ccgttccggt agacatgaca      9060
   tcctcaacag ttcggtactg ctgtccgaga gcgtctccct tgtcgtcaag acccaccccg      9120
   ggggtcagaa taagccagtc ctcagagtcg cccttaggtc ggttctgggc aatgaagcca      9180
   accacaaact cggggtcgga tcgggcaagc tcaatggtct gcttggagta ctcgccagtg      9240
   gccagagagc ccttgcaaga cagctcggcc agcatgagca gacctctggc cagcttctcg      9300
   ttgggagagg ggactaggaa ctccttgtac tgggagttct cgtagtcaga gacgtcctcc      9360
   ttcttctgtt cagagacagt ttcctcggca ccagctcgca ggccagcaat gattccggtt      9420
   ccgggtacac cgtgggcgtt ggtgatatcg gaccactcgg cgattcggtg acaccggtac      9480
   tggtgcttga cagtgttgcc aatatctgcg aactttctgt cctcgaacag gaagaaaccg      9540
   tgcttaagag caagttcctt gagggggagc acagtgccgg cgtaggtgaa gtcgtcaatg      9600
   atgtcgatat gggttttgat catgcacaca taaggtccga ccttatcggc aagctcaatg      9660
   agctccttgg tggtggtaac atccagagaa gcacacaggt tggttttctt ggctgccacg      9720
   agcttgagca ctcgagcggc aaaggcggac ttgtggacgt tagctcgagc ttcgtaggag      9780
   ggcattttgg tggtgaagag gagactgaaa taaatttagt ctgcagaact ttttatcgga      9840
   accttatctg gggcagtgaa gtatatgtta tggtaatagt tacgagttag ttgaacttat      9900
   agatagactg gactatacgg ctatcggtcc aaattagaaa gaacgtcaat ggctctctgg      9960
   gcgtcgcctt tgccgacaaa aatgtgatca tgatgaaagc cagcaatgac gttgcagctg     10020
   atattgttgt cggccaaccg cgccgaaaac gcagctgtca gacccacagc ctccaacgaa     10080
   gaatgtatcg tcaaagtgat ccaagcacac tcatagttgg agtcgtactc caaaggcggc     10140
   aatgacgagt cagacagata ctcgtcgacg tttaaaccat catctaaggg cctcaaaact     10200
   acctcggaac tgctgcgctg atctggacac cacagaggtt ccgagcactt taggttgcac     10260
   caaatgtccc accaggtgca ggcagaaaac gctggaacag cgtgtacagt ttgtcttaac     10320
   aaaaagtgag ggcgctgagg tcgagcaggg tggtgtgact tgttatagcc tttagagctg     10380
   cgaaagcgcg tatggatttg gctcatcagg ccagattgag ggtctgtgga cacatgtcat     10440
   gttagtgtac ttcaatcgcc ccctggatat agccccgaca ataggccgtg gcctcatttt     10500
   tttgccttcc gcacatttcc attgctcggt acccacacct tgcttctcct gcacttgcca     10560
   accttaatac tggtttacat tgaccaacat cttacaagcg gggggcttgt ctagggtata     10620
   tataaacagt ggctctccca atcggttgcc agtctctttt ttcctttctt tccccacaga     10680
   ttcgaaatct aaactacaca tcacac                                           10706
<210> 12
   <211> 1048
   <212> DNA
   <213> Artificial sequence
<220>
   <223> high-throughput cloning cassette
<400> 12
   gcgcacgtta attaaatttt ttttgatttt cttttttgac cccgtcttca attacacttc      60
   ccaactggga acacccctct ttatcgaccc attttaggta atttacccta gcccattgtc     120
   tccataagga atattaccct aacccacagt ccagggtgcc caggtccttc tttggccaaa     180
   ttttaacttc ggtcctatgg cacagcggta gcgcgtgaga ttgcaaatct taaggtcccg     240
   agttcgaatc tcggtgggac ctagttattt ttgatagata atttcgtgat gattagaaac     300
   ttaacgcaaa ataatggccg gcatggtccc agcctcctcg ctggcgccgg ctgggcaaca     360
   tgcttcggca tggcgaatgg gacgcaggtg atggcgggat cgttgtatat ttcttgacac     420
   cttttcggca tcgccctaaa ttcggcgtcc tcatattgtg tgaggacgtt ttattacgtg     480
   tttacgaagc aaaagctaaa accaggagct atttaatggc aacagttaac cagctggtac     540
   gcaaaccacg tgctcgcaaa gttgcgaaaa gcaacgtgcc tgcgctggaa gcatgcccgc     600
   aaaaacgtgg cgtatgtact cgtgtatata ctaccactcc taaaaaaccg aactccgcgc     660
   tgcgtaaagt atgccgtgtt cgtctgacta acggtttcga agtgacttcc tacatcggtg     720
   gtgaaggtca caacctgcag gagcactccg tgatcctgat ccgtggcggt cgtgttaaag     780
   acctcccggg tgttcgttac cacaccgtac gtggtgcgct tgactgctcc ggcgttaaag     840
   accgtaagca ggctcgttcc aagtatggcg tgaagcgtcc taaggcttag gttaataaca     900
   ggcctgctgg taatcgcagg cctttttatt tttacacctg cgttttagag ctagaaatag     960
   caagttaaaa taaggctagt ccgttatcaa cttgaaaaag tggcaccgag tcggtgcttt    1020
   tttttttgtt ttttatcgat gcgcgcac                                       1048
<210> 13
   <211> 300
   <212> DNA
   <213> Yarrowia lipolytica
<400> 13
   attttttttg attttctttt ttgaccccgt cttcaattac acttcccaac tgggaacacc     60
   cctctttatc gacccatttt aggtaattta ccctagccca ttgtctccat aaggaatatt    120
   accctaaccc acagtccagg gtgcccaggt ccttctttgg ccaaatttta acttcggtcc    180
   tatggcacag cggtagcgcg tgagattgca aatcttaagg tcccgagttc gaatctcggt    240
   gggacctagt tatttttgat agataatttc gtgatgatta gaaacttaac gcaaaataat    300
<210> 14
   <211> 68
   <212> DNA
   <213> Herpes Delta virus
<400> 14
   ggccggcatg gtcccagcct cctcgctggc gccggctggg caacatgctt cggcatggcg     60
   aatgggac                                                              68
<210> 15
   <211> 544
   <212> DNA
   <213> Escherischia coli
<400> 15
   atggcgggat cgttgtatat ttcttgacac cttttcggca tcgccctaaa ttcggcgtcc       60
   tcatattgtg tgaggacgtt ttattacgtg tttacgaagc aaaagctaaa accaggagct      120
   atttaatggc aacagttaac cagctggtac gcaaaccacg tgctcgcaaa gttgcgaaaa      180
   gcaacgtgcc tgcgctggaa gcatgcccgc aaaaacgtgg cgtatgtact cgtgtatata      240
   ctaccactcc taaaaaaccg aactccgcgc tgcgtaaagt atgccgtgtt cgtctgacta      300
   acggtttcga agtgacttcc tacatcggtg gtgaaggtca caacctgcag gagcactccg      360
   tgatcctgat ccgtggcggt cgtgttaaag acctcccggg tgttcgttac cacaccgtac      420
   gtggtgcgct tgactgctcc ggcgttaaag accgtaagca ggctcgttcc aagtatggcg      480
   tgaagcgtcc taaggcttag gttaataaca ggcctgctgg taatcgcagg cctttttatt     540
   ttta                                                                   544
<210> 16
   <211> 80
   <212> DNA
   <213> Artificial sequence
<220>
   <223> DNA encoding Cas9 CER domain
<400> 16
   gttttagagc tagaaatagc aagttaaaat aaggctagtc cgttatcaac ttgaaaaagt       60
   ggcaccgagt cggtgctttt                                                   80
<210> 17
   <211> 14
   <212> **DNA**
   <213> Saccharomyces cerevisiae
<400> 17
   tttttttgtt tttt 14
<210> 18
   <211> 11714
   <212> DNA
   <213> Artificial sequence
<220>
   <223> pRF291
<400> 18
   cgataaaaaa caaaaaaaaa agcaccgact cggtgccact ttttcaagtt gataacggac      60
   tagccttatt ttaacttgct atttctagct ctaaaacgca ggtgtaaaaa taaaaaggcc     120
   tgcgattacc agcaggcctg ttattaacct aagccttagg acgcttcacg ccatacttgg     180
   aacgagcctg cttacggtct ttaacgccgg agcagtcaag cgcaccacgt acggtgtggt     240
   aacgaacacc cgggaggtct ttaacacgac cgccacggat caggatcacg gagtgctcct     300
   gcaggttgtg accttcacca ccgatgtagg aagtcacttc gaaaccgtta gtcagacgaa     360
   cacggcatac tttacgcagc gcggagttcg gttttttagg agtggtagta tatacacgag     420
   tacatacgcc acgtttttgc gggcatgctt ccagcgcagg cacgttgctt ttcgcaactt     480
   tgcgagcacg tggtttgcgt accagctggt taactgttgc cattaaatag ctcctggttt     540
   tagcttttgc ttcgtaaaca cgtaataaaa cgtcctcaca caatatgagg acgccgaatt     600
   tagggcgatg ccgaaaaggt gtcaagaaat atacaacgat cccgccatca cctgcgtccc     660
   attcgccatg ccgaagcatg ttgcccagcc ggcgccagcg aggaggctgg gaccatgccg     720
   gccattattt tgcgttaagt ttctaatcat cacgaaatta tctatcaaaa ataactaggt     780
   cccaccgaga ttcgaactcg ggaccttaag atttgcaatc tcacgcgcta ccgctgtgcc     840
   ataggaccga agttaaaatt tggccaaaga aggacctggg caccctggac tgtgggttag     900
   ggtaatattc cttatggaga caatgggcta gggtaaatta cctaaaatgg gtcgataaag     960
   aggggtgttc ccagttggga agtgtaattg aagacggggt caaaaaagaa aatcaaaaaa    1020
   aatttaatta agtcatacac aagtcagctt tcttcgagcc tcatataagt ataagtagtt    1080
   caacgtatta gcactgtacc cagcatctcc gtatcgagaa acacaacaac atgccccatt    1140
   ggacagatca tgcggataca caggttgtgc agtatcatac atactcgatc agacaggtcg    1200
   tctgaccatc atacaagctg aacaagcgct ccatacttgc acgctctcta tatacacagt    1260
   taaattacat atccatagtc taacctctaa cagttaatct tctggtaagc ctcccagcca    1320
   gccttctggt atcgcttggc ctcctcaata ggatctcggt tctggccgta cagacctcgg    1380
   ccgacaatta tgatatccgt tccggtagac atgacatcct caacagttcg gtactgctgt    1440
   ccgagagcgt ctcccttgtc gtcaagaccc accccggggg tcagaataag ccagtcctca    1500
   gagtcgccct taggtcggtt ctgggcaatg aagccaacca caaactcggg gtcggatcgg    1560
   gcaagctcaa tggtctgctt ggagtactcg ccagtggcca gagagccctt gcaagacagc    1620
   tcggccagca tgagcagacc tctggccagc ttctcgttgg gagaggggac taggaactcc    1680
   ttgtactggg agttctcgta gtcagagacg tcctccttct tctgttcaga gacagtttcc    1740
   tcggcaccag ctcgcaggcc agcaatgatt ccggttccgg gtacaccgtg ggcgttggtg    1800
   atatcggacc actcggcgat tcggtgacac cggtactggt gcttgacagt gttgccaata    1860
   tctgcgaact ttctgtcctc gaacaggaag aaaccgtgct taagagcaag ttccttgagg    1920
   gggagcacag tgccggcgta ggtgaagtcg tcaatgatgt cgatatgggt tttgatcatg    1980
   cacacataag gtccgacctt atcggcaagc tcaatgagct ccttggtggt ggtaacatcc    2040
   agagaagcac acaggttggt tttcttggct gccacgagct tgagcactcg agcggcaaag    2100
   gcggacttgt ggacgttagc tcgagcttcg taggagggca ttttggtggt gaagaggaga    2160
   ctgaaataaa tttagtctgc agaacttttt atcggaacct tatctggggc agtgaagtat    2220
   atgttatggt aatagttacg agttagttga acttatagat agactggact atacggctat    2280
   cggtccaaat tagaaagaac gtcaatggct ctctgggcgt cgcctttgcc gacaaaaatg    2340
   tgatcatgat gaaagccagc aatgacgttg cagctgatat tgttgtcggc caaccgcgcc     2400
   gaaaacgcag ctgtcagacc cacagcctcc aacgaagaat gtatcgtcaa agtgatccaa     2460
   gcacactcat agttggagtc gtactccaaa ggcggcaatg acgagtcaga cagatactcg     2520
   tcgacgttta aaccatcatc taagggcctc aaaactacct cggaactgct gcgctgatct     2580
   ggacaccaca gaggttccga gcactttagg ttgcaccaaa tgtcccacca ggtgcaggca     2640
   gaaaacgctg gaacagcgtg tacagtttgt cttaacaaaa agtgagggcg ctgaggtcga     2700
   gcagggtggt gtgacttgtt atagccttta gagctgcgaa agcgcgtatg gatttggctc     2760
   atcaggccag attgagggtc tgtggacaca tgtcatgtta gtgtacttca atcgccccct    2820
   ggatatagcc ccgacaatag gccgtggcct catttttttg ccttccgcac atttccattg    2880
   ctcggtaccc acaccttgct tctcctgcac ttgccaacct taatactggt ttacattgac     2940
   caacatctta caagcggggg gcttgtctag ggtatatata aacagtggct ctcccaatcg     3000
   gttgccagtc tcttttttcc tttctttccc cacagattcg aaatctaaac tacacatcac     3060
   accatggaca agaaatactc catcggcctg gacattggaa ccaactctgt cggctgggct     3120
   gtcatcaccg acgagtacaa ggtgccctcc aagaaattca aggtcctcgg aaacaccgat     3180
   cgacactcca tcaagaaaaa cctcattggt gccctgttgt tcgattctgg cgagactgcc     3240
   gaagctacca gactcaagcg aactgctcgg cgacgttaca cccgacggaa gaaccgaatc     3300
   tgctacctgc aggagatctt ttccaacgag atggccaagg tggacgattc gttctttcat     3360
   cgactggagg aatccttcct cgtcgaggaa gacaagaaac acgagcgtca tcccatcttt     3420
   ggcaacattg tggacgaggt tgcttaccac gagaagtatc ctaccatcta ccatctccga     3480
   aagaaactcg tcgattccac cgacaaggcg gatctcagac ttatctacct cgctctggca     3540
   cacatgatca agtttcgagg tcatttcctc atcgagggcg atctcaatcc cgacaacagc     3600
   gatgtggaca agctgttcat tcagctcgtt cagacctaca accagctgtt cgaggaaaac     3660
   cccatcaatg cctccggagt cgatgcaaag gccatcttgt ctgctcgact ctcgaagagc     3720
   agacgactgg agaacctcat tgcccaactt cctggcgaga aaaagaacgg actgtttggc     3780
   aacctcattg ccctttctct tggtctcaca cccaacttca agtccaactt cgatctggcg     3840
   gaggacgcca agctccagct gtccaaggac acctacgacg atgacctcga caacctgctt     3900
   gcacagattg gcgatcagta cgccgacctg tttctcgctg ccaagaacct ttcggatgct     3960
   attctcttgt ctgacattct gcgagtcaac accgagatca caaaggctcc cctttctgcc     4020
   tccatgatca agcgatacga cgagcaccat caggatctca cactgctcaa ggctcttgtc     4080
   cgacagcaac tgcccgagaa gtacaaggag atctttttcg atcagtcgaa gaacggctac     4140
   gctggataca tcgacggcgg agcctctcag gaagagttct acaagttcat caagccaatt     4200
   ctcgagaaga tggacggaac cgaggaactg cttgtcaagc tcaatcgaga ggatctgctt     4260
   cggaagcaac gaaccttcga caacggcagc attcctcatc agatccacct cggtgagctg     4320
   cacgccattc ttcgacgtca ggaagacttc tacccctttc tcaaggacaa ccgagagaag   4380
   atcgagaaga ttcttacctt tcgaatcccc tactatgttg gtcctcttgc cagaggaaac     4440
   tctcgatttg cttggatgac tcgaaagtcc gaggaaacca tcactccctg gaacttcgag     4500
   gaagtcgtgg acaagggtgc ctctgcacag tccttcatcg agcgaatgac caacttcgac     4560
   aagaatctgc ccaacgagaa ggttcttccc aagcattcgc tgctctacga gtactttaca     4620
   gtctacaacg aactcaccaa agtcaagtac gttaccgagg gaatgcgaaa gcctgccttc     4680
   ttgtctggcg aacagaagaa agccattgtc gatctcctgt tcaagaccaa ccgaaaggtc     4740
   actgttaagc agctcaagga ggactacttc aagaaaatcg agtgtttcga cagcgtcgag     4800
   atttccggag ttgaggaccg attcaacgcc tctttgggca cctatcacga tctgctcaag     4860
   attatcaagg acaaggattt tctcgacaac gaggaaaacg aggacattct ggaggacatc     4920
   gtgctcactc ttaccctgtt cgaagatcgg gagatgatcg aggaacgact caagacatac     4980
   gctcacctgt tcgacgacaa ggtcatgaaa caactcaagc gacgtagata caccggctgg     5040
   ggaagacttt cgcgaaagct catcaacggc atcagagaca agcagtccgg aaagaccatt     5100
   ctggactttc tcaagtccga tggctttgcc aaccgaaact tcatgcagct cattcacgac     5160
   gattctctta ccttcaagga ggacatccag aaggcacaag tgtccggtca gggcgacagc     5220
   ttgcacgaac atattgccaa cctggctggt tcgccagcca tcaagaaagg cattctccag     5280
   actgtcaagg ttgtcgacga gctggtgaag gtcatgggac gtcacaagcc cgagaacatt     5340
   gtgatcgaga tggccagaga gaaccagaca actcaaaagg gtcagaaaaa ctcgcgagag     5400
   cggatgaagc gaatcgagga aggcatcaag gagctgggat cccagattct caaggagcat     5460
   cccgtcgaga acactcaact gcagaacgag aagctgtatc tctactatct gcagaatggt     5520
   cgagacatgt acgtggatca ggaactggac atcaatcgtc tcagcgacta cgatgtggac     5580
   cacattgtcc ctcaatcctt tctcaaggac gattctatcg acaacaaggt ccttacacga     5640
   tccgacaaga acagaggcaa gtcggacaac gttcccagcg aagaggtggt caaaaagatg     5700
   aagaactact ggcgacagct gctcaacgcc aagctcatta cccagcgaaa gttcgacaat     5760
   cttaccaagg ccgagcgagg cggtctgtcc gagctcgaca aggctggctt catcaagcgt     5820
   caactcgtcg agaccagaca gatcacaaag cacgtcgcac agattctcga ttctcggatg     5880
   aacaccaagt acgacgagaa cgacaagctc atccgagagg tcaaggtgat tactctcaag     5940
   tccaaactgg tctccgattt ccgaaaggac tttcagttct acaaggtgcg agagatcaac     6000
   aattaccacc atgcccacga tgcttacctc aacgccgtcg ttggcactgc gctcatcaag     6060
   aaatacccca agctcgaaag cgagttcgtt tacggcgatt acaaggtcta cgacgttcga     6120
   aagatgattg ccaagtccga acaggagatt ggcaaggcta ctgccaagta cttcttttac 6180
   tccaacatca tgaacttttt caagaccgag atcaccttgg ccaacggaga gattcgaaag 6240
   agaccactta tcgagaccaa cggcgaaact ggagagatcg tgtgggacaa gggtcgagac 6300
   tttgcaaccg tgcgaaaggt tctgtcgatg cctcaggtca acatcgtcaa gaaaaccgag 6360
   gttcagactg gcggattctc caaggagtcg attctgccca agcgaaactc cgacaagctc 6420
   atcgctcgaa agaaagactg ggatcccaag aaatacggtg gcttcgattc tcctaccgtc 6480
   gcctattccg tgcttgtcgt tgcgaaggtc gagaagggca agtccaaaaa gctcaagtcc 6540
   gtcaaggagc tgctcggaat taccatcatg gagcgatcga gcttcgagaa gaatcccatc 6600
   gacttcttgg aagccaaggg ttacaaggag gtcaagaaag acctcattat caagctgccc 6660
   aagtactctc tgttcgaact ggagaacggt cgaaagcgta tgctcgcctc cgctggcgag 6720
   ctgcagaagg gaaacgagct tgccttgcct tcgaagtacg tcaactttct ctatctggct 6780
   tctcactacg agaagctcaa gggttctccc gaggacaacg aacagaagca actcttcgtt 6840
   gagcagcaca aacattacct cgacgagatt atcgagcaga tttccgagtt ttcgaagcga 6900
   gtcatcctgg ctgatgccaa cttggacaag gtgctctctg cctacaacaa gcatcgggac 6960
   aaacccattc gagaacaggc ggagaacatc attcacctgt ttactcttac caacctgggt 7020
   gctcctgcag ctttcaagta cttcgatacc actatcgacc gaaagcggta cacatccacc 7080
   aaggaggttc tcgatgccac cctgattcac cagtccatca ctggcctgta cgagacccga 7140
   atcgacctgt ctcagcttgg tggcgactcc agagccgatc ccaagaaaaa gcgaaaggtc 7200
   taagcggccg caagtgtgga tggggaagtg agtgcccggt tctgtgtgca caattggcaa 7260
   tccaagatgg atggattcaa cacagggata tagcgagcta cgtggtggtg cgaggatata 7320
   gcaacggata tttatgtttg acacttgaga atgtacgata caagcactgt ccaagtacaa 7380
   tactaaacat actgtacata ctcatactcg tacccgggca acggtttcac ttgagtgcag 7440
   tggctagtgc tcttactcgt acagtgtgca atactgcgta tcatagtctt tgatgtatat 7500
   cgtattcatt catgttagtt gcgtacgagc cggaagcata aagtgtaaag cctggggtgc 7560
   ctaatgagtg agctaactca cattaattgc gttgcgctca ctgcccgctt tccagtcggg 7620
   aaacctgtcg tgccagctgc attaatgaat cggccaacgc gcggggagag gcggtttgcg 7680
   tattgggcgc tcttccgctt cctcgctcac tgactcgctg cgctcggtcg ttcggctgcg 7740
   gcgagcggta tcagctcact caaaggcggt aatacggtta tccacagaat caggggataa 7800
   cgcaggaaag aacatgtgag caaaaggcca gcaaaaggcc aggaaccgta aaaaggccgc 7860
   gttgctggcg tttttccata ggctccgccc ccctgacgag catcacaaaa atcgacgctc 7920
   aagtcagagg tggcgaaacc cgacaggact ataaagatac caggcgtttc cccctggaag 7980
   ctccctcgtg cgctctcctg ttccgaccct gccgcttacc ggatacctgt ccgcctttct 8040
   cccttcggga agcgtggcgc tttctcatag ctcacgctgt aggtatctca gttcggtgta 8100
   ggtcgttcgc tccaagctgg gctgtgtgca cgaacccccc gttcagcccg accgctgcgc 8160
   cttatccggt aactatcgtc ttgagtccaa cccggtaaga cacgacttat cgccactggc 8220
   agcagccact ggtaacagga ttagcagagc gaggtatgta ggcggtgcta cagagttctt 8280
   gaagtggtgg cctaactacg gctacactag aaggacagta tttggtatct gcgctctgct 8340
   gaagccagtt accttcggaa aaagagttgg tagctcttga tccggcaaac aaaccaccgc 8400
   tggtagcggt ggtttttttg tttgcaagca gcagattacg cgcagaaaaa aaggatctca 8460
   agaagatcct ttgatctttt ctacggggtc tgacgctcag tggaacgaaa actcacgtta 8520
   agggattttg gtcatgagat tatcaaaaag gatcttcacc tagatccttt taaattaaaa 8580
   atgaagtttt aaatcaatct aaagtatata tgagtaaact tggtctgaca gttaccaatg 8640
   cttaatcagt gaggcaccta tctcagcgat ctgtctattt cgttcatcca tagttgcctg 8700
   actccccgtc gtgtagataa ctacgatacg ggagggctta ccatctggcc ccagtgctgc 8760
   aatgataccg cgagacccac gctcaccggc tccagattta tcagcaataa accagccagc 8820
   cggaagggcc gagcgcagaa gtggtcctgc aactttatcc gcctccatcc agtctattaa 8880
   ttgttgccgg gaagctagag taagtagttc gccagttaat agtttgcgca acgttgttgc 8940
   cattgctaca ggcatcgtgg tgtcacgctc gtcgtttggt atggcttcat tcagctccgg 9000
   ttcccaacga tcaaggcgag ttacatgatc ccccatgttg tgcaaaaaag cggttagctc 9060
   cttcggtcct ccgatcgttg tcagaagtaa gttggccgca gtgttatcac tcatggttat 9120
   ggcagcactg cataattctc ttactgtcat gccatccgta agatgctttt ctgtgactgg 9180
   tgagtactca accaagtcat tctgagaata gtgtatgcgg cgaccgagtt gctcttgccc 9240
   ggcgtcaata cgggataata ccgcgccaca tagcagaact ttaaaagtgc tcatcattgg 9300
   aaaacgttct tcggggcgaa aactctcaag gatcttaccg ctgttgagat ccagttcgat 9360
   gtaacccact cgtgcaccca actgatcttc agcatctttt actttcacca gcgtttctgg 9420
   gtgagcaaaa acaggaaggc aaaatgccgc aaaaaaggga ataagggcga cacggaaatg 9480
   ttgaatactc atactcttcc tttttcaata ttattgaagc atttatcagg gttattgtct 9540
   catgagcgga tacatatttg aatgtattta gaaaaataaa caaatagggg ttccgcgcac 9600
   atttccccga aaagtgccac ctgacgcgcc ctgtagcggc gcattaagcg cggcgggtgt 9660
   ggtggttacg cgcagcgtga ccgctacact tgccagcgcc ctagcgcccg ctcctttcgc 9720
   tttcttccct tcctttctcg ccacgttcgc cggctttccc cgtcaagctc taaatcgggg 9780
   gctcccttta gggttccgat ttagtgcttt acggcacctc gaccccaaaa aacttgatta 9840
   gggtgatggt tcacgtagtg ggccatcgcc ctgatagacg gtttttcgcc ctttgacgtt 9900
   ggagtccacg ttctttaata gtggactctt gttccaaact ggaacaacac tcaaccctat      9960
   ctcggtctat tcttttgatt tataagggat tttgccgatt tcggcctatt ggttaaaaaa     10020
   tgagctgatt taacaaaaat ttaacgcgaa ttttaacaaa atattaacgc ttacaatttc     10080
   cattcgccat tcaggctgcg caactgttgg gaagggcgat cggtgcgggc ctcttcgcta     10140
   ttacgccagc tggcgaaagg gggatgtgct gcaaggcgat taagttgggt aacgccaggg     10200
   ttttcccagt cacgacgttg taaaacgacg gccagtgaat tgtaatacga ctcactatag     10260
   ggcgaattgg gtaccgggcc ccccctcgag gtcgatggtg tcgataagct tgatatcgaa     10320
   ttcatgtcac acaaaccgat cttcgcctca aggaaaccta attctacatc cgagagactg     10380
   ccgagatcca gtctacactg attaattttc gggccaataa tttaaaaaaa tcgtgttata     10440
   taatattata tgtattatat atatacatca tgatgatact gacagtcatg tcccattgct     10500
   aaatagacag actccatctg ccgcctccaa ctgatgttct caatatttaa ggggtcatct     10560
   cgcattgttt aataataaac agactccatc taccgcctcc aaatgatgtt ctcaaaatat     10620
   attgtatgaa cttattttta ttacttagta ttattagaca acttacttgc tttatgaaaa     10680
   acacttccta tttaggaaac aatttataat ggcagttcgt tcatttaaca atttatgtag     10740
   aataaatgtt ataaatgcgt atgggaaatc ttaaatatgg atagcataaa tgatatctgc     10800
   attgcctaat tcgaaatcaa cagcaacgaa aaaaatccct tgtacaacat aaatagtcat     10860
   cgagaaatat caactatcaa agaacagcta ttcacacgtt actattgaga ttattattgg     10920
   acgagaatca cacactcaac tgtctttctc tcttctagaa atacaggtac aagtatgtac     10980
   tattctcatt gttcatactt ctagtcattt catcccacat attccttgga tttctctcca     11040
   atgaatgaca ttctatcttg caaattcaac aattataata agatatacca aagtagcggt     11100
   atagtggcaa tcaaaaagct tctctggtgt gcttctcgta tttattttta ttctaatgat     11160
   ccattaaagg tatatattta tttcttgtta tataatcctt ttgtttatta catgggctgg     11220
   atacataaag gtattttgat ttaatttttt gcttaaattc aatcccccct cgttcagtgt     11280
   caactgtaat ggtaggaaat taccatactt ttgaagaagc aaaaaaaatg aaagaaaaaa     11340
   aaaatcgtat ttccaggtta gacgttccgc agaatctaga atgcggtatg cggtacattg     11400
   ttcttcgaac gtaaaagttg cgctccctga gatattgtac atttttgctt ttacaagtac     11460
   aagtacatcg tacaactatg tactactgtt gatgcatcca caacagtttg ttttgttttt     11520
   ttttgttttt tttttttcta atgattcatt accgctatgt atacctactt gtacttgtag     11580
   taagccgggt tattggcgtt caattaatca tagacttatg aatctgcacg gtgtgcgctg     11640
   cgagttactt ttagcttatg catgctactt gggtgtaata ttgggatctg ttcggaaatc     11700
   aacggatgct caat                                                       11714
<210> 19
   <211> 32
   <212> DNA
   <213> Artificial sequence
<220>
   <223> Can1-1F
<400> 19
   aatgggactc aaacgattac ccaccctcgt tt 32
<210> 20
   <211> 32
   <212> DNA
   <213> Artificial sequence
<220>
   <223> Can1-1R
<400> 20
   tctaaaacga gggtgggtaa tcgtttgagt cc 32
<210> 21
   <211> 20
   <212> DNA
   <213> Artificial sequence
<220>
   <223> DNA encoding Canl-1 VT domain
<400> 21
   tcaaacgatt acccaccctc 20
<210> 22
   <211> 23
   <212> DNA
   <213> Yarrowia lipolytica
<400> 22
   tcaaacgatt acccaccctc cgg 23
<210> 23
   <211> 1719
   <212> DNA
   <213> Yarrowia lipolytica
<400> 23
   atggaaaaga cattttcaaa cgattaccca ccctccggga ctgaggccca catccacatc      60
   aaccacacgg cccactcgga tgactcagag gaggtgccct cgcacaagga aaattacaac     120
   accagtggcc acgacctgga ggagtccgac ccggataacc atgtcggtga gaccctcgag     180
   gtcaagcgag gtctcaagat gcgacacatc tccatgatct cgcttggagg aaccattggt     240
   accggtctct tcattggtac cggaggagct ctccagcagg ccggtccctg tggcgccctc     300
   gtcgcctacg tgttcatggc caccattgtc tactctgttg ccgagtctct tggagaactg     360
   gctacgtaca ttcccatcac cggctccttt gccgtcttta ctacccgata tctgtcacag     420
   tcgtttggtg cctccatggg ctggctatac tggttctcgt gggcgatcac cttcgccatc     480
   gagctcaaca ccattggtcc cgtgattgag tactggactg acgccgttcc tactgctgcc     540
   tggattgcca tcttcttcgt catcctcact accatcaact tcttccccgt gggcttctat     600
   ggcgaagtcg agttctgggt ggcctccgtg aaggtcattg ccatcattgg atggctcatc     660
   tacgcgctct gcatgacgtg tggagcaggt gtaacaggtc ctgtgggatt cagatactgg     720
   aaccaccccg gacccatggg agacggaatc tggaccgacg gcgtgcccat tgtgcgaaac     780
   gcgcccggtc gacgattcat gggatggctc aattcgctcg ttaacgccgc cttcacctac     840
   cagggctgtg agctggtcgg agtcactgcc ggtgaggccc agaaccccag aaagtccgtc     900
   cctcgagcca tcaaccgagt ctttgctcga atttgcatct tctacattgg ctctatcttc     960
   ttcatgggca tgctcgtgcc ctttaacgac cccaagctga ccgatgactc ctccgtcatc    1020
   gcctcctctc cttttgttat tgccattatc aactctggca ccaaggtgct ccctcacatt    1080
   ttcaacgccg tcattctcat caccctgatt tcggcaggaa actccaacgt ctacattggc    1140
   tcgcgagtgg tctacgccct ggctgactcc ggaaccgcac caaagttctt caagcgaacc    1200
   accaagaagg gagtgccgta cgtggcagtc tgcttcacct cggcgtttgg tctgctggcc    1260
   ttcatgtctg tgtccgagtc gtcgtccact gtcttcgact ggttcatcaa catctccgct    1320
   gtggccggcc tcatctgttg ggccttcatc tctgcctccc acatccgatt catgcaagtg    1380
   cttaagcaca gagggatctc cagagatacg ctgcccttca aggcacgatg gcagccattc    1440
   tactcatggt acgcgctcgt ctccatcatc ttcatcactc tcatccaggg cttcacgtcc    1500
   ttctggcact ttaccgccgc caagttcatg actgcataca tctccgtcat tgtctgggtc    1560
   ggtttgtaca ttatcttcca gtgtctgttc cgatgcaagt tccttatccc tattgaggat    1620
   gtggacattg acaccggccg acgagagatt gacgacgatg tgtgggagga gaagatcccc    1680
   acaaagtggt acgagaagtt ttggaatatt attgcataa                           1719
<210> 24
   <211> 11176
   <212> DNA
   <213> Artificial Sequence
<220>
   <223> pRF303
<400> 24
   tctaaaacga gggtgggtaa tcgtttgagt cccattcgcc atgccgaagc atgttgccca      60
   gccggcgcca gcgaggaggc tgggaccatg ccggccatta ttttgcgtta agtttctaat     120
   catcacgaaa ttatctatca aaaataacta ggtcccaccg agattcgaac tcgggacctt     180
   aagatttgca atctcacgcg ctaccgctgt gccataggac cgaagttaaa atttggccaa     240
   agaaggacct gggcaccctg gactgtgggt tagggtaata ttccttatgg agacaatggg     300
   ctagggtaaa ttacctaaaa tgggtcgata aagaggggtg ttcccagttg ggaagtgtaa     360
   ttgaagacgg ggtcaaaaaa gaaaatcaaa aaaaatttaa ttaagtcata cacaagtcag     420
   ctttcttcga gcctcatata agtataagta gttcaacgta ttagcactgt acccagcatc     480
   tccgtatcga gaaacacaac aacatgcccc attggacaga tcatgcggat acacaggttg      540
   tgcagtatca tacatactcg atcagacagg tcgtctgacc atcatacaag ctgaacaagc      600
   gctccatact tgcacgctct ctatatacac agttaaatta catatccata gtctaacctc      660
   taacagttaa tcttctggta agcctcccag ccagccttct ggtatcgctt ggcctcctca      720
   ataggatctc ggttctggcc gtacagacct cggccgacaa ttatgatatc cgttccggta      780
   gacatgacat cctcaacagt tcggtactgc tgtccgagag cgtctccctt gtcgtcaaga      840
   cccaccccgg gggtcagaat aagccagtcc tcagagtcgc ccttaggtcg gttctgggca      900
   atgaagccaa ccacaaactc ggggtcggat cgggcaagct caatggtctg cttggagtac      960
   tcgccagtgg ccagagagcc cttgcaagac agctcggcca gcatgagcag acctctggcc     1020
   agcttctcgt tgggagaggg gactaggaac tccttgtact gggagttctc gtagtcagag     1080
   acgtcctcct tcttctgttc agagacagtt tcctcggcac cagctcgcag gccagcaatg     1140
   attccggttc cgggtacacc gtgggcgttg gtgatatcgg accactcggc gattcggtga     1200
   caccggtact ggtgcttgac agtgttgcca atatctgcga actttctgtc ctcgaacagg     1260
   aagaaaccgt gcttaagagc aagttccttg agggggagca cagtgccggc gtaggtgaag     1320
   tcgtcaatga tgtcgatatg ggttttgatc atgcacacat aaggtccgac cttatcggca     1380
   agctcaatga gctccttggt ggtggtaaca tccagagaag cacacaggtt ggttttcttg    1440
   gctgccacga gcttgagcac tcgagcggca aaggcggact tgtggacgtt agctcgagct     1500
   tcgtaggagg gcattttggt ggtgaagagg agactgaaat aaatttagtc tgcagaactt     1560
   tttatcggaa ccttatctgg ggcagtgaag tatatgttat ggtaatagtt acgagttagt     1620
   tgaacttata gatagactgg actatacggc tatcggtcca aattagaaag aacgtcaatg     1680
   gctctctggg cgtcgccttt gccgacaaaa atgtgatcat gatgaaagcc agcaatgacg     1740
   ttgcagctga tattgttgtc ggccaaccgc gccgaaaacg cagctgtcag acccacagcc     1800
   tccaacgaag aatgtatcgt caaagtgatc caagcacact catagttgga gtcgtactcc     1860
   aaaggcggca atgacgagtc agacagatac tcgtcgacgt ttaaaccatc atctaagggc     1920
   ctcaaaacta cctcggaact gctgcgctga tctggacacc acagaggttc cgagcacttt     1980
   aggttgcacc aaatgtccca ccaggtgcag gcagaaaacg ctggaacagc gtgtacagtt     2040
   tgtcttaaca aaaagtgagg gcgctgaggt cgagcagggt ggtgtgactt gttatagcct     2100
   ttagagctgc gaaagcgcgt atggatttgg ctcatcaggc cagattgagg gtctgtggac     2160
   acatgtcatg ttagtgtact tcaatcgccc cctggatata gccccgacaa taggccgtgg     2220
   cctcattttt ttgccttccg cacatttcca ttgctcggta cccacacctt gcttctcctg     2280
   cacttgccaa ccttaatact ggtttacatt gaccaacatc ttacaagcgg ggggcttgtc     2340
   tagggtatat ataaacagtg gctctcccaa tcggttgcca gtctcttttt tcctttcttt     2400
   ccccacagat tcgaaatcta aactacacat cacaccatgg acaagaaata ctccatcggc     2460
   ctggacattg gaaccaactc tgtcggctgg gctgtcatca ccgacgagta caaggtgccc     2520
   tccaagaaat tcaaggtcct cggaaacacc gatcgacact ccatcaagaa aaacctcatt     2580
   ggtgccctgt tgttcgattc tggcgagact gccgaagcta ccagactcaa gcgaactgct     2640
   cggcgacgtt acacccgacg gaagaaccga atctgctacc tgcaggagat cttttccaac     2700
   gagatggcca aggtggacga ttcgttcttt catcgactgg aggaatcctt cctcgtcgag     2760
   gaagacaaga aacacgagcg tcatcccatc tttggcaaca ttgtggacga ggttgcttac     2820
   cacgagaagt atcctaccat ctaccatctc cgaaagaaac tcgtcgattc caccgacaag     2880
   gcggatctca gacttatcta cctcgctctg gcacacatga tcaagtttcg aggtcatttc     2940
   ctcatcgagg gcgatctcaa tcccgacaac agcgatgtgg acaagctgtt cattcagctc     3000
   gttcagacct acaaccagct gttcgaggaa aaccccatca atgcctccgg agtcgatgca     3060
   aaggccatct tgtctgctcg actctcgaag agcagacgac tggagaacct cattgcccaa     3120
   cttcctggcg agaaaaagaa cggactgttt ggcaacctca ttgccctttc tcttggtctc     3180
   acacccaact tcaagtccaa cttcgatctg gcggaggacg ccaagctcca gctgtccaag     3240
   gacacctacg acgatgacct cgacaacctg cttgcacaga ttggcgatca gtacgccgac     3300
   ctgtttctcg ctgccaagaa cctttcggat gctattctct tgtctgacat tctgcgagtc     3360
   aacaccgaga tcacaaaggc tccccttcct gcctccatga tcaagcgata cgacgagcac    3420
   catcaggatc tcacactgct caaggctctt gtccgacagc aactgcccga gaagtacaag     3480
   gagatctttt tcgatcagtc gaagaacggc tacgctggat acatcgacgg cggagcctct     3540
   caggaagagt tctacaagtt catcaagcca attctcgaga agatggacgg aaccgaggaa     3600
   ctgcttgtca agctcaatcg agaggatctg cttcggaagc aacgaacctt cgacaacggc     3660
   agcattcctc atcagatcca cctcggtgag ctgcacgcca ttcttcgacg tcaggaagac     3720
   ttctacccct ttctcaagga caaccgagag aagatcgaga agattcttac ctttcgaatc     3780
   ccctactatg ttggtcctct tgccagagga aactctcgat ttgcttggat gactcgaaag     3840
   tccgaggaaa ccatcactcc ctggaacttc gaggaagtcg tggacaaggg tgcctctgca     3900
   cagtccttca tcgagcgaat gaccaacttc gacaagaatc tgcccaacga gaaggttctt     3960
   cccaagcatt cgctgctcta cgagtacttt acagtctaca acgaactcac caaagtcaag     4020
   tacgttaccg agggaatgcg aaagcctgcc ttcttgtctg gcgaacagaa gaaagccatt     4080
   gtcgatctcc tgttcaagac caaccgaaag gtcactgtta agcagctcaa ggaggactac     4140
   ttcaagaaaa tcgagtgttt cgacagcgtc gagatttccg gagttgagga ccgattcaac     4200
   gcctctttgg gcacctatca cgatctgctc aagattatca aggacaagga ttttctcgac     4260
   aacgaggaaa acgaggacat tctggaggac atcgtgctca ctcttaccct gttcgaagat 4320
   cgggagatga tcgaggaacg actcaagaca tacgctcacc tgttcgacga caaggtcatg 4380
   aaacaactca agcgacgtag atacaccggc tggggaagac tttcgcgaaa gctcatcaac 4440
   ggcatcagag acaagcagtc cggaaagacc attctggact ttctcaagtc cgatggcttt 4500
   gccaaccgaa acttcatgca gctcattcac gacgattctc ttaccttcaa ggaggacatc 4560
   cagaaggcac aagtgtccgg tcagggcgac agcttgcacg aacatattgc caacctggct 4620
   ggttcgccag ccatcaagaa aggcattctc cagactgtca aggttgtcga cgagctggtg 4680
   aaggtcatgg gacgtcacaa gcccgagaac attgtgatcg agatggccag agagaaccag 4740
   acaactcaaa agggtcagaa aaactcgcga gagcggatga agcgaatcga ggaaggcatc 4800
   aaggagctgg gatcccagat tctcaaggag catcccgtcg agaacactca actgcagaac 4860
   gagaagctgt atctctacta tctgcagaat ggtcgagaca tgtacgtgga tcaggaactg 4920
   gacatcaatc gtctcagcga ctacgatgtg gaccacattg tccctcaatc ctttctcaag 4980
   gacgattcta tcgacaacaa ggtccttaca cgatccgaca agaacagagg caagtcggac 5040
   aacgttccca gcgaagaggt ggtcaaaaag atgaagaact actggcgaca gctgctcaac 5100
   gccaagctca ttacccagcg aaagttcgac aatcttacca aggccgagcg aggcggtctg 5160
   tccgagctcg acaaggctgg cttcatcaag cgtcaactcg tcgagaccag acagatcaca 5220
   aagcacgtcg cacagattct cgattctcgg atgaacacca agtacgacga gaacgacaag 5280
   ctcatccgag aggtcaaggt gattactctc aagtccaaac tggtctccga tttccgaaag 5340
   gactttcagt tctacaaggt gcgagagatc aacaattacc accatgccca cgatgcttac 5400
   ctcaacgccg tcgttggcac tgcgctcatc aagaaatacc ccaagctcga aagcgagttc 5460
   gtttacggcg attacaaggt ctacgacgtt cgaaagatga ttgccaagtc cgaacaggag 5520
   attggcaagg ctactgccaa gtacttcttt tactccaaca tcatgaactt tttcaagacc 5580
   gagatcacct tggccaacgg agagattcga aagagaccac ttatcgagac caacggcgaa 5640
   actggagaga tcgtgtggga caagggtcga gactttgcaa ccgtgcgaaa ggttctgtcg 5700
   atgcctcagg tcaacatcgt caagaaaacc gaggttcaga ctggcggatt ctccaaggag 5760
   tcgattctgc ccaagcgaaa ctccgacaag ctcatcgctc gaaagaaaga ctgggatccc 5820
   aagaaatacg gtggcttcga ttctcctacc gtcgcctatt ccgtgcttgt cgttgcgaag 5880
   gtcgagaagg gcaagtccaa aaagctcaag tccgtcaagg agctgctcgg aattaccatc 5940
   atggagcgat cgagcttcga gaagaatccc atcgacttct tggaagccaa gggttacaag 6000
   gaggtcaaga aagacctcat tatcaagctg cccaagtact ctctgttcga actggagaac 6060
   ggtcgaaagc gtatgctcgc ctccgctggc gagctgcaga agggaaacga gcttgccttg 6120
   ccttcgaagt acgtcaactt tctctatctg gcttctcact acgagaagct caagggttct 6180
   cccgaggaca acgaacagaa gcaactcttc gttgagcagc acaaacatta cctcgacgag 6240
   attatcgagc agatttccga gttttcgaag cgagtcatcc tggctgatgc caacttggac 6300
   aaggtgctct ctgcctacaa caagcatcgg gacaaaccca ttcgagaaca ggcggagaac 6360
   atcattcacc tgtttactct taccaacctg ggtgctcctg cagctttcaa gtacttcgat 6420
   accactatcg accgaaagcg gtacacatcc accaaggagg ttctcgatgc caccctgatt 6480
   caccagtcca tcactggcct gtacgagacc cgaatcgacc tgtctcagct tggtggcgac 6540
   tccagagccg atcccaagaa aaagcgaaag gtctaagcgg ccgcaagtgt ggatggggaa 6600
   gtgagtgccc ggttctgtgt gcacaattgg caatccaaga tggatggatt caacacaggg 6660
   atatagcgag ctacgtggtg gtgcgaggat atagcaacgg atatttatgt ttgacacttg 6720
   agaatgtacg atacaagcac tgtccaagta caatactaaa catactgtac atactcatac 6780
   tcgtacccgg gcaacggttt cacttgagtg cagtggctag tgctcttact cgtacagtgt 6840
   gcaatactgc gtatcatagt ctttgatgta tatcgtattc attcatgtta gttgcgtacg 6900
   agccggaagc ataaagtgta aagcctgggg tgcctaatga gtgagctaac tcacattaat 6960
   tgcgttgcgc tcactgcccg ctttccagtc gggaaacctg tcgtgccagc tgcattaatg 7020
   aatcggccaa cgcgcgggga gaggcggttt gcgtattggg cgctcttccg cttcctcgct 7080
   cactgactcg ctgcgctcgg tcgttcggct gcggcgagcg gtatcagctc actcaaaggc 7140
   ggtaatacgg ttatccacag aatcagggga taacgcagga aagaacatgt gagcaaaagg 7200
   ccagcaaaag gccaggaacc gtaaaaaggc cgcgttgctg gcgtttttcc ataggctccg 7260
   cccccctgac gagcatcaca aaaatcgacg ctcaagtcag aggtggcgaa acccgacagg 7320
   actataaaga taccaggcgt ttccccctgg aagctccctc gtgcgctctc ctgttccgac 7380
   cctgccgctt accggatacc tgtccgcctt tctcccttcg ggaagcgtgg cgctttctca 7440
   tagctcacgc tgtaggtatc tcagttcggt gtaggtcgtt cgctccaagc tgggctgtgt 7500
   gcacgaaccc cccgttcagc ccgaccgctg cgccttatcc ggtaactatc gtcttgagtc 7560
   caacccggta agacacgact tatcgccact ggcagcagcc actggtaaca ggattagcag 7620
   agcgaggtat gtaggcggtg ctacagagtt cttgaagtgg tggcctaact acggctacac 7680
   tagaaggaca gtatttggta tctgcgctct gctgaagcca gttaccttcg gaaaaagagt 7740
   tggtagctct tgatccggca aacaaaccac cgctggtagc ggtggttttt ttgtttgcaa 7800
   gcagcagatt acgcgcagaa aaaaaggatc tcaagaagat cctttgatct tttctacggg 7860
   gtctgacgct cagtggaacg aaaactcacg ttaagggatt ttggtcatga gattatcaaa 7920
   aaggatcttc acctagatcc ttttaaatta aaaatgaagt tttaaatcaa tctaaagtat 7980
   atatgagtaa acttggtctg acagttacca atgcttaatc agtgaggcac ctatctcagc 8040
   gatctgtcta tttcgttcat ccatagttgc ctgactcccc gtcgtgtaga taactacgat      8100
   acgggagggc ttaccatctg gccccagtgc tgcaatgata ccgcgagacc cacgctcacc      8160
   ggctccagat ttatcagcaa taaaccagcc agccggaagg gccgagcgca gaagtggtcc      8220
   tgcaacttta tccgcctcca tccagtctat taattgttgc cgggaagcta gagtaagtag      8280
   ttcgccagtt aatagtttgc gcaacgttgt tgccattgct acaggcatcg tggtgtcacg      8340
   ctcgtcgttt ggtatggctt cattcagctc cggttcccaa cgatcaaggc gagttacatg      8400
   atcccccatg ttgtgcaaaa aagcggttag ctccttcggt cctccgatcg ttgtcagaag      8460
   taagttggcc gcagtgttat cactcatggt tatggcagca ctgcataatt ctcttactgt      8520
   catgccatcc gtaagatgct tttctgtgac tggtgagtac tcaaccaagt cattctgaga      8580
   atagtgtatg cggcgaccga gttgctcttg cccggcgtca atacgggata ataccgcgcc      8640
   acatagcaga actttaaaag tgctcatcat tggaaaacgt tcttcggggc gaaaactctc      8700
   aaggatctta ccgctgttga gatccagttc gatgtaaccc actcgtgcac ccaactgatc      8760
   ttcagcatct tttactttca ccagcgtttc tgggtgagca aaaacaggaa ggcaaaatgc      8820
   cgcaaaaaag ggaataaggg cgacacggaa atgttgaata ctcatactct tcctttttca      8880
   atattattga agcatttatc agggttattg tctcatgagc ggatacatat ttgaatgtat      8940
   ttagaaaaat aaacaaatag gggttccgcg cacatttccc cgaaaagtgc cacctgacgc      9000
   gccctgtagc ggcgcattaa gcgcggcggg tgtggtggtt acgcgcagcg tgaccgctac      9060
   acttgccagc gccctagcgc ccgctccttt cgctttcttc ccttcctttc tcgccacgtt      9120
   cgccggcttt ccccgtcaag ctctaaatcg ggggctccct ttagggttcc gatttagtgc      9180
   tttacggcac ctcgacccca aaaaacttga ttagggtgat ggttcacgta gtgggccatc      9240
   gccctgatag acggtttttc gccctttgac gttggagtcc acgttcttta atagtggact      9300
   cttgttccaa actggaacaa cactcaaccc tatctcggtc tattcttttg atttataagg      9360
   gattttgccg atttcggcct attggttaaa aaatgagctg atttaacaaa aatttaacgc      9420
   gaattttaac aaaatattaa cgcttacaat ttccattcgc cattcaggct gcgcaactgt      9480
   tgggaagggc gatcggtgcg ggcctcttcg ctattacgcc agctggcgaa agggggatgt     9540
   gctgcaaggc gattaagttg ggtaacgcca gggttttccc agtcacgacg ttgtaaaacg      9600
   acggccagtg aattgtaata cgactcacta tagggcgaat tgggtaccgg gccccccctc      9660
   gaggtcgatg gtgtcgataa gcttgatatc gaattcatgt cacacaaacc gatcttcgcc      9720
   tcaaggaaac ctaattctac atccgagaga ctgccgagat ccagtctaca ctgattaatt      9780
   ttcgggccaa taatttaaaa aaatcgtgtt atataatatt atatgtatta tatatataca      9840
   tcatgatgat actgacagtc atgtcccatt gctaaataga cagactccat ctgccgcctc      9900
   caactgatgt tctcaatatt taaggggtca tctcgcattg tttaataata aacagactcc      9960
   atctaccgcc tccaaatgat gttctcaaaa tatattgtat gaacttattt ttattactta     10020
   gtattattag acaacttact tgctttatga aaaacacttc ctatttagga aacaatttat     10080
   aatggcagtt cgttcattta acaatttatg tagaataaat gttataaatg cgtatgggaa     10140
   atcttaaata tggatagcat aaatgatatc tgcattgcct aattcgaaat caacagcaac     10200
   gaaaaaaatc ccttgtacaa cataaatagt catcgagaaa tatcaactat caaagaacag     10260
   ctattcacac gttactattg agattattat tggacgagaa tcacacactc aactgtcttt     10320
   ctctcttcta gaaatacagg tacaagtatg tactattctc attgttcata cttctagtca     10380
   tttcatccca catattcctt ggatttctct ccaatgaatg acattctatc ttgcaaattc     10440
   aacaattata ataagatata ccaaagtagc ggtatagtgg caatcaaaaa gcttctctgg     10500
   tgtgcttctc gtatttattt ttattctaat gatccattaa aggtatatat ttatttcttg     10560
   ttatataatc cttttgttta ttacatgggc tggatacata aaggtatttt gatttaattt     10620
   tttgcttaaa ttcaatcccc cctcgttcag tgtcaactgt aatggtagga aattaccata     10680
   cttttgaaga agcaaaaaaa atgaaagaaa aaaaaaatcg tatttccagg ttagacgttc     10740
   cgcagaatct agaatgcggt atgcggtaca ttgttcttcg aacgtaaaag ttgcgctccc     10800
   tgagatattg tacatttttg cttttacaag tacaagtaca tcgtacaact atgtactact     10860
   gttgatgcat ccacaacagt ttgttttgtt tttttttgtt tttttttttt ctaatgattc     10920
   attaccgcta tgtataccta cttgtacttg tagtaagccg ggttattggc gttcaattaa     10980
   tcatagactt atgaatctgc acggtgtgcg ctgcgagtta cttttagctt atgcatgcta     11040
   cttgggtgta atattgggat ctgttcggaa atcaacggat gctcaatcga taaaaaacaa     11100
   aaaaaaaagc accgactcgg tgccactttt tcaagttgat aacggactag ccttatttta     11160
   acttgctatt tctagc                                                     11176
<210> 25
   <211> 655
   <212> DNA
   <213> Artificial sequence
<220>
   <223> can1 upstream homology arm
<400> 25
   gggaagcctt gctacgttag gagaagacgc acggcgatga tacgggtacc cctcatgaca      60
   tcaatatccg ctgcccctct tgccagcaag gcgtcagcag gtgctttttt cgctattttc     120
   accagaccac agcctttttc cttgtgtctc atcttggatt ccttcaaagg caactcaccg      180
   cacctccgag tcgtgtgaac aatgtaataa taggctattg acttttttcc cacctgttta     240
   gcgccaaacc caaagcgctt ttcgccccca ctgcagcccg atggaaggca catatggcaa     300
   gggaaaagtc ttcaggtaat acatgcctgc tgcaactata tgtactctga ctcattccct     360
   cagacgtggg tcatagacag ctgttttaaa ccgggcaaat caatctctgt cgcacaggta     420
   tttctgccct tcaaaaccag gttgccacat cagattccat caaagttttt cagactaact     480
   tcaatcttaa acggcatctc acaacaagcg aattggacgg aaaaaaagcg tctatcatta     540
   ccggcaccta tccacactaa gacagtacta aaggacgacg ctccccacga aacgacgttt     600
   cgaccttaac gaccctgccg tctccatcca tccgaccact cccgacgctc tctcc          655
<210> 26
   <211> 29
   <212> DNA
   <213> Artificial sequence
<220>
   <223> Can1 upstream forward
<400> 26
   gggaagcttg ctacgttagg agaagacgc 29
<210> 27
   <211> 37
   <212> DNA
   <213> Artificial sequence
<220>
   <223> Can1 upstream reverse
<400> 27
   ggagagagcg tcgggagtgg tcggatggat ggagacg 37
<210> 28
   <211> 658
   <212> DNA
   <213> Artificial sequence
<220>
   <223> Can1 downstream homology arm
<400> 28
   cgtctccatc catccgacca ctcccgacgc tctctcctgg agcaaaccac tcttaccaag      60
   catatagcat atataataac gtattgaatt tattaactga ttgaattgag agtaaagcca     120
   gtagcgttgt acggctgtag ctttttagaa aagtggcaga tgagcgatgg tggatatgaa     180
   agtaccttta cggcatgtag cgacacaaga tcgcttccaa gaactcgaca ttcaagccca     240
   gctcgtacaa gaaaatgaac tagccaatca tatgaactag cacattgaag tcaccgcatc     300
   atctctgttg gaaacgacgc gcatgtactc gtgcgtagta aatccgtatc tgtacactcg     360
   aaagattaca gtatgtagta gtagcatgac taacgatgta acgtccaaat aacgctctgt     420
   gcctactcct gtagatgcat tagaccacct gctaacgtct acacgttatg tccgttagct     480
   ccaagattgc acttttccct caaagactct gctgggttac gtcatggtct ctttcgggtc     540
   tctggtccgt tctctgcccg cccatatccg cccaggctgc tacgatacag gataagctca     600
   taagcttaga ttatttttcc ggaatgacat cacgatgcag tggtggaagg atgtatgg       658
<210> 29
   <211> 37
   <212> DNA
   <213> Artificial sequence
<220>
   <223> Can1 downstream homology arm forward primer
<400> 29
   cgtctccatc catccgacca ctcccgacgc tctctcc 37
<210> 30
   <211> 22
   <212> DNA
   <213> Yarrowia lipolytica
<400> 30
   ccatacatcc ttccaccact gc 22
<210> 31
   <211> 1276
   <212> DNA
   <213> Artificial sequence
<220>
   <223> Can1 editing template clonign fragment
<400> 31
   gggaagcctt gctacgttag gagaagacgc acggcgatga tacgggtacc cctcatgaca     60
   tcaatatccg ctgcccctct tgccagcaag gcgtcagcag gtgctttttt cgctattttc    120
   accagaccac agcctttttc cttgtgtctc atcttggatt ccttcaaagg caactcaccg    180
   cacctccgag tcgtgtgaac aatgtaataa taggctattg acttttttcc cacctgttta    240
   gcgccaaacc caaagcgctt ttcgccccca ctgcagcccg atggaaggca catatggcaa    300
   gggaaaagtc ttcaggtaat acatgcctgc tgcaactata tgtactctga ctcattccct    360
   cagacgtggg tcatagacag ctgttttaaa ccgggcaaat caatctctgt cgcacaggta    420
   tttctgccct tcaaaaccag gttgccacat cagattccat caaagttttt cagactaact    480
   tcaatcttaa acggcatctc acaacaagcg aattggacgg aaaaaaagcg tctatcatta    540
   ccggcaccta tccacactaa gacagtacta aaggacgacg ctccccacga aacgacgttt    600
   cgaccttaac gaccctgccg tctccatcca tccgaccact cccgacgctc tctcctggag    660
   caaaccactc ttaccaagca tatagcatat ataataacgt attgaattta ttaactgatt    720
   gaattgagag taaagccagt agcgttgtac ggctgtagct ttttagaaaa gtggcagatg    780
   agcgatggtg gatatgaaag tacctttacg gcatgtagcg acacaagatc gcttccaaga    840
   actcgacatt caagcccagc tcgtacaaga aaatgaacta gccaatcata tgaactagca    900
   cattgaagtc accgcatcat ctctgttgga aacgacgcgc atgtactcgt gcgtagtaaa    960
   tccgtatctg tacactcgaa agattacagt atgtagtagt agcatgacta acgatgtaac   1020
   gtccaaataa cgctctgtgc ctactcctgt agatgcatta gaccacctgc taacgtctac   1080
   acgttatgtc cgttagctcc aagattgcac ttttccctca aagactctgc tgggttacgt   1140
   catggtctct ttcgggtctc tggtccgttc tctgcccgcc catatccgcc caggctgcta   1200
   cgatacagga taagctcata agcttagatt atttttccgg aatgacatca cgatgcagtg   1260
   gtggaaggat gtatgg                                                    1276
<210> 32
   <211> 2686
   <212> DNA
   <213> Artificial sequence
<220>
   <223> pUC18
<400> 32
   tcgcgcgttt cggtgatgac ggtgaaaacc tctgacacat gcagctcccg gagacggtca     60
   cagcttgtct gtaagcggat gccgggagca gacaagcccg tcagggcgcg tcagcgggtg    120
   ttggcgggtg tcggggctgg cttaactatg cggcatcaga gcagattgta ctgagagtgc    180
   accatatgcg gtgtgaaata ccgcacagat gcgtaaggag aaaataccgc atcaggcgcc    240
   attcgccatt caggctgcgc aactgttggg aagggcgatc ggtgcgggcc tcttcgctat    300
   tacgccagct ggcgaaaggg ggatgtgctg caaggcgatt aagttgggta acgccagggt    360
   tttcccagtc acgacgttgt aaaacgacgg ccagtgccaa gcttgcatgc ctgcaggtcg    420
   actctagagg atccccgggt accgagctcg aattcgtaat catggtcata gctgtttcct    480
   gtgtgaaatt gttatccgct cacaattcca cacaacatac gagccggaag cataaagtgt    540
   aaagcctggg gtgcctaatg agtgagctaa ctcacattaa ttgcgttgcg ctcactgccc    600
   gctttccagt cgggaaacct gtcgtgccag ctgcattaat gaatcggcca acgcgcgggg    660
   agaggcggtt tgcgtattgg gcgctcttcc gcttcctcgc tcactgactc gctgcgctcg    720
   gtcgttcggc tgcggcgagc ggtatcagct cactcaaagg cggtaatacg gttatccaca    780
   gaatcagggg ataacgcagg aaagaacatg tgagcaaaag gccagcaaaa ggccaggaac    840
   cgtaaaaagg ccgcgttgct ggcgtttttc cataggctcc gcccccctga cgagcatcac    900
   aaaaatcgac gctcaagtca gaggtggcga aacccgacag gactataaag ataccaggcg    960
   tttccccctg gaagctccct cgtgcgctct cctgttccga ccctgccgct taccggatac   1020
   ctgtccgcct ttctcccttc gggaagcgtg gcgctttctc atagctcacg ctgtaggtat   1080
   ctcagttcgg tgtaggtcgt tcgctccaag ctgggctgtg tgcacgaacc ccccgttcag   1140
   cccgaccgct gcgccttatc cggtaactat cgtcttgagt ccaacccggt aagacacgac   1200
   ttatcgccac tggcagcagc cactggtaac aggattagca gagcgaggta tgtaggcggt   1260
   gctacagagt tcttgaagtg gtggcctaac tacggctaca ctagaaggac agtatttggt   1320
   atctgcgctc tgctgaagcc agttaccttc ggaaaaagag ttggtagctc ttgatccggc   1380
   aaacaaacca ccgctggtag cggtggtttt tttgtttgca agcagcagat tacgcgcaga   1440
   aaaaaaggat ctcaagaaga tcctttgatc ttttctacgg ggtctgacgc tcagtggaac   1500
   gaaaactcac gttaagggat tttggtcatg agattatcaa aaaggatctt cacctagatc   1560
   cttttaaatt aaaaatgaag ttttaaatca atctaaagta tatatgagta aacttggtct   1620
   gacagttacc aatgcttaat cagtgaggca cctatctcag cgatctgtct atttcgttca   1680
   tccatagttg cctgactccc cgtcgtgtag ataactacga tacgggaggg cttaccatct   1740
   ggccccagtg ctgcaatgat accgcgagac ccacgctcac cggctccaga tttatcagca   1800
   ataaaccagc cagccggaag ggccgagcgc agaagtggtc ctgcaacttt atccgcctcc   1860
   atccagtcta ttaattgttg ccgggaagct agagtaagta gttcgccagt taatagtttg   1920
   cgcaacgttg ttgccattgc tacaggcatc gtggtgtcac gctcgtcgtt tggtatggct   1980
   tcattcagct ccggttccca acgatcaagg cgagttacat gatcccccat gttgtgcaaa   2040
   aaagcggtta gctccttcgg tcctccgatc gttgtcagaa gtaagttggc cgcagtgtta   2100
   tcactcatgg ttatggcagc actgcataat tctcttactg tcatgccatc cgtaagatgc   2160
   ttttctgtga ctggtgagta ctcaaccaag tcattctgag aatagtgtat gcggcgaccg   2220
   agttgctctt gcccggcgtc aatacgggat aataccgcgc cacatagcag aactttaaaa   2280
   gtgctcatca ttggaaaacg ttcttcgggg cgaaaactct caaggatctt accgctgttg   2340
   agatccagtt cgatgtaacc cactcgtgca cccaactgat cttcagcatc ttttactttc   2400
   accagcgttt ctgggtgagc aaaaacagga aggcaaaatg ccgcaaaaaa gggaataagg   2460
   gcgacacgga aatgttgaat actcatactc ttcctttttc aatattattg aagcatttat   2520
   cagggttatt gtctcatgag cggatacata tttgaatgta tttagaaaaa taaacaaata   2580
   ggggttccgc gcacatttcc ccgaaaagtg ccacctgacg tctaagaaac cattattatc   2640
   atgacattaa cctataaaaa taggcgtatc acgaggccct ttcgtc                  2686
<210> 33
   <211> 3901
   <212> DNA
   <213> Artificial sequence
<220>
   <223> pRF80
<400> 33
   agcttgctac gttaggagaa gacgcacggc gatgatacgg gtacccctca tgacatcaat      60
   atccgctgcc cctcttgcca gcaaggcgtc agcaggtgct tttttcgcta ttttcaccag     120
   accacagcct ttttccttgt gtctcatctt ggattccttc aaaggcaact caccgcacct     180
   ccgagtcgtg tgaacaatgt aataataggc tattgacttt tttcccacct gtttagcgcc     240
   aaacccaaag cgcttttcgc ccccactgca gcccgatgga aggcacatat ggcaagggaa     300
   aagtcttcag gtaatacatg cctgctgcaa ctatatgtac tctgactcat tccctcagac     360
   gtgggtcata gacagctgtt ttaaaccggg caaatcaatc tctgtcgcac aggtatttct     420
   gcccttcaaa accaggttgc cacatcagat tccatcaaag tttttcagac taacttcaat     480
   cttaaacggc atctcacaac aagcgaattg gacggaaaaa aagcgtctat cattaccggc     540
   acctatccac actaagacag tactaaagga cgacgctccc cacgaaacga cgtttcgacc     600
   ttaacgaccc tgccgtctcc atccatccga ccactcccga cgctctctcc tggagcaaac     660
   cactcttacc aagcatatag catatataat aacgtattga atttattaac tgattgaatt     720
   gagagtaaag ccagtagcgt tgtacggctg tagcttttta gaaaagtggc agatgagcga     780
   tggtggatat gaaagtacct ttacggcatg tagcgacaca agatcgcttc caagaactcg     840
   acattcaagc ccagctcgta caagaaaatg aactagccaa tcatatgaac tagcacattg     900
   aagtcaccgc atcatctctg ttggaaacga cgcgcatgta ctcgtgcgta gtaaatccgt     960
   atctgtacac tcgaaagatt acagtatgta gtagtagcat gactaacgat gtaacgtcca    1020
   aataacgctc tgtgcctact cctgtagatg cattagacca cctgctaacg tctacacgtt    1080
   atgtccgtta gctccaagat tgcacttttc cctcaaagac tctgctgggt tacgtcatgg    1140
   tctctttcgg gtctctggtc cgttctctgc ccgcccatat ccgcccaggc tgctacgata     1200
   caggataagc tcataagctt gcatgcctgc aggtcgactc tagaggatcc ccgggtaccg     1260
   agctcgaatt cgtaatcatg gtcatagctg tttcctgtgt gaaattgtta tccgctcaca     1320
   attccacaca acatacgagc cggaagcata aagtgtaaag cctggggtgc ctaatgagtg     1380
   agctaactca cattaattgc gttgcgctca ctgcccgctt tccagtcggg aaacctgtcg     1440
   tgccagctgc attaatgaat cggccaacgc gcggggagag gcggtttgcg tattgggcgc     1500
   tcttccgctt cctcgctcac tgactcgctg cgctcggtcg ttcggctgcg gcgagcggta     1560
   tcagctcact caaaggcggt aatacggtta tccacagaat caggggataa cgcaggaaag     1620
   aacatgtgag caaaaggcca gcaaaaggcc aggaaccgta aaaaggccgc gttgctggcg     1680
   tttttccata ggctccgccc ccctgacgag catcacaaaa atcgacgctc aagtcagagg     1740
   tggcgaaacc cgacaggact ataaagatac caggcgtttc cccctggaag ctccctcgtg     1800
   cgctctcctg ttccgaccct gccgcttacc ggatacctgt ccgcctttct cccttcggga     1860
   agcgtggcgc tttctcatag ctcacgctgt aggtatctca gttcggtgta ggtcgttcgc     1920
   tccaagctgg gctgtgtgca cgaacccccc gttcagcccg accgctgcgc cttatccggt     1980
   aactatcgtc ttgagtccaa cccggtaaga cacgacttat cgccactggc agcagccact     2040
   ggtaacagga ttagcagagc gaggtatgta ggcggtgcta cagagttctt gaagtggtgg     2100
   cctaactacg gctacactag aaggacagta tttggtatct gcgctctgct gaagccagtt     2160
   accttcggaa aaagagttgg tagctcttga tccggcaaac aaaccaccgc tggtagcggt     2220
   ggtttttttg tttgcaagca gcagattacg cgcagaaaaa aaggatctca agaagatcct     2280
   ttgatctttt ctacggggtc tgacgctcag tggaacgaaa actcacgtta agggattttg     2340
   gtcatgagat tatcaaaaag gatcttcacc tagatccttt taaattaaaa atgaagtttt     2400
   aaatcaatct aaagtatata tgagtaaact tggtctgaca gttaccaatg cttaatcagt     2460
   gaggcaccta tctcagcgat ctgtctattt cgttcatcca tagttgcctg actccccgtc     2520
   gtgtagataa ctacgatacg ggagggctta ccatctggcc ccagtgctgc aatgataccg     2580
   cgagacccac gctcaccggc tccagattta tcagcaataa accagccagc cggaagggcc     2640
   gagcgcagaa gtggtcctgc aactttatcc gcctccatcc agtctattaa ttgttgccgg     2700
   gaagctagag taagtagttc gccagttaat agtttgcgca acgttgttgc cattgctaca     2760
   ggcatcgtgg tgtcacgctc gtcgtttggt atggcttcat tcagctccgg ttcccaacga     2820
   tcaaggcgag ttacatgatc ccccatgttg tgcaaaaaag cggttagctc cttcggtcct     2880
   ccgatcgttg tcagaagtaa gttggccgca gtgttatcac tcatggttat ggcagcactg     2940
   cataattctc ttactgtcat gccatccgta agatgctttt ctgtgactgg tgagtactca     3000
   accaagtcat tctgagaata gtgtatgcgg cgaccgagtt gctcttgccc ggcgtcaata     3060
   cgggataata ccgcgccaca tagcagaact ttaaaagtgc tcatcattgg aaaacgttct     3120
   tcggggcgaa aactctcaag gatcttaccg ctgttgagat ccagttcgat gtaacccact     3180
   cgtgcaccca actgatcttc agcatctttt actttcacca gcgtttctgg gtgagcaaaa     3240
   acaggaaggc aaaatgccgc aaaaaaggga ataagggcga cacggaaatg ttgaatactc     3300
   atactcttcc tttttcaata ttattgaagc atttatcagg gttattgtct catgagcgga     3360
   tacatatttg aatgtattta gaaaaataaa caaatagggg ttccgcgcac atttccccga     3420
   aaagtgccac ctgacgtcta agaaaccatt attatcatga cattaaccta taaaaatagg     3480
   cgtatcacga ggccctttcg tctcgcgcgt ttcggtgatg acggtgaaaa cctctgacac     3540
   atgcagctcc cggagacggt cacagcttgt ctgtaagcgg atgccgggag cagacaagcc     3600
   cgtcagggcg cgtcagcggg tgttggcggg tgtcggggct ggcttaacta tgcggcatca     3660
   gagcagattg tactgagagt gcaccatatg cggtgtgaaa taccgcacag atgcgtaagg     3720
   agaaaatacc gcatcaggcg ccattcgcca ttcaggctgc gcaactgttg ggaagggcga     3780
   tcggtgcggg cctcttcgct attacgccag ctggcgaaag ggggatgtgc tgcaaggcga     3840
   ttaagttggg taacgccagg gttttcccag tcacgacgtt gtaaaacgac ggccagtgcc     3900
   a                                                                     3901
<210> 34
   <211> 1210
   <212> DNA
   <213> Artificial sequence
<220>
   <223> Can1 polynucleotide modification (editing) template
<400> 34
   gctacgttag gagaagacgc acggcgatga tacgggtacc cctcatgaca tcaatatccg      60
   ctgcccctct tgccagcaag gcgtcagcag gtgctttttt cgctattttc accagaccac     120
   agcctttttc cttgtgtctc atcttggatt ccttcaaagg caactcaccg cacctccgag     180
   tcgtgtgaac aatgtaataa taggctattg acttttttcc cacctgttta gcgccaaacc     240
   caaagcgctt ttcgccccca ctgcagcccg atggaaggca catatggcaa gggaaaagtc     300
   ttcaggtaat acatgcctgc tgcaactata tgtactctga ctcattccct cagacgtggg     360
   tcatagacag ctgttttaaa ccgggcaaat caatctctgt cgcacaggta tttctgccct     420
   tcaaaaccag gttgccacat cagattccat caaagttttt cagactaact tcaatcttaa     480
   acggcatctc acaacaagcg aattggacgg aaaaaaagcg tctatcatta ccggcaccta     540
   tccacactaa gacagtacta aaggacgacg ctccccacga aacgacgttt cgaccttaac     600
   gaccctgccg tctccatcca tccgaccact cccgacgctc tctcctggag caaaccactc     660
   ttaccaagca tatagcatat ataataacgt attgaattta ttaactgatt gaattgagag     720
   taaagccagt agcgttgtac ggctgtagct ttttagaaaa gtggcagatg agcgatggtg     780
   gatatgaaag tacctttacg gcatgtagcg acacaagatc gcttccaaga actcgacatt     840
   caagcccagc tcgtacaaga aaatgaacta gccaatcata tgaactagca cattgaagtc     900
   accgcatcat ctctgttgga aacgacgcgc atgtactcgt gcgtagtaaa tccgtatctg     960
   tacactcgaa agattacagt atgtagtagt agcatgacta acgatgtaac gtccaaataa    1020
   cgctctgtgc ctactcctgt agatgcatta gaccacctgc taacgtctac acgttatgtc    1080
   cgttagctcc aagattgcac ttttccctca aagactctgc tgggttacgt catggtctct    1140
   ttcgggtctc tggtccgttc tctgcccgcc catatccgcc caggctgcta cgatacagga    1200
   taagctcata                                                           1210
<210> 35
   <211> 20
   <212> DNA
   <213> Artificial sequence
<220>
   <223> C3S forward
<400> 35
   agcttgctac gttaggagaa 20
<210> 36
   <211> 20
   <212> DNA
   <213> Artificial seqeunce
<220>
   <223> C3S reverse
<400> 36
   tatgagctta tcctgtatcg 20
<210> 37
   <211> 20
   <212> DNA
   <213> Artificial sequence
<220>
   <223> PT forward
<400> 37
   agcttgctac gttaggagaa 20
<210> 38
   <211> 20
   <212> DNA
   <213> Artificial sequence
<220>
   <223> PT reverse
<400> 38
   tatgagctta tcctgtatcg 20
<210> 39
   <211> 2125
   <212> DNA
   <213> Artificial sequence
<220>
   <223> CAN1 locus
<400> 39
   ggaaggcaca tatggcaagg gaaaagtctt caggtaatac atgcctgctg caactatatg      60
   tactctgact cattccctca gacgtgggtc atagacagct gttttaaacc gggcaaatca     120
   atctctgtcg cacaggtatt tctgcccttc aaaaccaggt tgccacatca gattccatca     180
   aagtttttca gactaacttc aatcttaaac ggcatctcac aacaagcgaa ttggacggaa     240
   aaaaagcgtc tatcattacc ggcacctatc cacactaaga cagtactaaa ggacgacgct     300
   ccccacgaaa cgacgtttcg accttaacga ccctgccgtc tccatccatc cgaccacaat     360
   ggaaaagaca ttttcaaacg attacccacc ctccgggact gaggcccaca tccacatcaa     420
   ccacacggcc cactcggatg actcagagga ggtgccctcg cacaaggaaa attacaacac     480
   cagtggccac gacctggagg agtccgaccc ggataaccat gtcggtgaga ccctcgaggt     540
   caagcgaggt ctcaagatgc gacacatctc catgatctcg cttggaggaa ccattggtac     600
   cggtctcttc attggtaccg gaggagctct ccagcaggcc ggtccctgtg gcgccctcgt     660
   cgcctacgtg ttcatggcca ccattgtcta ctctgttgcc gagtctcttg gagaactggc     720
   tacgtacatt cccatcaccg gctcctttgc cgtctttact acccgatatc tgtcacagtc     780
   gtttggtgcc tccatgggct ggctatactg gttctcgtgg gcgatcacct tcgccatcga     840
   gctcaacacc attggtcccg tgattgagta ctggactgac gccgttccta ctgctgcctg     900
   gattgccatc ttcttcgtca tcctcactac catcaacttc ttccccgtgg gcttctatgg     960
   cgaagtcgag ttctgggtgg cctccgtgaa ggtcattgcc atcattggat ggctcatcta    1020
   cgcgctctgc atgacgtgtg gagcaggtgt aacaggtcct gtgggattca gatactggaa    1080
   ccaccccgga cccatgggag acggaatctg gaccgacggc gtgcccattg tgcgaaacgc    1140
   gcccggtcga cgattcatgg gatggctcaa ttcgctcgtt aacgccgcct tcacctacca    1200
   gggctgtgag ctggtcggag tcactgccgg tgaggcccag aaccccagaa agtccgtccc    1260
   tcgagccatc aaccgagtct ttgctcgaat ttgcatcttc tacattggct ctatcttctt    1320
   catgggcatg ctcgtgccct ttaacgaccc caagctgacc gatgactcct ccgtcatcgc    1380
   ctcctctcct tttgttattg ccattatcaa ctctggcacc aaggtgctcc ctcacatttt    1440
   caacgccgtc attctcatca ccctgatttc ggcaggaaac tccaacgtct acattggctc    1500
   gcgagtggtc tacgccctgg ctgactccgg aaccgcacca aagttcttca agcgaaccac    1560
   caagaaggga gtgccgtacg tggcagtctg cttcacctcg gcgtttggtc tgctggcctt    1620
   catgtctgtg tccgagtcgt cgtccactgt cttcgactgg ttcatcaaca tctccgctgt    1680
   ggccggcctc atctgttggg ccttcatctc tgcctcccac atccgattca tgcaagtgct    1740
   taagcacaga gggatctcca gagatacgct gcccttcaag gcacgatggc agccattcta    1800
   ctcatggtac gcgctcgtct ccatcatctt catcactctc atccagggct tcacgtcctt    1860
   ctggcacttt accgccgcca agttcatgac tgcatacatc tccgtcattg tctgggtcgg    1920
   tttgtacatt atcttccagt gtctgttccg atgcaagttc cttatcccta ttgaggatgt    1980
   ggacattgac accggccgac gagagattga cgacgatgtg tgggaggaga agatccccac    2040
   aaagtggtac gagaagtttt ggaatattat tgcataagaa gatcggggat tcccgacgct    2100
   ctctcctgga gcaaaccact cttac                                          2125
<210> 40
   <211> 20
   <212> DNA
   <213> Artificial sequence
<220>
   <223> unmodified forward
<400> 40
   agcttgctac gttaggagaa 20
<210> 41
   <211> 20
   <212> DNA
   <213> Artificial sequence
<220>
   <223> unmodified reverse
<400> 41
   tatgagctta tcctgtatcg 20
<210> 42
   <211> 20
   <212> DNA
   <213> Yarrowia lipolytica
<400> 42
   ggaaggcaca tatggcaagg 20
<210> 43
   <211> 21
   <212> DNA
   <213> Yarrowia lipolytica
<400> 43
   gtaagagtgg tttgctccag g 21
<210> 44
   <211> 2125
   <212> DNA
   <213> Yarrowia lipolytica
<400> 44
   ggaaggcaca tatggcaagg gaaaagtctt caggtaatac atgcctgctg caactatatg      60
   tactctgact cattccctca gacgtgggtc atagacagct gttttaaacc gggcaaatca     120
   atctctgtcg cacaggtatt tctgcccttc aaaaccaggt tgccacatca gattccatca     180
   aagtttttca gactaacttc aatcttaaac ggcatctcac aacaagcgaa ttggacggaa     240
   aaaaagcgtc tatcattacc ggcacctatc cacactaaga cagtactaaa ggacgacgct     300
   ccccacgaaa cgacgtttcg accttaacga ccctgccgtc tccatccatc cgaccacaat     360
   ggaaaagaca ttttcaaacg attacccacc ctccgggact gaggcccaca tccacatcaa     420
   ccacacggcc cactcggatg actcagagga ggtgccctcg cacaaggaaa attacaacac     480
   cagtggccac gacctggagg agtccgaccc ggataaccat gtcggtgaga ccctcgaggt     540
   caagcgaggt ctcaagatgc gacacatctc catgatctcg cttggaggaa ccattggtac     600
   cggtctcttc attggtaccg gaggagctct ccagcaggcc ggtccctgtg gcgccctcgt     660
   cgcctacgtg ttcatggcca ccattgtcta ctctgttgcc gagtctcttg gagaactggc     720
   tacgtacatt cccatcaccg gctcctttgc cgtctttact acccgatatc tgtcacagtc     780
   gtttggtgcc tccatgggct ggctatactg gttctcgtgg gcgatcacct tcgccatcga     840
   gctcaacacc attggtcccg tgattgagta ctggactgac gccgttccta ctgctgcctg     900
   gattgccatc ttcttcgtca tcctcactac catcaacttc ttccccgtgg gcttctatgg     960
   cgaagtcgag ttctgggtgg cctccgtgaa ggtcattgcc atcattggat ggctcatcta    1020
   cgcgctctgc atgacgtgtg gagcaggtgt aacaggtcct gtgggattca gatactggaa    1080
   ccaccccgga cccatgggag acggaatctg gaccgacggc gtgcccattg tgcgaaacgc    1140
   gcccggtcga cgattcatgg gatggctcaa ttcgctcgtt aacgccgcct tcacctacca    1200
   gggctgtgag ctggtcggag tcactgccgg tgaggcccag aaccccagaa agtccgtccc    1260
   tcgagccatc aaccgagtct ttgctcgaat ttgcatcttc tacattggct ctatcttctt    1320
   catgggcatg ctcgtgccct ttaacgaccc caagctgacc gatgactcct ccgtcatcgc    1380
   ctcctctcct tttgttattg ccattatcaa ctctggcacc aaggtgctcc ctcacatttt    1440
   caacgccgtc attctcatca ccctgatttc ggcaggaaac tccaacgtct acattggctc    1500
   gcgagtggtc tacgccctgg ctgactccgg aaccgcacca aagttcttca agcgaaccac    1560
   caagaaggga gtgccgtacg tggcagtctg cttcacctcg gcgtttggtc tgctggcctt    1620
   catgtctgtg tccgagtcgt cgtccactgt cttcgactgg ttcatcaaca tctccgctgt    1680
   ggccggcctc atctgttggg ccttcatctc tgcctcccac atccgattca tgcaagtgct    1740
   taagcacaga gggatctcca gagatacgct gcccttcaag gcacgatggc agccattcta    1800
   ctcatggtac gcgctcgtct ccatcatctt catcactctc atccagggct tcacgtcctt    1860
   ctggcacttt accgccgcca agttcatgac tgcatacatc tccgtcattg tctgggtcgg    1920
   tttgtacatt atcttccagt gtctgttccg atgcaagttc cttatcccta ttgaggatgt    1980
   ggacattgac accggccgac gagagattga cgacgatgtg tgggaggaga agatccccac    2040
   aaagtggtac gagaagtttt ggaatattat tgcataagaa gatcggggat tcccgacgct    2100
   ctctcctgga gcaaaccact cttac                                          2125
<210> 45
   <211> 392
   <212> DNA
   <213> Artificial sequence
<220>
   <223> Can1 locus deletion
<400> 45
   ggaaggcaca tatggcaagg gaaaagtctt caggtaatac atgcctgctg caactatatg      60
   tactctgact cattccctca gacgtgggtc atagacagct gttttaaacc gggcaaatca     120
   atctctgtcg cacaggtatt tctgcccttc aaaaccaggt tgccacatca gattccatca     180
   aagtttttca gactaacttc aatcttaaac ggcatctcac aacaagcgaa ttggacggaa     240
   aaaaagcgtc tatcattacc ggcacctatc cacactaaga cagtactaaa ggacgacgct     300
   ccccacgaaa cgacgtttcg accttaacga ccctgccgtc tccatccatc cgaccactcc     360
   cgacgctctc tcctggagca aaccactctt ac                                   392
<210> 46
   <211> 62
   <212> DNA
   <213> Yarrowia lipolytica
<220>
   <221> misc_feature
   <222> (1)..(62)
   <223> Copy number analysis fragment
<400> 46
   agcgccaaac ccaaagcgct tttcgccccc actgcagccc gatggaaggc acatatggca        60
   ag                                                                        62
<210> 47
   <211> 17
   <212> DNA
   <213> Artificial sequence
<220>
   <223> Can1 copy number F
<400> 47
   agcgccaaac ccaaagc 17
<210> 48
   <211> 20
   <212> DNA
   <213> Artificial Seqeunce
<220>
   <223> Can1 copy number R
<400> 48
   cttgccatat gtgccttcca 20
<210> 49
   <211> 21
   <212> DNA
   <213> Artificial sequence
<220>
   <223> Can1 copy number probe
<400> 49
   cttttcgccc ccactgcagc c 21
<210> 50
   <211> 69
   <212> DNA
   <213> Yarrowia lipolytica
<400> 50
   tgaccgtcct tggagatacc agcctcgaac tcaccaacac caccagcaat gatgaggatg        60
   gcacagtcg                                                                 69
<210> 51
   <211> 20
   <212> DNA
   <213> Artificial sequence
<220>
   <223> TEF1 forward
<400> 51
   cgactgtgcc atcctcatca 20
<210> 52
   <211> 21
   <212> DNA
   <213> Artificial sequence
<220>
   <223> TEF1 reverse
<400> 52
   tgaccgtcct tggagatacc a 21
<210> 53
   <211> 21
   <212> DNA
   <213> Yarrowia lipolytica
<400> 53
   tgctggtggt gttggtgagt t 21
<210> 54
   <211> 12167
   <212> DNA
   <213> Artificial sequence
<220>
   <223> pRF434
<400> 54
   cgataaaaaa caaaaaaaaa agcaccgact cggtgccact ttttcaagtt gataacggac      60
   tagccttatt ttaacttgct atttctagct ctaaaacgca ggtgtaaaaa taaaaaggcc     120
   tgcgattacc agcaggcctg ttattaacct aagccttagg acgcttcacg ccatacttgg     180
   aacgagcctg cttacggtct ttaacgccgg agcagtcaag cgcaccacgt acggtgtggt     240
   aacgaacacc cgggaggtct ttaacacgac cgccacggat caggatcacg gagtgctcct     300
   gcaggttgtg accttcacca ccgatgtagg aagtcacttc gaaaccgtta gtcagacgaa     360
   cacggcatac tttacgcagc gcggagttcg gttttttagg agtggtagta tatacacgag     420
   tacatacgcc acgtttttgc gggcatgctt ccagcgcagg cacgttgctt ttcgcaactt     480
   tgcgagcacg tggtttgcgt accagctggt taactgttgc cattaaatag ctcctggttt     540
   tagcttttgc ttcgtaaaca cgtaataaaa cgtcctcaca caatatgagg acgccgaatt     600
   tagggcgatg ccgaaaaggt gtcaagaaat atacaacgat cccgccatca cctgcgtccc     660
   attcgccatg ccgaagcatg ttgcccagcc ggcgccagcg aggaggctgg gaccatgccg     720
   gccattattt tgcgttaagt ttctaatcat cacgaaatta tctatcaaaa ataactaggt     780
   cccaccgaga ttcgaactcg ggaccttaag atttgcaatc tcacgcgcta ccgctgtgcc     840
   ataggaccga agttaaaatt tggccaaaga aggacctggg caccctggac tgtgggttag     900
   ggtaatattc cttatggaga caatgggcta gggtaaatta cctaaaatgg gtcgataaag     960
   aggggtgttc ccagttggga agtgtaattg aagacggggt caaaaaagaa aatcaaaaaa      1020
   aatttaatta agactatgat aacttcgtat aatgtatgct atacgaacgg tagcacactg      1080
   tacgagtaag agcactagcc actgcactca agtgaaaccg ttgcccgggt acgagtatga      1140
   gtatgtacag tatgtttagt attgtacttg gacagtgctt gtatcgtaca ttctcaagtg      1200
   tcaaacataa atatccgttg ctatatcctc gcaccaccac gtagctcgct atatccctgt      1260
   gttgaatcca tccatcttgg attgccaatt gtgcacacag aaccgggcac tcacttcccc      1320
   atccacactt gcggccgcta ttcctttgcc ctcggacgag tgctggggcg tcggtttcca      1380
   ctatcggcga gtacttctac acagccatcg gtccagacgg ccgcgcttct gcgggcgatt      1440
   tgtgtacgcc cgacagtccc ggctccggat cggacgattg cgtcgcatcg accctgcgcc      1500
   caagctgcat catcgaaatt gccgtcaacc aagctctgat agagttggtc aagaccaatg      1560
   cggagcatat acgcccggag ccgcggcgat cctgcaagct ccggatgcct ccgctcgaag      1620
   tagcgcgtct gctgctccat acaagccaac cacggcctcc agaagaagat gttggcgacc      1680
   tcgtattggg aatccccgaa catcgcctcg ctccagtcaa tgaccgctgt tatgcggcca      1740
   ttgtccgtca ggacattgtt ggagccgaaa tccgcgtgca cgaggtgccg gacttcgggg      1800
   cagtcctcgg cccaaagcat cagctcatcg agagcctgcg cgacggacgc actgacggtg      1860
   tcgtccatca cagtttgcca gtgatacaca tggggatcag caatcgcgca tatgaaatca      1920
   cgccatgtag tgtattgacc gattccttgc ggtccgaatg ggccgaaccc gctcgtctgg      1980
   ctaagatcgg ccgcagcgat cgcatccata gcctccgcga ccggctgcag aacagcgggc      2040
   agttcggttt caggcaggtc ttgcaacgtg acaccctgtg cacggcggga gatgcaatag      2100
   gtcaggctct cgctgaactc cccaatgtca agcacttccg gaatcgggag cgcggccgat      2160
   gcaaagtgcc gataaacata acgatctttg tagaaaccat cggcgcagct atttacccgc      2220
   aggacatatc cacgccctcc tacatcgaag ctgaaagcac gagattcttc gccctccgag      2280
   agctgcatca ggtcggagac gctgtcgaac ttttcgatca gaaacttctc gacagacgtc      2340
   gcggtgagtt caggcttttt ggccatggtt gatgtgtgtt taattcaaga atgaatatag      2400
   agaagagaag aagaaaaaag attcaattga gccggcgatg cagaccctta tataaatgtt      2460
   gccttggaca gacggagcaa gcccgcccaa acctacgttc ggtataatat gttaagcttt      2520
   ttaacacaaa ggtttggctt ggggtaacct gatgtggtgc aaaagaccgg gcgttggcga      2580
   gccattgcgc gggcgaatgg ggccgtgact cgtctcaaat tcgagggcgt gcctcaattc      2640
   gtgcccccgt ggctttttcc cgccgtttcc gccccgtttg caccactgca gccgcttctt      2700
   tggttcggac accttgctgc gagctaggtg ccttgtgcta cttaaaaagt ggcctcccaa      2760
   caccaacatg acatgagtgc gtgggccaag acacgttggc ggggtcgcag tcggctcaat      2820
   ggcccggaaa aaacgctgct ggagctggtt cggacgcagt ccgccgcggc gtatggatat      2880
   ccgcaaggtt ccatagcgcc attgccctcc gtcggcgtct atcccgcaac ctaccgttcg      2940
   tataatgtat gctatacgaa gttatgagcg ggcttaaggt ttaaaccatc atctaagggc      3000
   ctcaaaacta cctcggaact gctgcgctga tctggacacc acagaggttc cgagcacttt      3060
   aggttgcacc aaatgtccca ccaggtgcag gcagaaaacg ctggaacagc gtgtacagtt      3120
   tgtcttaaca aaaagtgagg gcgctgaggt cgagcagggt ggtgtgactt gttatagcct      3180
   ttagagctgc gaaagcgcgt atggatttgg ctcatcaggc cagattgagg gtctgtggac      3240
   acatgtcatg ttagtgtact tcaatcgccc cctggatata gccccgacaa taggccgtgg      3300
   cctcattttt ttgccttccg cacatttcca ttgctcggta cccacacctt gcttctcctg      3360
   cacttgccaa ccttaatact ggtttacatt gaccaacatc ttacaagcgg ggggcttgtc      3420
   tagggtatat ataaacagtg gctctcccaa tcggttgcca gtctcttttt tcctttcttt      3480
   ccccacagat tcgaaatcta aactacacat cacaccatgg acaagaaata ctccatcggc      3540
   ctggacattg gaaccaactc tgtcggctgg gctgtcatca ccgacgagta caaggtgccc      3600
   tccaagaaat tcaaggtcct cggaaacacc gatcgacact ccatcaagaa aaacctcatt      3660
   ggtgccctgt tgttcgattc tggcgagact gccgaagcta ccagactcaa gcgaactgct      3720
   cggcgacgtt acacccgacg gaagaaccga atctgctacc tgcaggagat cttttccaac      3780
   gagatggcca aggtggacga ttcgttcttt catcgactgg aggaatcctt cctcgtcgag      3840
   gaagacaaga aacacgagcg tcatcccatc tttggcaaca ttgtggacga ggttgcttac      3900
   cacgagaagt atcctaccat ctaccatctc cgaaagaaac tcgtcgattc caccgacaag      3960
   gcggatctca gacttatcta cctcgctctg gcacacatga tcaagtttcg aggtcatttc      4020
   ctcatcgagg gcgatctcaa tcccgacaac agcgatgtgg acaagctgtt cattcagctc      4080
   gttcagacct acaaccagct gttcgaggaa aaccccatca atgcctccgg agtcgatgca      4140
   aaggccatct tgtctgctcg actctcgaag agcagacgac tggagaacct cattgcccaa      4200
   cttcctggcg agaaaaagaa cggactgttt ggcaacctca ttgccctttc tcttggtctc      4260
   acacccaact tcaagtccaa cttcgatctg gcggaggacg ccaagctcca gctgtccaag      4320
   gacacctacg acgatgacct cgacaacctg cttgcacaga ttggcgatca gtacgccgac      4380
   ctgtttctcg ctgccaagaa cctttcggat gctattctct tgtctgacat tctgcgagtc      4440
   aacaccgaga tcacaaaggc tcccctttct gcctccatga tcaagcgata cgacgagcac      4500
   catcaggatc tcacactgct caaggctctt gtccgacagc aactgcccga gaagtacaag      4560
   gagatctttt tcgatcagtc gaagaacggc tacgctggat acatcgacgg cggagcctct      4620
   caggaagagt tctacaagtt catcaagcca attctcgaga agatggacgg aaccgaggaa      4680
   ctgcttgtca agctcaatcg agaggatctg cttcggaagc aacgaacctt cgacaacggc      4740
   agcattcctc atcagatcca cctcggtgag ctgcacgcca ttcttcgacg tcaggaagac     4800
   ttctacccct ttctcaagga caaccgagag aagatcgaga agattcttac ctttcgaatc     4860
   ccctactatg ttggtcctct tgccagagga aactctcgat ttgcttggat gactcgaaag     4920
   tccgaggaaa ccatcactcc ctggaacttc gaggaagtcg tggacaaggg tgcctctgca     4980
   cagtccttca tcgagcgaat gaccaacttc gacaagaatc tgcccaacga gaaggttctt     5040
   cccaagcatt cgctgctcta cgagtacttt acagtctaca acgaactcac caaagtcaag     5100
   tacgttaccg agggaatgcg aaagcctgcc ttcttgtctg gcgaacagaa gaaagccatt     5160
   gtcgatctcc tgttcaagac caaccgaaag gtcactgtta agcagctcaa ggaggactac     5220
   ttcaagaaaa tcgagtgttt cgacagcgtc gagatttccg gagttgagga ccgattcaac     5280
   gcctctttgg gcacctatca cgatctgctc aagattatca aggacaagga ttttctcgac     5340
   aacgaggaaa acgaggacat tctggaggac atcgtgctca ctcttaccct gttcgaagat     5400
   cgggagatga tcgaggaacg actcaagaca tacgctcacc tgttcgacga caaggtcatg     5460
   aaacaactca agcgacgtag atacaccggc tggggaagac tttcgcgaaa gctcatcaac     5520
   ggcatcagag acaagcagtc cggaaagacc attctggact ttctcaagtc cgatggcttt     5580
   gccaaccgaa acttcatgca gctcattcac gacgattctc ttaccttcaa ggaggacatc     5640
   cagaaggcac aagtgtccgg tcagggcgac agcttgcacg aacatattgc caacctggct     5700
   ggttcgccag ccatcaagaa aggcattctc cagactgtca aggttgtcga cgagctggtg     5760
   aaggtcatgg gacgtcacaa gcccgagaac attgtgatcg agatggccag agagaaccag     5820
   acaactcaaa agggtcagaa aaactcgcga gagcggatga agcgaatcga ggaaggcatc     5880
   aaggagctgg gatcccagat tctcaaggag catcccgtcg agaacactca actgcagaac     5940
   gagaagctgt atctctacta tctgcagaat ggtcgagaca tgtacgtgga tcaggaactg     6000
   gacatcaatc gtctcagcga ctacgatgtg gaccacattg tccctcaatc ctttctcaag     6060
   gacgattcta tcgacaacaa ggtccttaca cgatccgaca agaacagagg caagtcggac     6120
   aacgttccca gcgaagaggt ggtcaaaaag atgaagaact actggcgaca gctgctcaac     6180
   gccaagctca ttacccagcg aaagttcgac aatcttacca aggccgagcg aggcggtctg     6240
   tccgagctcg acaaggctgg cttcatcaag cgtcaactcg tcgagaccag acagatcaca     6300
   aagcacgtcg cacagattct cgattctcgg atgaacacca agtacgacga gaacgacaag     6360
   ctcatccgag aggtcaaggt gattactctc aagtccaaac tggtctccga tttccgaaag     6420
   gactttcagt tctacaaggt gcgagagatc aacaattacc accatgccca cgatgcttac     6480
   ctcaacgccg tcgttggcac tgcgctcatc aagaaatacc ccaagctcga aagcgagttc     6540
   gtttacggcg attacaaggt ctacgacgtt cgaaagatga ttgccaagtc cgaacaggag     6600
   attggcaagg ctactgccaa gtacttcttt tactccaaca tcatgaactt tttcaagacc     6660
   gagatcacct tggccaacgg agagattcga aagagaccac ttatcgagac caacggcgaa     6720
   actggagaga tcgtgtggga caagggtcga gactttgcaa ccgtgcgaaa ggttctgtcg     6780
   atgcctcagg tcaacatcgt caagaaaacc gaggttcaga ctggcggatt ctccaaggag     6840
   tcgattctgc ccaagcgaaa ctccgacaag ctcatcgctc gaaagaaaga ctgggatccc     6900
   aagaaatacg gtggcttcga ttctcctacc gtcgcctatt ccgtgcttgt cgttgcgaag     6960
   gtcgagaagg gcaagtccaa aaagctcaag tccgtcaagg agctgctcgg aattaccatc     7020
   atggagcgat cgagcttcga gaagaatccc atcgacttct tggaagccaa gggttacaag     7080
   gaggtcaaga aagacctcat tatcaagctg cccaagtact ctctgttcga actggagaac     7140
   ggtcgaaagc gtatgctcgc ctccgctggc gagctgcaga agggaaacga gcttgccttg     7200
   ccttcgaagt acgtcaactt tctctatctg gcttctcact acgagaagct caagggttct     7260
   cccgaggaca acgaacagaa gcaactcttc gttgagcagc acaaacatta cctcgacgag     7320
   attatcgagc agatttccga gttttcgaag cgagtcatcc tggctgatgc caacttggac     7380
   aaggtgctct ctgcctacaa caagcatcgg gacaaaccca ttcgagaaca ggcggagaac     7440
   atcattcacc tgtttactct taccaacctg ggtgctcctg cagctttcaa gtacttcgat     7500
   accactatcg accgaaagcg gtacacatcc accaaggagg ttctcgatgc caccctgatt     7560
   caccagtcca tcactggcct gtacgagacc cgaatcgacc tgtctcagct tggtggcgac     7620
   tccagagccg atcccaagaa aaagcgaaag gtctaagcgg ccgcaagtgt ggatggggaa     7680
   gtgagtgccc ggttctgtgt gcacaattgg caatccaaga tggatggatt caacacaggg     7740
   atatagcgag ctacgtggtg gtgcgaggat atagcaacgg atatttatgt ttgacacttg     7800
   agaatgtacg atacaagcac tgtccaagta caatactaaa catactgtac atactcatac     7860
   tcgtacccgg gcaacggttt cacttgagtg cagtggctag tgctcttact cgtacagtgt     7920
   gcaatactgc gtatcatagt ctttgatgta tatcgtattc attcatgtta gttgcgtacg     7980
   agccggaagc ataaagtgta aagcctgggg tgcctaatga gtgagctaac tcacattaat     8040
   tgcgttgcgc tcactgcccg ctttccagtc gggaaacctg tcgtgccagc tgcattaatg     8100
   aatcggccaa cgcgcgggga gaggcggttt gcgtattggg cgctcttccg cttcctcgct     8160
   cactgactcg ctgcgctcgg tcgttcggct gcggcgagcg gtatcagctc actcaaaggc     8220
   ggtaatacgg ttatccacag aatcagggga taacgcagga aagaacatgt gagcaaaagg     8280
   ccagcaaaag gccaggaacc gtaaaaaggc cgcgttgctg gcgtttttcc ataggctccg     8340
   cccccctgac gagcatcaca aaaatcgacg ctcaagtcag aggtggcgaa acccgacagg     8400
   actataaaga taccaggcgt ttccccctgg aagctccctc gtgcgctctc ctgttccgac     8460
   cctgccgctt accggatacc tgtccgcctt tctcccttcg ggaagcgtgg cgctttctca     8520
   tagctcacgc tgtaggtatc tcagttcggt gtaggtcgtt cgctccaagc tgggctgtgt     8580
   gcacgaaccc cccgttcagc ccgaccgctg cgccttatcc ggtaactatc gtcttgagtc     8640
   caacccggta agacacgact tatcgccact ggcagcagcc actggtaaca ggattagcag     8700
   agcgaggtat gtaggcggtg ctacagagtt cttgaagtgg tggcctaact acggctacac     8760
   tagaaggaca gtatttggta tctgcgctct gctgaagcca gttaccttcg gaaaaagagt     8820
   tggtagctct tgatccggca aacaaaccac cgctggtagc ggtggttttt ttgtttgcaa     8880
   gcagcagatt acgcgcagaa aaaaaggatc tcaagaagat cctttgatct tttctacggg     8940
   gtctgacgct cagtggaacg aaaactcacg ttaagggatt ttggtcatga gattatcaaa     9000
   aaggatcttc acctagatcc ttttaaatta aaaatgaagt tttaaatcaa tctaaagtat     9060
   atatgagtaa acttggtctg acagttacca atgcttaatc agtgaggcac ctatctcagc     9120
   gatctgtcta tttcgttcat ccatagttgc ctgactcccc gtcgtgtaga taactacgat     9180
   acgggagggc ttaccatctg gccccagtgc tgcaatgata ccgcgagacc cacgctcacc     9240
   ggctccagat ttatcagcaa taaaccagcc agccggaagg gccgagcgca gaagtggtcc     9300
   tgcaacttta tccgcctcca tccagtctat taattgttgc cgggaagcta gagtaagtag     9360
   ttcgccagtt aatagtttgc gcaacgttgt tgccattgct acaggcatcg tggtgtcacg     9420
   ctcgtcgttt ggtatggctt cattcagctc cggttcccaa cgatcaaggc gagttacatg     9480
   atcccccatg ttgtgcaaaa aagcggttag ctccttcggt cctccgatcg ttgtcagaag     9540
   taagttggcc gcagtgttat cactcatggt tatggcagca ctgcataatt ctcttactgt     9600
   catgccatcc gtaagatgct tttctgtgac tggtgagtac tcaaccaagt cattctgaga     9660
   atagtgtatg cggcgaccga gttgctcttg cccggcgtca atacgggata ataccgcgcc     9720
   acatagcaga actttaaaag tgctcatcat tggaaaacgt tcttcggggc gaaaactctc     9780
   aaggatctta ccgctgttga gatccagttc gatgtaaccc actcgtgcac ccaactgatc     9840
   ttcagcatct tttactttca ccagcgtttc tgggtgagca aaaacaggaa ggcaaaatgc     9900
   cgcaaaaaag ggaataaggg cgacacggaa atgttgaata ctcatactct tcctttttca     9960
   atattattga agcatttatc agggttattg tctcatgagc ggatacatat ttgaatgtat    10020
   ttagaaaaat aaacaaatag gggttccgcg cacatttccc cgaaaagtgc cacctgacgc    10080
   gccctgtagc ggcgcattaa gcgcggcggg tgtggtggtt acgcgcagcg tgaccgctac    10140
   acttgccagc gccctagcgc ccgctccttt cgctttcttc ccttcctttc tcgccacgtt    10200
   cgccggcttt ccccgtcaag ctctaaatcg ggggctccct ttagggttcc gatttagtgc    10260
   tttacggcac ctcgacccca aaaaacttga ttagggtgat ggttcacgta gtgggccatc    10320
   gccctgatag acggtttttc gccctttgac gttggagtcc acgttcttta atagtggact    10380
   cttgttccaa actggaacaa cactcaaccc tatctcggtc tattcttttg atttataagg    10440
   gattttgccg atttcggcct attggttaaa aaatgagctg atttaacaaa aatttaacgc    10500
   gaattttaac aaaatattaa cgcttacaat ttccattcgc cattcaggct gcgcaactgt    10560
   tgggaagggc gatcggtgcg ggcctcttcg ctattacgcc agctggcgaa agggggatgt   10620
   gctgcaaggc gattaagttg ggtaacgcca gggttttccc agtcacgacg ttgtaaaacg    10680
   acggccagtg aattgtaata cgactcacta tagggcgaat tgggtaccgg gccccccctc    10740
   gaggtcgatg gtgtcgataa gcttgatatc gaattcatgt cacacaaacc gatcttcgcc    10800
   tcaaggaaac ctaattctac atccgagaga ctgccgagat ccagtctaca ctgattaatt    10860
   ttcgggccaa taatttaaaa aaatcgtgtt atataatatt atatgtatta tatatataca    10920
   tcatgatgat actgacagtc atgtcccatt gctaaataga cagactccat ctgccgcctc    10980
   caactgatgt tctcaatatt taaggggtca tctcgcattg tttaataata aacagactcc    11040
   atctaccgcc tccaaatgat gttctcaaaa tatattgtat gaacttattt ttattactta    11100
   gtattattag acaacttact tgctttatga aaaacacttc ctatttagga aacaatttat    11160
   aatggcagtt cgttcattta acaatttatg tagaataaat gttataaatg cgtatgggaa    11220
   atcttaaata tggatagcat aaatgatatc tgcattgcct aattcgaaat caacagcaac    11280
   gaaaaaaatc ccttgtacaa cataaatagt catcgagaaa tatcaactat caaagaacag    11340
   ctattcacac gttactattg agattattat tggacgagaa tcacacactc aactgtcttt    11400
   ctctcttcta gaaatacagg tacaagtatg tactattctc attgttcata cttctagtca    11460
   tttcatccca catattcctt ggatttctct ccaatgaatg acattctatc ttgcaaattc    11520
   aacaattata ataagatata ccaaagtagc ggtatagtgg caatcaaaaa gcttctctgg    11580
   tgtgcttctc gtatttattt ttattctaat gatccattaa aggtatatat ttatttcttg    11640
   ttatataatc cttttgttta ttacatgggc tggatacata aaggtatttt gatttaattt    11700
   tttgcttaaa ttcaatcccc cctcgttcag tgtcaactgt aatggtagga aattaccata    11760
   cttttgaaga agcaaaaaaa atgaaagaaa aaaaaaatcg tatttccagg ttagacgttc    11820
   cgcagaatct agaatgcggt atgcggtaca ttgttcttcg aacgtaaaag ttgcgctccc    11880
   tgagatattg tacatttttg cttttacaag tacaagtaca tcgtacaact atgtactact    11940
   gttgatgcat ccacaacagt ttgttttgtt tttttttgtt tttttttttt ctaatgattc    12000
   attaccgcta tgtataccta cttgtacttg tagtaagccg ggttattggc gttcaattaa    12060
   tcatagactt atgaatctgc acggtgtgcg ctgcgagtta cttttagctt atgcatgcta    12120
   cttgggtgta atattgggat ctgttcggaa atcaacggat gctcaat                   12167
<210> 55
   <211> 1963
   <212> DNA
   <213> Artificial sequence
<220>
   <223> Hygromycin resistance cassette
<400> 55
   gtttaaacct taagcccgct cataacttcg tatagcatac attatacgaa cggtaggttg      60
   cgggatagac gccgacggag ggcaatggcg ctatggaacc ttgcggatat ccatacgccg     120
   cggcggactg cgtccgaacc agctccagca gcgttttttc cgggccattg agccgactgc     180
   gaccccgcca acgtgtcttg gcccacgcac tcatgtcatg ttggtgttgg gaggccactt     240
   tttaagtagc acaaggcacc tagctcgcag caaggtgtcc gaaccaaaga agcggctgca     300
   gtggtgcaaa cggggcggaa acggcgggaa aaagccacgg gggcacgaat tgaggcacgc     360
   cctcgaattt gagacgagtc acggccccat tcgcccgcgc aatggctcgc caacgcccgg     420
   tcttttgcac cacatcaggt taccccaagc caaacctttg tgttaaaaag cttaacatat     480
   tataccgaac gtaggtttgg gcgggcttgc tccgtctgtc caaggcaaca tttatataag     540
   ggtctgcatc gccggctcaa ttgaatcttt tttcttcttc tcttctctat attcattctt     600
   gaattaaaca cacatcaacc atggccaaaa agcctgaact caccgcgacg tctgtcgaga     660
   agtttctgat cgaaaagttc gacagcgtct ccgacctgat gcagctctcg gagggcgaag     720
   aatctcgtgc tttcagcttc gatgtaggag ggcgtggata tgtcctgcgg gtaaatagct     780
   gcgccgatgg tttctacaaa gatcgttatg tttatcggca ctttgcatcg gccgcgctcc     840
   cgattccgga agtgcttgac attggggagt tcagcgagag cctgacctat tgcatctccc     900
   gccgtgcaca gggtgtcacg ttgcaagacc tgcctgaaac cgaactgccc gctgttctgc     960
   agccggtcgc ggaggctatg gatgcgatcg ctgcggccga tcttagccag acgagcgggt    1020
   tcggcccatt cggaccgcaa ggaatcggtc aatacactac atggcgtgat ttcatatgcg    1080
   cgattgctga tccccatgtg tatcactggc aaactgtgat ggacgacacc gtcagtgcgt    1140
   ccgtcgcgca ggctctcgat gagctgatgc tttgggccga ggactgcccc gaagtccggc    1200
   acctcgtgca cgcggatttc ggctccaaca atgtcctgac ggacaatggc cgcataacag    1260
   cggtcattga ctggagcgag gcgatgttcg gggattccca atacgaggtc gccaacatct    1320
   tcttctggag gccgtggttg gcttgtatgg agcagcagac gcgctacttc gagcggaggc    1380
   atccggagct tgcaggatcg ccgcggctcc gggcgtatat gctccgcatt ggtcttgacc    1440
   aactctatca gagcttggtt gacggcaatt tcgatgatgc agcttgggcg cagggtcgat    1500
   gcgacgcaat cgtccgatcc ggagccggga ctgtcgggcg tacacaaatc gcccgcagaa    1560
   gcgcggccgt ctggaccgat ggctgtgtag aagtactcgc cgatagtgga aaccgacgcc    1620
   ccagcactcg tccgagggca aaggaatagc ggccgcaagt gtggatgggg aagtgagtgc    1680
   ccggttctgt gtgcacaatt ggcaatccaa gatggatgga ttcaacacag ggatatagcg    1740
   agctacgtgg tggtgcgagg atatagcaac ggatatttat gtttgacact tgagaatgta    1800
   cgatacaagc actgtccaag tacaatacta aacatactgt acatactcat actcgtaccc    1860
   gggcaacggt ttcacttgag tgcagtggct agtgctctta ctcgtacagt gtgctaccgt    1920
   tcgtatagca tacattatac gaagttatca tagtcttaat taa                      1963
<210> 56
   <211> 1714
   <212> DNA
   <213> Yarrowia lipolytica
<400> 56
   catgacatgt gtccacagac cctcaatctg gcctgatgag ccaaatccat acgcgctttc      60
   gcagctctaa aggctataac aagtcacacc accctgctcg acctcagcgc cctcactttt     120
   tgttaagaca aactgtacac gctgttccag cgttttctgc ctgcacctgg tgggacattt     180
   ggtgcaacct aaagtgctcg gaacctctgt ggtgtccaga tcagcgcagc agttccgagg     240
   tagttttgag gcccttagat gatggtttaa acgtcgacga gtatctgtct gactcgtcat     300
   tgccgccttt ggagtacgac tccaactatg agtgtgcttg gatcactttg acgatacatt     360
   cttcgttgga ggctgtgggt ctgacagctg cgttttcggc gcggttggcc gacaacaata     420
   tcagctgcaa cgtcattgct ggctttcatc atgatcacat ttttgtcggc aaaggcgacg     480
   cccagagagc cattgacgtt ctttctaatt tggaccgata gccgtatagt ccagtctatc     540
   tataagttca actaactcgt aactattacc ataacatata cttcactgcc ccagataagg     600
   ttccgataaa aagttctgca gactaaattt atttcagtct cctcttcacc accaaaatgc     660
   cctcctacga agctcgagct aacgtccaca agtccgcctt tgccgctcga gtgctcaagc     720
   tcgtggcagc caagaaaacc aacctgtgtg cttctctgga tgttaccacc accaaggagc     780
   tcattgagct tgccgataag gtcggacctt atgtgtgcat gatcaaaacc catatcgaca     840
   tcattgacga cttcacctac gccggcactg tgctccccct caaggaactt gctcttaagc     900
   acggtttctt cctgttcgag gacagaaagt tcgcagatat tggcaacact gtcaagcacc     960
   agtaccggtg tcaccgaatc gccgagtggt ccgatatcac caacgcccac ggtgtacccg    1020
   gaaccggaat cattgctggc ctgcgagctg gtgccgagga aactgtctct gaacagaaga    1080
   aggaggacgt ctctgactac gagaactccc agtacaagga gttcctagtc cctctcccca    1140
   acgagaagct ggccagaggt ctgctcatgc tggccgagct gtcttgcaag ggctctctgg    1200
   ccactggcga gtactccaag cagaccattg agcttgcccg atccgacccc gagtttgtgg    1260
   ttggcttcat tgcccagaac cgacctaagg gcgactctga ggactggctt attctgaccc    1320
   ccggggtggg tcttgacgac aagggagacg ctctcggaca gcagtaccga actgttgagg    1380
   atgtcatgtc taccggaacg gatatcataa ttgtcggccg aggtctgtac ggccagaacc    1440
   gagatcctat tgaggaggcc aagcgatacc agaaggctgg ctgggaggct taccagaaga    1500
   ttaactgtta gaggttagac tatggatatg taatttaact gtgtatatag agagcgtgca    1560
   agtatggagc gcttgttcag cttgtatgat ggtcagacga cctgtctgat cgagtatgta    1620
   tgatactgca caacctgtgt atccgcatga tctgtccaat ggggcatgtt gttgtgtttc    1680
   tcgatacgga gatgctgggt acagtgctaa tacg                                1714
<210> 57
   <211> 23
   <212> DNA
   <213> Yarrowia lipolytica
<220>
   <221> misc_feature
   <222> (1)..(23)
   <223> ura3-1 target site
<400> 57
   cgctcgagtg ctcaagctcg tgg 23
<210> 58
   <211> 34
   <212> DNA
   <213> Artificial sequence
<220>
   <223> ura3-1F
<400> 58
   aatgggacgc cgctcgagtg ctcaagctcg gttt 34
<210> 59
   <211> 34
   <212> DNA
   <213> Artificial sequence
<220>
   <223> ura3-1R
<400> 59
   tctaaaaccg agcttgagca ctcgagcggc gtcc 34
<210> 60
   <211> 20
   <212> DNA
   <213> Artificial sequence
<220>
   <223> DNA encoding Ura3-1 VT domain
<400> 60
   cgctcgagtg ctcaagctcg 20
<210> 61
   <211> 11631
   <212> DNA
   <213> Artificial sequence
<220>
   <223> pRF421
<400> 61
   taagactatg ataacttcgt ataatgtatg ctatacgaac ggtagcacac tgtacgagta      60
   agagcactag ccactgcact caagtgaaac cgttgcccgg gtacgagtat gagtatgtac     120
   agtatgttta gtattgtact tggacagtgc ttgtatcgta cattctcaag tgtcaaacat     180
   aaatatccgt tgctatatcc tcgcaccacc acgtagctcg ctatatccct gtgttgaatc     240
   catccatctt ggattgccaa ttgtgcacac agaaccgggc actcacttcc ccatccacac     300
   ttgcggccgc tattcctttg ccctcggacg agtgctgggg cgtcggtttc cactatcggc     360
   gagtacttct acacagccat cggtccagac ggccgcgctt ctgcgggcga tttgtgtacg     420
   cccgacagtc ccggctccgg atcggacgat tgcgtcgcat cgaccctgcg cccaagctgc     480
   atcatcgaaa ttgccgtcaa ccaagctctg atagagttgg tcaagaccaa tgcggagcat     540
   atacgcccgg agccgcggcg atcctgcaag ctccggatgc ctccgctcga agtagcgcgt     600
   ctgctgctcc atacaagcca accacggcct ccagaagaag atgttggcga cctcgtattg     660
   ggaatccccg aacatcgcct cgctccagtc aatgaccgct gttatgcggc cattgtccgt     720
   caggacattg ttggagccga aatccgcgtg cacgaggtgc cggacttcgg ggcagtcctc     780
   ggcccaaagc atcagctcat cgagagcctg cgcgacggac gcactgacgg tgtcgtccat     840
   cacagtttgc cagtgataca catggggatc agcaatcgcg catatgaaat cacgccatgt     900
   agtgtattga ccgattcctt gcggtccgaa tgggccgaac ccgctcgtct ggctaagatc     960
   ggccgcagcg atcgcatcca tagcctccgc gaccggctgc agaacagcgg gcagttcggt    1020
   ttcaggcagg tcttgcaacg tgacaccctg tgcacggcgg gagatgcaat aggtcaggct    1080
   ctcgctgaac tccccaatgt caagcacttc cggaatcggg agcgcggccg atgcaaagtg    1140
   ccgataaaca taacgatctt tgtagaaacc atcggcgcag ctatttaccc gcaggacata    1200
   tccacgccct cctacatcga agctgaaagc acgagattct tcgccctccg agagctgcat    1260
   caggtcggag acgctgtcga acttttcgat cagaaacttc tcgacagacg tcgcggtgag    1320
   ttcaggcttt ttggccatgg ttgatgtgtg tttaattcaa gaatgaatat agagaagaga    1380
   agaagaaaaa agattcaatt gagccggcga tgcagaccct tatataaatg ttgccttgga    1440
   cagacggagc aagcccgccc aaacctacgt tcggtataat atgttaagct ttttaacaca     1500
   aaggtttggc ttggggtaac ctgatgtggt gcaaaagacc gggcgttggc gagccattgc    1560
   gcgggcgaat ggggccgtga ctcgtctcaa attcgagggc gtgcctcaat tcgtgccccc    1620
   gtggcttttt cccgccgttt ccgccccgtt tgcaccactg cagccgcttc tttggttcgg    1680
   acaccttgct gcgagctagg tgccttgtgc tacttaaaaa gtggcctccc aacaccaaca    1740
   tgacatgagt gcgtgggcca agacacgttg gcggggtcgc agtcggctca atggcccgga    1800
   aaaaacgctg ctggagctgg ttcggacgca gtccgccgcg gcgtatggat atccgcaagg    1860
   ttccatagcg ccattgccct ccgtcggcgt ctatcccgca acctaccgtt cgtataatgt    1920
   atgctatacg aagttatgag cgggcttaag gtttaaacca tcatctaagg gcctcaaaac    1980
   tacctcggaa ctgctgcgct gatctggaca ccacagaggt tccgagcact ttaggttgca    2040
   ccaaatgtcc caccaggtgc aggcagaaaa cgctggaaca gcgtgtacag tttgtcttaa    2100
   caaaaagtga gggcgctgag gtcgagcagg gtggtgtgac ttgttatagc ctttagagct    2160
   gcgaaagcgc gtatggattt ggctcatcag gccagattga gggtctgtgg acacatgtca    2220
   tgttagtgta cttcaatcgc cccctggata tagccccgac aataggccgt ggcctcattt    2280
   ttttgccttc cgcacatttc cattgctcgg tacccacacc ttgcttctcc tgcacttgcc    2340
   aaccttaata ctggtttaca ttgaccaaca tcttacaagc ggggggcttg tctagggtat    2400
   atataaacag tggctctccc aatcggttgc cagtctcttt tttcctttct ttccccacag    2460
   attcgaaatc taaactacac atcacaccat ggacaagaaa tactccatcg gcctggacat    2520
   tggaaccaac tctgtcggct gggctgtcat caccgacgag tacaaggtgc cctccaagaa    2580
   attcaaggtc ctcggaaaca ccgatcgaca ctccatcaag aaaaacctca ttggtgccct    2640
   gttgttcgat tctggcgaga ctgccgaagc taccagactc aagcgaactg ctcggcgacg    2700
   ttacacccga cggaagaacc gaatctgcta cctgcaggag atcttttcca acgagatggc    2760
   caaggtggac gattcgttct ttcatcgact ggaggaatcc ttcctcgtcg aggaagacaa    2820
   gaaacacgag cgtcatccca tctttggcaa cattgtggac gaggttgctt accacgagaa    2880
   gtatcctacc atctaccatc tccgaaagaa actcgtcgat tccaccgaca aggcggatct    2940
   cagacttatc tacctcgctc tggcacacat gatcaagttt cgaggtcatt tcctcatcga    3000
   gggcgatctc aatcccgaca acagcgatgt ggacaagctg ttcattcagc tcgttcagac    3060
   ctacaaccag ctgttcgagg aaaaccccat caatgcctcc ggagtcgatg caaaggccat    3120
   cttgtctgct cgactctcga agagcagacg actggagaac ctcattgccc aacttcctgg    3180
   cgagaaaaag aacggactgt ttggcaacct cattgccctt tctcttggtc tcacacccaa    3240
   cttcaagtcc aacttcgatc tggcggagga cgccaagctc cagctgtcca aggacaccta    3300
   cgacgatgac ctcgacaacc tgcttgcaca gattggcgat cagtacgccg acctgtttct    3360
   cgctgccaag aacctttcgg atgctattct cttgtctgac attctgcgag tcaacaccga     3420
   gatcacaaag gctccccttt ctgcctccat gatcaagcga tacgacgagc accatcagga     3480
   tctcacactg ctcaaggctc ttgtccgaca gcaactgccc gagaagtaca aggagatctt     3540
   tttcgatcag tcgaagaacg gctacgctgg atacatcgac ggcggagcct ctcaggaaga     3600
   gttctacaag ttcatcaagc caattctcga gaagatggac ggaaccgagg aactgcttgt     3660
   caagctcaat cgagaggatc tgcttcggaa gcaacgaacc ttcgacaacg gcagcattcc     3720
   tcatcagatc cacctcggtg agctgcacgc cattcttcga cgtcaggaag acttctaccc     3780
   ctttctcaag gacaaccgag agaagatcga gaagattctt acctttcgaa tcccctacta     3840
   tgttggtcct cttgccagag gaaactctcg atttgcttgg atgactcgaa agtccgagga     3900
   aaccatcact ccctggaact tcgaggaagt cgtggacaag ggtgcctctg cacagtcctt     3960
   catcgagcga atgaccaact tcgacaagaa tctgcccaac gagaaggttc ttcccaagca     4020
   ttcgctgctc tacgagtact ttacagtcta caacgaactc accaaagtca agtacgttac     4080
   cgagggaatg cgaaagcctg ccttcttgtc tggcgaacag aagaaagcca ttgtcgatct     4140
   cctgttcaag accaaccgaa aggtcactgt taagcagctc aaggaggact acttcaagaa     4200
   aatcgagtgt ttcgacagcg tcgagatttc cggagttgag gaccgattca acgcctcttt     4260
   gggcacctat cacgatctgc tcaagattat caaggacaag gattttctcg acaacgagga     4320
   aaacgaggac attctggagg acatcgtgct cactcttacc ctgttcgaag atcgggagat     4380
   gatcgaggaa cgactcaaga catacgctca cctgttcgac gacaaggtca tgaaacaact     4440
   caagcgacgt agatacaccg gctggggaag actttcgcga aagctcatca acggcatcag     4500
   agacaagcag tccggaaaga ccattctgga ctttctcaag tccgatggct ttgccaaccg     4560
   aaacttcatg cagctcattc acgacgattc tcttaccttc aaggaggaca tccagaaggc     4620
   acaagtgtcc ggtcagggcg acagcttgca cgaacatatt gccaacctgg ctggttcgcc     4680
   agccatcaag aaaggcattc tccagactgt caaggttgtc gacgagctgg tgaaggtcat     4740
   gggacgtcac aagcccgaga acattgtgat cgagatggcc agagagaacc agacaactca     4800
   aaagggtcag aaaaactcgc gagagcggat gaagcgaatc gaggaaggca tcaaggagct     4860
   gggatcccag attctcaagg agcatcccgt cgagaacact caactgcaga acgagaagct     4920
   gtatctctac tatctgcaga atggtcgaga catgtacgtg gatcaggaac tggacatcaa     4980
   tcgtctcagc gactacgatg tggaccacat tgtccctcaa tcctttctca aggacgattc     5040
   tatcgacaac aaggtcctta cacgatccga caagaacaga ggcaagtcgg acaacgttcc     5100
   cagcgaagag gtggtcaaaa agatgaagaa ctactggcga cagctgctca acgccaagct     5160
   cattacccag cgaaagttcg acaatcttac caaggccgag cgaggcggtc tgtccgagct     5220
   cgacaaggct ggcttcatca agcgtcaact cgtcgagacc agacagatca caaagcacgt     5280
   cgcacagatt ctcgattctc ggatgaacac caagtacgac gagaacgaca agctcatccg     5340
   agaggtcaag gtgattactc tcaagtccaa actggtctcc gatttccgaa aggactttca     5400
   gttctacaag gtgcgagaga tcaacaatta ccaccatgcc cacgatgctt acctcaacgc     5460
   cgtcgttggc actgcgctca tcaagaaata ccccaagctc gaaagcgagt tcgtttacgg     5520
   cgattacaag gtctacgacg ttcgaaagat gattgccaag tccgaacagg agattggcaa     5580
   ggctactgcc aagtacttct tttactccaa catcatgaac tttttcaaga ccgagatcac     5640
   cttggccaac ggagagattc gaaagagacc acttatcgag accaacggcg aaactggaga     5700
   gatcgtgtgg gacaagggtc gagactttgc aaccgtgcga aaggttctgt cgatgcctca     5760
   ggtcaacatc gtcaagaaaa ccgaggttca gactggcgga ttctccaagg agtcgattct     5820
   gcccaagcga aactccgaca agctcatcgc tcgaaagaaa gactgggatc ccaagaaata     5880
   cggtggcttc gattctccta ccgtcgccta ttccgtgctt gtcgttgcga aggtcgagaa     5940
   gggcaagtcc aaaaagctca agtccgtcaa ggagctgctc ggaattacca tcatggagcg     6000
   atcgagcttc gagaagaatc ccatcgactt cttggaagcc aagggttaca aggaggtcaa     6060
   gaaagacctc attatcaagc tgcccaagta ctctctgttc gaactggaga acggtcgaaa     6120
   gcgtatgctc gcctccgctg gcgagctgca gaagggaaac gagcttgcct tgccttcgaa     6180
   gtacgtcaac tttctctatc tggcttctca ctacgagaag ctcaagggtt ctcccgagga     6240
   caacgaacag aagcaactct tcgttgagca gcacaaacat tacctcgacg agattatcga     6300
   gcagatttcc gagttttcga agcgagtcat cctggctgat gccaacttgg acaaggtgct     6360
   ctctgcctac aacaagcatc gggacaaacc cattcgagaa caggcggaga acatcattca     6420
   cctgtttact cttaccaacc tgggtgctcc tgcagctttc aagtacttcg ataccactat     6480
   cgaccgaaag cggtacacat ccaccaagga ggttctcgat gccaccctga ttcaccagtc     6540
   catcactggc ctgtacgaga cccgaatcga cctgtctcag cttggtggcg actccagagc     6600
   cgatcccaag aaaaagcgaa aggtctaagc ggccgcaagt gtggatgggg aagtgagtgc     6660
   ccggttctgt gtgcacaatt ggcaatccaa gatggatgga ttcaacacag ggatatagcg     6720
   agctacgtgg tggtgcgagg atatagcaac ggatatttat gtttgacact tgagaatgta     6780
   cgatacaagc actgtccaag tacaatacta aacatactgt acatactcat actcgtaccc     6840
   gggcaacggt ttcacttgag tgcagtggct agtgctctta ctcgtacagt gtgcaatact     6900
   gcgtatcata gtctttgatg tatatcgtat tcattcatgt tagttgcgta cgagccggaa     6960
   gcataaagtg taaagcctgg ggtgcctaat gagtgagcta actcacatta attgcgttgc     7020
   gctcactgcc cgctttccag tcgggaaacc tgtcgtgcca gctgcattaa tgaatcggcc     7080
   aacgcgcggg gagaggcggt ttgcgtattg ggcgctcttc cgcttcctcg ctcactgact     7140
   cgctgcgctc ggtcgttcgg ctgcggcgag cggtatcagc tcactcaaag gcggtaatac     7200
   ggttatccac agaatcaggg gataacgcag gaaagaacat gtgagcaaaa ggccagcaaa     7260
   aggccaggaa ccgtaaaaag gccgcgttgc tggcgttttt ccataggctc cgcccccctg     7320
   acgagcatca caaaaatcga cgctcaagtc agaggtggcg aaacccgaca ggactataaa     7380
   gataccaggc gtttccccct ggaagctccc tcgtgcgctc tcctgttccg accctgccgc     7440
   ttaccggata cctgtccgcc tttctccctt cgggaagcgt ggcgctttct catagctcac     7500
   gctgtaggta tctcagttcg gtgtaggtcg ttcgctccaa gctgggctgt gtgcacgaac     7560
   cccccgttca gcccgaccgc tgcgccttat ccggtaacta tcgtcttgag tccaacccgg     7620
   taagacacga cttatcgcca ctggcagcag ccactggtaa caggattagc agagcgaggt     7680
   atgtaggcgg tgctacagag ttcttgaagt ggtggcctaa ctacggctac actagaagga     7740
   cagtatttgg tatctgcgct ctgctgaagc cagttacctt cggaaaaaga gttggtagct     7800
   cttgatccgg caaacaaacc accgctggta gcggtggttt ttttgtttgc aagcagcaga     7860
   ttacgcgcag aaaaaaagga tctcaagaag atcctttgat cttttctacg gggtctgacg     7920
   ctcagtggaa cgaaaactca cgttaaggga ttttggtcat gagattatca aaaaggatct     7980
   tcacctagat ccttttaaat taaaaatgaa gttttaaatc aatctaaagt atatatgagt     8040
   aaacttggtc tgacagttac caatgcttaa tcagtgaggc acctatctca gcgatctgtc     8100
   tatttcgttc atccatagtt gcctgactcc ccgtcgtgta gataactacg atacgggagg     8160
   gcttaccatc tggccccagt gctgcaatga taccgcgaga cccacgctca ccggctccag     8220
   atttatcagc aataaaccag ccagccggaa gggccgagcg cagaagtggt cctgcaactt     8280
   tatccgcctc catccagtct attaattgtt gccgggaagc tagagtaagt agttcgccag     8340
   ttaatagttt gcgcaacgtt gttgccattg ctacaggcat cgtggtgtca cgctcgtcgt     8400
   ttggtatggc ttcattcagc tccggttccc aacgatcaag gcgagttaca tgatccccca     8460
   tgttgtgcaa aaaagcggtt agctccttcg gtcctccgat cgttgtcaga agtaagttgg     8520
   ccgcagtgtt atcactcatg gttatggcag cactgcataa ttctcttact gtcatgccat     8580
   ccgtaagatg cttttctgtg actggtgagt actcaaccaa gtcattctga gaatagtgta     8640
   tgcggcgacc gagttgctct tgcccggcgt caatacggga taataccgcg ccacatagca     8700
   gaactttaaa agtgctcatc attggaaaac gttcttcggg gcgaaaactc tcaaggatct     8760
   taccgctgtt gagatccagt tcgatgtaac ccactcgtgc acccaactga tcttcagcat     8820
   cttttacttt caccagcgtt tctgggtgag caaaaacagg aaggcaaaat gccgcaaaaa     8880
   agggaataag ggcgacacgg aaatgttgaa tactcatact cttccttttt caatattatt     8940
   gaagcattta tcagggttat tgtctcatga gcggatacat atttgaatgt atttagaaaa     9000
   ataaacaaat aggggttccg cgcacatttc cccgaaaagt gccacctgac gcgccctgta     9060
   gcggcgcatt aagcgcggcg ggtgtggtgg ttacgcgcag cgtgaccgct acacttgcca     9120
   gcgccctagc gcccgctcct ttcgctttct tcccttcctt tctcgccacg ttcgccggct     9180
   ttccccgtca agctctaaat cgggggctcc ctttagggtt ccgatttagt gctttacggc     9240
   acctcgaccc caaaaaactt gattagggtg atggttcacg tagtgggcca tcgccctgat     9300
   agacggtttt tcgccctttg acgttggagt ccacgttctt taatagtgga ctcttgttcc     9360
   aaactggaac aacactcaac cctatctcgg tctattcttt tgatttataa gggattttgc     9420
   cgatttcggc ctattggtta aaaaatgagc tgatttaaca aaaatttaac gcgaatttta     9480
   acaaaatatt aacgcttaca atttccattc gccattcagg ctgcgcaact gttgggaagg     9540
   gcgatcggtg cgggcctctt cgctattacg ccagctggcg aaagggggat gtgctgcaag     9600
   gcgattaagt tgggtaacgc cagggttttc ccagtcacga cgttgtaaaa cgacggccag     9660
   tgaattgtaa tacgactcac tatagggcga attgggtacc gggccccccc tcgaggtcga     9720
   tggtgtcgat aagcttgata tcgaattcat gtcacacaaa ccgatcttcg cctcaaggaa     9780
   acctaattct acatccgaga gactgccgag atccagtcta cactgattaa ttttcgggcc     9840
   aataatttaa aaaaatcgtg ttatataata ttatatgtat tatatatata catcatgatg     9900
   atactgacag tcatgtccca ttgctaaata gacagactcc atctgccgcc tccaactgat     9960
   gttctcaata tttaaggggt catctcgcat tgtttaataa taaacagact ccatctaccg    10020
   cctccaaatg atgttctcaa aatatattgt atgaacttat ttttattact tagtattatt    10080
   agacaactta cttgctttat gaaaaacact tcctatttag gaaacaattt ataatggcag    10140
   ttcgttcatt taacaattta tgtagaataa atgttataaa tgcgtatggg aaatcttaaa    10200
   tatggatagc ataaatgata tctgcattgc ctaattcgaa atcaacagca acgaaaaaaa    10260
   tcccttgtac aacataaata gtcatcgaga aatatcaact atcaaagaac agctattcac    10320
   acgttactat tgagattatt attggacgag aatcacacac tcaactgtct ttctctcttc    10380
   tagaaataca ggtacaagta tgtactattc tcattgttca tacttctagt catttcatcc    10440
   cacatattcc ttggatttct ctccaatgaa tgacattcta tcttgcaaat tcaacaatta    10500
   taataagata taccaaagta gcggtatagt ggcaatcaaa aagcttctct ggtgtgcttc    10560
   tcgtatttat ttttattcta atgatccatt aaaggtatat atttatttct tgttatataa    10620
   tccttttgtt tattacatgg gctggataca taaaggtatt ttgatttaat tttttgctta    10680
   aattcaatcc cccctcgttc agtgtcaact gtaatggtag gaaattacca tacttttgaa    10740
   gaagcaaaaa aaatgaaaga aaaaaaaaat cgtatttcca ggttagacgt tccgcagaat    10800
   ctagaatgcg gtatgcggta cattgttctt cgaacgtaaa agttgcgctc cctgagatat    10860
   tgtacatttt tgcttttaca agtacaagta catcgtacaa ctatgtacta ctgttgatgc    10920
   atccacaaca gtttgttttg tttttttttg tttttttttt ttctaatgat tcattaccgc 10980
   tatgtatacc tacttgtact tgtagtaagc cgggttattg gcgttcaatt aatcatagac 11040
   ttatgaatct gcacggtgtg cgctgcgagt tacttttagc ttatgcatgc tacttgggtg 11100
   taatattggg atctgttcgg aaatcaacgg atgctcaatc gataaaaaac aaaaaaaaaa 11160
   gcaccgactc ggtgccactt tttcaagttg ataacggact agccttattt taacttgcta 11220
   tttctagctc taaaaccgag cttgagcact cgagcggcgt cccattcgcc atgccgaagc 11280
   atgttgccca gccggcgcca gcgaggaggc tgggaccatg ccggccatta ttttgcgtta 11340
   agtttctaat catcacgaaa ttatctatca aaaataacta ggtcccaccg agattcgaac 11400
   tcgggacctt aagatttgca atctcacgcg ctaccgctgt gccataggac cgaagttaaa 11460
   atttggccaa agaaggacct gggcaccctg gactgtgggt tagggtaata ttccttatgg 11520
   agacaatggg ctagggtaaa ttacctaaaa tgggtcgata aagaggggtg ttcccagttg 11580
   ggaagtgtaa ttgaagacgg ggtcaaaaaa gaaaatcaaa aaaaatttaa t 11631
<210> 62
   <211> 378
   <212> DNA
   <213> Yarrowia lipolytica
<400> 62
   gagtatctgt ctgactcgtc attgccgcct ttggagtacg actccaacta tgagtgtgct 60
   tggatcactt tgacgataca ttcttcgttg gaggctgtgg gtctgacagc tgcgttttcg 120
   gcgcggttgg ccgacaacaa tatcagctgc aacgtcattg ctggctttca tcatgatcac 180
   atttttgtcg gcaaaggcga cgcccagaga gccattgacg ttctttctaa tttggaccga 240
   tagccgtata gtccagtcta tctataagtt caactaactc gtaactatta ccataacata 300
   tacttcactg ccccagataa ggttccgata aaaagttctg cagactaaat ttatttcagt 360
   ctcctcttca ccaccaaa 378
<210> 63
   <211> 258
   <212> DNA
   <213> Yarrowia lipolytica
<400> 63
   tagaggttag actatggata tgtaatttaa ctgtgtatat agagagcgtg caagtatgga 60
   gcgcttgttc agcttgtatg atggtcagac gacctgtctg atcgagtatg tatgatactg 120
   cacaacctgt gtatccgcat gatctgtcca atggggcatg ttgttgtgtt tctcgatacg 180
   gagatgctgg gtacagtgct aatacgttga actacttata cttatatgag gctcgaagaa 240
   agctgacttg tgtatgac 258
<210> 64
   <211> 648
   <212> DNA
   <213> Artificial sequence
<220>
   <223> URA3 deletion polynucleotide editing template
<400> 64
   gaattcgagt atctgtctga ctcgtcattg ccgcctttgg agtacgactc caactatgag       60
   tgtgcttgga tcactttgac gatacattct tcgttggagg ctgtgggtct gacagctgcg      120
   ttttcggcgc ggttggccga caacaatatc agctgcaacg tcattgctgg ctttcatcat      180
   gatcacattt ttgtcggcaa aggcgacgcc cagagagcca ttgacgttct ttctaatttg      240
   gaccgatagc cgtatagtcc agtctatcta taagttcaac taactcgtaa ctattaccat      300
   aacatatact tcactgcccc agataaggtt ccgataaaaa gttctgcaga ctaaatttat      360
   ttcagtctcc tcttcaccac caaatagagg ttagactatg gatatgtaat ttaactgtgt      420
   atatagagag cgtgcaagta tggagcgctt gttcagcttg tatgatggtc agacgacctg      480
   tctgatcgag tatgtatgat actgcacaac ctgtgtatcc gcatgatctg tccaatgggg      540
   catgttgttg tgtttctcga tacggagatg ctgggtacag tgctaatacg ttgaactact      600
   tatacttata tgaggctcga agaaagctga cttgtgtatg acaagctt                   648
<210> 65
   <211> 3277
   <212> DNA
   <213> Artificial sequence
<220>
   <223> pRF263
<400> 65
   agcttgtcat acacaagtca gctttcttcg agcctcatat aagtataagt agttcaacgt      60
   attagcactg tacccagcat ctccgtatcg agaaacacaa caacatgccc cattggacag     120
   atcatgcgga tacacaggtt gtgcagtatc atacatactc gatcagacag gtcgtctgac     180
   catcatacaa gctgaacaag cgctccatac ttgcacgctc tctatataca cagttaaatt     240
   acatatccat agtctaacct ctatttggtg gtgaagagga gactgaaata aatttagtct     300
   gcagaacttt ttatcggaac cttatctggg gcagtgaagt atatgttatg gtaatagtta     360
   cgagttagtt gaacttatag atagactgga ctatacggct atcggtccaa attagaaaga     420
   acgtcaatgg ctctctgggc gtcgcctttg ccgacaaaaa tgtgatcatg atgaaagcca     480
   gcaatgacgt tgcagctgat attgttgtcg gccaaccgcg ccgaaaacgc agctgtcaga     540
   cccacagcct ccaacgaaga atgtatcgtc aaagtgatcc aagcacactc atagttggag     600
   tcgtactcca aaggcggcaa tgacgagtca gacagatact cgaattcgta atcatggtca     660
   tagctgtttc ctgtgtgaaa ttgttatccg ctcacaattc cacacaacat acgagccgga     720
   agcataaagt gtaaagcctg gggtgcctaa tgagtgagct aactcacatt aattgcgttg     780
   cgctcactgc ccgctttcca gtcgggaaac ctgtcgtgcc agctgcatta atgaatcggc     840
   caacgcgcgg ggagaggcgg tttgcgtatt gggcgctctt ccgcttcctc gctcactgac     900
   tcgctgcgct cggtcgttcg gctgcggcga gcggtatcag ctcactcaaa ggcggtaata     960
   cggttatcca cagaatcagg ggataacgca ggaaagaaca tgtgagcaaa aggccagcaa    1020
   aaggccagga accgtaaaaa ggccgcgttg ctggcgtttt tccataggct ccgcccccct    1080
   gacgagcatc acaaaaatcg acgctcaagt cagaggtggc gaaacccgac aggactataa    1140
   agataccagg cgtttccccc tggaagctcc ctcgtgcgct ctcctgttcc gaccctgccg    1200
   cttaccggat acctgtccgc ctttctccct tcgggaagcg tggcgctttc tcatagctca    1260
   cgctgtaggt atctcagttc ggtgtaggtc gttcgctcca agctgggctg tgtgcacgaa    1320
   ccccccgttc agcccgaccg ctgcgcctta tccggtaact atcgtcttga gtccaacccg    1380
   gtaagacacg acttatcgcc actggcagca gccactggta acaggattag cagagcgagg    1440
   tatgtaggcg gtgctacaga gttcttgaag tggtggccta actacggcta cactagaagg    1500
   acagtatttg gtatctgcgc tctgctgaag ccagttacct tcggaaaaag agttggtagc    1560
   tcttgatccg gcaaacaaac caccgctggt agcggtggtt tttttgtttg caagcagcag    1620
   attacgcgca gaaaaaaagg atctcaagaa gatcctttga tcttttctac ggggtctgac    1680
   gctcagtgga acgaaaactc acgttaaggg attttggtca tgagattatc aaaaaggatc    1740
   ttcacctaga tccttttaaa ttaaaaatga agttttaaat caatctaaag tatatatgag    1800
   taaacttggt ctgacagtta ccaatgctta atcagtgagg cacctatctc agcgatctgt    1860
   ctatttcgtt catccatagt tgcctgactc cccgtcgtgt agataactac gatacgggag    1920
   ggcttaccat ctggccccag tgctgcaatg ataccgcgag acccacgctc accggctcca    1980
   gatttatcag caataaacca gccagccgga agggccgagc gcagaagtgg tcctgcaact    2040
   ttatccgcct ccatccagtc tattaattgt tgccgggaag ctagagtaag tagttcgcca    2100
   gttaatagtt tgcgcaacgt tgttgccatt gctacaggca tcgtggtgtc acgctcgtcg    2160
   tttggtatgg cttcattcag ctccggttcc caacgatcaa ggcgagttac atgatccccc    2220
   atgttgtgca aaaaagcggt tagctccttc ggtcctccga tcgttgtcag aagtaagttg    2280
   gccgcagtgt tatcactcat ggttatggca gcactgcata attctcttac tgtcatgcca    2340
   tccgtaagat gcttttctgt gactggtgag tactcaacca agtcattctg agaatagtgt    2400
   atgcggcgac cgagttgctc ttgcccggcg tcaatacggg ataataccgc gccacatagc    2460
   agaactttaa aagtgctcat cattggaaaa cgttcttcgg ggcgaaaact ctcaaggatc    2520
   ttaccgctgt tgagatccag ttcgatgtaa cccactcgtg cacccaactg atcttcagca    2580
   tcttttactt tcaccagcgt ttctgggtga gcaaaaacag gaaggcaaaa tgccgcaaaa    2640
   aagggaataa gggcgacacg gaaatgttga atactcatac tcttcctttt tcaatattat    2700
   tgaagcattt atcagggtta ttgtctcatg agcggataca tatttgaatg tatttagaaa    2760
   aataaacaaa taggggttcc gcgcacattt ccccgaaaag tgccacctga cgtctaagaa    2820
   accattatta tcatgacatt aacctataaa aataggcgta tcacgaggcc ctttcgtctc    2880
   gcgcgtttcg gtgatgacgg tgaaaacctc tgacacatgc agctcccgga gacggtcaca    2940
   gcttgtctgt aagcggatgc cgggagcaga caagcccgtc agggcgcgtc agcgggtgtt    3000
   ggcgggtgtc ggggctggct taactatgcg gcatcagagc agattgtact gagagtgcac    3060
   catatgcggt gtgaaatacc gcacagatgc gtaaggagaa aataccgcat caggcgccat    3120
   tcgccattca ggctgcgcaa ctgttgggaa gggcgatcgg tgcgggcctc ttcgctatta    3180
   cgccagctgg cgaaaggggg atgtgctgca aggcgattaa gttgggtaac gccagggttt    3240
   tcccagtcac gacgttgtaa aacgacggcc agtgcca                             3277
<210> 66
   <211> 26
   <212> DNA
   <213> Artificial sequence
<220>
   <223> HY007
<400> 66
   cgggcagtga gcgcaacgca attaat 26
<210> 67
   <211> 29
   <212> DNA
   <213> Artificial sequence
<220>
   <223> oligo 297
<400> 67
   ggggaattct gcaaggcgat taagttggg 29
<210> 68
   <211> 873
   <212> DNA
   <213> Artificial sequence
<220>
   <223> EcoRI flanked URA3 deletion template
<400> 68
   cgggcagtga gcgcaacgca attaatgtga gttagctcac tcattaggca ccccaggctt      60
   tacactttat gcttccggct cgtatgttgt gtggaattgt gagcggataa caatttcaca     120
   caggaaacag ctatgaccat gattacgaat tcgagtatct gtctgactcg tcattgccgc     180
   ctttggagta cgactccaac tatgagtgtg cttggatcac tttgacgata cattcttcgt     240
   tggaggctgt gggtctgaca gctgcgtttt cggcgcggtt ggccgacaac aatatcagct     300
   gcaacgtcat tgctggcttt catcatgatc acatttttgt cggcaaaggc gacgcccaga     360
   gagccattga cgttctttct aatttggacc gatagccgta tagtccagtc tatctataag     420
   ttcaactaac tcgtaactat taccataaca tatacttcac tgccccagat aaggttccga     480
   taaaaagttc tgcagactaa atttatttca gtctcctctt caccaccaaa tagaggttag     540
   actatggata tgtaatttaa ctgtgtatat agagagcgtg caagtatgga gcgcttgttc     600
   agcttgtatg atggtcagac gacctgtctg atcgagtatg tatgatactg cacaacctgt     660
   gtatccgcat gatctgtcca atggggcatg ttgttgtgtt tctcgatacg gagatgctgg     720
   gtacagtgct aatacgttga actacttata cttatatgag gctcgaagaa agctgacttg     780
   tgtatgacaa gcttggcact ggccgtcgtt ttacaacgtc gtgactggga aaaccctggc     840
   gttacccaac ttaatcgcct tgcagaattc ccc                                  873
<210> 69
   <211> 12349
   <212> **DNA**
   <213> Artificial sequence
<220>
   <223> pRF437
<400> 69
   aattctgcaa ggcgattaag ttgggtaacg ccagggtttt cccagtcacg acgttgtaaa      60
   acgacggcca gtgccaagct tgtcatacac aagtcagctt tcttcgagcc tcatataagt     120
   ataagtagtt caacgtatta gcactgtacc cagcatctcc gtatcgagaa acacaacaac     180
   atgccccatt ggacagatca tgcggataca caggttgtgc agtatcatac atactcgatc     240
   agacaggtcg tctgaccatc atacaagctg aacaagcgct ccatacttgc acgctctcta     300
   tatacacagt taaattacat atccatagtc taacctctat ttggtggtga agaggagact     360
   gaaataaatt tagtctgcag aactttttat cggaacctta tctggggcag tgaagtatat     420
   gttatggtaa tagttacgag ttagttgaac ttatagatag actggactat acggctatcg     480
   gtccaaatta gaaagaacgt caatggctct ctgggcgtcg cctttgccga caaaaatgtg      540
   atcatgatga aagccagcaa tgacgttgca gctgatattg ttgtcggcca accgcgccga      600
   aaacgcagct gtcagaccca cagcctccaa cgaagaatgt atcgtcaaag tgatccaagc      660
   acactcatag ttggagtcgt actccaaagg cggcaatgac gagtcagaca gatactcgaa      720
   ttcatgtcac acaaaccgat cttcgcctca aggaaaccta attctacatc cgagagactg      780
   ccgagatcca gtctacactg attaattttc gggccaataa tttaaaaaaa tcgtgttata      840
   taatattata tgtattatat atatacatca tgatgatact gacagtcatg tcccattgct      900
   aaatagacag actccatctg ccgcctccaa ctgatgttct caatatttaa ggggtcatct      960
   cgcattgttt aataataaac agactccatc taccgcctcc aaatgatgtt ctcaaaatat     1020
   attgtatgaa cttattttta ttacttagta ttattagaca acttacttgc tttatgaaaa     1080
   acacttccta tttaggaaac aatttataat ggcagttcgt tcatttaaca atttatgtag     1140
   aataaatgtt ataaatgcgt atgggaaatc ttaaatatgg atagcataaa tgatatctgc     1200
   attgcctaat tcgaaatcaa cagcaacgaa aaaaatccct tgtacaacat aaatagtcat     1260
   cgagaaatat caactatcaa agaacagcta ttcacacgtt actattgaga ttattattgg     1320
   acgagaatca cacactcaac tgtctttctc tcttctagaa atacaggtac aagtatgtac     1380
   tattctcatt gttcatactt ctagtcattt catcccacat attccttgga tttctctcca     1440
   atgaatgaca ttctatcttg caaattcaac aattataata agatatacca aagtagcggt     1500
   atagtggcaa tcaaaaagct tctctggtgt gcttctcgta tttattttta ttctaatgat      1560
   ccattaaagg tatatattta tttcttgtta tataatcctt ttgtttatta catgggctgg     1620
   atacataaag gtattttgat ttaatttttt gcttaaattc aatcccccct cgttcagtgt     1680
   caactgtaat ggtaggaaat taccatactt ttgaagaagc aaaaaaaatg aaagaaaaaa     1740
   aaaatcgtat ttccaggtta gacgttccgc agaatctaga atgcggtatg cggtacattg     1800
   ttcttcgaac gtaaaagttg cgctccctga gatattgtac atttttgctt ttacaagtac     1860
   aagtacatcg tacaactatg tactactgtt gatgcatcca caacagtttg ttttgttttt     1920
   ttttgttttt tttttttcta atgattcatt accgctatgt atacctactt gtacttgtag   1980
   taagccgggt tattggcgtt caattaatca tagacttatg aatctgcacg gtgtgcgctg     2040
   cgagttactt ttagcttatg catgctactt gggtgtaata ttgggatctg ttcggaaatc     2100
   aacggatgct caatcgataa aaaacaaaaa aaaaagcacc gactcggtgc cactttttca     2160
   agttgataac ggactagcct tattttaact tgctatttct agctctaaaa ccgagcttga     2220
   gcactcgagc ggcgtcccat tcgccatgcc gaagcatgtt gcccagccgg cgccagcgag     2280
   gaggctggga ccatgccggc cattattttg cgttaagttt ctaatcatca cgaaattatc     2340
   tatcaaaaat aactaggtcc caccgagatt cgaactcggg accttaagat ttgcaatctc     2400
   acgcgctacc gctgtgccat aggaccgaag ttaaaatttg gccaaagaag gacctgggca     2460
   ccctggactg tgggttaggg taatattcct tatggagaca atgggctagg gtaaattacc     2520
   taaaatgggt cgataaagag gggtgttccc agttgggaag tgtaattgaa gacggggtca     2580
   aaaaagaaaa tcaaaaaaaa tttaattaag actatgataa cttcgtataa tgtatgctat     2640
   acgaacggta gcacactgta cgagtaagag cactagccac tgcactcaag tgaaaccgtt     2700
   gcccgggtac gagtatgagt atgtacagta tgtttagtat tgtacttgga cagtgcttgt     2760
   atcgtacatt ctcaagtgtc aaacataaat atccgttgct atatcctcgc accaccacgt     2820
   agctcgctat atccctgtgt tgaatccatc catcttggat tgccaattgt gcacacagaa     2880
   ccgggcactc acttccccat ccacacttgc ggccgctatt cctttgccct cggacgagtg     2940
   ctggggcgtc ggtttccact atcggcgagt acttctacac agccatcggt ccagacggcc     3000
   gcgcttctgc gggcgatttg tgtacgcccg acagtcccgg ctccggatcg gacgattgcg     3060
   tcgcatcgac cctgcgccca agctgcatca tcgaaattgc cgtcaaccaa gctctgatag     3120
   agttggtcaa gaccaatgcg gagcatatac gcccggagcc gcggcgatcc tgcaagctcc     3180
   ggatgcctcc gctcgaagta gcgcgtctgc tgctccatac aagccaacca cggcctccag     3240
   aagaagatgt tggcgacctc gtattgggaa tccccgaaca tcgcctcgct ccagtcaatg     3300
   accgctgtta tgcggccatt gtccgtcagg acattgttgg agccgaaatc cgcgtgcacg     3360
   aggtgccgga cttcggggca gtcctcggcc caaagcatca gctcatcgag agcctgcgcg     3420
   acggacgcac tgacggtgtc gtccatcaca gtttgccagt gatacacatg gggatcagca     3480
   atcgcgcata tgaaatcacg ccatgtagtg tattgaccga ttccttgcgg tccgaatggg     3540
   ccgaacccgc tcgtctggct aagatcggcc gcagcgatcg catccatagc ctccgcgacc     3600
   ggctgcagaa cagcgggcag ttcggtttca ggcaggtctt gcaacgtgac accctgtgca     3660
   cggcgggaga tgcaataggt caggctctcg ctgaactccc caatgtcaag cacttccgga     3720
   atcgggagcg cggccgatgc aaagtgccga taaacataac gatctttgta gaaaccatcg     3780
   gcgcagctat ttacccgcag gacatatcca cgccctccta catcgaagct gaaagcacga     3840
   gattcttcgc cctccgagag ctgcatcagg tcggagacgc tgtcgaactt ttcgatcaga     3900
   aacttctcga cagacgtcgc ggtgagttca ggctttttgg ccatggttga tgtgtgttta     3960
   attcaagaat gaatatagag aagagaagaa gaaaaaagat tcaattgagc cggcgatgca     4020
   gacccttata taaatgttgc cttggacaga cggagcaagc ccgcccaaac ctacgttcgg     4080
   tataatatgt taagcttttt aacacaaagg tttggcttgg ggtaacctga tgtggtgcaa     4140
   aagaccgggc gttggcgagc cattgcgcgg gcgaatgggg ccgtgactcg tctcaaattc     4200
   gagggcgtgc ctcaattcgt gcccccgtgg ctttttcccg ccgtttccgc cccgtttgca     4260
   ccactgcagc cgcttctttg gttcggacac cttgctgcga gctaggtgcc ttgtgctact 4320
   taaaaagtgg cctcccaaca ccaacatgac atgagtgcgt gggccaagac acgttggcgg 4380
   ggtcgcagtc ggctcaatgg cccggaaaaa acgctgctgg agctggttcg gacgcagtcc 4440
   gccgcggcgt atggatatcc gcaaggttcc atagcgccat tgccctccgt cggcgtctat 4500
   cccgcaacct accgttcgta taatgtatgc tatacgaagt tatgagcggg cttaaggttt 4560
   aaaccatcat ctaagggcct caaaactacc tcggaactgc tgcgctgatc tggacaccac 4620
   agaggttccg agcactttag gttgcaccaa atgtcccacc aggtgcaggc agaaaacgct 4680
   ggaacagcgt gtacagtttg tcttaacaaa aagtgagggc gctgaggtcg agcagggtgg 4740
   tgtgacttgt tatagccttt agagctgcga aagcgcgtat ggatttggct catcaggcca 4800
   gattgagggt ctgtggacac atgtcatgtt agtgtacttc aatcgccccc tggatatagc 4860
   cccgacaata ggccgtggcc tcattttttt gccttccgca catttccatt gctcggtacc 4920
   cacaccttgc ttctcctgca cttgccaacc ttaatactgg tttacattga ccaacatctt 4980
   acaagcgggg ggcttgtcta gggtatatat aaacagtggc tctcccaatc ggttgccagt 5040
   ctcttttttc ctttctttcc ccacagattc gaaatctaaa ctacacatca caccatggac 5100
   aagaaatact ccatcggcct ggacattgga accaactctg tcggctgggc tgtcatcacc 5160
   gacgagtaca aggtgccctc caagaaattc aaggtcctcg gaaacaccga tcgacactcc 5220
   atcaagaaaa acctcattgg tgccctgttg ttcgattctg gcgagactgc cgaagctacc 5280
   agactcaagc gaactgctcg gcgacgttac acccgacgga agaaccgaat ctgctacctg 5340
   caggagatct tttccaacga gatggccaag gtggacgatt cgttctttca tcgactggag 5400
   gaatccttcc tcgtcgagga agacaagaaa cacgagcgtc atcccatctt tggcaacatt 5460
   gtggacgagg ttgcttacca cgagaagtat cctaccatct accatctccg aaagaaactc 5520
   gtcgattcca ccgacaaggc ggatctcaga cttatctacc tcgctctggc acacatgatc 5580
   aagtttcgag gtcatttcct catcgagggc gatctcaatc ccgacaacag cgatgtggac 5640
   aagctgttca ttcagctcgt tcagacctac aaccagctgt tcgaggaaaa ccccatcaat 5700
   gcctccggag tcgatgcaaa ggccatcttg tctgctcgac tctcgaagag cagacgactg 5760
   gagaacctca ttgcccaact tcctggcgag aaaaagaacg gactgtttgg caacctcatt 5820
   gccctttctc ttggtctcac acccaacttc aagtccaact tcgatctggc ggaggacgcc 5880
   aagctccagc tgtccaagga cacctacgac gatgacctcg acaacctgct tgcacagatt 5940
   ggcgatcagt acgccgacct gtttctcgct gccaagaacc tttcggatgc tattctcttg 6000
   tctgacattc tgcgagtcaa caccgagatc acaaaggctc ccctttctgc ctccatgatc 6060
   aagcgatacg acgagcacca tcaggatctc acactgctca aggctcttgt ccgacagcaa 6120
   ctgcccgaga agtacaagga gatctttttc gatcagtcga agaacggcta cgctggatac 6180
   atcgacggcg gagcctctca ggaagagttc tacaagttca tcaagccaat tctcgagaag 6240
   atggacggaa ccgaggaact gcttgtcaag ctcaatcgag aggatctgct tcggaagcaa 6300
   cgaaccttcg acaacggcag cattcctcat cagatccacc tcggtgagct gcacgccatt 6360
   cttcgacgtc aggaagactt ctaccccttt ctcaaggaca accgagagaa gatcgagaag 6420
   attcttacct ttcgaatccc ctactatgtt ggtcctcttg ccagaggaaa ctctcgattt 6480
   gcttggatga ctcgaaagtc cgaggaaacc atcactccct ggaacttcga ggaagtcgtg 6540
   gacaagggtg cctctgcaca gtccttcatc gagcgaatga ccaacttcga caagaatctg 6600
   cccaacgaga aggttcttcc caagcattcg ctgctctacg agtactttac agtctacaac 6660
   gaactcacca aagtcaagta cgttaccgag ggaatgcgaa agcctgcctt cttgtctggc 6720
   gaacagaaga aagccattgt cgatctcctg ttcaagacca accgaaaggt cactgttaag 6780
   cagctcaagg aggactactt caagaaaatc gagtgtttcg acagcgtcga gatttccgga 6840
   gttgaggacc gattcaacgc ctctttgggc acctatcacg atctgctcaa gattatcaag 6900
   gacaaggatt ttctcgacaa cgaggaaaac gaggacattc tggaggacat cgtgctcact 6960
   cttaccctgt tcgaagatcg ggagatgatc gaggaacgac tcaagacata cgctcacctg 7020
   ttcgacgaca aggtcatgaa acaactcaag cgacgtagat acaccggctg gggaagactt 7080
   tcgcgaaagc tcatcaacgg catcagagac aagcagtccg gaaagaccat tctggacttt 7140
   ctcaagtccg atggctttgc caaccgaaac ttcatgcagc tcattcacga cgattctctt 7200
   accttcaagg aggacatcca gaaggcacaa gtgtccggtc agggcgacag cttgcacgaa 7260
   catattgcca acctggctgg ttcgccagcc atcaagaaag gcattctcca gactgtcaag 7320
   gttgtcgacg agctggtgaa ggtcatggga cgtcacaagc ccgagaacat tgtgatcgag 7380
   atggccagag agaaccagac aactcaaaag ggtcagaaaa actcgcgaga gcggatgaag 7440
   cgaatcgagg aaggcatcaa ggagctggga tcccagattc tcaaggagca tcccgtcgag 7500
   aacactcaac tgcagaacga gaagctgtat ctctactatc tgcagaatgg tcgagacatg 7560
   tacgtggatc aggaactgga catcaatcgt ctcagcgact acgatgtgga ccacattgtc 7620
   cctcaatcct ttctcaagga cgattctatc gacaacaagg tccttacacg atccgacaag 7680
   aacagaggca agtcggacaa cgttcccagc gaagaggtgg tcaaaaagat gaagaactac 7740
   tggcgacagc tgctcaacgc caagctcatt acccagcgaa agttcgacaa tcttaccaag 7800
   gccgagcgag gcggtctgtc cgagctcgac aaggctggct tcatcaagcg tcaactcgtc 7860
   gagaccagac agatcacaaa gcacgtcgca cagattctcg attctcggat gaacaccaag 7920
   tacgacgaga acgacaagct catccgagag gtcaaggtga ttactctcaa gtccaaactg 7980
   gtctccgatt tccgaaagga ctttcagttc tacaaggtgc gagagatcaa caattaccac 8040
   catgcccacg atgcttacct caacgccgtc gttggcactg cgctcatcaa gaaatacccc      8100
   aagctcgaaa gcgagttcgt ttacggcgat tacaaggtct acgacgttcg aaagatgatt      8160
   gccaagtccg aacaggagat tggcaaggct actgccaagt acttctttta ctccaacatc      8220
   atgaactttt tcaagaccga gatcaccttg gccaacggag agattcgaaa gagaccactt      8280
   atcgagacca acggcgaaac tggagagatc gtgtgggaca agggtcgaga ctttgcaacc      8340
   gtgcgaaagg ttctgtcgat gcctcaggtc aacatcgtca agaaaaccga ggttcagact      8400
   ggcggattct ccaaggagtc gattctgccc aagcgaaact ccgacaagct catcgctcga      8460
   aagaaagact gggatcccaa gaaatacggt ggcttcgatt ctcctaccgt cgcctattcc      8520
   gtgcttgtcg ttgcgaaggt cgagaagggc aagtccaaaa agctcaagtc cgtcaaggag      8580
   ctgctcggaa ttaccatcat ggagcgatcg agcttcgaga agaatcccat cgacttcttg      8640
   gaagccaagg gttacaagga ggtcaagaaa gacctcatta tcaagctgcc caagtactct      8700
   ctgttcgaac tggagaacgg tcgaaagcgt atgctcgcct ccgctggcga gctgcagaag      8760
   ggaaacgagc ttgccttgcc ttcgaagtac gtcaactttc tctatctggc ttctcactac      8820
   gagaagctca agggttctcc cgaggacaac gaacagaagc aactcttcgt tgagcagcac      8880
   aaacattacc tcgacgagat tatcgagcag atttccgagt tttcgaagcg agtcatcctg       8940
   gctgatgcca acttggacaa ggtgctctct gcctacaaca agcatcggga caaacccatt      9000
   cgagaacagg cggagaacat cattcacctg tttactctta ccaacctggg tgctcctgca      9060
   gctttcaagt acttcgatac cactatcgac cgaaagcggt acacatccac caaggaggtt      9120
   ctcgatgcca ccctgattca ccagtccatc actggcctgt acgagacccg aatcgacctg      9180
   tctcagcttg gtggcgactc cagagccgat cccaagaaaa agcgaaaggt ctaagcggcc      9240
   gcaagtgtgg atggggaagt gagtgcccgg ttctgtgtgc acaattggca atccaagatg      9300
   gatggattca acacagggat atagcgagct acgtggtggt gcgaggatat agcaacggat      9360
   atttatgttt gacacttgag aatgtacgat acaagcactg tccaagtaca atactaaaca      9420
   tactgtacat actcatactc gtacccgggc aacggtttca cttgagtgca gtggctagtg      9480
   ctcttactcg tacagtgtgc aatactgcgt atcatagtct ttgatgtata tcgtattcat      9540
   tcatgttagt tgcgtacgag ccggaagcat aaagtgtaaa gcctggggtg cctaatgagt      9600
   gagctaactc acattaattg cgttgcgctc actgcccgct ttccagtcgg gaaacctgtc      9660
   gtgccagctg cattaatgaa tcggccaacg cgcggggaga ggcggtttgc gtattgggcg      9720
   ctcttccgct tcctcgctca ctgactcgct gcgctcggtc gttcggctgc ggcgagcggt      9780
   atcagctcac tcaaaggcgg taatacggtt atccacagaa tcaggggata acgcaggaaa      9840
   gaacatgtga gcaaaaggcc agcaaaaggc caggaaccgt aaaaaggccg cgttgctggc      9900
   gtttttccat aggctccgcc cccctgacga gcatcacaaa aatcgacgct caagtcagag      9960
   gtggcgaaac ccgacaggac tataaagata ccaggcgttt ccccctggaa gctccctcgt     10020
   gcgctctcct gttccgaccc tgccgcttac cggatacctg tccgcctttc tcccttcggg     10080
   aagcgtggcg ctttctcata gctcacgctg taggtatctc agttcggtgt aggtcgttcg     10140
   ctccaagctg ggctgtgtgc acgaaccccc cgttcagccc gaccgctgcg ccttatccgg     10200
   taactatcgt cttgagtcca acccggtaag acacgactta tcgccactgg cagcagccac     10260
   tggtaacagg attagcagag cgaggtatgt aggcggtgct acagagttct tgaagtggtg     10320
   gcctaactac ggctacacta gaaggacagt atttggtatc tgcgctctgc tgaagccagt     10380
   taccttcgga aaaagagttg gtagctcttg atccggcaaa caaaccaccg ctggtagcgg     10440
   tggttttttt gtttgcaagc agcagattac gcgcagaaaa aaaggatctc aagaagatcc     10500
   tttgatcttt tctacggggt ctgacgctca gtggaacgaa aactcacgtt aagggatttt     10560
   ggtcatgaga ttatcaaaaa ggatcttcac ctagatcctt ttaaattaaa aatgaagttt     10620
   taaatcaatc taaagtatat atgagtaaac ttggtctgac agttaccaat gcttaatcag     10680
   tgaggcacct atctcagcga tctgtctatt tcgttcatcc atagttgcct gactccccgt     10740
   cgtgtagata actacgatac gggagggctt accatctggc cccagtgctg caatgatacc     10800
   gcgagaccca cgctcaccgg ctccagattt atcagcaata aaccagccag ccggaagggc     10860
   cgagcgcaga agtggtcctg caactttatc cgcctccatc cagtctatta attgttgccg     10920
   ggaagctaga gtaagtagtt cgccagttaa tagtttgcgc aacgttgttg ccattgctac     10980
   aggcatcgtg gtgtcacgct cgtcgtttgg tatggcttca ttcagctccg gttcccaacg     11040
   atcaaggcga gttacatgat cccccatgtt gtgcaaaaaa gcggttagct ccttcggtcc     11100
   tccgatcgtt gtcagaagta agttggccgc agtgttatca ctcatggtta tggcagcact     11160
   gcataattct cttactgtca tgccatccgt aagatgcttt tctgtgactg gtgagtactc     11220
   aaccaagtca ttctgagaat agtgtatgcg gcgaccgagt tgctcttgcc cggcgtcaat     11280
   acgggataat accgcgccac atagcagaac tttaaaagtg ctcatcattg gaaaacgttc     11340
   ttcggggcga aaactctcaa ggatcttacc gctgttgaga tccagttcga tgtaacccac     11400
   tcgtgcaccc aactgatctt cagcatcttt tactttcacc agcgtttctg ggtgagcaaa     11460
   aacaggaagg caaaatgccg caaaaaaggg aataagggcg acacggaaat gttgaatact     11520
   catactcttc ctttttcaat attattgaag catttatcag ggttattgtc tcatgagcgg     11580
   atacatattt gaatgtattt agaaaaataa acaaataggg gttccgcgca catttccccg     11640
   aaaagtgcca cctgacgcgc cctgtagcgg cgcattaagc gcggcgggtg tggtggttac     11700
   gcgcagcgtg accgctacac ttgccagcgc cctagcgccc gctcctttcg ctttcttccc     11760
   ttcctttctc gccacgttcg ccggctttcc ccgtcaagct ctaaatcggg ggctcccttt     11820
   agggttccga tttagtgctt tacggcacct cgaccccaaa aaacttgatt agggtgatgg    11880
   ttcacgtagt gggccatcgc cctgatagac ggtttttcgc cctttgacgt tggagtccac    11940
   gttctttaat agtggactct tgttccaaac tggaacaaca ctcaacccta tctcggtcta    12000
   ttcttttgat ttataaggga ttttgccgat ttcggcctat tggttaaaaa atgagctgat    12060
   ttaacaaaaa tttaacgcga attttaacaa aatattaacg cttacaattt ccattcgcca    12120
   ttcaggctgc gcaactgttg ggaagggcga tcggtgcggg cctcttcgct attacgccag    12180
   ctggcgaaag ggggatgtgc tgcaaggcga ttaagttggg taacgccagg gttttcccag    12240
   tcacgacgtt gtaaaacgac ggccagtgaa ttgtaatacg actcactata gggcgaattg    12300
   ggtaccgggc cccccctcga ggtcgatggt gtcgataagc ttgatatcg                 12349
<210> 70
   <211> 20
   <212> DNA
   <213> Artificial sequence
<220>
   <223> oligo 308
<400> 70
   cgtattagca ctgtacccag 20
<210> 71
   <211> 20
   <212> DNA
   <213> Artificial Sequence
<220>
   <223> oligo 309
<400> 71
   catgacatgt gtccacagac 20
<210> 72
   <211> 859
   <212> DNA
   <213> Artificial sequence
<220>
   <223> URA3 locus deletion PCR product
<400> 72
   catgacatgt gtccacagac cctcaatctg gcctgatgag ccaaatccat acgcgctttc      60
   gcagctctaa aggctataac aagtcacacc accctgctcg acctcagcgc cctcactttt     120
   tgttaagaca aactgtacac gctgttccag cgttttctgc ctgcacctgg tgggacattt     180
   ggtgcaacct aaagtgctcg gaacctctgt ggtgtccaga tcagcgcagc agttccgagg     240
   tagttttgag gcccttagat gatggtttaa acgtcgacga gtatctgtct gactcgtcat     300
   tgccgccttt ggagtacgac tccaactatg agtgtgcttg gatcactttg acgatacatt     360
   cttcgttgga ggctgtgggt ctgacagctg cgttttcggc gcggttggcc gacaacaata     420
   tcagctgcaa cgtcattgct ggctttcatc atgatcacat ttttgtcggc aaaggcgacg     480
   cccagagagc cattgacgtt ctttctaatt tggaccgata gccgtatagt ccagtctatc     540
   tataagttca actaactcgt aactattacc ataacatata cttcactgcc ccagataagg     600
   ttccgataaa aagttctgca gactaaattt atttcagtct cctcttcacc accaaaaggt     660
   tagactatgg atatgtaatt taactgtgta tatagagagc gtgcaagtat ggagcgcttg     720
   ttcagcttgt atgatggtca gacgacctgt ctgatcgagt atgtatgata ctgcacaacc     780
   tgtgtatccg catgatctgt ccaatggggc atgttgttgt gtttctcgat acggagatgc     840
   tgggtacagt gctaatacg                                                  859